(19) Europäisches Patentamt
European Patent Office
Office européen des brevets

(11) EP 0 819 125 B1

(12) EUROPEAN PATENT SPECIFICATION

(45) Date of publication and mention of the grant of the patent: **18.06.2003 Bulletin 2003/25**

(21) Application number: **96912600.2**

(22) Date of filing: **04.04.1996**

(51) Int Cl.[7]: **C07D 261/16**, C07D 261/14, C07D 413/12, A61K 31/42, C07D 413/14, C07D 417/14

(86) International application number: **PCT/US96/04759**

(87) International publication number: **WO 96/031492 (10.10.1996 Gazette 1996/45)**

(54) **THIENYL-, FURYL-, PYRROLYL- AND BIPHENYLSULFONAMIDES AND DERIVATIVES THEREOF THAT MODULATE THE ACTIVITY OF ENDOTHELIN**

THIENYL-, FURYL-, PYRROLYL- UND BIPHENYLSULFONAMIDE UND DERIVATE ZUR MODULATION DER ENDOTHELIN-AKTIVITÄT

THIENYL-, FURYL-, PYRROLYL- ET BIPHENYL SULFONAMIDES ET LEURS DERIVES MODULANT L'ACTIVITE DE L'ENDOTHELINE

(84) Designated Contracting States:
**AT BE CH DE DK ES FI FR GB GR IE IT LI LU MC NL PT SE**
Designated Extension States:
**AL LT LV SI**

(30) Priority: **04.04.1995 US 416199**
**04.04.1995 US 417075**
**06.06.1995 US 477223**

(43) Date of publication of application:
**21.01.1998 Bulletin 1998/04**

(60) Divisional application:
**00113076.4 / 1 048 657**

(73) Proprietor: **TEXAS BIOTECHNOLOGY CORPORATION**
**Houston, TX 77030 (US)**

(72) Inventors:
- **CHAN, Ming, Fai**
  **San Diego, CA 92128 (US)**
- **RAJU, Bore, Gowda**
  **San Diego, CA 92128 (US)**
- **KOIS, Adam**
  **San Diego, CA 92127 (US)**
- **VERNER, Erik, Joel**
  **San Diego, CA 92127 (US)**
- **WU, Chengde**
  **San Diego, CA 92128 (US)**
- **CASTILLO, Rosario, Silverstre**
  **San Diego, CA 92126 (US)**
- **YALAMOORI, Venkatachalapathi**
  **San Diego, CA 92128 (US)**
- **BALAJI, Vitukudi, Narayanaiyengar**
  **Encinitas, CA 92024 (US)**
- **KOGAN, Timothy**
  **Sugar Land, Texas 77479 (US)**

(74) Representative: **Goldin, Douglas Michael**
**J.A. KEMP & CO.**
**14 South Square**
**Gray's Inn**
**London WC1R 5JJ (GB)**

(56) References cited:
**EP-A- 0 558 258** **EP-A- 0 569 193**
**WO-A-94/27979** **US-A- 3 660 383**

- **JOURNAL OF MEDICINAL CHEMISTRY, vol. 37, no. 3, 4 February 1994, WASHINGTON US, pages 329-331, XP002007977 PHILIP D.STEIN ET AL: "The discovery of sulfonamide endothelin antagonists and the development of the orally active ETa antagonist 5-(dimethylamino)-N-(3,4-dimethyl-5-isoxaz olyl)-1-naphthalenesulfonamide"**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 819 125 B1

Printed by Jouve, 75001 PARIS (FR)

## Description

## FIELD OF THE INVENTION

The present invention relates to the compounds that modulate the activity of the endothelin family of peptides. In particular, the invention relates to the use of sulfonamides and sulfonamide pro-drugs as endothelin agonists and antagonists.

## BACKGROUND OF THE INVENTION

The vascular endothelium releases a variety of vasoactive substances, including the endothelium-derived vasoconstrictor peptide, endothelin (ET) (see, e.g., Vanhoutte et al. (1986) Annual Rev. Physiol. 48: 307-320; Furchgott and Zawadski (1980) Nature 288: 373-376). Endothelin, which was originally identified in the culture supernatant of porcine aortic endothelial cells (see, Yanagisawa et al. (1988) Nature 332: 411-415), is a potent twenty-one amino acid peptide vasoconstrictor. It is the most potent vasopressor known and is produced by numerous cell types, including the cells of the endothelium, trachea, kidney and brain. Endothelin is synthesized as a two hundred and three amino acid precursor preproendothelin that contains a signal sequence which is cleaved by an endogenous protease to produce a thirty-eight (human) or thirty-nine (porcine) amino acid peptide. This intermediate, referred to as big endothelin, is processed in vivo to the mature biologically active form by a putative endothelin-converting enzyme (ECE) that appears to be a metal-dependent neutral protease (see, e.g., Kashiwabara et al. (1989) FEBS Lttrs. 247: 337-340). Cleavage is required for induction of physiological responses (see, e.g., von Geldern et al. (1991) Peptide Res. 4: 32-35). In porcine aortic endothelial cells, the thirty-nine amino acid intermediate, big endothelin, is hydrolyzed at the $Trp^{21}$-$Val^{22}$ bond to generate endothelin-1 and a C-terminal fragment. A similar cleavage occurs in human cells from a thirty-eight amino acid intermediate. Three distinct endothelin isopeptides, endothelin-1, endothelin-2 and endothelin-3, that exhibit potent vasoconstrictor activity have been identified.

The family of three isopeptides endothelin-1, endothelin-2 and endothelin-3 are encoded by a family of three genes (see, Inoue et al. (1989) Proc. Natl. Acad. Sci. USA 86: 2863-2867; see, also Saida et al. (1989)J. Biol. Chem. 264: 14613-14616). The nucleotide sequences of the three human genes are highly conserved within the region encoding the mature 21 amino acid peptides and the C-terminal portions of the peptides are identical. Endothelin-2 is ($Trp^6$,$Leu^7$) endothelin-1 and endothelin-3 is ($Thr^2$,$Phe^4$,$Thr^5$,$Tyr^6$,$Lys^7$,$Tyr^{14}$) endothelin-1. These peptides are, thus, highly conserved at the C-terminal ends.

Release of endothelins from cultured endothelial cells is modulated by a variety of chemical and physical stimuli and appears to be regulated at the level of transcription and/or translation. Expression of the gene encoding endothelin-1 is increased by chemical stimuli, including adrenaline, thrombin and $Ca^{2+}$ ionophore. The production and release of endothelin from the endothelium is stimulated by angiotensin II, vasopressin, endotoxin, cyclosporine and other factors (see, Brooks et al. (1991) Eur. J. Pharm. 194: 115-117), and is inhibited by nitric oxide. Endothelial cells appear to secrete short-lived endothelium-derived relaxing factors (EDRF), including nitric oxide or a related substance (Palmer et al. (1987) Nature 327: 524-526), when stimulated by vasoactive agents, such as acetylcholine and bradykinin. Endothelin-induced vasoconstriction is also attenuated by atrial natriuretic peptide (ANP).

The endothelin peptides exhibit numerous biological activities in vitro and in vivo. Endothelin provokes a strong and sustained vasoconstriction in vivo in rats and in isolated vascular smooth muscle preparations; it also provokes the release of eicosanoids and endothelium-derived relaxing factor (EDRF) from perfused vascular beds. Intravenous administration of endothelin-1 and in vitro addition to vascular and other smooth muscle tissues produce long-lasting pressor effects and contraction, respectively (see, e.g., Bolger et al. (1991) Can. J. Physiol. Pharmacol. 69: 406-413). In isolated vascular strips, for example, endothelin-1 is a potent ($EC_{50}$ = 4 x $10^{-10}$ M), slow acting, but persistent, contractile agent. In vivo, a single dose elevates blood pressure in about twenty to thirty minutes. Endothelin-induced vasoconstriction is not affected by antagonists to known neurotransmitters or hormonal factors, but is abolished by calcium channel antagonists. The effect of calcium channel antagonists, however, is most likely the result of inhibition of calcium influx, since calcium influx appears to be required for the long-lasting contractile response to endothelin.

Endothelin also mediates renin release, stimulates ANP release and induces a positive inotropic action in guinea pig atria. In the lung, endothelin-1 acts as a potent bronchoconstrictor (Maggi et al. (1989) Eur. J. Pharmacol. 160: 179-182). Endothelin increases renal vascular resistance, decreases renal blood flow, and decreases glomerular filtrate rate. It is a potent mitogen for glomerular mesangial cells and invokes the phosphoinoside cascade in such cells (Simonson et al. (1990) J. Clin. Invest. 85: 790-797).

There are specific high affinity binding sites (dissociation constants in the range of 2-6 x $10^{-10}$ M) for the endothelins in the vascular system and in other tissues, including the intestine, heart, lungs, kidneys, spleen, adrenal glands and brain. Binding is not inhibited by catecholamines, vasoactive peptides, neurotoxins or calcium channel antagonists. Endothelin binds and interacts with receptor sites that are distinct from other autonomic receptors and

voltage dependent calcium channels. Competitive binding studies indicate that there are multiple classes of receptors with different affinities for the endothelin isopeptides. The sarafotoxins, a group of peptide toxins from the venom of the snake Atractaspis eingadensis that cause severe coronary vasospasm in snake bite victims, have structural and functional homology to endothelin-1 and bind competitively to the same cardiac membrane receptors (Kloog et al. (1989) Trends Pharmacol. Sci. 10: 212-214).

Two distinct endothelin receptors, designated $ET_A$ and $ET_B$, have been identified and DNA clones encoding each receptor have been isolated (Arai et al. (1990) Nature 348: 730-732; Sakurai et al. (1990) Nature 348: 732-735). Based on the amino acid sequences of the proteins encoded by the cloned DNA, it appears that each receptor contains seven membrane spanning domains and exhibits structural similarity to G-protein-coupled membrane proteins. Messenger RNA encoding both receptors has been detected in a variety of tissues, including heart, lung, kidney and brain. The distribution of receptor subtypes is tissue specific (Martin et al. (1989) Biochem. Biophys. Res. Commun. 162: 130-137). $ET_A$ receptors appear to be selective for endothelin-1 and are predominant in cardiovascular tissues. $ET_B$ receptors are predominant in noncardiovascular tissues, including the central nervous system and kidney, and interact with the three endothelin isopeptides (Sakurai et al. (1990) Nature 348: 732-734). In addition, $ET_A$ receptors occur on vascular smooth muscle, are linked to vasoconstriction and have been associated with cardiovascular, renal and central nervous system diseases; whereas $ET_B$ receptors are located on the vascular endothelium, linked to vasodilation (Takayanagi et al. (1991) FEBS Lttrs. 282: 103-106) and have been associated with bronchoconstrictive disorders.

By virtue of the distribution of receptor types and the differential affinity of each isopeptide for each receptor type, the activity of the endothelin isopeptides varies in different tissues. For example, endothelin-1 inhibits $^{125}$I-labelled endothelin-1 binding in cardiovascular tissues forty to seven hundred times more potently than endothelin-3. $^{125}$I-labelled endothelin-1 binding in non-cardiovascular tissues, such as kidney, adrenal gland, and cerebellum, is inhibited to the same extent by endothelin-1 and endothelin-3, which indicates that $ET_A$ receptors predominate in cardiovascular tissues and $ET_B$ receptors predominate in non-cardiovascular tissues.

Endothelin plasma levels are elevated in certain disease states (see, e.g., International PCT Application WO 94/27979, and U.S. Patent No. 5,382,569).

Endothelin-1 plasma levels in healthy individuals, as measured by radioimmunoassay (RIA), are about 0.26-5 pg/ml. Blood levels of endothelin-1 and its precursor, big endothelin, are elevated in shock, myocardial infarction, vasospastic angina, kidney failure and a variety of connective tissue disorders. In patients undergoing hemodialysis or kidney transplantation or suffering from cardiogenic shock, myocardial infarction or pulmonary hypertension levels as high as 35 pg/ml have been observed (see, Stewart et al. (1991) Annals Internal Med. 114: 464-469). Because endothelin is likely to be a local, rather than a systemic, regulating factor, it is probable that the levels of endothelin at the endothelium/smooth muscle interface are much higher than circulating levels.

Elevated levels of endothelin have also been measured in patients suffering from ischemic heart disease (Yasuda et al. (1990) Amer. Heart J. 119:801-806, Ray et al. (1992) Br. Heart J. 67:383-386). Circulating and tissue endothelin immunoreactivity is increased more than twofold in patients with advanced atherosclerosis (Lerman et al. (1991) New Engl. J. Med. 325:997-1001). Increased endothelin immunoreactivity has also been associated with Buerger's disease (Kanno et al. (1990) J. Amer. Med. Assoc. 264:2868) and Raynaud's phenomenon (Zamora et al. (1990) Lancet 336 1144-1147). Increased circulating endothelin levels were observed in patients who underwent percutaneous transluminal coronary angioplasty (PTCA) (Tahara et al. (1991) Metab. Clin. Exp. 40:1235-1237; Sanjay et al. (1991) Circulation 84(Suppl. 4):726), and in individuals (Miyauchi et al. (1992) Jpn. J. Pharmacol.58:279P; Stewart et al. (1991) Ann.Internal Medicine 114:464-469) with pulmonary hypertension. Thus, there is clinical human data supporting the correlation between increased endothelin levels and numerous disease states.

**Endothelin agonists and antagonists**

Because endothelin is associated with certain disease states and is implicated in numerous physiological effects, compounds that can interfere with or potentiate endothelin-associated activities, such as endothelin-receptor interaction and vasoconstrictor activity, are of interest. Compounds that exhibit endothelin antagonistic activity have been identified. For example, a fermentation product of Streptomyces misakiensis, designated BE-18257B, has been identified as an $ET_A$ receptor antagonist. BE-18257B is a cyclic pentapeptide, cyclo(D-Glu-L-Ala-allo-D-Ile-L-Leu-D-Trp), which inhibits $^{125}$I-labelled endothelin-1 binding in cardiovascular tissues in a concentration-dependent manner ($IC_{50}$ 1.4 μM in aortic smooth muscle, 0.8 μM in ventricle membranes and 0.5 μM in cultured aortic smooth muscle cells), but fails to inhibit binding to receptors in tissues in which $ET_B$ receptors predominate at concentrations up to 100 μM. Cyclic pentapeptides related to BE-18257B, such as cyclo(D-Asp-Pro-D-Val-Leu-D-Trp) (BQ-123), have been synthesized and shown to exhibit activity as $ET_A$ receptor antagonists (see, U.S. Patent No. 5,114,918 to Ishikawa et al.; see, also, EP A1 0 436 189 to BANYU PHARMACEUTICAL CO., LTD (October 7, 1991)). Studies that measure the inhibition by these cyclic peptides of endothelin-1 binding to endothelin-specific receptors indicate that these cyclic peptides bind preferentially to $ET_A$ receptors. Other peptide and non-peptidic $ET_A$ antagonists have been identified

(see, e.g., 5,352,800, 5,334,598, 5,352,659, 5,248,807, 5,240,910, 5,198,548, 5,187,195, 5,082,838). These include other cyclic pentapeptides, acyltripeptides, hexapeptide analogs, certain antraquinone derivatives, indanecarboxylic acids, certain N-pyriminylbenzenesulfonamides, certain benzenesulfonamides, and certain naphthalenesulfonamides (Nakajima et al. (1991) J. Antibiot. 44:1348-1356; Miyata et al. (1992) J. Antibiot. 45:74-8; Ishikawa et al. (1992) J. Med. Chem. 35:2139-2142; U.S. Patent No. 5,114,918 to Ishikawa et al.; EP A1 0 569 193; EP A1 0 558 258; EP A1 0 436 189 to BANYU PHARMACEUTICAL CO., LTD (October 7, 1991); Canadian Patent Application 2,067,288; Canadian Patent Application 2,071,193; U.S. Patent No. 5,208,243; U.S. Patent No. 5,270,313; U.S. Patent No. 5,464,853 to Chan et al.; Cody et al. (1993) Med. Chem. Res. 3:154-162; Miyata et al. (1992) J. Antibiot 45:1041-1046; Miyata et al. (1992) J. Antibiot 45:1029-1040, Fujimoto et al. (1992) FEBS Lett. 305:41-44; Oshashi et al. (1002) J. Antibiot 45:1684-1685; EP A1 0 496 452; Clozel et al. (1993) Nature 365:759-761; International Patent Application WO93/08799; Nishikibe et al. (1993) Life Sci. 52:717-724; and Benigni et al. (1993) Kidney Int. 44:440-444). In general, the identified compounds have activities in in vitro assays as $ET_A$ antagonists at concentrations on the order of about 50-100 μM or less. A number of such compounds have also been shown to possess activity in in vivo animal models. Very few selective $ET_B$ antagonists have been identified.

## Endothelin antagonists and agonists as therapeutic agents

It has been recognized that compounds that exhibit activity at $IC_{50}$ or $EC_{50}$ concentrations on the order of $10^{-4}$ or lower in standard in vitro assays that assess endothelin antagonist or agonist activity have pharmacological utility (see, e.g., U.S. Patent Nos. 5,352,800, 5,334,598, 5,352,659, 5,248,807, 5,240,910, 5,198,548, 5,187,195, 5,082,838). By virtue of this activity, such compounds are considered to be useful for the treatment of hypertension such as peripheral circulatory failure, heart disease such as angina pectoris, cardiomyopathy, arteriosclerosis, myocardial infarction, pulmonary hypertension, vasospasm, vascular restenosis, Raynaud's disease, cerebral stroke such as cerebral arterial spasm, cerebral ischemia, late phase cerebral spasm after subarachnoid hemorrhage, asthma, bronchoconstriction, renal failure, particularly post-ischemic renal failure, cyclosporine nephrotoxicity such as acute renal failure, colitis, as well as other inflammatory diseases, endotoxic shock caused by or associated with endothelin, and other diseases in which endothelin has been implicated.

In view of the numerous physiological effects of endothelin and its association with certain diseases, endothelin is believed to play a critical role in these pathophysiological conditions (see, e.g., Saito et al. (1990) Hypertension 15: 734-738; Tomita et al. (1989) N. Engl. J. Med. 321: 1127; Kurihara et al. (1989) J. Cardiovasc. Pharmacol. 13(Suppl. 5): S13-S17; Doherty (1992) J. Med. Chem. 35: 1493-1508; Morel et al. (1989) Eur. J. Pharmacol. 167: 427-428). More detailed knowledge of the function and structure of the endothelin peptide family should provide insight in the progression and treatment of such conditions.

To aid in gaining further understanding of and to develop treatments for endothelin-mediated or related disorders, there is a need to identify compounds that modulate or alter endothelin activity. Identification of compounds that modulate endothelin activity, such as those that act as specific antagonists or agonists, may not only aid in elucidating the function of endothelin, but may yield in therapeutically useful compounds. In particular, compounds that specifically interfere with the interaction of endothelin peptides with the $ET_A$ or $ET_B$ receptors should be useful in identifying essential characteristics of endothelin peptides, should aid in the design of therapeutic agents, and may be useful as disease specific therapeutic agents.

Therefore, it is an object herein to provide compounds that have the ability to modulate the biological activity of one or more of the endothelin isopeptides. It is another object to provide compounds that have use as specific endothelin antagonists. It is also an object to use compounds that specifically interact with or inhibit the interaction of endothelin peptides with $ET_A$ or $ET_B$ receptors. Such compounds should be useful as therapeutic agents for the treatment of endothelin-mediated diseases and disorders and also for the identification of endothelin receptor subtypes.

## SUMMARY OF THE INVENTION

Sulfonamides and methods for modulating the interaction of an endothelin peptide with $ET_A$ and/or $ET_B$ receptors are provided. In particular, sulfonamides and methods for inhibiting the binding of an endothelin peptide to $ET_A$ or $ET_B$ receptors are provided. The methods are effected by contacting the receptors with one or more sulfonamides prior to, simultaneously with, or subsequent to contacting the receptors with an endothelin peptide.

The present invention provides a compound of formula I:

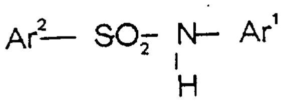

or a pharmaceutically acceptable salt, acid or ester thereof, wherein:

$Ar^1$ is:

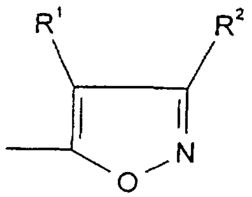

or

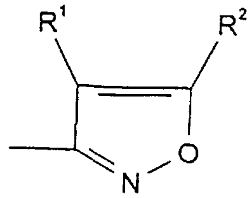

where $R^1$ is selected from halide, $CH_3$, $C_2H_5$, $CF_3$, $C_2F_5$, n-$C_3H_7$ and cyclo-$C_3H_7$ and $R^2$ is selected from H, $CH_3$, $C_2H_5$, $CF_3$, $C_2F_5$, n-$C_3H_7$ and cyclo-$C_3H_7$; and
$Ar^2$ has formula VI:

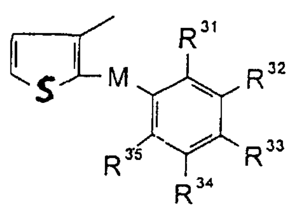

wherein:

M is selected from

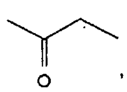, 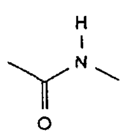, 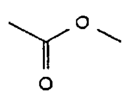, 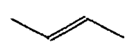,

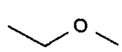, 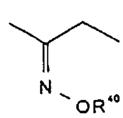, 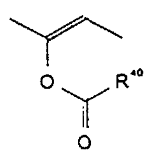 and 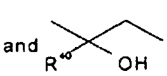

where $R^{40}$ is methyl, ethyl or hydrogen.

$R^{31}$, $R^{32}$, $R^{33}$, $R^{34}$ and $R^{35}$ are each independently selected from (i) or (ii) as follows:

(i) $R^{31}$, $R^{32}$ and $R^{34}$ are each independently selected from hydrogen, $C_{1-6}$alkyl, halo$C_{1-6}$alkyl, phenyl, $C_{1-6}$alkoxy, $C_{1-6}$alkylsulfonylamino$C_{1-6}$alkyl, cyano$C_{1-6}$alkyl, acetyl, $C_{1-6}$alkoxycarbonyl, cyano, OH, acetoxy$C_{1-6}$alkyl, hydroxy$C_{1-6}$alkyl, acetoxy$C_{1-6}$alkoxy or $C_{1-6}$alkoxycarbonyl; $R^{33}$ and $R^{35}$ are other than hydrogen and are selected from $C_{1-6}$alkyl and $C_{1-6}$alkoxy; or
(ii) $R^{35}$ is $C_{1-6}$alkyl or $C_{1-6}$alkoxy, and $R_{32}$ and $R_{33}$ or $R^{33}$ and $R^{34}$ form methylenedioxy or ethylenedioxy, and the

others of $R^{31}$, $R^{32}$, $R^{33}$ and $R^{34}$ are selected as in (i), with the proviso that when M is $(CH_2)_mC(O)NH(CH_2)_r$, then at least two of $R^{31}$, $R^{32}$, $R^{33}$, $R^{34}$ and $R^{35}$ are not hydrogen.

In the embodiments provided herein, the alkyl, alkynyl and alkenyl portions of each listed substituent are straight or branched chains, acyclic or cyclic, and preferably have from about 1 up to about 10 carbons; in more preferred embodiments they have from 1-6 carbons. The aryl, alicyclic, aromatic rings and heterocyclic groups can have from 3 to 16, generally, 3-7, more often 5-7 members in the rings, and may be single or fused rings. The ring size and carbon chain length are selected up to an amount that the resulting molecule binds and retains activity as an endothelin antagonist or agonist, such that the resulting compound inhibits binding by 50%, compared to binding in the absence of the sulfonamide, of an endothelin peptide to an endothelin receptor at a concentration of less than about 100 µM.

Of the compounds described herein, those that inhibit or increase an endothelin-mediated activity by about 50% at concentrations of less than about 10 µM are preferred. More preferred are those that inhibit or increase an endothelin-mediated activity by about 50% at concentrations of less than about 1 µM, more preferably less than about 0.1 µM, even more preferably less than about 0.01 µM, and most preferably less than about 0.001 µM. It is noted that, as described below, the $IC_{50}$ concentration determined in the in vitro assays is a non-linear function of incubation temperature. The preferred values recited herein refer to the assays that are performed at 4° C. When the assays are performed at 24° C, somewhat higher (see, Table 1) $IC_{50}$ concentrations are observed. Accordingly, the preferred $IC_{50}$ concentrations are about 10-fold higher.

Also among the most preferred compounds for use in methods provided herein, are those that are $ET_A$ selective, i.e., they interact with $ET_A$ receptors at substantially lower concentratons (at an $IC_{50}$ at least about 10-fold lower, preferably 100-fold lower) than they interact with $ET_B$ receptors. In particular, compounds that interact with $ET_A$ with an $IC_{50}$ of less than about 10 µM, preferably less than 1 µM, more preferably less than 0.1 µM, but with $ET_B$ with an $IC_{50}$ of greater than about about 10 µM or compounds that interact with $ET_B$ with an $IC_{50}$ of less than about 10 µM, preferably less than 1 µM, more preferably less than 0.1 µM, but with $ET_A$ with an $IC_{50}$ of greater than about 10 µM are preferred.

Preferred compounds also include compounds that are $ET_B$ receptor selective or that bind to $ET_B$ receptors with an $IC_{50}$ of less than about 1 µM. $ET_B$ selective compounds interact with $ET_B$ receptors at $IC_{50}$ concentrations that are at least about 10-fold lower than the concentrations at which they interact with $ET_A$ receptors. In these compounds, $R^2$ is selected from among alkyl, lower alkenyl, lower alkynyl, lower haloalkyl, halide or H; and $R^1$ is halide or loweralkyl, and in preferred embodiments, $R^1$ is bromide or chloride, preferabaly chloride; $R^9$ and $R^{10}$ are selected independently from hydrogen, loweralkyl, preferably methyl or ethyl, or halide, and $R^8$, which is the substituent at the 5-position (see, e.g., formulae III and IV), is aryl or a heterocycle, particularly phenyl and isoxazolyl, which are unsubstituted or substituted with Z, which is preferably loweralkyl or halide.

Pharmaceutical compositions formulated for administration by an appropriate route and means containing effective concentrations of one or more of the compounds provided herein or pharmaceutically acceptable salts or acids thereof (referred to below as "the compounds") that deliver amounts effective for the treatment of hypertension, stroke, asthma, shock, ocular hypertension, glaucoma, renal failure, inadequate retinal perfusion and other conditions that are in some manner mediated by an endothelin peptide or that involve vasoconstriction or whose symptoms can be ameliorated by administration of an endothelin antagonist or agonist, are also provided. Particularly preferred compositions are those that deliver amounts effective for the treatment of hypertension or renal failure. The effective amounts and concentrations are effective for ameliorating any of the symptoms of any of the disorders.

Use of the compounds in the preparation of a medicament for inhibiting binding of an endothelin peptide to an endothelin receptor is provided. The inhibition is practiced by contacting the receptor with one or more of the compounds provided herein simultaneously, prior to, or subsequent to contacting the receptor with an endothelin peptide.

Use of the compounds in the preparation of a medicament for treatment of endothelin-mediated disorders, including but not limited to, hypertension, asthma, shock, ocular hypertension, glaucoma, inadequate retinal perfusion and other conditions that are in some manner mediated by an endothelin peptide, or for treatment of disorder that involve vasoconstriction or that are ameliorated by administration of an endothelin antagonist or agonist are provided.

In particular, treating endothelin-mediated disorders by administering effective amounts of the sulfonamides, prodrugs or other suitable derivatives of the sulfonamides is envisaged. In particular, treating endothelin-mediated disorders, including hypertension, cardiovascular diseases, cardiac diseases including myocardial infarction, pulmonary hypertension, erythropoietin-mediated hypertension, respiratory diseases and inflammatory diseases, including asthma, bronchoconstriction, ophthalmologic diseases, gastroenteric diseases, renal failure, endotoxin shock, menstrual disorders, obstetric conditions, wounds, anaphylactic shock, hemorrhagic shock, and other diseases in which endothelin mediated physiological responses are implicated, by administering effective amounts of one or more of the compounds provided herein in pharmaceutically acceptable carriers are envisaged. Preferred are treatments of hypertension and renal failure.

More treatments are those in which the compositions contain at least one compound that inhibits the interaction of endothelin-1 with $ET_A$ receptors at an $IC_{50}$ of less than about 10 μM, and preferably less than about 5 μM, more preferably less than about 1 μM, even more preferably less than 0.1 μM, and most preferably less than 0.05 μM Other preferred treatments are those in which the compositions contain one or more compounds that is (are) $ET_A$ selective or one or more compounds that is (are) $ET_B$ selective. Uses in which the compounds are $ET_A$ selective are for treatment of disorders, such as hypertension; and uses in which the compounds are $ET_B$ selective are for treatment of disorders, such as asthma, that require bronchodilation.

In practicing the invention, effective amounts of compositions containing therapeutically effective concentrations of the compounds formulated for oral, intravenous, local and topical application for the treatment of hypertension, cardiovascular diseases, cardiac diseases, including myocardial infarction, respiratory diseases, including asthma, inflammatory diseases, ophthalmologic diseases, gastroenteric diseases, renal failure, immunosuppressant-mediated renal vasoconstriction, erythropoietin-mediated vasoconstriction, endotoxin shock, anaphylactic shock, hemorrhagic shock, pulmonary hypertension, and other diseases in which endothelin mediated physiological responses are implicated are administered to an individual exhibiting the symptoms of one or more of these disorders. The amounts are effective to ameliorate or eliminate one or more symptoms of the disorders.

The compounds of the invention may also be used in the identification and isolation of endothelin receptor subtypes. In particular, methods for detecting, distinguishing and isolating endothelin receptors using the disclosed compounds are considered, In particular, methods for detecting, distinguishing and isolating endothelin receptors using the compounds provided herein.

In addition, the compounds may be used for identifying compounds that are suitable for use in treating particular diseases based on their preferential affinity for a particular endothelin receptor subtype.

Articles of manufacture containing packaging material, a compound provided herein, which is effective for ameliorating the symptoms of an endothelin-mediated disorder, antagonizing the effects of endothelin or inhibiting binding of an endothelin peptide to an ET receptor with an $IC_{50}$ of less than about 10 μM, within the packaging material, and a label that indicates that the compound or salt thereof is used for antagonizing the effects of endothelin, treating an endothelin-mediated disorder, or inhibiting the binding of an endothelin peptide to an ET receptor are provided.

## DETAILED DESCRIPTION OF PREFERRED EMBODIMENTS

### Definitions

Unless defined otherwise, all technical and scientific terms used herein have the same meaning as is commonly understood by one of skill in the art to which this invention belongs. All patents and publications referred to herein are incorporated by reference.

As used herein, endothelin (ET) peptides include peptides that have substantially the amino acid sequence of endothelin-1, endothelin-2 or endothelin-3 and that act as potent endogenous vasoconstrictor peptides.

As used herein, an endothelin-mediated condition is a condition that is caused by abnormal endothelin activity or one in which compounds that inhibit endothelin activity have therapeutic use. Such diseases include, but are not limited to hypertension, cardiovascular disease, asthma, inflammatory diseases, ophthalmologic disease, menstrual disorders, obstetric conditions, gastroenteric disease, renal failure, pulmonary hypertension, endotoxin shock, anaphylactic shock, or hemorrhagic shock. Endothelin-mediated conditions also include conditions that result from therapy with agents, such as erythropoietin and immunosuppressants, that elevate endothelin levels.

As used herein an effective amount of a compound for treating a particular disease is an amount that is sufficient to ameliorate, or in some manner reduce the symptoms associated with the disease. Such amount may be administered as a single dosage or may be administered according to a regimen, whereby it is effective. The amount may cure the disease but, typically, is administered in order to ameliorate the symptoms of the disease. Typically, repeated administration is required to achieve the desired amelioration of symptoms.

As used herein, an endothelin agonist is a compound that potentiates or exhibits a biological activity associated with or possessed by an endothelin peptide.

As used herein, an endothelin antagonist is a compound, such as a drug or an antibody, that inhibits endothelin-stimulated vasoconstriction and contraction and other endothelin-mediated physiological responses. The antagonist may act by interfering with the interaction of the endothelin with an endothelin-specific receptor or by interfering with the physiological response to or bioactivity of an endothelin isopeptide, such as vasoconstriction. Thus, as used herein, an endothelin antagonist interferes with endothelin-stimulated vasoconstriction or other response or interferes with the interaction of an endothelin with an endothelin-specific receptor, such as $ET_A$ receptors, as assessed by assays known to those of skill in the art.

The effectiveness of potential agonists and antagonists can be assessed using methods known to those of skill in the art. For example, endothelin agonist activity can be identified by its ability to stimulate vasoconstriction of isolated rat thoracic aorta or portal vein ring segments (Borges et al. (1989) "Tissue selectivity of endothelin" Eur. J.

Pharmacol. 165: 223-230). Endothelin antagonist activity can be assessed by the ability to interfere with endothelin-induced vasoconstriction. Exemplary assays are set forth in the EXAMPLES. As noted above, the preferred $IC_{50}$ concentration ranges are set forth with reference to assays in which the test compound is incubated with the ET receptor-bearing cells at 4° C. Data presented for assays in which the incubation step is performed at the less preferred 24° C are identified. It is understood that for purposes of comparison, these concentrations are somewhat higher than the concentrations determined at 4° C.

As used herein, the biological activity or bioactivity of endothelin includes any activity induced, potentiated or influenced by endothelin in vivo. It also includes the ability to bind to particular receptors and to induce a functional response, such as vasoconstriction. It may be assessed by in vivo assays or by in vitro assays, such as those exemplified herein. The relevant activities include, but are not limited to, vasoconstriction, vasorelaxation and bronchodilation. For example, $ET_B$ receptors appear to be expressed in vascular endothelial cells and may mediate vasodilation and other such responses; whereas $ET_A$ receptors, which are endothelin-1-specific, occur on smooth muscle and are linked to vasoconstriction Any assay known to those of skill in the art to measure or detect such activity may be used to assess such activity (see, e.g., Spokes et al. (1989) J. Cardiovasc. Pharmacol. 13(Suppl. 5):S191-S192; Spinella et al. (1991) Proc. Natl. Acad. Sci. USA 88: 7443-7446; Cardell et al. (1991) Neurochem. Int. 18:571-574); and the Examples herein).

As used herein, the $IC_{50}$ refers to an amount, concentration or dosage of a particular test compound that achieves a 50% inhibition of a maximal response, such as binding of endothelin to tissue receptors, in an assay that measures such response.

As used herein, $EC_{50}$ refers to a dosage, concentration or amount of a particular test compound that elicits a dose-dependent response at 50% of maximal expression of a particular response that is induced, provoked or potentiated by the particular test compound.

As used herein a sulfonamide that is $ET_A$ selective refers to sulfonamides that exhibit an $IC_{50}$ that is at least about 10-fold lower with respect to $ET_A$ receptors than $ET_B$ receptors.

As used herein, a sulfonamide that is $ET_B$ selective refers to sulfonamides that exhibit an $IC_{50}$ that is at least about 10-fold lower with respect to $ET_B$ receptors than $ET_A$ receptors.

As used herein, pharmaceutically acceptable salts, esters or other derivatives of the compounds include any salts, esters or derivatives that may be readily prepared by those of skill in this art using known methods for such derivatization and that produce compounds that may be administered to animals or humans without substantial toxic effects and that either are pharmaceutically active or are prodrugs. For example, hydroxy groups can be esterified or etherified.

As used herein, treatment means any manner in which the symptoms of a conditions, disorder or disease are ameliorated or otherwise beneficially altered. Treatment also encompasses any pharmaceutical use of the compositions herein, such as use as contraceptive agents.

As used herein, amelioration of the symptoms of a particular disorder by administration of a particular pharmaceutical composition refers to any lessening, whether permanent or temporary, lasting or transient that can be attributed to or associated with administration of the composition.

As used herein, substantially pure means sufficiently homogeneous to appear free of readily detectable impurities as determined by standard methods of analysis, such as thin layer chromatography (TLC), gel electrophoresis and high performance liquid chromatography (HPLC), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers. In such instances, further purification might increase the specific activity of the compound.

As used herein, biological activity refers to the in vivo activities of a compound or physiological responses that result upon in vivo administration of a compound, composition or other mixture. Biological activity, thus, encompasses therapeutic effects and pharmaceutical activity of such compounds, compositions and mixtures.

As used herein, a prodrug is a compound that, upon in vivo administration, is metabolized or otherwise converted to the biologically, pharmaceutically or therapeutically active form of the compound. To produce a prodrug, the pharmaceutically active compound is modified such that the active compound will be regenerated by metabolic processes. The prodrug may be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism in vivo, those of skill in this art, once a pharmaceutically active compound is known, can design prodrugs of the compound (see, e.g., Nogrady (1985) Medicinal Chemistry A Biochemical Approach, Oxford University Press, New York, pages 388-392). For example, succinyl-sulfathiazole is a prodrug of 4-amino-N-(2-thiazoyl)benzenesulfonamide (sulfathiazole) that exhibits altered transport characteristics.

As used herein, acid isostere means a group that is significantly ionized at physiological pH. Examples of

suitable acid isosteres include sulfo, phosphono, alkylsulfonylcarbamoyl, tetrazolyl, arylsulfonylcarbamoyl or heteroarylsulfonylcarbamoyl.

As used herein, halo or halide refers to the halogen atoms; F, Cl, Br and I.

As used herein, pseudohalides are compounds that behave substantially similar to halides. Such compounds can be used in the same manner and treated in the same manner as halides ($X^-$, in which X is a halogen, such as Cl or Br). Pseudohalides include, but are not limited to cyanide, cyanate, thiocyanate, selenocyanate and azide.

As used herein, haloalkyl refers to a loweralkyl radical in which one or more of the hydrogen atoms are replaced by halogen including, but not limited to, chloromethyl, trifluoromethyl, 1-chloro-2-fluoroethyl and the like.

As used herein, alkyl means an aliphatic hydrocarbon group that is a straight or branched chain preferably having about 1 to 12 carbon atoms in the chain. Preferred alkyl groups are loweralkyl groups which are alkyls containing 1 to about 6 carbon atoms in the chain. Branched means that one or more loweralkyl groups such as methyl, ethyl or propyl are attached to a linear alkyl chain. The alkyl group may be unsubstituted or independently substituted by one or more groups, such as, but not limited to: halo, carboxy, formyl, sulfo, sulfino, carbamoyl, amino and imino. Exemplary alkyl groups include methyl, ethyl, propyl, methanoic acid, ethanoic acid, propanoic acid, ethanesulfinic acid and ethane sulfonic acid.

As used herein the term lower describes alkyl, alkenyl and alkynyl groups containing about 6 carbon atoms or fewer. It is also used to describe aryl groups or heteroaryl groups that contain 6 or fewer atoms in the ring. Loweralkyl, lower alkenyl, and lower alkynyl refer to carbon chains having less than about 6 carbons. In preferred embodiments of the compounds provided herein that include alkyl, alkenyl, or alkynyl portions include loweralkyl, lower alkenyl, and lower alkynyl portions.

As used herein, alkenyl means an aliphatic hydrocarbon group containing a carbon-carbon double bond and which may be straight or branched chained having from about 2 to about 10 carbon atoms in the chain. Preferred alkenyl groups have 2 to about 4 carbon atoms in the chain. Branched means that one or more loweralkyl or lower alkenyl groups are attached to a linear alkenyl chain. The alkenyl group may be unsubstituted or independently substituted by one or more groups, such as halo, carboxy, formyl, sulfo, sulfino, carbamoyl, amino and imino. Exemplary alkenyl groups include ethenyl, propenyl, carboxyethenyl, carboxypropenyl, sulfinoethenyl and sulfonoethenyl.

As used herein, alkynyl means an aliphatic hydrocarbon group containing a carbon-carbon triple bond and which may be straight or branched having about 2 to 10 carbon atoms in the chain. Branched means that one or more loweralkyl, alkenyl or alkynyl groups are attached to a linear alkynyl chain. An exemplary alkynyl group is ethynyl.

As used herein, aryl means an aromatic monocyclic or multicyclic hydrocarbon ring system containing from 3 to 15 or 16 carbon atoms, preferably from 5 to 10. Aryl groups include, but are not limited to groups, such as phenyl, substituted phenyl, napthyl, substituted naphthyl, in which the substitunent is loweralkyl, halogen, or lower alkoxy. Preferred aryl groups are lower aryl groups that contain less than 7 carbons in the ring structure.

As used herein, the nomenclature alkyl, alkoxy, carbonyl, etc. are used as is generally understood by those of skill in this art. For example, as used herein alkyl refers to saturated carbon chains that contain one or more carbons; the chains may be straight or branched or include cyclic portions or be cyclic. As used herein, alicyclic refers to aryl groups that are cyclic.

As used herein, cycloalkyl refers to saturated cyclic carbon chains; cycloalkyenyl and cycloalkynyl refer to cyclic carbon chains that include at least one unsaturated double or triple bond, respectively. The cyclic portions of the carbon chains may include one ring or two or more fused rings.

As used herein, cycloalkenyl means a non-aromatic monocyclic or multicyclic ring system containing a carbon-carbon double bond and having about 3 to about 10 carbon atoms. Exemplary monocyclic cycloalkenyl rings include cyclopentenyl or cyclohexenyl; preferred is cyclohexenyl. An exemplary multicyclic cycloalkenyl ring is norbornylenyl. The cycloalkenyl group may be independently substituted by one or more halo or alkyl.

As used herein, "haloalkyl" refers to a loweralkyl radical in which one or more of the hydrogen atoms are replaced by halogen including, but not limited to, chloromethyl, trifluoromethyl, 1-chloro-2-fluoroethyl and the like.

As used herein, "haloalkoxy" refers to RO- in which R is a haloalkyl group.

As used herein, "carboxamide" refers to groups of formula $R_pCONH_2$ in which R is selected from alkyl or aryl, preferably loweralkyl or lower aryl and p is 0 or 1.

As used herein, "alkylaminocarbonyl" refers to -C(O)NHR in which R is hydrogen, alkyl, preferably loweralkyl or aryl, preferably lower aryl.

As used herein "dialkylaminocarbonyl" as used herein refers to -C(O)NR'R in which R' and R are independently selected from alkyl or aryl, preferably loweralkyl or loweraryl; "carboxamide" refers to groups of formula NR'COR.

As used herein, "alkoxycarbonyl" as used herein refers to -C(O)OR in which R is alkyl, preferably loweralkyl or aryl, preferably lower aryl.

As used herein, "alkoxy" and "thioalkoxy" refer to RO- and RS-, in which R is alkyl, preferably loweralkyl or aryl, preferably lower aryl.

As used herein, "haloalkoxy" refers to RO- in which R is a haloalkyl group.

As used herein, "aminocarbonyl" refers to $-C(O)NH_2$.

As used herein, "alkylaminocarbonyl" refers to -C(O)NHR in which R is alkyl, preferably loweralkyl or aryl, preferably lower aryl.

As used herein, "alkoxycarbonyl" refers to -C(O)OR in which R is alkyl, preferably loweralkyl.

As used herein, cycloalkyl refers to satured cyclic carbon chains; cycloalkyenyl and cycloalkynyl refer to cyclic carbon chains that include at least one unsaturated triple bond. The cyclic portions of the carbon chains may include one ring or two or more fused rings.

As used herein, alkylenedioxy means an -O-alkyl-O- group in which the alkyl group is as previously described. A replacement analog of alkylenedioxy means an alkylenedioxy in which one or both of the oxygen atoms is replaced by a similar behaving atom or group of atoms such as, S, N, NH, Se. An exemplary replacement alkylenedioxy group is ethylenebis(sulfandiyl). Alkylenethioxyoxy is -S-alkyl-O- , -O-alkyl-S- and alkylenedithioxy is -S-alkyl-S-.

As used herein, heteroaryl means an aromatic monocyclic or fused ring system in which one or more of the carbon atoms in the ring system is(are) replaced by an element(s) other than carbon, for example nitrogen, oxygen or sulfur. Preferred cyclic groups contain one or two fused rings and include from about 3 to about 7 members in each ring. Similar to "aryl groups", the heteroaryl groups may be unsubstituted or substituted by one or more substituents. Exemplary heteroaryl groups include pyrazinyl, pyrazolyl, tetrazolyl, furanyl, (2- or 3-)thienyl, (2-,3- or 4-)pyridyl, imidazoyl, pyrimidinyl, isoxazolyl, thiazolyl, isothiazolyl, quinolinyl, indolyl, isoquinolinyl, oxazolyl and 1,2,4-oxadiazolyl. Preferred heteroaryl groups include 5 to 6-membered nitrogen-containing rings, such as pyrmidinyl.

As used herein, alkoxycarbonyl means an alkyl-O-CO- group. Exemplary alkoxycarbonyl groups include methoxy- and ethoxycarbonyl.

As used herein, carbamoyl means $-CONH_2$. As with all groups described herein, these groups may be unsubstituted or substituted. Substituted carbamoyl includes groups such as $-CONY^2Y^3$ in which $Y^2$ and $Y^3$ are independently hydrogen, alkyl, cyano(loweralkyl), aryalkyl, heteroaralkyl, carboxy(loweralkyl), carboxy(aryl substituted loweralkyl), carboxy(carboxy substituted loweralkyl), carboxy(hydroxy substituted loweralkyl), carboxy(heteroaryl substituted loweralkyl), carbamoyl(loweralkyl), alkoxycarbonyl(loweralkyl) or alkoxycarbonyl(aryl substituted loweralkyl), provided that only one of $Y^2$ and $Y^3$ may be hydrogen and when one of $Y^2$ and $Y^3$ is carboxy(loweralkyl), carboxy(aryl substituted loweralkyl), carbamoyl(loweralkyl), alkoxycarbonyl(loweralkyl) or alkoxycarbonyl(aryl substituted loweralkyl) then the other of $Y^2$ and $Y^3$ is hydrogen or alkyl. Preferred for $Y^2$ and $Y^3$ are independently hydrogen, alkyl, cyano (loweralkyl), aryalkyl, heteroaralkyl, carboxy(loweralkyl), carboxy(aryl substituted loweralkyl) and carbamoyl(loweralkyl).

As used herein, any corresponding N-(4-halo-3-methyl-5-isoxazolyl), N-(4-halo-5-methyl-3-isoxazolyl), N-(3,4-dimethyl-5-isoxazolyl), N-(4-halo-5-methyl-3-isoxazolyl), N-(4-halo-3-methyl-5-isoxazolyl), N-(4,5-dimethyl-3-isoxazolyl) derivative thereof refers to compounds in which $Ar^2$ is the same as the compound specifcally set forth, but $Ar^1$ is N-(4-halo-3-methyl-5-isoxazolyl), N-(4-halo-5-methyl-3-isoxazolyl), N-(3,4-dimethyl-5-isoxazolyl), N-(4-halo-5-methyl-3-isoxazolyl), N-(4-halo-3-methyl-5-isoxazolyl), or N-(4,5-dimethyl-3-isoxazolyl) in which halo is any halide, peferably Cl or Br.

As used herein, the abbreviations for any protective groups, amino acids and other compounds, are, unless indicated otherwise, in accord with their common usage, recognized abbreviations, or the IUPAC-IUB Commission on Biochemical Nomenclature (see, (1972) Biochem. 11:942-944).

### A. Compounds for use in treating endothelin-mediated diseases

Compounds and their use in preparation of a medicament for treating endothelin-mediated diseases are provided. In the compounds provided herein, $Ar^1$ is N-(5-isoxazolyl) or N-(3-isoxazolyl).

The most preferred compounds provided herein have an $IC_{50}$ for $ET_A$ receptors in the assays exemplified herein less than 0.1 μM, more prefereably less than 0.01 μM, and more preferably less than 0.001 (see, e.g., Table 1 for representative experimental results), when measured at 4° C, as described in the Examples. When measured at 24° C, the $IC_{50}$ concentrations are somewhat higher (2- to 10-fold; see, Table 1 for some comparative values).

M is most preferably chosen from

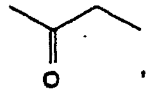,
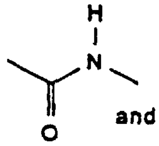
and
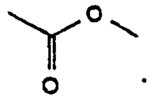.

In more preferred compounds, M is $C(O)CH_2$, C(O)NH, -CH=CH-, $CH_2CH_2C(O)(CH)_2$, $CH_2CHC(O)CH_2$, and

most preferably has formula VII:

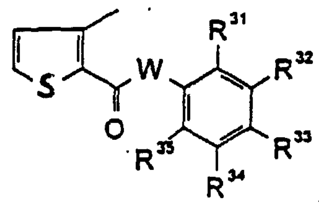

in which W is $CH_2$ or NH.

In preferred embodiments, $R^{31}$, $R^{33}$, $R^{35}$ are other then hydrogen and are preferably loweralkyl or lower alkoxy, or $R^{31}$ or $R^{35}$ is other than hydrogen, preferably loweralkyl or lower alkoxy, and $R^{32}$ and $R^{33}$ or $R^{33}$ and $R^{34}$ form methylenedioxy.

In all embodiments, preferred substituents also can be determined by reference to Table 1, which sets forth exemplary compounds. Preferred compounds are those of Table 1 that have the highest activities, and preferred substituents are those on the compounds with the highest activities. Compounds of the invention are marked "Inv." is Table 1.

TABLE 1

| COMPOUND | $ET_A$ (μM)* | $ET_B$ (μM)* | |
|---|---|---|---|
| N-(3,4-dimethyl-5-isoxazolyl)-2-methylbenzo[b]thiophene-3-sulfonamide | 0.167 | 16.6 | |
| N-(4-bromo-3-methyl-5-isoxazolyl)-2-methylbenzo[b]thiophene-3-sulfonamide | 0.0486 | 3.5 | |
| N-(4-bromo-3-methyl-5-isoxazolyl)-2-ethylbenzo[b]thiophene-3-sulfonamide | 0.0067 | 5.13 | |
| N-(4-bromo-3-methyl-5-isoxazolyl)-2-n-benzylbenzo[b]thiophene-3-sulfonamide | 0.0182 | ~1 | |
| N-(4-bromo-3-methyl-5-isoxazolyl)-2-butylbenzo[b]thiophene-3-sulfonamide | 0.0226 | ~3 | |
| N-(4-bromo-3-methyl-5-isoxazolyl)-2-*i*-propylbenzo[b]thiophene-3-sulfonamide | 0.005<br>0.03† | 5.7<br>10.7† | |
| N-(4-bromo-3-methyl-5-isoxazolyl)-2-n-propylbenzo[b]thiophene-3-sulfonamide | 0.024<br>0.074† | 7.95<br>16.6† | |
| N-(4-bromo-3-methyl-5-isoxazolyl)-2-(4-ethylbenzyl)benzo[b]thiophene-3-sulfonamide | 0.048† | 1.1† | |
| N-(4-bromo-3-methyl-5-isoxazolyl)-2-[3,4-(methylenedioxy)benzyl]benzo[b]thiophene-3-sulfonamide | 0.0015±0.0014<br>0.0074±0.0011† | 0.324±0.78<br>0.939±0.262† | Inv. |
| N-(4-bromo-3-methyl-5-isoxazolyl)-2-(3,4,5-trimethoxybenzyl)-benzo[b]-thiophene-3-sulfonamide | 0.013† | 1.2† | Inv. |
| N-(4-bromo-3-methyl-5-isoxazolyl)-2-ethyl-5-methylbenzo[b]thiophene-3-sulfonamide | 1.89±0.431† | 54.3±2.6† | |
| N-(4-chloro-3-methyl-5-isoxazolyl)-2-[(3,4-methylenedioxy)benzyl]benzo[b]thiophene-3-sulfonamide | 0.011±0.005† | 0.936±0.095† | Inv. |

| COMPOUND | $ET_A$ ($\mu M$)* | $ET_B$ ($\mu M$)* | |
|---|---|---|---|
| N-(4-bromo-3-methyl-5-isoxazolyl)-2-(3,4-dimethoxybenzyl)benzo[b]thiophene-3-sulfonamide | 0.021 ±0.017† | 2.94 ± 1.32† | Inv. |
| N-(4-bromo-3-methyl-5-isoxazolyl)-5-(benzo[b]thien-2-yl)thiophene-2- sulfonamide | 16† | 0.80† | |
| N-(4-bromo-3-methyl-5-isoxazolyl)-2-(4-methoxybenzyl)benzo[b]thiophene-3-sulfonamide | 0.051† | 1.5† | |
| N-(4-bromo-3-methyl-5-isoxozolyl)-2-(2-methoxybenzyl)-benzo[b]thiophene-3-sulfonamide | 0.19† | 2.2† | |
| N-(3,4-dimethyl-5-isoxazolyl)-2-(4-chlorobenzyl)benzo[b]thiophene-3-sulfonamide | 0.21† | 4.7† | |
| N-(4-chloro-3-methyl-5-isoxazolyl)-2-(4-dimethylaminobenzyl)benzo[b]thiophene-3-sulfonamide | 0.041†<br>0.014 | 1.3†<br>0.477 | |
| N-(4-chloro-3-methyl-5-isoxazolyl)-2-ethylbenzo[b]furan-3-sulfonamide | 0.15† | 22† | |
| N-(4-chloro-3-methyl-5-isoxazolyl)-2-phenylbenzo[b]thiophene sulfonamide | 0.932† | 46.8† | |
| N-(4-chloro-3-methyl-5-isoxazolyl)-6-methoxy-2-[3,4-(methylenedioxy)benzyl]benzo[b]thiophene-3-sulfonamide | ~2***† | 2.39† | Inv. |
| N-(4-chloro-5-methyl-3-isoxazolyl)-2-[3,4-(methylenedioxy)benzyl]benzo[b]thiophene-3-sulfonamide | 0.0055† | 0.364† | Inv. |
| N-(4-chloro-3-methyl-5-isoxazolyl)-2-methoxycarbonylthiophene-3-sulfonamide | 0.631 | 53.2 | |
| N-(4-bromo-3-methyl-5-isoxazolyl)-4-(4-propylphenyl)thiophene-2-sulfonamide | 0.962† | 0.435† | |
| N-(4-bromo-3-methyl-5-isoxazolyl)-3-(phenylthio)thiophene-2-sulfonamide | 0.0801† | 3.68† | |
| N-(3,4-dimethyl-5-isoxazolyl))-3-(phenylaminocarbonyl)thiophene-2-sulfonamide | 0.163 | >100 | |
| N-(4-chloro-3-methyl-5-isoxazolyl)-2-[(4-tolyl)aminocarbonyl]thiophene-3-sulfonamide | 0.00116<br>0.0105† | 2.93<br>14† | |
| N-(4-bromo-3-methyl-5-isoxazolyl)-5-(4-methoxyphenyl)thiophene-2-sulfonamide | 8.69<br>26.3† | 0.363<br>2.4† | |
| N-(4-bromo-3-methyl-5-isoxazolyl)-5-(3-methoxyphenyl)thiophene-2-sulfonamide | 3.26<br>23.4† | 0.776<br>4.7† | |

| COMPOUND | $ET_A$ (μM)* | $ET_B$ (μM)* |
|---|---|---|
| N-(4-bromo-3-methyl-5-isoxazolyl)-5-(3-thienyl)thiophene-2-sulfonamide | 4.49 | 0.380 |
| N-(4-bromo-3-methyl-5-isoxazolyl)-3-methylthiophene-2-sulfonamide | 0.651 | 7.15 |
| N-(4-bromo-3-methyl-5-isoxazolyl)-3-(phenethyl)thiophene-2-sulfonamide | 0.16<br>0.676† | 10.77<br>37.2† |
| N-(4-bromo-3-methyl-5-isoxazolyl)-4-(phenethyl)thiophene-2-sulfonamide | 6.64 | 3.97 |
| N-(3,4-dimethyl-5-isoxazolyl)-2-[(4-methylphenyl)-aminocarbonyl]thiophene-3-sulfonamide | 0.00336 | 11.3 |
| N-(4-bromo-3-methyl-5-isoxazolyl)-2,5-dimethyl-4-phenylthiophene-3-sulfonamide | 1.40 | ~100 |
| N-(4-bromo-3-methyl-5-isoxazolyl)-2-[(methyl)phenylaminocarbonyl]thiophene-3-sulfonamide | 0.188 | 16.0 |
| N-(4-bromo-3-methyl-5-isoxazolyl)-2-(α-hydroxybenzyl)thiophene-3-sulfonamide | 0.337 | 9.37 |
| N-(4-bromo-5-methyl-3-isoxazolyl)-5-(4-methylphenyl)thiophene-2-sulfonamide | 7.10<br>15.8† | 0.3593<br>0.25† |
| N-(4-bromo-3-methyl-5-isoxazolyl)-5-phenylthiophene-2-sulfonamide | 3.53<br>36.6† | 0.417<br>2.4† |
| N-(4-bromo-3-methyl-5-isoxazolyl)-5-[4-(trifluoromethyl)phenyl]thiophene-2-sulfonamide | 6.39<br>6.31† | 0.0835<br>.282† |
| N,N'-bis{3-[(4-bromo-3-methyl-5-isoxazolyl)aminosulfonyl)thien-2-yl} urea | 0.0692<br>0.295† | 0.290<br>1.19† |
| N-(4-bromo-3-methyl-5-isoxazolyl)-2-(hydroxymethyl)thiophene-3- sulfonamide | 0.160<br>1.55† | 44.1<br>-- |
| N-(4-bromo-3-methyl-5-isoxazolyl)-5-(2-formylphenyl)thiophene-3-sulfonamide | 3.46<br>12.31† | 0.529<br>1.28±0.71† |
| N,N'-bis{3-[3,4-dimethyl-5-isoxazolyl)aminosulfonyl]thien-2-yl}urea | 1.01±1.03<br>2.7† | 3.7±2.7<br>5.9† |
| N-(3,4-dimethyl-5-isoxazolyl))-2-[(3-methoxyanilino)methyl]thiophene-3- sulfonamide | 0.214<br>0.933† | 5.34<br>7.7† |
| N-(4-bromo-3-methyl-5-isoxazolyl)-5-(3-aminophenyl)thiophene-2-sulfonamide | 0.537<br>1.44† | 1.07<br>2.63† |
| N-(4-bromo-3-methyl-5-isoxazolyl)-5-[3,5-bis(triflouromethyl)phenyl]thiophene-2-sulfonamide | 0.794<br>5.9† | 12.0<br>15.5† |

| COMPOUND | $ET_A$ ($\mu$M)* | $ET_B$ ($\mu$M)* |
|---|---|---|
| N-(4-bromo-3-methyl-5-isoxazolyl)-5-(3,3-dimethylbutyn-1-yl)thiophene-2-sulfonamide | 1.12<br>7.24† | 24.0<br>35.5† |
| N-(4-bromo-3-methyl-5-isoxazolyl)-5-(2-methoxyphenyl)thiophene-2- sulfonamide | 0.381 | 1.097 |
| N-(4-bromo-3-methyl-5-isoxazolyl)-5-(2-tolyl)thiophene-2-sulfonamide | 0.432 | 0.313 |
| N-(4-bromo-3-methyl-5-isoxazolyl)-2-[(3-carboxyphenyl)aminocarbonyl]thiophene-3-sulfonamide | 0.062† | >100† |
| N-(4-bromo-3-methyl-5-isoxazolyl)-2-[2-carboyxylphenyl)aminocarbonyl]-thiophene-3-sulfonamide | 0.21† | 20† |
| N-(4-bromo-3-methyl-5-isoxazolyl)-2-(aminocarbonyl)thiophene-3-sulfonamide | 0.84† | >100† |
| N-(4-bromo-3-methyl-5-isoxazolyl)-2-[(5-dimethylamino-1-naphthyl)sulfonyl-aminocarbonyl]thiophene-3-sulfonamide | 0.97† | 3.9† |
| N-(4-bromo-3-methyl-5-isoxazolyl)-5-(5-methyl-2-thienyl)thiophene-2-sulfonamide | 17† | 0.21† |
| N-(4-bromo-3-methyl-5-isoxazolyl)-2-[(3,4-methylenedioxyphenyl)aminocarbonyl]thiophene-3-sulfonamide | 0.017† | 9.8† |
| N-(4-bromo-3-methyl-5-isoxazolyl)-2-[(3,4-methylenedioxy)phenoxycarbonyl]thiophene-3-sulfonamide | 0.0073† | 6.0† |
| N-(4-bromo-3-methyl-5-isoxazolyl)-3-[(3,4-methylenedioxy)phenyl]thiophene-2-sulfonamide | 0.50† | 79† |
| N-(4-bromo-3-methyl-5-isoxazolyl)-3-[(3,4-methylenedioxy)benzyl]thiophene-2-sulfonamide | 8.1† | 3.2† |
| N-(4-bromo-3-methyl-5-isoxazolyl)-3-benzylthiophene-2-sulfonamide | 1.6† | 39† |
| N-(4-bromo-3-methyl-5-isoxazolyl)-5-(3-tolyl)thiophene-2-sulfonamide | 15† | 4.2† |
| N-(4-bromo-3-methyl-5-isoxazolyl)-2-[(3,4-methylenedioxy)benzyl]thiophene-3-sulfonamide | 0.27† | 7.7† |
| N-(4-bromo-3-methyl-5-isoxazolyl)-2-[(3,4-methylenedioxy)benzoyl]thiophene-3-sulfonamide | 2.0† | 15† |

| COMPOUND | $ET_A$ (μM)* | $ET_B$ (μM)* |
|---|---|---|
| N-(4-bromo-3-methyl-5-isoxazolyl)-2-[(2-hydroxyphenyl)aminocarbonyl]thiophene-3-sulfonamide | 0.013† | 38† |
| N-(3,4-dimenthyl-5-isoxazolyl)-2-[3,4-(methylenedioxy)phenoxycarbonyl]thiophene-3-sulfonamide | 6.1† | >-50† |
| N-(4-bromo-3-methyl-5-isoxazolyl)-5-(5-ethylthien-2-yl)thiophene-2- sulfonamide | 24† | 7.7† |
| N-(4-bromo-3-methyl-5-isoxazolyl)-2-[(3,4-methylenedioxy)benzoyl]aminocarbonyl]thiophene-3-sulfonamide | 0.089† | 37† |
| N-(4-chloro-3-methyl-5-isoxazolyl)-2-[3,4-(methylenedioxy)phenoxycarbonyl]thiophene-3-sulfonamide | 0.0065† | 7.4† |
| N-(4-bromo-3-methyl-5-isoxazolyl)-5-(1-pentynyl)thiophene-2-sulfonamide | 29† | 5.6† |
| N-(4-chloro-3-methyl-5-isoxazolyl)-5-(5-ethylthien-2-yl)thiophene-2- sulfonamide | 12† | 0.71† |
| N-(4-bromo-3-methyl-5-isoxazolyl)-2-[(3,4-methylenedioxy)phenylacetyl]thiophene-3-sulfonamide | 0.0091† | 5.5† |
| N-(4-bromo-3-methyl-5-isoxazolyl)-2-[3,4-(methylenedioxy)phenoxycarbonylamino]thiophene-3-sulfonamide | 0.087† | 5.9† |
| N-(4-bromo-3-methyl-5-isoxazolyl)-2-[(2-chloro-3,4-methylenedioxy)phenoxymethyl]thiophene-3-sulfonamide | 13† | 0.76† |
| N-(4-bromo-3-methyl-5-isoxazolyl)-2-[*trans*-(3,4-methylenedioxy)cinnamyl]thiophene-3-sulfonamide | 0.14† | 1.4† |
| N-(4-bromo-3-methyl-5-isoxazolyl)-5-(1-naphthyl)-thiophene-2-sulfonamide | 14† | 1.4† |
| N-(4-bromo-3-methyl-5-isoxazolyl)-5-(3-nitrophenyl)thiophene-2-sulfonamide | 26† | 4.5† |
| N-(4-bromo-3-methyl-5-isoxazolyl)-2-[(3,4-methylenedioxy)phenylureido]thiophene-3-sulfonamide | 0.57† | 1.3† |
| N-(4-chloro-3-methyl-5-isoxazolyl)-2-[(3,4-(methylenedioxy)phenylacetyl]thiophene-3-sulfonamide | 0.021† | 6.5† |

Inv.

| COMPOUND | $ET_A$ (μM)* | $ET_B$ (μM)* |
|---|---|---|
| N-(4-bromo-3-methyl-5-isoxazolyl)-5-(4-methyoxycarbonylphenyl)thiophene-2-sulfonamide | >100† | 17† |
| N-(4-bromo-3-methyl-5-isoxazolyl)-5-(4-carboxyphenyl)thiophene-2-sulfonamide | >100† | 31† |
| N-(4-bromo-3-methyl-5-isoxazolyl)-5-(4-tolyl)aminocarbonyl)thiophene-2-sulfonamide | 28† | 8.6† |
| N-(4-bromo-3-methyl-5-isoxazolyl)-5-(2-methyfuranyl)thiophene-2-sulfonamide | 32† | 7.5† |
| N-(4-chloro-3-methyl-5-isoxazolyl)-2-[3,4-(methylenedioxy)benzyloxycarbonyl]thiophene-3-sulfonamide | .42† | 12† |
| N-(4-chloro-3-methyl-5-isoxazolyl)-2-[2-(3,4-methylenedioxyphenyl)]ethoxycarbonyl-3-sulfonamide | .23† | 6.2† |
| N-(4-chloro-3-methyl-5-isoxazolyl)-2-{[4-(3,4-methylenedioxybenzyl)piperazin-1-yl]carbonyl}thiophene-3-sulfonamide | 20† | >~100† |
| N-(4-chloro-3-methyl-5-isoxazolyl)-2-aminothiophene-3-sulfonamide | 14† | 6.2† |
| N-(4-bromo-3-methyl-5-isoxazolyl)-5-(benzyloxymethyl)thiophene-2-sulfonamide | 12† | 9.0† |
| N-(4-chloro-3-methyl-5-isoxazolyl)-2-{1-cyano-1-[(3,4-methylenedioxy)phenyl]acetyl}thiophene-3-sulfonamide | 2.1† | 27† |
| N-(4-bromo-3-methyl-5-isoxazolyl)-2-[(3,4-methylenedioxy)phenethyl]thiophene-3-sulfonamide | 0.21† | 9.2† |
| N-(4-chloro-3-methyl-5-isoxazolyl)-2-[(3-dimethylamino)phenoxycarbonyl]thiophene-3-sulfonamide | 1.4† | 60† |
| N-(4-bromo-3-methyl-5-isoxazolyl)-1-methylindole-2-sulfonamide | 77† | ~100† |
| N-(4-chloro-3-methyl-5-isoxozolyl-2-(cyclohexyloxycarbonyl)thiophene-3-sulfonamide | 0.44† | 34† |
| N-(4-chloro-3-methyl-5-isoxazolyl)-2-[β-hydroxy(3,4-methylenedioxy)phenylethyl]thiophene-3-sulfonamide | 0.053† | 16† |
| N-(4-bromo-3-methyl-5-isoxazolyl)-2-carboxyl-1-methylidole-3-sulfonamide | 0.59† | 104† |

| COMPOUND | $ET_A$ ($\mu$M)* | $ET_B$ ($\mu$M)* |
|---|---|---|
| N-(4-chloro-3-methyl-5-isoxazolyl)-2-[(4-oxacyclohexyl)oxycarbonyl]thiophene-3-sulfonamide | 1.37† | — |
| N-2-[3,4-(methylenedioxy)phenylacetyl]thiophene-3-sulfonamide | 1.8† | 32.5† |
| N-(4-chloro-3-methyl-5-isoxazolyl)-{2-[3,4-(methylenedioxy)phenyl]acetyl}thiophene-3-sulfonamide oxime | — | — |
| N-(4-chloro-3-methyl-5-isoxazolyl)-2-[(4-tolyl)aminocarbonyl]-1-methylindole-3-sulfonamide | 31.3† | 14.7† |
| N-(4-chloro-3-methyl-5-isoxazolyl)-2-[(4-methoxyphenoxy)carbonyl]thiophene-3-sulfonamide | 0.023† | 15† |
| N-(4-bromo-3-methyl-5-isoxazolyl)-1-[3,4-(methylenedioxy)benzyl]indole-2-sulfonamide | 5.29† | 18.6† |
| N-(4-bromo-3-methyl-5-isoxazolyl)-2-[(4-methylphenoxy)carbonyl]thiophene-3-sulfonamide | 122† | 9.7† |
| N-(4-chloro-3-methyl-5-isoxazolyl)-2-[(4-methoxy-phenyl)acetyl]thiophene-3-sulfonamide | 0.043† | 10.1† |
| N-(4-bromo-3-methyl-5-isoxazolyl)-3-[(4-methylphenoxy)methyl]thiophene-2-sulfonamide | 1.64† | 22.8† |
| N-(4-bromo-3-methyl-5-isoxazolyl)-2-[(4-methylphenoxy)methyl]thiophene-3-sulfonamide | 1.2† | 15† |
| N-(4-bromo-3-methyl-5-isoxazolyl)-3-(4-methyl-*trans*-styryl)thiophene-2-sulfonamide | 0.94† | 0.66† |
| N-(4-bromo-3-methyl-5-isoxazolyl)-3-(4-methyl-phenethyl)thiophene-2-sulfonamide | 0.347† | 9.4† |
| N-(4-bromo-3-methyl-5-isoxazolyl)-2-[(4-methyl-phenyl)acetyl]thiophene-3-sulfonamide | 0.198† | 9.13† |
| N-(4-bromo-3-methyl-5-isoxazolyl)-2-[(3-methoxyphenyl)acetyl]thiophene-3-sulfonamide | 0.030† | 19.1† |
| N-(4-bromo-3-methyl-5-isoxazolyl)-3-(4-methylphenethyl)-5-(4-tolyl)thiophene-2-sulfonamide | 6.1† | 2.09† |
| N-(4-bromo-3-methyl-5-isoxazolyl)-3-(4-methylbenzyl)-5-(4-tolyl)thiophene-2-sulfonamide | 4.69† | 1.56† |
| N-(4-bromo-3-methyl-5-isoxazolyl)-3-(4-methyl-*trans*-styryl)-5-(4-tolyl)thiophene-2-sulfonamide | 6.9† | 1.58† |

| COMPOUND | $ET_A$ (μM)* | $ET_B$ (μM)* |
|---|---|---|
| N-(4-chloro-3-methyl-5-isoxazolyl)-2-[β,β-(ethylenedioxy)-3,4-(methylenedioxy)phenethyl]thiophene-3-sulfonamide | 0.128† | 2.09† |
| N-(4-chloro-3-methyl-5-isoxazolyl)-2-[β-(dimethylamino)-3,4-(methylenedioxy)phenethy]thiophene-3-sulfonamide | 20.9† | ~100† |
| N-(4-chloro-3-methyl-5-isoxazolyl)-2-{α-hydroxy-[3,4-(methylenedioxy)phenyl]acetyl}thiophene-3-sulfonamide | 2.5† | 30† |
| N-(4-chloro-3-methyl-5-isoxazolyl)-2-[(5-methyl-3-isoxazolyl)aminocarbonyl]thiophene-3-sulfonamide | 0.056† | 92† |
| N-(4-bromo-3-methyl-5-isoxazolyl)-2-[(3-hydroxyl-6-pyridazinyl)aminocarbonyl]thiophene-3-sulfonamide | 0.066† | 81.3† |
| N-(4-chloro-3-methyl-5-isoxazolyl)2-{[2-acetyl-4,5-(methylenedioxy)phenyl]aminocarbonyl}thiophene-3-sulfonamide | 0.010† | 31.6† |
| N-(4-bromo-3-methyl-5-isoxazolyl)-3-{[3,4-(methylenedioxy)phenoxy]methyl}thiophene-2-sulfonamide | 0.513† | 9.6† |
| N-(4-bromo-3-methyl-5-isoxazolyl)-2-[(4-methyl)(cinnamyl)] thiophene-3-sulfonamide | 0.26† | 0.413† |
| N-(4-chloro-3-methyl-5-isoxazolyl)-2-[(4,5-dimethoxy-2-methoxycarbonylphenyl)aminocarbonyl]thiophene-3-sulfonamide | 0.55† | -- |
| N-(4-chloro-3-methyl-5-isoxazolyl)-2-[(2-methyl-1,3,4-thiadiazol-5-yl)aminocarbonyl]thiophene-3-sulfonamide | 0.13† | -- |
| N-(4-chloro-3-methyl-5-isoxazolyl)2-{[4,5-dimethoxy-2,4,5-dimethoxy-2-methoxycarbonyl)phenyl]phenylaminocarbonyl}thiophene-3-sulfonamide | 3.80† | -- |
| N-(4-chloro-3-methyl-5-isoxazolyl)2-{[2-carboxyl-4,5-(methylenedioxy)phenyl]aminocarbonyl}thiophene-3-sulfonamide | 1.43† | — |
| N-(4-bromo-3-methyl-5-isoxazolyl)-3-[3,4-(methylenedioxy)phenethyl]thiophene-2-sulfonamide | 0.236† | 18† |
| N-(4-bromo-3-methyl-5-isoxazolyl)-3-[3,4-(methylenedioxy)-*trans*-styryl]thiophene-2-sulfonamide | 0.218† | 10† |

| COMPOUND | $ET_A$ ($\mu M$)* | $ET_B$ ($\mu M$)* | |
|---|---|---|---|
| N-(4-bromo-3-methyl-5-isoxazolyl)-2-[(4-methyl)-phenethyl]thiophene-3-sulfonamide | $0.106^{\dagger}$ | $40.1^{\dagger}$ | |
| N-(3,4-dimethyl-5-isoxazolyl)-2-{[2-acetyl-4,5-(methylenedioxy)phenyl]aminocarbonyl}thiophene-3-sulfonamide | $0.032^{\dagger}$ | -- | |
| N-(4-chloro-3-methyl-5-isoxazolyl)-2-[4-methoxy-2-methylphenyl)aminocarbonyl]thiophene-3-sulfonamide | $0.027^{\dagger}$ | $0.14^{\dagger}$ | |
| N-(4-chloro-3-methyl-5-isoxazolyl)-2-[(2-cyano-4,5-dimethoxyphenyl)aminocarbonyl]thiophene-3-sulfonamide | $0.0039^{\dagger}$ | $12.2^{\dagger}$ | Inv. |
| N-(3,4-dimethyl-5-isoxazolyl)-2-(4-tolylacetylphenyl)-thiophene-3-sulfonamide | $.0027^{\dagger}$ | $29.2^{\dagger}$ | |
| N-(3,4-dimethyl-5-isoxazolyl)-2-[3,4-(methylenedioxy)phenylacetyl]thiophene-3-sulfonamide | $0.0273^{\dagger}$ | $12.2^{\dagger}$ | |
| N-(4-chloro-3-methyl-5-isoxazolyl)-2-[(2,4-dimethoxyphenyl)aminocarbonyl]thiophene-3-sulfonamide | $0.158^{\dagger}$ | $63.1^{\dagger}$ | Inv. |
| N-(4-chloro-3-methyl-5-isoxazolyl)-2-[(3-methyl-6-pyridyl)aminocarbonyl]thiophene-3-sulfonamide | $0.023^{\dagger}$ | $43.7^{\dagger}$ | |
| N-(4-chloro-3-methyl-5-isoxazolyl)-2-[2-hydroxy-4-methylphenyl)aminocarbonyl]thiophene-3-sulfonamide | $.006^{\dagger}$ | -- | Inv. |
| N-(4-chloro-3-methyl-5-isoxazolyl)-2-{[2-cyano-4,5-(methylenedioxy)phenyl]aminocarbonyl}thiophene-3-sulfonamide | $0.0034^{\dagger}$ | $40.4^{\dagger}$ | Inv. |
| N-(4-chloro-3-methyl-5-isoxazolyl)-2-[2-methyl-4,5-(methylenedioxy)phenylaminocarbonyl]thiophene-3-sulfonamide | $0.0030^{\dagger}$ | $355^{\dagger}$ | Inv. |
| N-(4-chloro-3-methyl-5-isoxazolyl)-2-(2-carboxamido-4,5-dimethoxyphenylaminocarbonyl)thiophene-3-sulfonamide | $0.011^{\dagger}$ | $61^{\dagger}$ | Inv. |
| N-(3,4-dimethyl-5-isoxazolyl)-2-(2,4-dimethylphenylacetyl)thiophene-3-sulfonamide | $0.0027^{\dagger}$ | $17.4^{\dagger}$ | Inv. |
| N-(4-chloro-3-methyl-5-isoxazolyl)-2-(2,4-dimethylphenylacetyl)thiophene-3-sulfonamide | $0.0004^{\dagger}$ | $4.8^{\dagger}$ | Inv. |
| N-(4-bromo-3-methyl-5-isoxazolyl)-2-(2,4-dimethylphenylacetyl)thiophene-3-sulfonamide | $0.0008^{\dagger **}$ | $3.6^{\dagger}$ | Inv. |

| COMPOUND | $ET_A$ ($\mu$M)* | $ET_B$ ($\mu$M)* | |
|---|---|---|---|
| N-(4-chloro-3-methyl-5-isoxazolyl)-2-[3,4-(methylenedioxy)]phenylaminocarbonyl-3-thiophenesulfonamide | $0.0073^{\dagger}$ | $9.2^{\dagger}$ | Inv. |
| N-(4-chloro-3-methyl-5-isoxazolyl)-2-[2-methyl-4,5-(methylenedioxy)phenylacetyl]thiophene-3-sulfonamide | $0.0032^{\dagger}$ | $9^{\dagger}$ | Inv. |
| N-(4-chloro-3-methyl-5-isoxazolyl)-2-[3,4-(methylenedioxy)-6-(2-acetoxyethyl)phenylamino-carbonyl]thiophene-3-sulfonamide | $0.0045^{\dagger}$ | $25.7^{\dagger}$ | |
| N-(4-chloro-3-methyl-5-isoxazolyl)-2-[3,4-(methylenedioxy)-6-(2-hydroxyethyl)phenyl-aminocarbonyl]thiophene-3-sulfonamide | $0.0056^{\dagger}$ | $16.8^{\dagger}$ | |
| N-(4-chloro-3-methyl-5-isoxazolyl)-2-(3,5-dimethyl-phenylacetyl)thiophene-3-sulfonamide | $0.045^{\dagger}$ | $17.7^{\dagger}$ | |
| N-(4-chloro-3-methyl-5-isoxazolyl)-2-(2,5-dimethylphenylacetyl)thiophene-3-sulfonamide | $0.007^{\dagger}$ | $18^{\dagger}$ | Inv. |
| N-(4-chloro-3-methyl-5-isoxazolyl)-2-[2-methanesulfonylaminomethyl)-4,5-(methylenedioxy)phenylaminocarbonyl]thiophene-3-sulfonamide | $0.0068^{\dagger}$ | $19.8^{\dagger}$ | Inv. |
| N-(4-chloro-3-methyl-5-isoxazolyl)-2-[2-cyanomethyl-4,5-(methylenedioxy)-6-cyanomethyl]-phenylaminocarbonyl-3-thiophenesulfonamide | $0.0038^{\dagger}$ | $25^{\dagger}$ | Inv. |
| N-(4-chloro-3-methyl-5-isoxazolyl)-2-[2-hyroxyproyl-4,5-(methylenedioxy)phenylaminocarbonyl]-thiophene-3-sulfonamide | $0.0073^{\dagger}$ | $8.3^{\dagger}$ | Inv. |
| N-(4-bromo-3-methyl-5-isoxazolyl)-3-[2-methyl-4,5-(methylenedioxy)cinnamyl]thiophene-2-sulfonamide | $\sim 0.1^{\dagger**}$ | $\sim 6^{\dagger**}$ | |
| N-(4-bromo-3-methyl-5-isoxazolyl)-3-[2-methyl-4,5-(methylenedioxy)phenethyl]thiophene-2-sulfonamide | $\sim 0.1^{\dagger**}$ | $\sim 5^{\dagger**}$ | |
| N-(4-bromo-3-methyl-5-isoxazolyl)-3-{[2-propyl-4,5-(methylenedioxy)phenoxy]methyl}thiophene-2-sulfonamide | $\sim 0.2^{\dagger**}$ | $\sim 1.5^{\dagger**}$ | |
| N-(4-chloro-3-methyl-5-isoxazolyl)-2-[3,4-(methylenedioxy)-6-(2-acetoxyethoxy)]phenylamino-carbonyl]thiophene-3-sulfonamide | $\sim 0.02^{\dagger**}$ | $\sim 18^{\dagger}$ | |
| N-(4-chloro-3-methyl-5-isoxazolyl)-2-[3,4-(methylenedioxy)-6-(2-hydroxyethoxy)phenyl-aminocarbonyl]thiophene-3-sulfonamide | $\sim 0.01^{\dagger**}$ | $\sim 18^{\dagger}$ | |

| COMPOUND | $ET_A$ ($\mu$M)* | $ET_B$ ($\mu$M)* | |
|---|---|---|---|
| N-(4-chloro-3-methyl-5-isoxazolyl)-2-[2-cyano-4,5-(methylenedioxy)phenylacetyl]thiophene-3-sulfonamide | ~0.3$^{\dagger}$** | ~0.7$^{\dagger}$ | |
| N-(4-chloro-3-methyl-5-isoxazolyl)-2-{2-[(dimethylamino)carbonylmethyl]-4,5-(methylenedioxy)phenylaminocarbonyl}thiophene-3-sulfonamide | 0.009$^{\dagger}$ | 13.8$^{\dagger}$ | Inv. |
| N-(4-chloro-3-methyl-5-isoxazolyl)-2-[2-methyl-4,5-(methylenedioxy)phenylhydroxyimino]thiophene-3-sulfonamide | 0.794$^{\dagger}$ | 6.49$^{\dagger}$ | |
| N-(4-bromo-3-methyl-5-isoxazolyl)-2-[2-methyl-4,5-(methylenedioxy)phenethyl]thiophene-3-sulfonamide | 0.0619$^{\dagger}$ | 8.90$^{\dagger}$ | |
| N-(4-bromo-3-methyl-5-isoxazolyl)-3-[2-(hydroxymethyl)-4,5-(methylenedioxy)cinnamyl]thiophene-2-sulfonamide | 0.0795$^{\dagger}$ | 3.24$^{\dagger}$ | |
| N-(4-bromo-3-methyl-5-isoxazolyl)-3-{2-[(tetrahydro-4H-pyran-2-ylxoy)methyl]-4,5-(methylenedioxy)cinnamyl}thiophene-2-sulfonamide | 0.0967$^{\dagger}$ | 4.14 | |
| N-(4-bromo-3-methyl-5-isoxazolyl)-3-(2,4-dimethylphenethyl)thiophene-2-sulfonamide | 0.1006$^{\dagger}$ | 4.30$^{\dagger}$ | Inv. |
| N-(4-bromo-3-methyl-5-isoxazolyl)-3-(2,4-dimethylcinnamyl)thiophene-2-sulfonamide | 0.180$^{\dagger}$ | 2.97$^{\dagger}$ | |
| N-(4-bromo-3-methyl-5-isoxazolyl)-2-(2,4-dimethylcinnamyl)thiophene-3-sulfonamide | 0.166$^{\dagger}$ | 2.97$^{\dagger}$ | |
| N-(4-bromo-3-methyl-5-isoxazolyl)-3-[(2,4-dimethylphenoxy)methyl]thiophene-2-sulfonamide | 0.346$^{\dagger}$ | 7.45$^{\dagger}$ | |
| N-(4-bromo-3-methyl-5-isoxazolyl)-2-[(2,4-dimethylphenoxy)methyl]thiophene-3-sulfonamide | 0.308$^{\dagger}$ | 4.48$^{\dagger}$ | Inv. |
| N-(4-chloro-3-methyl-5-isoxazolyl)-5-(phenylaminocarbonyl)thiophene-2-sulfonamide | 28.1$^{\dagger}$ | 60.6$^{\dagger}$ | |
| N-(4-chloro-3-methyl-5-isoxazolyl)-2-[$\beta$-acetoxy-2-methyl-4,5-(methylenedioxy)styryl]thiophene-3-sulfonamide | 0.00544 | 3.74$^{\dagger}$ | Inv. |
| N-(4-chloro-3-methyl-5-isoxazolyl)-2-[(2,3,4-trimethoxy-6-cyano)phenylaminocarbonyl]thiophene-3-sulfonamide | 0.000169$^{\dagger}$ | 12.5$^{\dagger}$ | Inv. |
| N-(4-chloro-3-methyl-5-isoxazolyl)-2-[2-(cyano)phenyl]benzo[b]thiophene-3-sulfonamide | 6.33$^{\dagger}$ | 8.82$^{\dagger}$ | |

| COMPOUND | $ET_A$ ($\mu$M)* | $ET_B$ ($\mu$M)* | |
|---|---|---|---|
| N-(4-chloro-3-methyl-5-isoxazolyl)-2-[3,4-(methylenedioxy)phenyl]benzo[b]thiophene-3-sulfonamide | 0.550† | 52.6† | |
| N-(4-bromo-3-methyl-5-isoxazolyl)-3-(2-tolyl)thiophene-2-sulfonamide | 0.324† | 55.1† | |
| N-(4-bromo-3-methyl-5-isoxazolyl)-3-(3-tolyl)thiophene-2-sulfonamide | 0.832† | 21.2† | |
| N-(4-bromo-3-methyl-5-isoxazolyl)-3-(2-tolyl)thiophene-2-sulfonamide | 0.302† | 31%@100† | |
| N-(4-bromo-3-methyl-5-isoxazolyl)-3-(3-methoxyphenyl)thiophene-2-sulfonamide | 0.334† | ** | |
| N-(4-bromo-3-methyl-5-isoxazolyl)-3-(3-methoxyphenyl)thiophene-2-sulfonamide | 1.32† | 56.3† | |
| N-(4-bromo-3-methyl-5-isoxazolyl)-3-(2-methoxyphenyl)thiophene-2-sulfonamide | 1.71† | 59.1† | |
| N-(4-bromo-3-methyl-5-isoxazolyl)-3-(4-ethylphenyl)thiophene-2-sulfonamide | 0.184 | 43.9† | |
| N-(4-bromo-3-methyl-5-isoxazolyl)-3-(4-propylphenyl)thiophene-2-sulfonamide | 0.0873 | 8.48† | |
| N-(4-bromo-3-methyl-5-isoxazolyl)-3-(4-*iso*-propylphenyl)thiophene-2-sulfonamide | 0.218 | 28.3† | |
| N-(4-bromo-3-methyl-5-isoxazolyl)-3-(4-butylphenyl)thiophene-2-sulfonamide | 0.160 | 6.11† | |
| N-(3,4-dimethyl-5-isoxazolyl)-2-[2-methyl-4,5-(methylenedioxy)phenylacetyl]thiophene-3-sulfonamide | 0.00328† | 34.3† | Inv. |
| N-(4-chloro-3-methyl-5-isoxazolyl)-2-(2,4,6-trimethylphenylaminocarbonyl)thiophene-3-sulfonamide | 0.000626† | 8.27† | Inv. |
| N-(4-chloro-3-methyl-5-isoxazolyl)-2-(2,4,6-trimethylphenylacetyl)thiophene-3-sulfonamide | 0.000238† | 3.82† | Inv. |
| N-(4-chloro-5-methyl-3-isoxazolyl)-2-[2-methyl-4,5-(methylenedioxy)phenylacetyl]thiophene-3-sulfonamide | 0.000625† | 3.69† | Inv. |
| N-(4-bromo-3-methyl-5-isoxazolyl)-2-[2-methyl-4,5-(methylenedioxy)cinnamyl]thiophene-3-sulfonamide | 0.0804† | 3.28† | Inv. |
| N-(4-bromo-3-methyl-5-isoxazolyl)-2-(2,4-dimethylphenethyl)thiophene-3-sulfonamide | 0.0555† | 3.48† | Inv. |

| COMPOUND | $ET_A$ (μM)[*] | $ET_B$ (μM)[*] | |
|---|---|---|---|
| N-(4-chloro-3-methyl-5-isoxazolyl)-2-[(4-methoxycarbonyl-2,6-dimethyl)-phenylaminocarbonyl]thiophene-3-sulfonamide | 0.000266[†] | 9.78[†] | Inv. |
| N-(4-chloro-3-methyl-5-isoxazolyl)-2-(phenoxycarbonyl)thiophene-3-sulfonamide | 4.41[†] | 31%@100[†] | |
| N-(4-bromo-3-methyl-5-isoxazolyl)-2-(phenoxycarbonyl)thiophene-3-sulfonamide | 2.71[†] | 20%@100[†] | |
| N-(3,4-dimethyl-5-isoxazolyl)-2-{[3,4-(methylenedioxy)phenoxy]carbonyl}thiophene-3-sulfonamide | 3.61[†] | 30%@100[†] | |
| N-(4-bromo-3-methyl-5-isoxazolyl)-2-[(2-methylphenoxy)carbonyl]thiophene-3-sulfonamide | 0.684[†] | 105[†] | |
| N-(4-bromo-3-methyl-5-isoxazolyl)-2-[(3-methylphenoxy)carbonyl]thiophene-3-sulfonamide | 1.20[†] | 111[†] | |
| N-(4-bromo-3-methyl-5-isoxazolyl)-2-[(2,4-dimethylphenoxy)carbonyl]thiophene-3-sulfonamide | 0.291[†] | 43.2[†] | Inv. |
| N-(4-bromo-3-methyl-5-isoxazolyl)-2-[(2-methoxylphenoxy)carbonyl]thiophene-3-sulfonamide | 0.761[†] | 29%@100[†] | |
| N-(4-bromo-3-methyl-5-isoxazolyl)-2-[(3-methoxylphenoxy)carbonyl]thiophene-3-sulfonamide | 0.78[†] | 90[†] | |
| N-(4-bromo-3-methyl-5-isoxazolyl)-2-[(4-methoxylphenoxy)carbonyl]thiophene-3-sulfonamide | 1.73[†] | 111[†] | |
| N-(3,4-dimethyl-5-isoxazolyl)-2-[(4-methoxylphenoxy)carbonyl]thiophene-3-sulfonamide | [†]0.324 | 68.5[†] | |
| N-(3,4-dimethyl-5-isoxazolyl)-2-[(4-methylphenoxy)carbonyl]thiophene-3-sulfonamide | 0.324[†] | 68.5[†] | |
| N-(4-chloro-3-methyl-5-isoxazolyl)-2-[(4-methylphenoxy)carbonyl]thiophene-3-sulfonamide | 2.52[†] | 19%@100[†] | |
| N-(4-chloro-3-methyl-5-isoxazolyl)-2-[(2,4-dimethylphenoxy)carbonyl]thiophene-3-sulfonamide | 3.22[†] | 43%@100[†] | Inv. |
| N-(3,4-dimethyl-5-isoxazolyl)-2-[(2,4-dimethylphenoxy)carbonyl]thiophene-3-sulfonamide | 0.648[†] | 68.5[†] | Inv. |
| N-(4-bromo-3-methyl-5-isoxazolyl)-2-{[2-propyl-4,5-(methylenedioxy)phenoxy]carbonyl}thiophene-3-sulfonamide | 0.274[†] | 21%@100[†] | Inv. |
| N-(4-chloro-3-methyl-5-isoxazolyl)-2-(3-methoxycarbonyl-2,4,6-trimethylphenylamino-carbonyl)thiophene-3-sulfonamide | 0.138[†] | 11.9[†] | Inv. |

| COMPOUND | $ET_A$ ($\mu M$)* | $ET_B$ ($\mu M$)* | |
|---|---|---|---|
| N-(4-bromo-3-methyl-5-isoxazolyl)-3-(2,4-dimethylphenyl)thiophene-2-sulfonamide | 0.1002† | 60.3† | |
| N-(3,4-dimethyl-5-isoxazolyl)-2-(phenoxycarbonyl)thiophene-3-sulfonamide | 2.85† | 31%† | |
| N-(4-bromo-3-methyl-5-isoxazolyl)-3-(4-*iso*-butylphenyl)thiophene-2-sulfonamide | 0.0823† | 2.76† | |
| N-(4-bromo-3-methyl-5-isoxazolyl)-3-(4-*iso*-pentylphenyl)thiophene-2-sulfonamide | 0.155† | 3.31† | |
| N-(4-bromo-3-methyl-5-isoxazolyl)-3-[(2,4,6-trimethylphenoxy)methyl]thiophene-2-sulfonamide | 0.0457† | 4.68† | |
| N-(4-bromo-3-methyl-5-isoxazolyl)-2-[(2,4,6-trimethylphenoxy)methyl]thiophene-3-sulfonamide | 0.0562† | 3.39† | Inv. |
| N-(4-bromo-3-methyl-5-isoxazolyl)-3-(2,4,6-trimethylcinnamyl)thiophene-2-sulfonamide | 0.0490† | 1.86† | |
| N-(4-bromo-3-methyl-5-isoxazolyl)-3-(2-methyl-4-propylphenyl)thiophene-2-sulfonamide | 0.0468† | 3.63† | |
| N-(4-bromo-3-methyl-5-isoxazolyl)-3-(4-*iso*-butyl-2-methylphenyl)thiophene-2-sulfonamide | 0.0468† | 1.66† | |
| N-(4-bromo-3-methyl-5-isoxazolyl)-3-(4-*iso*-pentyl-2-methylphenyl)thiophene-2-sulfonamide | 0.107† | 2.40† | |
| N-(4-bromo-3-methyl-5-isoxazolyl)-2-{[3,4-(methylenedioxy)phenoxy]methyl}thiophene-3-sulfonamide | 0.302† | 6.61† | |
| N-(4-bromo-3-methyl-5-isoxazolyl)-2-{[4,5-(methylenedioxy)-2-propylphenoxy]methyl}thiophene-3-sulfonamide | 0.107† | 0.407† | Inv. |
| N-(4-bromo-3-methyl-5-isoxazolyl)-2-(2,4,6-trimethylphenethyl)thiophene-3-sulfonamide | 0.0417† | 1.23† | |
| N-(4-bromo-3-methyl-5-isoxazolyl)-3-(2,4,6-trimethylphenethyl)thiophene-2-sulfonamide | 0.055† | 1.62† | |
| N-(3,4-dimethyl-5-isoxazolyl)-2-[(2,4,6-trimethylphenoxy)carbonyl]thiophene-3-sulfonamide | 0.537† | 8%@100† | Inv. |
| N-(4-chloro-3-methyl-5-isoxazolyl)-2-[(2,4,6-trimethylphenoxy)carbonyl]thiophene-3-sulfonamide | 0.0776† | 30.2† | Inv. |
| N-(4-bromo-3-methyl-5-isoxazolyl)-2-[(2,4,6-trimethylphenoxy)carbonyl]thiophene-3-sulfonamide | 0.479† | 24.5† | Inv. |

* results are generally the average of 2 to 5 experiments
** preliminary results or results in which one or more data points were only determined approximately
† assay performed with incubation at 24° C. As described in the Examples, incubation at the higher temperature reduces the activity by a factor of 2- to about 10-compared to the activity at 4° C
– data not available or measured as % inhibition @ 100 μM
% = % inhibition @ 100 μM

It is understood that 4-bromo or 4-chloro groups can be replaced by other 4-halo substituents or other suitable substituents for $R^1$, such as alkyl.

**B. Preparation of the compounds**

The preparation of some of the above and other compounds that possess the requisite acitivities are set forth in the Examples. Compounds whose synthesis is not explicitly exemplified can be synthesized by routine modification of one or more methods described in detail in the Examples by substituting appropriate readily available reagents.

The preparation of the above compounds are described in detail in the examples. Any such compound or similar compound may be synthesized according to a method discussed in general below and set forth in the Examples by selecting appropriate starting materials as exemplified.

In general, most of the syntheses involve the condensation of a sulfonyl chloride with an aminoisoxazole in dry pryidine or in tetrahydrofuran (THF) and sodium hydride. The sulfonyl chorides and aminoisoxazoles either can be obtained commercially or synthesized according to methods described in the Examples or using other methods available to those of skill in this art (see, e.g., U.S. Patent Nos. 4,659,369, 4,861,366 and 4,753,672).

The N-(alkylisoxazolyl)sulfonamides can be prepared by condensing an aminoisoxazole with a sulfonyl chloride in dry pyridine with or without the catalyst 4-(dimethylamino)pyridine. The N-(3,4-dimethyl-5-isoxazolyl)sulfonamides and N-(4,5-dimethyl-5-isoxazolyl)sulfonamides can be prepared from the corresponding aminodimethylisoxazole, such as 5-amino-3,4-dimethylisoxazole. For example, N-(3,4-dimethyl-5-isoxazolyl)-2-(carbomethoxy)thiophene-3-sulfonamide was prepared from 2-methoxycarbonylthiophene-3-sulfonyl chloride and 5-amino-3,4-dimethylisoxazole in dry pyridine.

The N-(4-haloisoxazolyl)sulfonamides can be prepared by condensation of amino-4-haloisoxazole with a sulfonyl chloride in THF with sodium hydride as a base. For example, N-(4-bromo-3-methyl-5-isoxazolyl)thiophene-2-sulfonamide was prepared from 5-amino-4-bromo-3-methylisoxazole and thiophene-2-sulfonyl chloride in THF and sodium hydride. N-(4-bromo-3-methyl-5-isoxazolyl)-5-(3-isoxazolyl)thiophene-2-sulfonamide was prepared from 5-amino-4-bromo-3-methylisoxazole and 5-(3-isoxazolyl)thiophene-2-sulphonyl chloride.

Alternatively, compounds in which $Ar^2$ is thienyl herein, may be prepared by reacting an appropriate sulfonyl chloride with a 5-aminoisoxazole substituted at the 3 and 4 positions, such as 5-amino-4-bromo-3-methylisoxazole, in tetrahydrofuran (THF) solution containing a base, such as sodium hydride. Following the reaction, the THF is removed under reduced pressure, the residue dissolved in water, acidified and extracted with methylene chloride. The organic layer is washed and then dried over anhydrous magnesium sulfate, the solvents are evaporated and the residue is purified by recrystallization using hexanes/ethylacetate to yield pure product.

These sulfonamides also can be prepared from the corresponding sulfonyl chloride and the aminoisoxazole in pyridine with or without a catalytic amount of 4-dimethylaminopyridine (DMAP). In some cases, the bis-sulfonyl compound is obtained as the major or exclusive product. The bis-sulfonated products can be readily hydrolyzed to the sulfonamide using aqueous sodium hydroxide and a suitable co-solvent, such as methanol or tetrahydrofuran, generally at room temperature. For example:

(a) N-(4-bromo-3-methyl-5-isoxazolyl)-2-(N-phenylaminocarbonyl)thiophene-3-sulfonamide was prepared from N-(4-bromo-3-methyl-5-isoxazolyl)-2-carboxylthiophene-3-sulfonamide and aniline and 1-ethyl-3'-[3-dimethylaminopropyl]carbodiimide (EDCI). N-(4-bromo-3-methyl-5-isoxazolyl)-2-[(4-methoxyphenyl)aminocarbonyl]thiophene-3-sulfonamide was prepared from 4-methoxyaniline, N,N'-diisopropylethylamine and N-(4-bromo-3-methyl-5-isoxazolyl)-2-carboxylthiophene-3-sulfonamide. N-(4-bromo-3-methyl-5-isoxazolyl)-2-(benzylaminocarbonyl)-thiophene-3-sulfonamide was prepared from N-(4-bromo-3-methyl-5-isoxazolyl)-2-carboxylthiophene-3-sulfonamide and benzylamine as described above.

N-(4-bromo-3-methyl-5-isoxazolyl)-2-carboxylthiophene-3-sulfonamide was prepared from N-(4-bromo-3-methyl-5-isoxazolyl)-2-(carbomethoxy)thiophene-3-sulfonamide, which was prepared from the condensation of 5-amino-4-bromo-3-methylisoxazole and 2-(carbomethoxy)thiophene-3-sulfonyl chloride.

(b) N-(4-bromo-3-methyl-5-isoxazolyl)-1-(4'-isopropylphenyl)pyrrole-2-sulfonamide and N-(4-bromo-3-methyl-5-isoxazolyl)-1-(4'-isopropylphenyl)pyrrole-3-sulfonamide were prepared from 5-amino-4-bromo-3-methylisoxazole and a mixture of 1-(4'-isopropylphenyl)pyrrole-2-sulfonyl chloride and 1-(4'-isopropylphenyl)pyrrole-3-sulfonyl chloride. These sulfonyl chlorides were prepared from 1-(4'-isopropylphenyl)pyrrole-2-sulfonic acid in phosphorus oxychloride and phosphorus pentachloride. 1-(4'-isopropylphenyl)pyrrole-2-sulfonic acid was prepared from 1-(4'-isopropylphenyl)pyrrole and chtorosulfonic acid. 1-(4'-isopropylphenyl)pyrrole was prepared from 4-isopropylaniline and 2,5-dimethoxytetrahydrofuran.

Prodrugs and other derivatives of the compounds suitable for administration to humans may also be designed and prepared by methods known to those of skill in the art (see, e.g., Nogrady (1985) Medicinal Chemistry A Biochemical Approach, Oxford University Press, New York, pages 388-392).

Compounds described herein have been synthesized and tested for activity in in vitro assays and, in some cases, in in vivo animal models. Nuclear magnetic resonance spectroscopic (NMR), mass spectrometric, infrared spectroscopic and high performance liquid chromatographic analyses indicated that the synthesized compounds have structures consistent with those expected for such compounds and are generally at least about 98% pure. All of the compounds exemplified or described herein exhibited activity as endothelin antagonists.

**C. Evaluation of the bioactivity of the compounds**

Standard physiological, pharmacological and biochemical procedures are available for testing the compounds to identify those that possess any biological activities of an endothelin peptide or the ability to interfere with or inhibit endothelin peptides. Compounds that exhibit in vitro activities, such as the ability to bind to endothelin receptors or to compete with one or more of the endothelin peptides for binding to endothelin receptors can be used in the methods for isolation of endothelin receptors and the methods for distinguishing the specificities of endothelin receptors, and are candidates for use in the methods of treating endothelin-mediated disorders.

Thus, other preferred compounds of formula I, in addition to those specifically identified herein, that are endothelin antagonists or agonists may be identified using such screening assays.

**1. Identifying compounds that modulate the activity of an endothelin peptide**

The compounds are tested for the ability to modulate the activity of endothelin-1. Numerous assays are known to those of skill in the art for evaluating the ability of compounds to modulate the activity of endothelin (see, e.g., U.S. Patent No. 5,114,918 to Ishikawa et al.; EP A1 0 436 189 to BANYU PHARMACEUTICAL CO., LTD. (October 7, 1991); Borges et al. (1989) Eur. J. Pharm. 165: 223-230; Filep et al. (1991) Biochem. Biophys. Res. Commun. 177: 171-176). In vitro studies may be corroborated with in vivo studies (see, e.g., U.S. Patent No. 5,114,918 to Ishikawa et al.; EP A1 0 436 189 to BANYU PHARMACEUTICAL CO., LTD. (October 7, 1991)) and pharmaceutical activity thereby evaluated. Such assays are described in the Examples herein and include the ability to compete for binding to $ET_A$ and $ET_B$ receptors present on membranes isolated from cell lines that have been genetically engineered to express either $ET_A$ or $ET_B$ receptors on their cell surfaces.

The properties of a potential antagonist may be assessed as a function of its ability to inhibit an endothelin induced activity in vitro using a particular tissue, such as rat portal vein and aorta as well as rat uterus, trachea and vas deferens (see e.g., Borges, R., Von Grafenstein, H. and Knight, D.E., "Tissue selectivity of endothelin," Eur. J. Pharmacol 165:223-230, (1989)). The ability to act as an endothelin antagonist in vivo can be tested in hypertensive rats, ddy mice or other recognized animal models (see, Kaltenbronn et al. (1990) J. Med. Chem. 33:838-845, see, also, U.S. Patent No. 5,114,918 to Ishikawa et al.; and EP A1 0 436 189 to BANYU PHARMACEUTICAL CO., LTD (October 7, 1991); see, also Bolger et al. (1983) J. Pharmacol. Exp. Ther. 225291-309). Using the results of such animal studies, pharmaceutical effectiveness may be evaluated and pharmaceutically effective dosages determined. A potential agonist may also be evaluated using in vitro and in vivo assays known to those of skill in the art.

Endothelin activity can be identified by the ability of a test compound to stimulate constriction of isolated rat thoracic aorta (Borges et al. (1989) "Tissue selectivity of endothelin" Eur. J. Pharmacol. 165: 223-230). To perform the assay, the endothelium is abraded and ring segments mounted under tension in a tissue bath and treated with endothelin in the presence of the test compound. Changes in endothelin induced tension are recorded. Dose response curves may be generated and used to provide information regarding the relative inhibitory potency of the test compound. Other tissues, including heart, skeletal muscle, kidney, uterus, trachea and vas deferens, may be used for evaluating the effects of a particular test compound on tissue contraction.

Endothelin isotype specific antagonists may be identified by the ability of a test compound to interfere with endothelin binding to different tissues or cells expressing different endothelin-receptor subtypes, or to interfere with the biological effects of endothelin or an endothelin isotype (Takayanagi et al. (1991) Reg. Pep. 32: 23-37, Panek et

al. (1992) Biochem. Biophys. Res. Commun. 183: 566-571). For example, $ET_B$ receptors are expressed in vascular endothelial cells, possibly mediating the release of prostacyclin and endothelium-derived relaxing factor (De Nucci et al. (1988) Proc. Natl. Acad. Sci. USA 85:9797). $ET_A$ receptors are not detected in cultured endothelial cells, which express $ET_B$ receptors.

The binding of compounds or inhibition of binding of endothelin to $ET_B$ receptors can be assessed by measuring the inhibition of endothelin-1-mediated release of prostacyclin, as measured by its major stable metabolite, 6-keto $PGF_{1\alpha}$, from cultured bovine aortic endothelial cells (see, e.g., Filep et al. (1991) Biochem. and Biophys Res. Commun. 177: 171-176). Thus, the relative affinity of the compounds for different endothelin receptors may be evaluated by determining the inhibitory dose response curves using tissues that differ in receptor subtype.

Using such assays, the relative affinities of the compounds for $ET_A$ receptors and $ET_B$ receptors have been and can be assessed. Those that possess the desired properties, such as specific inhibition of binding of endothelin-1, are selected. The selected compounds that exhibit desirable activities may be therapeutically useful and are tested for such uses using the above-described assays from which in vivo effectiveness may be evaluated (see, e.g., U.S. Patent No. 5,248,807; U.S. Patent No. 5,240,910; U.S. Patent No. 5,198,548; U.S. Patent No. 5,187,195; U.S. Patent No. 5,082,838; U.S. Patent No. 5,230,999; published Canadian Application Nos. 2,067,288 and 2071193; published Great Britain Application No. 2,259,450; Published International PCT Application No. WO 93/08799; Benigi et al. (1993) Kidney International 44:440-444; and Nirei et al. (1993) Life Sciences 52:1869-1874). Compounds that exhibit in vitro activities that correlate with in vivo effectiveness will then be formulated in suitable pharmaceutical compositions and used as therapeutics.

The compounds also may be used in methods for identifying and isolating endothelin-specific receptors and aiding in the design of compounds that are more potent endothelin antagonists or agonists or that are more specific for a particular endothelin receptor.

### 2. Isolation of endothelin receptors

The compounds of the invention may be used in a method for identifying endothelin receptors. In practicing this method, one or more of the compounds is linked to a support and used in methods of affinity purification of receptors. By selecting compounds with particular specificities, distinct subclasses of ET receptors may be identified.

One or more of the compounds may be linked to an appropriate resin, such as Affi-gel, covalently or by other linkage, by methods known to those of skill in the art for linking endothelin to such resins (see, Schvartz et al. (1990) Endocrinology 126: 3218-3222). The linked compounds can be those that are specific for $ET_A$ or $ET_B$ receptors or other subclass of receptors.

The resin is pre-equilibrated with a suitable buffer generally at a physiological pH (7 to 8). A composition containing solubilized receptors from a selected tissue are mixed with the resin to which the compound is linked and the receptors are selectively eluted. The receptors can be identified by testing them for binding to an endothelin isopeptide or analog or by other methods by which proteins are identified and characterized. Preparation of the receptors, the resin and the elution method may be performed by modification of standard protocols known to those of skill in the art (see, e.g., Schvartz et al. (1990) Endocrinology 126: 3218-3222).

Other methods for distinguishing receptor type based on differential affinity to any of the compounds herein are provided. Any of the assays described herein for measuring the affinity of selected compounds for endothelin receptors may also be used to distinguish receptor subtypes based on affinity for particular compounds provided herein. In particular, an unknown receptor may be identified as an $ET_A$ or $ET_B$ receptor by measuring the binding affinity of the unknown receptor for a compound provided herein that has a known affinity for one receptor over the other. Such preferential interaction is useful for determining the particular disease that may be treated with a compound prepared as described herein. For example, compounds with high affinity for $ET_A$ receptors and little or no affinity for $ET_B$ receptors are candidates for use as hypertensive agents; whereas, compounds that preferentially interact with $ET_B$ receptors are candidates for use as anti-asthma agents.

### D. Formulation and administration of the compositions

Effective concentrations of one or more of the sulfonamide compounds of formula I or pharmaceutically acceptable salts, esters or other derivatives thereof are mixed with a suitable pharmaceutical carrier or vehicle. In instances in which the compounds exhibit insufficient solubility, methods for solubilizing compounds may be used. Such methods are known to those of skill in this art, and include, but are not limited to, using cosolvents, such as dimethylsulfoxide (DMSO), using surfactants, such as tween, or dissolution in aqueous sodium bicarbonate. Derivatives of the compounds, such as salts of the compounds or prodrugs of the compounds may also be used in formulating effective pharmaceutical compositions.

The concentrations or the compounds are effective for delivery of an amount, upon administration, that ameliorates the symptoms of the endothelin-mediated disease. Typically, the compositions are formulated for single dosage administration.

Upon mixing or addition of the sulfonamide compound(s), the resulting mixture may be a solution, suspension, emulsion or the like. The form of the resulting mixture depends upon a number of factors, including the intended mode of administration and the solubility of the compound in the selected carrier or vehicle. The effective concentration is sufficient for ameliorating the symptoms of the disease, disorder or condition treated and may be empirically determined.

Pharmaceutical carriers or vehicles suitable for administration of the compounds provided herein include any such carriers known to those skilled in the art to be suitable for the particular mode of administration. In addition, the compounds may be formulated as the sole pharmaceutically active ingredient in the composition or may be combined with other active ingredients.

The active compounds can be administered by any appropriate route, for example, orally, parenterally, intravenously, intradermally, subcutaneously, or topically, in liquid, semi-liquid or solid form and are formulated in a manner suitable for each route of administration. Preferred modes of administration include oral and parenteral modes of administration.

The active compound is included in the pharmaceutically acceptable carrier in an amount sufficient to exert a therapeutically useful effect in the absence of undesirable side effects on the patient treated. The therapeutically effective concentration may be determined empirically by testing the compounds in known in vitro and in vivo systems (see, e.g., U.S. Patent No. 5,114,918 to Ishikawa et al.; EP A1 0 436 189 to BANYU PHARMACEUTICAL CO., LTD (October 7, 1991); Borges et al. (1989) Eur. J. Pharm. 165: 223-230;: Filep et al. (1991) Biochem. Biophys. Res. Commun. 177: 171-176) and then extrapolated therefrom for dosages for humans.

The concentration of active compound in the drug composition will depend on absorption, inactivation and excretion rates of the active compound, the dosage schedule, and amount administered as well as other factors known to those of skill in the art. For example, the amount that is delivered is sufficient to treat the symptoms of hypertension. The effective amounts for treating endothelin-mediated disorders are expected to be higher than the amount of the sulfonamide compound that would be administered for treating bacterial infections.

Typically a therapeutically effective dosage should produce a serum concentration of active ingredient of from about 0.1 ng/ml to about 50-100 µg/ml. The pharmaceutical compositions typically should provide a dosage of from about 0.01 mg to about 2000 mg of compound per kilogram of body weight per day. The active ingredient may be administered at once, or may be divided into a number of smaller doses to be administered at intervals of time. It is understood that the precise dosage and duration of treatment is a function of the disease being treated and may be determined empirically using known testing protocols or by extrapolation from in vivo or in vitro test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

If oral administration is desired, the compound should be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

Oral compositions will generally include an inert diluent or an edible carrier and may be compressed into tablets or enclosed in gelatin capsules. For the purpose of oral therapeutic administration, the active compound or compounds can be incorporated with excipients and used in the form of tablets, capsules or troches. Pharmaceutically compatible binding agents and adjuvant materials can be included as part of the composition.

The tablets, pills, capsules, troches and the like can contain any of the following ingredients, or compounds of a similar nature: a binder, such as microcrystalline cellulose, gum tragacanth and gelatin; an excipient such as starch and lactose, a disintegrating agent such as, but not limited to, alginic acid and corn starch; a lubricant such as, but not limited to, magnesium stearate; a glidant, such as, but not limited to, colloidal silicon dioxide; a sweetening agent such as sucrose or saccharin; and a flavoring agent such as peppermint, methyl salicylate, and fruit flavoring.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The compounds can also be administered as a component of an elixir, suspension, syrup, wafer, chewing gum or the like. A syrup may contain, in addition to the active compounds, sucrose as a sweetening agent and certain preservatives, dyes and colorings and flavors.

The active materials can also be mixed with other active materials which do not impair the desired action, or with materials that supplement the desired action, such as antacids, H2 blockers, and diuretics. For example, if the compound is used for treating asthma or hypertension, it may be used with other bronchodilators and antihypertensive agents, respectively.

Solutions or suspensions used for parenteral, intradermal, subcutaneous, or topical application can include any of the following components: a sterile diluent, such as water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose. Parenteral preparations can be enclosed in ampules, disposable syringes or multiple dose vials made of glass, plastic or other suitable material.

If administered intravenously, suitable carriers include physiological saline or phosphate buffered saline (PBS), and solutions containing thickening and solubilizing agents, such as glucose, polyethylene glycol, and polypropylene glycol and mixtures thereof. Liposomal suspensions, including tissue-targeted liposomes, may also be suitable as pharmaceutically acceptable carriers. These may be prepared according to methods known to those skilled in the art. For example, liposome formulations may be prepared as described in U.S. Patent No. 4,522,811.

The active compounds may be prepared with carriers that protect the compound against rapid elimination from the body, such as time release formulations or coatings. Such carriers include controlled release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparation of such formulations are known to those skilled in the art.

The compounds may be formulated for local or topical application, such as for topical application to the skin and mucous membranes, such as in the eye, in the form of gels, creams, and lotions and for application to the eye or for intracisternal or intraspinal application. Such solutions, particularly those intended for ophthalmic use, may be formulated as 0.01% - 10% isotonic solutions, pH about 5-7, with appropriate salts. The compounds may be formulated as aeorsols for topical application, such as by inhalation (see, e.g., U.S. Patent Nos. 4,044,126, 4,414,209, and 4,364,923, which describe aerosols for delivery of a steroid useful for treatment inflammatory diseases, particularly asthma).

Finally, the compounds may be packaged as articles of manufacture containing packaging material, a compound provided herein, which is effective for antagonizing the effects of endothelin, ameliorating the symptoms of an endothelin-mediated disorder, or inhibiting binding of an endothelin peptide to an ET receptor with an $IC_{50}$ of less than about 10 µM, within the packaging material, and a label that indicates that the compound or salt thereof is used for antagonizing the effects of endothelin, treating endothelin-mediated disorders or inhibiting the binding of an endothelin peptide to an ET receptor.

The following examples are included for illustrative purposes only and are not intended to limit the scope of the invention.

**REFERENCE EXAMPLE 1**

**N-(4-Bromo-3-methyl-5-isoxazolyl)thiophene-2-sulfonamide**

A solution of 5-amino-4-bromo-3-methylisoxazole (177 mg, 1.0 mmol) in dry tetrahydrofuran (THF, 2 ml) was added to a suspension of sodium hydride (60% dispersion in mineral oil, 90 mg, 2.2 mmol) in dry THF (1 ml) at 0 - 5° C. After stirring at 0 - 5° C for 5 min., the reaction was stirred at room temperature for 10 min to complete the reaction. The reaction mixture was re-cooled to 0° C and thiophene-2-sulfonyl chloride (200 mg, 1.1 mmol) dissolved in dry THF (2 ml) was added dropwise. Stirring was continued for 1 h; during this period the reaction mixture slowly attained ambient temperature. THF was removed under reduced pressure. The residue was dissolved in water (10 ml), the pH was adjusted to 10 - 11 by adding 5 N sodium hydroxide solution, and was extracted with ethyl acetate (3 X 10 ml) to remove the neutral impurities. The aqueous layer was acidified with concentrated HCl (pH 2 - 3) and extracted with methylene chloride (3 X 10 ml). The combined organic layers was dried over anhydrous magnesium sulfate and concentrated under reduced pressure to give N-(4-bromo-3-methyl-5-isoxazolyl)thiophene-2-sulfonamide. The pure material was obtained by recrystallization using hexanes/ethyl acetate (110 mg, 34 % yield), m.p. 125- 127° C.

**REFERENCE EXAMPLE 2**

**N-(4-Bromo-3-methyl-5-isoxazolyl)-5-(3-isoxazolyl)thiophene-2-sulfonamide**

A solution of 5-amino-4-bromo-3-methylisoxazole (177 mg, 1.0 mmol) in dry THF (2 ml) was added to a suspension of sodium hydride (60% dispersion in mineral oil, 90 mg, 2.2 mmol) in dry THF (1 ml) at 0 - 5° C. After stirring at 0 - 5° C for 5 min, the reaction was warmed to room temperature for 10 min to complete the reaction. The reaction mixture was re-cooled to 0° C, and 5-(3-isoxazolyl)thiophene-2-sulphonyl chloride (273 mg, 1.1 mmol), which had been dissolved in dry THF (2 ml), was added slowly. Stirring was continued for 1 h; during this period the reaction mixture

slowly attained ambient temperature. THF was removed under reduced pressure. The residue was dissolved in water (10 ml), the pH was adjusted to 2 - 3 by adding concentrated HCl, and was extracted with methylene chloride (3 X 10 ml). The combined organic layers was dried over anhydrous magnesium sulfate and concentrated under reduced pressure to give N-(4-bromo-3-methyl-5-isoxazolyl)-5-(3-isoxazolyl)thiophene-2-sulfonamide. The pure material was obtained by recrystallization using hexanes/ethyl acetate (160 mg, 41% yield), m.p. 120 - 123° C.

**REFERENCE EXAMPLE 3**

**N-(4-Bromo-3-methyl-5-isoxazolyl)-5-(2-pyridyl)thiophene-2-sulfonamide**

N-(4-bromo-3-methyl-5-isoxazolyl)-5-(2-pyridyl)thiophene-2-sulfonamide was prepared in the same manner as described in Reference Example 2 from 5-amino-4-bromo-3-methylisoxazole and 5-(2-pyridyl)thiophene-2-sulphonyl chloride in 40% yield. Purification was achieved by recrystallization from ethyl acetate to give a crystalline solid, m.p. 186 - 188° C.

**REFERENCE EXAMPLE 4**

**N-(4-Bromo-3-methyl-5-isoxazolyl)-4,5-dibromothiophene-2-sulfonamide**

N-(4-bromo-3-methyl-5-isoxazolyl)-4,5-dibromothiophene-2-sulfonamide was prepared in the same manner as described in Reference Example 2 from 5-amino-4-bromo-3-methylisoxazole and 4,5-dibromothiophene-2-sulphonyl chloride in 45% yield. Purification was achieved by recrystallization from ethyl acetate/hexanes to give a crystalline solid, m.p. 153 - 155° C.

**REFERENCE EXAMPLE 5**

**N-(4-Bromo-3-methyl-5-isoxazolyl)-5-chloro-3-methylbenzo[b]thiophene-2-sulfonamide**

N-(4-bromo-3-methyl-5-isoxazolyl)-5-chloro-3-methylbenzo[b]thiophene-2-sulfonamide was prepared in the same manner as described in Reference Example 2 from 5-amino-4-bromo-3-methylisoxazole and 5-chloro-3-methylbenzo[b]thiophene-2-sulphonyl chloride in 18% yield. Purification was achieved by recrystallization from ethyl acetate/hexanes to give a crystalline solid, m.p. 153 - 155° C.

**REFERENCE EXAMPLE 6**

**N-(4-Bromo-3-methyl-5-isoxazolyl)-5-(4-chlorobenzamidomethyl)thiophene-2-sulfonamide**

N-(4-Bromo-3-methyl-5-isoxazolyl)-5-(4-chlorobenzamidomethyl) thiophene-2-sulfonamide was prepared in the same manner as described in Reference Example 2 from 5-amino-4-bromo-3-methylisoxazole and 5-(4-chlorobenzamidomethyl)thiophene-2-sulphonyl chloride in 27% yield. The crude product was passed through a silica gel column using hexanes/ethyl acetate as eluent. Purification was effected by recrystallization from ethyl acetate/hexanes to give a crystalline solid, m.p. 210° C (dec).

**REFERENCE EXAMPLE 7**

**N-(4-Bromo-3-methyl-5-isoxazolyl)-4-(benzenesulfonyl)thiophene-2-sulfonamide**

N-(4-Bromo-3-methyl-5-isoxazolyl)-4-(benzenesulfonyl)thiophene-2-sulfonamide was prepared in the same manner as described in Reference Example 2 from 5-amino-4-bromo-3-methylisoxazole and 4-benzenesulfonylthiophene-2-sulphonyl chloride in 26% yield. Purification was achieved by recrystallization from ethyl acetate/hexanes to give a crystalline solid, m.p. 181 - 184° C.

**REFERENCE EXAMPLE 8**

**N-(4-Bromo-3-methyl-5-isoxazolyl)-4-bromo-5-chloro-thiophene-2-sulfonamide**

N-(4-Bromo-3-methyl-5-isoxazolyl)-4-bromo-5-chloro-thiophene-2-sulfonamide was prepared in the same manner as described in Reference Example 2 from 5-amino-4-bromo-3-methylisoxazole and 4-bromo-5-chlorothiophene-2-sulphonyl chloride in 25% yield. Purification was achieved by recrystallization from ethyl acetate/hexanes to give a crystalline solid, m.p. 143 - 145° C.

### REFERENCE EXAMPLE 9

#### N-(4-Bromo-3-methyl-5-isoxazolyl)-2,5-dichlorothiophene-3-sulfonamide

N-(4-Bromo-3-methyl-5-isoxazolyl)-2,5-dichlorothiophene-3-sulfonamide was prepared in the same manner as described in Reference Example 2 from 5-amino-4-bromo-3-methylisoxazole and 2,5-dichlorolthiophene-3-sulphonyl chloride in 47% yield. Purification was achieved by recrystallization from ethyl acetate/hexanes to give a crystalline solid, m.p. 135 - 138° C.

### REFERENCE EXAMPLE 10

#### N-(4-Bromo-3-methyl-5-isoxazolyl)-2,5-dimethylthiophene-3-sulfonamide

N-(4-Bromo-3-methyl-5-isoxazolyl)-2,5-dimethylthiophene-3-sulfonamide was prepared in the same manner as described in Reference Example 1 from 5-amino-4-bromo-3-methylisoxazole and 2,5-dimethylthiophene-3-sulphonyl chloride in 55% yield. Purification was achieved by recrystallization from ethyl acetate/hexanes to give a crystalline solid, m.p. 77 - 80° C.

### REFERENCE EXAMPLE 11

#### N-(4-Bromo-3-methyl-5-isoxazolyl)-4,5-dichlorothiophene-2-sulfonamide

N-(4-Bromo-3-methyl-5-isoxazolyl)-4,5-dichlorothiophene-2-sulfonamide was prepared in the same manner as described in Reference Example 1 from 5-amino-4-bromo-3-methylisoxazole and 4,5-dichlorothiophene-2-sulphonyl chloride in 42% yield. Purification was achieved by recrystallization from ethyl acetate/hexanes to give a crystalline solid, m.p. 135 - 138° C.

### REFERENCE EXAMPLE 12

#### N-(4-Bromo-3-methyl-5-isoxazolyl)-2,5-dichloro-4-bromothiophene-3-sulfonamide

N-(4-Bromo-3-methyl-5-isoxazolyl)-2,5-dichloro-4-bromothiophene-3-sulfonamide was prepared in the same manner as described in Reference Example 1 from 5-amino-4-bromo-3-methylisoxazole and 4-bromo-2,5-dichlorothiophene-3-sulfonyl chloride in 58% yield. Purification was achieved by recrystallization from ethyl acetate/hexanes to give a crystalline solid, m.p. 146 - 149° C.

### REFERENCE EXAMPLE 13

#### N-(4-Bromo-3-methyl-5-isoxazolyl)-2-{3-[1-methyl-5-(trifluoromethyl)pyrazolyl]}thiophene-5-sulfonamide

N-(4-Bromo-3-methyl-5-isoxazolyl)-2-{3-[1 -methyl-5-(trifluoromethyl)pyrazolyl]}thiophene-5-sulfonamide was prepared in the same manner as described in Reference Example 1 from 5-amino-4-bromo-3-methylisoxazole and 2-{3-[1-methyl-5-(trifluoromethyl)pyrazolyl]}thiophene-5-sulphonyl chloride in 30% yield. Purification was achieved by recrystallization from ethyl acetate/hexanes to give a crystalline solid, m.p. 121 - 123° C.

### REFERENCE EXAMPLE 14

#### N-(4-Bromo-5-methyl-3-isoxazolyl)thiophene-2-sulfonamide

Thiophene-2-sulfonyl Chloride (183 mg, 1 mmol) was added to a solution of 3-amino-4-bromo-5-methylisoxazole (177 mg, 1 mmol) in dry pyridine (0.5 ml). The reaction mixture was stirred at room temperature for 3 h. Pyridine was removed under reduced pressure and the residue was partitioned between water and ethyl acetate. The organic layer was washed with 1N HCl (3 X 10 ml), brine (10 ml) and dried over anhydrous magnesium sulfate. Evaporation of the solvents left an oily residue which was solidified at -20° C and then purified by recrystallization from ethyl acetate/hexanes, to give the pure product (51% yield) as a tan solid, m.p. 156 - 158° C.

**REFERENCE EXAMPLE 15**

**N-{4-Bromo-3-methyl-5-isoxazolyl)-5-(benzenesulfonyl)thiophene-2-sulfonamide**

N-(4-Bromo-3-methyl-5-isoxazolyl)-5-(benzenesulfonyl)thiophene-2-sulfonamide was prepared in the same manner as described in Reference Example 2 from 5-amino-4-bromo-3-methylisoxazole and 5-benzenesulfonylthiophene-2-sulfonyl chloride in 59% yield. Purification was achieved by recrystallization from ethyl acetate/hexanes to give a crystalline solid, m.p. 139 - 142° C.

**REFERENCE EXAMPLE 16**

**N-(4-Bromo-3-methyl-5-isoxazolyl)-2-(carbomethoxy)thiophene-3-sulfonamide**

N-(4-Bromo-3-methyl-5-isoxazolyl)-2-(carbomethoxy)thiophene-3-sulfonamide was prepared in the same manner as described in Reference Example 2 from 5-amino-4-bromo-3-methylisoxazole and 2-(carbomethoxy)thiophene-3-sulfonyl chloride in 73% yield. Purification was achieved by recrystallization from ethyl acetate/hexanes to give a crystalline solid, m.p. 198 - 200° C.

**REFERENCE EXAMPLE 17**

**N-(4-Bromo-3-methyl-5-isoxazolyl)-2-(carboxyl)thiophene-3-sulfonamide**

N-(4-Bromo-3-methyl-5-isoxazolyl)-2-(carbomethoxy)thiophene-3-sulfonamide (Reference Example 16) (1.5 g, 3.95 mmol) was dissolved in methanol (10 ml). Sodium hydroxide pellets (1 g, 25 mmol) and a few drops of water were then added. The resultant solution was stirred for 16 h at ambient temperature. Methanol was removed under reduced pressure. The residue was diluted with water and was extracted with ethyl acetate (2 X 10 ml). The aqueous layer was acidified (pH = 2) with concentrated hydrochloric acid and was extracted with ethyl acetate (2 X 60 ml). The combined organic layers was dried over anhydrous magnesium sulfate and filtered. Removal of the solvent gave N-(4-bromo-3-methyl-5-isoxazolyl)-2-(carbomethoxy)thiophene-3-sulfonamide (1.2 g, 82% yield), which was purified by silica gel column chromatography using ethyl acetate as eluent, m.p. 188 - 194° C.

**REFERENCE EXAMPLE 18**

**N-(4-Bromo-3-methyl-5-isoxazolyl)-2-(N-phenylaminocarbonyl)thiophene-3-sulfonamide**

Aniline (0.093 g, 1 mmol) and 1-ethyl-3'[3-dimethylaminopropyl]-carbodiimide (EDCI) (0.191 g, 1mmol) were added to N-(4-bromo-3-methyl-5-isoxazolyl)-2-(carboxyl)thiophene-3-sulfonamide (0.368 g, 1 mmol) that had been suspended in methylene chloride (5 ml) to produce a clear solution. Stirring was continued for 1 h at ambient temperature. The reaction mixture was diluted with methylene chloride (50 ml) and washed with 3 N hydrochloric acid solution (3 X 50 ml). The combined organic layers was dried over anhydrous magnesium sulfate and filtered. Removal of the solvent under reduced pressure gave N-(4-bromo-3-methyl-5-isoxazolyl)-2-(N-phenylaminocarbonyl)thiophene-3-sulfonamide. The crude product thus obtained was purified by column chromatography using ethyl acetate as eluent to yield the product (0.32 g, 72% yield, m.p. 168 - 170° C.

**REFERENCE EXAMPLE 19**

**N-(4-Bromo-3-methyl-5-isoxazolyl) 1-(4'-isopropylphenyl)pyrrole-2-sulfonamide and N-(4-bromo-3-methyl-5-isoxazolyl) 1-(4'-isopropylphenyl)pyrrole-3-sulfonamide**

**A. 1-(4'-isopropylphenyl)pyrrole**

Glacial acetic acid (100 ml) was added to a mixture of 4-isopropylaniline (10 ml, 72.4 mmol) and 2,5-dimethoxytetrahydrofuran (9.6 ml, 72.4 mmol) and the resulting mixture was refluxed for 1.5 h. The reaction mixture was allowed to cool and acetic acid was removed under reduced pressure. The resulting brown syrup was dissolved in ethyl acetate (200 ml) and washed with water (2 X 200 ml). The organic layer was dried over magnesium sulfate and filtered. Removal of the solvent gave 1-(4'-isopropylphenyl)pyrrole (13.28 g, 99% yield) as a brown syrup.

**B. 1-(4'-isopropylphenyl)pyrrole-2-sulfonic acid**

Chlorosulfonic acid (1.82 ml, 27.08 mmol) was slowly added to a solution of 1-(4'-isopropylphenyl)pyrrole (5.01 g, 27.08 mmol) in chloroform (100 ml) at 0° C. The resulting solution was stirred at 0° C for 1 h and for an additional 1 h at room temperature. Chloroform was removed under reduced pressure. The resultant brown liquid was diluted with ethyl acetate (200 ml) and washed with 1 N sodium hydroxide. The aqueous layer was then acidified with concentrated hydrochloric acid (pH <1) and then extracted with chloroform (2 X 150 ml). The combined organic layers was dried over magnesium sulfate and was filtered. Removal of the solvent gave 1-(4'-isopropylphenyl)pyrrole-2-sulfonic acid as a brown syrup (3 g, 42 % yield).

**C. 1-(4'-isopropylphenyl)pyrrole-2-sulfonyl chloride and 1-(4'-isopropylphenyl)pyrrole-3-sulfonyl chloride**

Phosphorus pentachloride (4.7 g, 22.64 mmol) was slowly added to a solution of 1-(4'-isopropylphenyl)pyrrole-2-sulfonic acid (3 g, 11.32 mmol)in phosphorus oxychloride (8.4 ml, 90.57 mmol). The resulting mixture was heated at 70° C for 10 h. The reaction mixture was allowed to cool to room temperature, then carefully poured on to crushed ice (500 g) and extracted with chloroform (200 ml). The combined organic layers was dried over anhydrous magnesium sulfate. This was filtered and removal of the solvent yielded a 4:1 mixture of 1-(4'-isopropylphenyl)-pyrrole-2-sulfonyl chloride and 1-[4'-isopropylphenyl)pyrrole-3-sulfonyl chloride (2.5 g, 78%) as a brown oil.

**D. N-(4-bromo-3-methyl-5-isoxazolyl) 1-(4'-isopropylphenyl)pyrrole-2-sulfonamide and N-(4-bromo-3-methyl-5-isoxazolyl) 1-(4'-isopropylphenyl)pyrrole-3-sulfonamide**

N-(4-bromo-3-methyl-5-isoxazolyl) 1-(4'-isopropylphenyl)pyrrole-2-sulfonamide and N-(4-bromo-3-methyl-5-isoxazolyl) 1-(4'-isopropylphenyl)pyrrole-3-sulfonamide were prepared in the same manner as described in Reference Example 2 from 5-amino-4-bromo-3-methylisoxazole and a mixture of 1-(4'-isopropylphenyl)pyrrole-2-sulfonyl chloride and 1-(4'-isopropylphenyl)pyrrole-3-sulfonyl chloride in 65% combined yield. The mixture was subjected to preparative HPLC to give N-(4-bromo-3-methyl-5-isoxazolyl) 1-(4'-isopropylphenyl) pyrrole-2-sulfonamide (retention time 22.85 min, 5% to 95% acetonitrile in water with 0.1% TFA over 30 min period, $C_{18}$ analytical column) and N-(4-bromo-3-methyl-5-isoxazolyl) 1-(4'-isopropylphenyl)pyrrole-3-sulfonamide (retention time 24.56 min, 5% to 95% acetonitrile in water with 0.1% TFA over 30 min period, $C_{18}$ analytical column) as oils.

**REFERENCE EXAMPLE 20**

**N-(4-Bromo-3-methyl-5-isoxazolyl)-5-bromothiophene-2-sulfonamide**

N-(4-Bromo-3-methyl-5-isoxazolyl)-5-bromothiophene-2-sulfonamide was prepared in the same manner as described in Reference Example 2 from 5-amino-4-bromo-3-methylisoxazole and 5-bromothiophene-2-sulfonyl chloride in 30% yield. Purification was achieved by recrystallization from ethyl acetate/hexanes to give a crystalline solid, m.p. 240° C (dec).

**REFERENCE EXAMPLE 21**

**N-(4-Bromo-3-methyl-5-isoxazolyl)-2-[N-(4-methoxyphenyl)aminocarbonyl]-thiophene-3-sulfonamide**

4-Methoxyaniline (0.246 g, 2 mmol), bromo-tris-pyrrolidinophosphonium hexafluorophosphate (PyBrop) (0.466 g, 1 mmol) and N,N'-diisopropylethylamine (0.15 ml) were added to N-(4-bromo-3-methyl-5-isoxazolyl)-2-(carboxyl)thiophene-3-sulfonamide (0.368 g, 1 mmol), which had been suspended in methylene chloride (3 ml), resulting in a clear solution. Stirring was continued for 24 h at ambient temperature. The reaction mixture was diluted with methylene chloride (50 ml) and washed with 3 N hydrochloric acid solution (3 X 50 ml) followed by 5% sodium carbonate solution (2 X 50 ml). The combined organic layers was dried over anhydrous magnesium sulfate and filtered. Removal of the solvent under reduced pressure gave N-(4-bromo-3-methyl-5-isoxazolyl) -2-[N-(4-methoxyphenyl)aminocarbonyl]thiophene-3-sulfonamide. The crude product thus obtained was purified by column chromatography using ethyl acetate as eluent. This was recrystallized from ethyl acetate/hexanes to give a crystalline solid, m.p. 202 - 205° C (0.08 g, 17% yield).

## REFERENCE EXAMPLE 22

### N-(4-Bromo-3-methyl-5-isoxazolyl)-2-[N-(3-methoxyphenyl)aminocarbonyl]thiophene-3-sulfonamide

N-(4-Bromo-3-methyl-5-isoxazolyl)-2-[N-(3-methoxyphenyl)aminocarbonyl]thiophene-3-sulfonamide was prepared in the same manner as described in Reference Example 21 from N-(4-bromo-3-methyl-5-isoxazolyl)-2-(carboxyl)thiophene-3-sulfonamide and 3-methoxyaniline in 23% yield. The crude product was purified by column chromatography using ethyl acetate as eluent. This was recrystallized from ethyl acetate/hexanes to give a crystalline solid, m.p. 200 - 202° C.

## REFERENCE EXAMPLE 23

### N-(4-Bromo-3-methyl-5-isoxazolyl)-2-[N-(2-methoxyphenyl)aminocarbonyl]thiophene-3-sulfonamide

N-(4-bromo-3-methyl-5-isoxazolyl)-2-[N-(2-methoxyphenyl)aminocarbonyl]thiophene-3-sulfonamide was prepared in the same manner as described in Reference Example 21 from N-(4-bromo-3-methyl-5-isoxazolyl)-2-(carboxyl)thiophene-3-sulfonamide and 2-methoxyaniline in 26% yield. The crude product was purified by column chromatography using ethyl acetate as eluent. This was recrystallized from ethyl acetate/hexanes to give a crystalline solid, m.p. 74 - 80° C.

## REFERENCE EXAMPLE 24

### N-(4-Bromo-3-methyl-5-isoxazolyl)-2-(N-benzylaminocarbonyl)thiophene-3-sulfonamide

Benzylamine (0.214 g, 2 mmol), benzotriazole-1-yl-oxy-tris(dimethylamino)phosphonium hexafluorophosphate (Bop) (0.442 g, 1 mmol) and N,N'-diisopropylethylamine (0.15 ml) were added to N-(4-bromo-3-methyl-5-isoxazolyl)-2-(carboxyl)thiophene-3-sulfonamide (0.368 g, 1 mmol), which had been suspended in methylene chloride (3 ml). The resultant solution was stirred for 14 h at ambient temperature. This was diluted with methylene chloride (50 ml) and washed with 3 N hydrochloric acid (3 X 50 ml) followed by 5% sodium carbonate solution (2 X 50 ml). The combined organic layers was dried over anhydrous magnesium sulfate and filtered. Removal of the solvent under reduced pressure gave N-(4-bromo-3-methyl-5-isoxazolyl)-2-(N-benzylaminocarbonyl)thiophene-3-sulfonamide. The crude product was purified by column chromatography using ethyl acetate as eluent. Recrystallization from ethyl acetate/hexanes gave a crystalline solid, m.p. 186 - 190° C (0.14 g, 30% yield).

## REFERENCE EXAMPLE 25

### N-(4-Bromo-3-methyl-5-isoxazolyl)-2-(N-(4-ethylphenyl)aminocarbonyl]-thiophene-3-sulfonamide

N-(4-bromo-3-methyl-5-isoxazolyl)-2-[N-(4-ethylphenyl)aminocarbonyl]thiophene-3-sulfonamide was prepared in the same manner as described in Reference Example 24 from N-(4-bromo-3-methyl-5-isoxazolyl)-2-(carboxyl) thiophene-3-sulfonamide and 4-ethylaniline in 31% yield. The crude product was purified by column chromatography using ethyl acetate as eluent. This was recrystallized from ethyl acetate/hexanes to give a crystalline solid, m.p. 187 - 190° C.

## REFERENCE EXAMPLE 26

### N-(4-Bromo-3-methyl-5-isoxazolyl)-2-[N-(4-biphenyl)aminocarbonyl]-thiophane-3-sulfonamide

N-(4-bromo-3-methyl-5-isoxazolyl)-2-[N-(4-biphenyl)aminocarbonyl]-thiophene-3-sulfonamide compound was prepared in the same manner as described in Example 24 from N-(4-bromo-3-methyl-5-isoxazolyl)-2-(carboxyl) thiophene-3-sulfonamide and 4-phenylaniline in 26% yield. The crude product was purified by column chromatography using ethyl acetate as eluent. This was recrystallized from ethyl acetate/hexanes to give a crystalline solid, m.p. 205 - 212° C (dec).

**REFERENCE EXAMPLE 27**

**N-(3,4-dimethyl-5-isoxazolyl)-2-(carbomethoxy)thiophene-3-sulfonamide**

2-Methoxycarbonylthophene-3-sulfonyl chloride (2.50 g, 10.05 mmol) was added to a solution of 5-amino-3,4-dimethylisoxazole(0.98 g, 8.75 mmol) in dry pyridine (5.0 ml). The reaction mixture was stirred at room temperature for 16 h. Pyridine was removed under reduced pressure and the residue was partitioned between water and dichloromethane. The organic layer was washed with 1 N HCl (2 X 50 ml) and dried over anhydrous magnesium sulfate. Evaporation of the solvents left an oily residue, which, after purification by column chromatography over silica gel (1:1 hexaneslethyl acetate as eluent), yielded 2.20 mg (65%) of a brown solid. Further purification was achieved by recrystallization from ethyl acetate/hexanes, giving the pure product as a white solid, m.p. 113-116° C.

**REFERENCE EXAMPLE 28**

**N-(3,4-dimethyl-5-isoxazolyl)-2-(carboxyl)thiophene-3-sulfonamide**

N-(3,4-dimethyl-5-isoxazolyl)-2-(carboxyl)thiophene-3-sulfonamide was prepared in the same manner as described in Reference Example 17 from N-(3,4-dimethyl-5-isoxazolyl)-2-(carbomethoxy)thiophene-3-sulfonamide by basic hydrolysis in 94% yield. Purification was achieved by recrystallization from ethyl acetate/hexanes to give a crystalline solid, m.p. 202 - 203° C.

**REFERENCE EXAMPLE 29**

**N-(3,4-dimethyl-5-isoxazolyl)-2-(N-phenylaminocarbonyl)thiophene-3-sulfonamide**

N-(3,4-dimethyl-5-isoxazolyl)-2-(N-phenylaminocarbonyl)thiophene-3-sulfonamide was prepared in the same manner as described in Reference Example 18 from N-(3,4-dimethyl-5-isoxazolyl)-2-(carboxyl)thiophene-3-sulfonamide in 40% yield. Purification was achieved by recrystallization from ethyl methanol/water to give a crystalline solid, m.p. 176 - 178 C.

**REFERENCE EXAMPLE 30**

**N-(4-Bromo-3-methyl-5-isoxazolyl)-5-(2-thienyl)thiophene-2-sulfonamide A. 5-bromo-2,2'-bithiophene**

N-Bromosuccinimide (NBS, 1.12 g, 6.3 mmol) was added in small portions to a stirred solution of 1.0 g (6.01 mmol) of 2,2'-bithiophene in 10 ml of glacial acetic acid and 10 ml of chloroform. After stirring for 1 h at room temperature, the mixture was poured into ice-water and extracted into chloroform (75 ml). The organic layer was washed with aqueous sodium bicarbonate solution, water, and then dried over magnesium sulfate and evaporated. The residue was subjected to flash chromatography on silica gel using hexane to give 1.3 g (88%) of a light green solid, m.p. 55 - 56° C.

**B. 5-Chlorosulfonyl-2,2'-bithiophene**

A stirred solution of 5-bromo-2,2'-bithiophene (1.5 g, 6.1 mmol) in 10 ml of dry ether was placed under an argon atmosphere, cooled to -78° C and 4.3 ml of a 1.7 M solution of t-butyllithium was added over 20 min. Stirring was continued at this temperature for an additional 20 min. Sulfur dioxide gas was then bubbled in at -78° C until a yellow precipitate formed. Bubbling of the sulfur dioxide gas was continued for an additional 3 min and was immediately followed by a dropwise addition of N-chlorosuccinimide (NCS, 902 mg, 6.76 mmol) that had been dissolved in THF. The mixture was warmed to room temperature and stirring was continued for an additional 1.5 h. The mixture was then concentrated and the residue dissolved in ether. The organic layer was washed with water, brine solution and dried over magnesium sulfate. Evaporation of solvent left a pale yellow solid, which was recrystallized from hexane to give 700 mg (44%) of a yellow solid, m.p. 63-64° C.

**C. N-(4-Bromo-3-methyl-5-isoxazolyl)-5-(2-thienyl)thiophene-2-sulfonamide**

N-(4-bromo-3-methyl-5-isoxazolyl)-5-{2-thienyl)thiophene-2-sulfonamide was prepared in the same manner as described in Reference Example 2. Reaction of 2-chlorosulfonyl-5,2'-bithiophene (300 mg, 1.14 mmol) with 5-amino-4-bromo-3-methylisoxazole (183 mg, 1.03 mmol) yielded, after flash chromatography using 10% MeOH/$CHCl_3$, 430 mg (94%) of a pale brown solid, m.p. 210° C.

**REFERENCE EXAMPLE 31**

**N-(4-Bromo-3-methyl-5-isoxazolyl)thiophene-3-sulfonamide**

**A. Thiophene-3-sulfonyl chloride**

A stirred solution of 3-bromothiophene (1.5 g, 9.2 mmol) in 10 ml of dry ether was placed under an argon atmosphere and cooled to -78° C. Over the course of 20 min, a solution of t-butyllithium (5.6 ml of a 1.7 M) was added and stirring was continued at this temperature for an additional 20 min. Sulfur dioxide gas was then bubbled in at -78° C and the solution was warmed to 0° C, whereupon NCS (1.47 g, 12 mmol) in 8 ml of THF, was added dropwise. After warming to room temperature, stirring was continued for an additional 1 hour, after which, evaporation of solvents left 1.55 g of a brown oil. Flash chromatography over silica gel using hexanes yielded 1.24 g (74%) of a yellow oil which solidified on standing to give a yellow crystalline solid, m.p. 38-39° C.

**B. N-(4-Bromo-3-methyl-5-isoxazolyl)thiophene-3-sulfonamide**

N-(4-bromo-3-methyl-5-isoxazolyl)thiophene-3-sulfonamide was prepared in the same manner as described in Reference Example 2 from thiophene-3-sulfonyl chloride with 5-amino-4-bromo-3-methylisoxazole in 22% yield. Purification by column chromatography using 10% MeOH/$CHCl_3$ as eluent gave a pale brown oil.

**REFERENCE EXAMPLE 32**

**N-(3,4-dimethyl-5-isoxazolyl)-5-phenylthiophene-2-sulfonamide**

**A. N-(3,4-dimethyl-5-isoxazolyl)-5-bromothiophene-2-sulfonamide**

A solution of 5-bromothiophene-2-sulfonyl chloride (2.75 g, 10 mmol) and 5-amino-3,4-dimethylisoxazole (1.07 g, 9.57 mmol) in pyridine containing a catalytic amount of 4-dimethylaminopyridine (DMAP, 10 mg) was stirred at room temperature for a period of 3 h. The solution was heated at 50° C for an additional 1.5 h to drive the reaction to completion as judged by TLC. The pyridine was removed under reduced pressure and the residue, after extraction into ethyl acetate, was washed with 1 N HCl (2 x 25 ml), water (1 x 25), brine solution, (1 x 25 ml) and dried over magnesium sulfate. Evaporation of solvent left a viscous brown gum, which was subjected to flash chromatography. Elution with 3% methanol hexanes gave 246 mg (10%) of pure sulfonamide.

**B. N-(methoxyethoxymethyl)-N-(3,4-dimethyl-5-isoxazolyl)-5-bromothiophene-2-sulfonamide**

N-(3,4-dimethyl-5-isoxazolyl)-5-bromothiophene-2-sulfonamide (680 mg, 2 mmol) in dry THF (2 ml) was added to sodium hydride (121 mg of a 60% oil dispersion, 3 mmol) in dry THF (1 ml). The resulting suspension was cooled to 0° C and methoxyethoxymethyl chloride (334 mg, 2.68 mmol) was added dropwise via syringe. The solution was warmed to room temperature, and stirring continued overnight. Evaporation of solvent left an oil that was extracted into ethyl acetate, washed with brine, dried over magnesium sulfate and evaporated. Flash chromatography of the residue on silica gel using 10-15% ethylacetate/hexanes yielded 480 mg (56%) of a colorless oil.

**C. N-(methoxyethoxymethyl)-N-(3,4-dimethyl-5-isoxazolyl)-5-phenylthiophene-2-sulfonamide**

Sodium carbonate (2 ml of a 2 M aqueous solution) followed by phenyl boronic acid (86 mg, 0.71 mmol) in 2 ml of 95% ethanol were added to a solution of N-(methoxyethoxymethyl)-N-(3,4-dimethyl-5-isoxazolyl)-5-bromothiophene-2-sulfonamide (200 mg, 0.47 mmol) and tetrakis (triphenylphosphine) palladium (0) (23 mg, 0.02 mmol) in dry benzene (4 ml) under argon. The mixture was refluxed for 12 h, diluted with 5 ml of water and extracted into ethyl acetate (3 X 25 ml). The combined organic extracts was washed with brine (1 x 25 ml), dried and evaporated. The residue was flash chromatographed on silica gel using 25% ethylacetate/hexanes to afford 123 mg (62%) of the sulfonamide as a colorless gum.

**D. N-(3,4-dimethyl-5-isoxazolyl)-5-phenylthiophene-2-sulfonamide**

HCl (3 ml of a 3 N aqueous solution) was added to a solution of N-(methoxyethoxymethyl)-N-(3,4-dimethyl-5-isoxazolyl)-5-phenylthiophene-2-sulfonamide (100 mg, 0.24 mmol) in 3 ml of 95% ethanol and the resulting mixture was refluxed for 6 h. The mixture was then concentrated, diluted with 5 ml of water, neutralized with saturated aqueous

sodium bicarbonate solution and acidified to pH 4 using glacial acetic acid. The mixture was extracted with ethyl acetate (2 x 25 ml) and the combined organic extract was washed with brine (1 x 5 ml), dried and evaporated. Flash chromatography of the residue on silica gel using 2% $MeOH/CHCl_3$ and further purification by reverse phase HPLC yielded 33.4 mg (42%) of the pure sulfonamide as a white powder, m.p. 176-178° C.

**REFERENCE EXAMPLE 33**

**N-(4-Bromo-3-methyl-5-isoxazolyl)-5-(4-ethylphenyl)thiophene-2-sulfonamide**

**A. N-(5-bromothiophene-2-sulfonyl)-pyrrole**

Sodium hydride (60% oil dispersion, 191 m.g., 4.78 mmol) was suspended in dry tetrahydrofuran (2 ml) and the resulting cloudy suspension was cooled to 0° C in an ice bath. Pyrrole (385 mg, 5.75 mmol) in dry tetrahydrofuran (2 ml) was added dropwise over a period of 10 min. The ice bath was removed and the solution was stirred at room temperature until gas evolution ceased (15 minutes), whereupon 5-bromothiophene-2-sulfonyl chloride (1.0 g, 3.82 mmol) previously dissolved in tetrahydrofuran (4.0 ml) was added dropwise through a steel cannula. After stirring for 1 h at room temperature, the mixture was filtered through Celite. The filter pad was rinsed with tetrahydrofuran, and the filtrate was evaporated, which left a light brown solid that was recrystallized from methanol to produce the sulfonamide (821 mg, 74% yield) as a white powder.

**B. 4-Ethylphenylboronic acid**

A solution of 1-bromo-4-ethyl benzene (2.0 g, 11 mmol) in dry ether (5 ml) was added to magnesium turnings (311 mg, 13 mmol), which had been suspended in dry ether, by dropwise addition. After addition was complete, the suspension was refluxed for a period of 15 min, by which time nearly all of the magnesium had reacted. The solution was then added to trimethy borate (1.12 g, 11 mmol), previously dissolved in ether (5 ml) at -78° C, warmed to room temperature and stirred for 90 min. The reaction was quenched by the addition of 10% aqueous HCl (2 ml) and the solution was extracted with ether. The combined ether extract was extracted with 1 M NaOH (2 X 20 ml), the aqueous extracts were acidified with dilute HCl to pH 2 and extracted with ether (2 X 25 ml). The resulting combined ether extract was washed once with water (10 ml), dried and evaporated to produce a white solid (676 mg, 38% yield), m.p. 138-140° C.

**C. N-[5-(4-ethylphenyl)thiophene-2-sulfonyl]pyrrole**

N-[5-(4-ethylphenyl)thiophene-2-sulfonyl]pyrrole was prepared, in the same manner as described in Reference Example 32C, from 4-ethylphenylboronic acid and N-(5-bromothiophenesulfonyl)pyrrole. Purification by column chromatography using 10% ethyl acetate/hexanes gave the pure sulfonamide as a tan solid in 81 % yield.

**D. 5-Chlorosulfonyl-2-(4-ethylphenyl)thiophene**

A solution of N-t5-(4-ethylphenyl)thiophene-2-sulfonyl]pyrrole (100 mg, 0.32 mmol) and 6 N sodium hydroxide (1 ml) in methanol (1.5 ml) was refluxed for approximately 6 h. Evaporation of solvents and drying *in vacuo* resulted in an oil. Phosphorus oxychloride (258 ml, 2.52 mmol) and phosphorus pentachloride (131 mg, 0.63 mmol) were added to the oil and the resulting brown suspension was heated at 50° C for 3 h. The resulting clear brown solution was carefully added to about 20 ml of crushed ice and then extracted with ethyl acetate (3x25 ml). The combined organic layers was washed with brine (2x5 ml), dried ($MgSO_4$) and evaporated to leave an oily residue. Flash chromatography over silica gel using 2% ethyl acetate/hexanes yielded (53 mg, 59%) of the pure sulfonyl chloride as a pale yellow oil.

**E. N-(4-bromo-3-methyl-5-isoxazolyl)-5-(4-ethylphenyl)thiophene-2-sulfonamide**

N-(4-bromo-3-methyl-5-isoxazolyl)-5-(4-ethylphenyl) thiophene-2-sulfonamide was prepared in the same manner as described in Reference Example 2. Reaction of 5-chlorosulfonyl-2-(4-ethylphenyl) thiophene (47.1 mg, 11.16 mmol) with 5-amino-4-bromo-3-methyl isoxazole (29 mg, 0.16 mmol) yielded, after flash chromatography using 10% $MeOH/CHCl_3$, a pale brown solid (46 mg, 66% yield), m.p. 172-175° C.

## REFERENCE EXAMPLE 34

### N-(3,4-dimethyl-5-isoxazolyl)benzo[b]thiophene-2-sulfonamide

### A. Benzo[b]thiophene-2-sulfonyl chloride

Benzo[b]thiophene (1.50 g, 11.2 mmol) was stirred at 0° C in 20 ml of THF. t-Butyllithium (1.7 M, 16.8 mmol, 9.9 ml) was slowly added over a 5 minute period. Fifteen minutes later, $SO_2$ was flushed into the reaction flask and a thick white precepitate formed. The reaction mixture was stirred for 15 minutes at 0° C and then NCS (1.64 g, 12.3 mmol) was added. The reaction was warmed to 25° C and stirred for 30 min. It was then poured into ethyl acetate (150 ml) and washed with brine (3x100 ml). The organic phase was dried with $MgSO_4$, filtered and concentrated to collect 2.29 g of a brown oil. The brown oil was subjected to flash chromatography (5% ethyl acetate/hexanes), which provided a yellow tan solid (1.39 g, 53% yield).

### B. N-(3,4-Dimethyl-5-isoxazolyl)benzo[b]thiophene-2-sulfonamide

3,4-Dimethyl-5-amino-isoxazole (0.224 g, 2.0 mmol) and 50 mg of DMAP were stirred in 5 ml of pyridine at 25° C. The benzo[b]thiophene-2-sulfonyl chloride (0.16 g, 2.6 mmol) was added and the dark brown-yellow reaction mixture was stirred for 18 h at ambient temperature, poured into 100 ml of ethyl acetate and washed with 2% HCl (3x50 ml). The organic phase was dried with $MgSO_4$, filtered and concentrated to collect 0.61 g of a brown oil/solid. The brown oil/solid was subjected to flash chromatography (30% ethyl acetate/hexanes) to provide 0.37 g of a light brown solid. This was stirred in 10 ml of methanol and 0.5 g of NaOH. The methanolic solution was heated for reflux for 1 h, then cooled to 25° C and the methanol was removed *in vacuo*. The resulting residue was acidified to pH 1 with 2% HCl (100 ml) and extracted with ethyl acetate (2x50 ml) The organic phase was dried with $MgSO_4$, filtered and concentrated to collect 0.225 g of a yellow-orange solid. This was recrystallized from $CHCl_3$/hexanes to produce a light tan-yellow solid (0.194 g, 31% yield), m.p. 157-160° C.

## REFERENCE EXAMPLE 35

### N-(3,4-Dimethyl-5-isoxazolyl)benzo[b]furan-2-sulfonamide

### A. Benzo[b]furan-2-sulfonyl chloride

Benzo[b]furan-2-sulfonyl chloride was prepared as in Reference Example 34A from benzo[b]furan (1.61 g, 13.6 mmol), t-BuLi (1.7 M, 17.7 mmol, 10.4 ml) and NCS (2.0 g, 15.0 mmol). Flash chromatography (5% ethyl acetate/hexanes) yielded a brown solid (1.84 g, 62% yield).

### B. N-(3,4-Dimethyl-5-isoxazolyl)benzo[b]furan-2-sulfonamide

N-(3,4-Dimethyl-5-isoxazolyl)benzo[b]furan-2-sulfonamide was prepared, in the same manner as described in Reference Example 34B, from 3,4-dimethyl-5-amino isoxazole (78 mg, 0.70 mmol) and benzo[b]furan-2-sulfonyl chloride (0.46 g, 2.1 mmol) Flash chromatography (30% ethyl acetate/hexanes) provided 0.186 g of a light yellow solid, which was treated with 31 mg of NaOH in 10 ml of methanol at 25° C for 30 minutes. Recrystallization from $CHCl_3$/hexanes yielded a light tan solid (90 mg, 44% yield), m.p. 160.5-163° C.

## REFERENCE EXAMPLE 36

### N-(3,4-dimethyl-5-isoxazolyl)furan-2-sulfonamide

### A. Furan-2-sulfonyl chloride

Furan-2-sulfonyl chloride was prepared as in Reference Example 34A from furan (0.96 g, 14.2 mmol), t-BuLi (1.7 M, 17 mmol, 10 ml) and NCS (2.27 g, 17 mmol) using ether (30 ml) as the solvent. Flash chromatography (5% ethyl acetate/hexanes) produced a yellow liquid (1.22 g, 52% yield).

### B. N-(3,4-dimethyl-5-isoxazolyl)furan-2-sulfonamide

N-(3,4-dimethyl-5-isoxazolyl)furan-2-sulfonamide was prepared as described in Example 34B from 3,4-dimethyl-5-amino isoxazole (0.122 g, 1.0 mmol), furan-2-sulfonyl chloride (0.50 g, 3.0 mmol) and NaOH (64 mg). Flash chromatography (50% ethyl acetate/hexanes) yielded 70 mg of a yellow solid. Recrystallization from $CHCl_3$/hexanes produced an off-white solid (46 mg, 29% yield), m.p 107 - 110° C.

## REFERENCE EXAMPLE 37

### N-(3,4-Dimethyl-5-isoxazolyl)-3-methoxy-2-thiophene sulfonamide

### A. 3-methoxy-2-thiophenesulfonyl chloride

Chlorosulfonic acid ($ClSO_3H$, 2.31 g, 19.62 mmol) was slowly added to a solution of 3-methoxythiophene (2.29 g, 19.62 mmol) in $CHCl_3$ (80 ml) at 0° C. The resulting mixture was stirred at 0° C for 30 min. The solvent was evaporated under reduced pressure at room temperature, the residue was suspended in $POCl_3$ (15 ml, 156.96 mmol), and $PCl_5$ (8.2 g, 39.24 mmol) was added slowly. The reaction was stirred at 60° C for 18 h, then cooled to room temperature and poured onto crushed ice (200 g). The aqueous mixture was extracted with $CHCl_3$ (2x150 ml) and the combined organic layers was dried ($MgSO_4$). The solid was removed by filtration and the filtrate was concentrated to give 3-methoxy-2-thiophenesulfonyl chloride as a brown oil (1.81 g, 43% yield).

### B. N-(3,4-dimethyl-5-isoxazolyl)-3-methoxy-2-thiophene sulfonamide

Sodium hydride (1.02 g, 25.56 mmol, 60% dispersion in mineral oil) was slowly added to a solution of 3-methoxy-2-thiophenesulfonyl chloride (1.18 g, 8.52 mmol) and 3,4-dimethyl-5-aminoisoxazole (1.05 g, 9.37 mmol) in THF (20 ml) at room temperature. The resulting mixture was refluxed for 4 h. THE was removed under reduced pressure. The residue was dissolved in water (10 ml), the pH was adjusted to 10 - 11 by adding 5 N sodium hydroxide solution, and was extracted with ethyl acetate (3 X 10 ml) to remove the neutral impurities. The aqueous layer was acidified with concentrated HCl (pH 2 - 3) and extracted with methylene chloride (3 X 10 ml). The combined organic layers was dried over anhydrous magnesium sulfate to produce a crude oil. Further purification by reverse phase HPLC yielded a yellow oil (retention time 14.94 min, 5% to 95% acetonitrile in $H_2O$ with 0.1% TFA over 30 min period, $C_{18}$ analytical column).

## REFERENCE EXAMPLE 38

### N-(4-Bromo-3-methyl-5-isoxazolyl)-3-phenyl-2-thiophene sulfonamide and N-(4-bromo-3-methyl-5-isoxazolyl)-4-phenyl-2-thiophene sulfonamide

### A. 3-phenyl-2-thiophenesulfonyl chloride and 4-phenyl-2-thiophenesulfonyl chloride

n-Butyllithium (2.38 M, 17.2 ml, 41.03 mmol) was slowly added to a solution of 3-phenylthiophene (5.47 g, 34.2 mmol) in $Et_2O$ (25 ml) at 0° C. The ice bath was removed, the mixture was stirred at room temperature for 2 h, cooled to -30° C ($CO_2$/acetone) and $SO_2$ gas was bubbled through the reaction mixture for 20 min. A solution of NCS (6.06 g, 44.5 mmol) in THF (20 ml) was then added. The reaction was allowed to warm to room temperature and stirred for 16 h. The crude mixture was filtered, and the solid was washed with $Et_2O$. The combined organic layers was concentrated and the residue was chromatographed (hexanes/$CHCl_3$) to give 3-phenyl-2-thiophenesulfonyl chloride and 4-phenyl-2-thiophenesulfonyl chloride as a 1:1 mixture (1.46 g, 16.5%, while solid).

### B. N-(4-Bromo-3-methyl-5-isoxazolyl)-3-phenyl-2-thiophene sulfonamide and N-(4-bromo-3-methyl-5-isoxazolyl)-4-phenyl-2-thiophene sulfonamide

N-(4-bromo-3-methyl-5-isoxazolyl)-3-phenyl-2-thiophene sulfonamide and N-(4-bromo-3-methyl-5-isoxazolyl)-4-phenyl-2-thiophene sulfonamide were prepared as described in Reference Example 1. A fraction of the crude mixture of products was purified by HPLC to give N-(4-bromo-3-methyl-5-isoxazolyl)-3-phenyl-2-thiophene sulfonamide (light brown solid, retention time 20.48 min, 5% to 95% acetonitrile in water with 0.1% TFA over 30 min $C_{18}$ analytical column, m.p. 105-107° C) and N-(4-bromo-3-methyl-5-isoxazolyl)-4-phenyl-2-thiophene sulfonamide (dull yellow solid, m.p. 108-110°C, retention time 21.35 min, same conditions).

## REFERENCE EXAMPLE 39

### 4-*tert*-Butyl-N-(4-bromo-3-methyl-5-isoxazolyl)benzenesulfonamide

A solution of 5-amino-4-bromo-3-methylisoxazole (354 mg, 2.0 mmol) in dry THF (1 ml) was added to a suspension of sodium hydride (60% dispersion in mineral oil, 188 mg, 4.4 mmol) in dry THF (1 ml) at 0-5° C. After stirring at 0-5° C for 10 min., the reaction was warmed to room temperature for 10 min. to complete the reaction. The reaction mixture was re-cooled to 0° C and 4-*tert*-butylbenzenesulfonyl chloride (512 mg, 2.2 mmol) was added slowly. Stirring was continued for 20 min. at 0-5° C. Excess sodium hydride was decomposed by addition of methanol (0.4 ml) followed by water (0.5 ml). The mixture was acidified with hydrochloric acid and extracted with dichloromethane. The extract was dried over anhydrous magnesium sulfate and the solvent was removed under reduced pressure to give a crude product, which was purified by recrystallization from ethyl acetate/hexanes to give a white solid in 21% yield, m.p. 170° C (dec.).

## REFERENCE EXAMPLE 40

### N-(3,4-Dimethyl-5-isoxazolyl)-2-methylbenzo[b]thiophene-3-sulfonamide

#### A. 2-Methylbenzo[b]thiophene

t-BuLi (1.7 M, 26 mmoles, 15 ml) was added to a stirred solution of benzo[b]thiophene (17 mmoles, 2.3 g) and THF (30 ml) at -50°C. The resulting bright yellow reaction mixture was warmed to -30°C, and iodomethane (26 mmoles, 1.6 ml) was added. After 10 min. at -30°C, the solution was warmed to ambient temperature and stirred an additional 30 min., then diluted with ether (100 ml) and washed with brine (2 x 100 ml). The organic phase was dried ($MgSO_4$), filtered and concentrated to collect 2.48 g (98%) of 2-methylbenzo[b]thiophene as a light yellow solid.

#### B. 2-Methylbenzo[b]thiophene-3-sulfonyl chloride

Sulfuryl chloride (9.5 mmoles, 0.76 ml) was added to a stirred solution of dimethylformamide (DMF; 11.2 mmoles, 0.87 ml) at 0°C, and the resulting faint yellow solution was stirred for 20 min at 0°C. 2-Methylbenzo[b]thiophene (5.6 mmoles, 0.83 g) was then added, the reaction mixture was diluted with 2 ml of DMF, and then heated to 85°C. After 2.5 hrs at 85°C, the brown reaction mixture was cooled to ambient temperature and added to ice (≈ 100 ml). The aqueous phase was extracted with ethyl acetate (100 ml), and the organic phase was dried ($MgSO_4$), filtered and concentrated to collect an orange-brown solid. Flash chromatography (4% ethyl acetate/hexanes) provided 0.89 g (64%) of 2-methylbenzo[b]thiophene-3-sulfonyl chloride as a yellow solid.

#### C. N-(3,4-Dimethyl-5-isoxazolyl)-2-methylbenzo[b]thiophene-3-sulfonamide

2-Methylbenzo[b]thiophene-3-sulfonyl chloride (1.7 mmoles, 0.41 g) was added to a solution of 3,4-dimethyl-5-aminoisoxazole (0.75 mmoles, 84 mg), 4-dimethylaminopyridine (DMAP; 50 mg) and pyridine (5 ml) at ambient temperature. After 24 h, the reaction mixture was diluted with ethyl acetate (50 ml) and washed with 2% HCl (3 x 50 ml). The organic phase was dried ($MgSO_4$), filtered and concentrated to collect a brown-orange solid, which was dissolved in a solution of methanol (10 ml) and NaOH (60 mg). The solution was stirred 1 h at ambient temperature, then the methanol was evaporated and the resulting residue was diluted with 2% HCl (50 ml), and extracted with ethyl acetate (75 ml). The organic phase was dried ($MgSO_4$), filtered and concentrated to collect a tan solid. Recrystallization from chloroform and hexanes resulted in 93 mg (38%) of N-(3,4-dimethyl-5-isoxazolyl)-2-methylbenzo[b]thiophene-3-sulfonamide as light yellow crystals,
m.p. 174-176°C.

## REFERENCE EXAMPLE 41

### N-(4-Bromo-3-methyl-5-isoxazolyl)-2-methylbenzo[b]thiophene-3-sulfonamide

NaH (60% oil dispersion, 2.5 mmoles, 100 mg) was added to a solution of 4-bromo-3-methyl-5-aminoisoxazole (1.0 mmoles, 0.177 g). THF (5 ml) at 0°C was added, and the resulting reaction mixture was stirred 10 min at 0°C. 2-Methylbenzo[b]thiophene-3-sulfonyl chloride (1.2 mmoles, 0.28 g) was added, and the reaction mixture was stirred for 20 min at 0°C, then warmed to ambient temperature for 1 hr followed by addition of 2 ml of water. The mixture was diluted with ethyl acetate (100 ml) and washed with 2% HCl (2 x 50 ml), then brine (50 ml). The organic phase was

dried ($MgSO_4$), filtered and concentrated. Recrystallization of the crude reaction mixture resulted in 0.24 g (63%) of N-(4-bromo-3-methyl-5-isoxazolyl)-2-methylbenzo[b]thiophene-3-sulfonamide as an off-white solid, m.p. 131-133°C.

**REFERENCE EXAMPLE 42**

**N-(4-Bromo-3-methyl-5-isoxazolyl)-2-ethylbenzo[b]thiophene-3-sulfonamide**

**A. 2-Ethylbenzo[b]thiophene**

2-Ethylbenzo[b]thiophene was prepared by the method of Reference Example 40A with benzo[b]thiophene (7.5 mmoles, 1.0 g), t-BuLi (1.7 M, 8.9 mmoles, 5.3 ml), iodoethane (8.9 mmoles, 0.72 ml) and THF (20 ml). 1.2 g (99%) of a yellow liquid was isolated.

**B. 2-Ethylbenzo[b]thiophene-3-sulfonyl chloride**

2-Ethylbenzo[b]thiophene-3-sulfonyl chloride compound was prepared by the method of Reference Example 40B with dimethylformamide (DMF; 13.6 mmoles, 1.1 ml), sulfonyl chloride (11.5 mmoles, 0.93 ml). Flash chromatography (2% ethyl acetate/hexanes) provided 1.34 g (76%) of a light yellow solid.

**C. N-(4-bromo-3-methyl-5-isoxazolyl)-2-ethylbenzo[b]thiophene-3-sulfonamide**

N-(4-bromo-3-methyl-5-isoxazolyl)-2-ethylbenzo[b]thiophene-3-sulfonamide was prepared by the method of Reference Example 41 with 4-bromo-3-methyl-5-aminoisoxazole (1.0 mmoles, 0.177 g), NaH (2.5 mmoles, 100 mg), 2-ethylbenzo[b]thiophene-3-sulfonyl chloride (1.2 mmoles, 0.31 g) and THF (7 ml). Recrystallization from chloroform and hexanes provided 0.24 g (60%) of a tan crystalline solid, m.p. 118.5-120°C.

**REFERENCE EXAMPLE 43**

**N-(4-Bromo-3-methyl-5-isoxazolyl)-2-benzylbenzo[b]thiophene-3-sulfonamide**

**A. 2-Benzylbenzo[b]thiophene**

2-Benzylbenzo[b]thiophene was prepared by the method of Reference Example 40A with benzo[b]thiophene (7.5 mmoles, 1.0 g), t-BuLi (1.7 M, 11.2 mmoles, 6.6 ml), benzyl bromide (10.2 mmoles, 1.3 ml) and THF (20 ml). Flash chromatography (hexanes) provided 0.66 g (39%) of a yellow solid.

**B. 2-Benzylbenzo[b]thiophene-3-sulfonyl chloride**

2-Benzylbenzo[b]thiophene-3-sulfonyl chloride was prepared by the method of Reference Example 40B with DMF (5.4 mmoles, 0.41 ml), sulfuryl chloride (4.6 mmoles, 0.37 ml) and 2-benzylbenzo[b]thiophene. Flash chromatography (5% ethyl acetate/hexanes) provided 0.55 g (64%) of a yellow solid.

**C. N-(4-bromo-3-methyl-5-isoxazolyl)-2-benzylbenzo[b]thiophene-3-sulfonamide**

N-(4-bromo-3-methyl-5-isoxazolyl)-2-benzylbenzo[b]thiophene-3-sulfonamide was prepared by the method of Reference Example 41 with 4-bromo-3-methyl-5-aminoisoxazole (1.0 mmoles, 0.177 g), NaH (2.5 mmoles, 100 mg), 2-benzylbenzo[b]thiophene-3-sulfonyl chloride (1.2 mmoles, 0.39 g) and THF (7 ml). Flash chromatography (5% methanol/chloroform) followed by recrystallization from chloroform and hexanes provided 0.11 g (24%) of a tan crystalline solid,
m.p. 120-123°C.

**REFERENCE EXAMPLE 44**

**N-(4-Bromo-3-methyl-5-isoxazolyl)-2-butylbenzo[b]thiophene-3-sulfonamide**

**A. 2-Butylbenzo[b]thiophene**

2-n-Butylbenzo[b]thiophene was prepared by the method of Reference Example 40A with benzo[b]thiophene

(7.5 mmoles, 1.0 g), t-BuLi (1.7 M, 9.7 mmoles, 5.7 ml), 1-bromobutane (9.7 mmoles, 1.0 ml) and THF (20 ml). 0.65 g (46%) of a yellow liquid was isolated.

**B. 2-n-Butylbenzo[b]thiophene-3-sulfonyl chloride**

2-n-Butylbenzo[b]thiophene-3-sulfonyl chloride was prepared by the method of Reference Example 40B with DMF (6.6 mmoles, 0.51 ml), sulfuryl chloride (5.6 mmoles, 0.45 ml) and 2-n-butylbenzo[b]thiophene (3.3 mmoles, 0.63 g). Flash chromatography (2% ethyl acetate/hexanes) provided 0.68 g (71 %) of an orange solid.

**C. N-(4-bromo-3-methyl-5-isoxazolyl)-2-n-butylbenzo[b]thiophene-3-sulfonamide**

N-(4-bromo-3-methyl-5-isoxazolyl)-2-n-butylbenzo[b]thiophene-3-sulfonamide was prepared by the method of Reference Example 41 with 4-bromo-3-methyl-5-aminoisoxazole (1.0 mmoles, 0.177 g), NaH (2.5 mmoles, 100 mg), 2-n-butylbenzo[b]thiophene-3-sulfonyl chloride (1.2 mmoles, 0.35 g) and THF (6 ml). Recrystallization from ethyl acetate and hexanes provided 0.24 g (56%) of a yellow solid, m.p. 124.5-126°C.

**REFERENCE EXAMPLE 45**

**N-(4-Bromo-3-methyl-5-isoxazolyl)-2-n-propylbenzo[b]thiophene-3-sulfonamide**

**A. 2-n-propylbenzo[b]thiophene**

2-n-Propylbenzo[b]thiophene was prepared by the method of Reference Example 40A with benzo[b]thiophene (7.5 mmoles, 1.0 g), t-BuLi (1.7 M, 9.7 mmoles, 5.7 ml), n-bromopropane (9.7 mmoles, 0.88 ml) and THF (20 ml). 1.11 g (85%) of a light yellow liquid was isolated.

**B. 2-propylbenzo[b]thiophene-3-sulfonyl chloride**

2-Propylbenzo[b]thiophene-3-sulfonyl chloride was prepared by the method of Reference Example 40B with DMF (3.6 mmoles, 0.28 ml), sulfuryl chloride (3.1 mmoles, 0.25 ml) and 2-propylbenzo[b]thiophene (1.8 mmoles, 0.32 g). Flash chromatography (3% ethyl acetate/hexanes) provided 0.28 g (56%) of a yellow solid.

**C. N-(4-bromo-3-methyl-5-isoxazolyl)-2-n-propylbenzo[b]thiophene-3-sulfonamide**

N-(4-bromo-3-methyl-5-isoxazolyl)-2-n-propylbenzo[b]thiophene-3-sulfonamide was prepared by the method of Reference Example 41 with 4-bromo-3-methyl-5-aminoisoxazole (0.68 mmoles, 0.12 g) NaH (1.7 mmoles, 6.8 mg), 2-n-propylbenzo[b]thiophene-3-sulfonyl chloride (0.82 mmoles, 0.23 g) and THF (3 ml). Recrystallization from chloroform and hexanes provided 0.19 g (67%) of a yellow crystalline solid, m.p. 136-138°C.

**REFERENCE EXAMPLE 46**

**N-(4-Bromo-3-methyl-5-isoxazolyl)-2-*i*-propylbenzo[b]thiophene-3-sulfonamide**

**A. 2-*i*-propylbenzo[b]thiophene**

2-*i*-Propylbenzo[b]thiophene was prepared by the method of Reference Example 40A with benzo[b]thiophene (7.5 mmoles, 1.0 g), t-BuLi (1.7 M, 11.2 mmoles, 6.6 ml), 2-iodopropane (11.2 mmoles, 1.12 ml) and THF (20 ml) with stirring at ambient temperature for 24 hrs. It was isolated as a yellow oil (1.11 g; 85% yield).

**B. 2-*i*-propylbenzo[b]thiophene-3-sulfonyl chloride**

2-*i*-propylbenzo[b]thiophene-3-sulfonyl chloride compound was prepared by the method of Reference Example 40B with DMF (5.2 mmoles, 0.40 ml), using sulfuryl chloride (4.2 mmoles, 0.34 ml) and 2-*i*-propylbenzo[b]thiophene (2.1 mmoles, 0.37 g). Flash chromatography (1% ethyl acetate/hexanes) provided 0.17 g (29%) of a yellow solid.

**C. N-(4-bromo-3-methyl-5-isoxazolyl)-2-*i*-propylbenzo[b]thiophene-3-sulfonamide**

N-(4-bromo-3-methyl-5-isoxazolyl)-2-*i*-propylbenzo[b]thiophene-3-sulfonamide was prepared by the method

of Reference Example 41 using 4-bromo-3-methyl-5-aminoisoxazole (0.55 mmoles, 9.7 mg), NaH (1.4 mmoles, 5.5 mg), 2-*i*-propylbenzo[b]thiophene-3-sulfonyl chloride (0.60 mmoles, 0.17 g) and THF (2 ml). Recrystallization from chloroform and hexanes provided 89 mg (39%) of a tan crystalline solid, m.p. 157.5-159°C.

## REFERENCE EXAMPLE 47

### N-(4-Bromo-3-methyl-5-isoxazolyl)-2-(4-ethylbenzyl)benzo[b]-thiophene-3-sulfonamide

### A. α-(2-benzo[b]thiophene)-4-ethylbenzyl alcohol

α-(2-Benzo[b]thiophene)-4-ethylbenzyl alcohol was prepared by the method of Reference Example 40A with benzo[b]thiophene (7.5 mmoles, 1.0 g), t-BuLi (9.7 mmoles, 1.7 M, 5.7 ml), 4-ethylbenzaldehyde (8.9 mmoles, 1.22 ml) and THF (20 ml). Flash chromatography (10% ethyl acetate/hexanes) provided 1.79 g (89%) of a yellow solid.

### B. 2-(4-ethylbenzyl)benzo[b]thiophene

To a solution of α-(2-benzo[b]thiophene)-4-ethylbenzyl alcohol (4.0 mmoles, 1.1 g), triethylsilane (4.4 mmoles, 0.11 ml) and $CH_2Cl_2$ (20 ml) at 0°C was added TFA (8.1 mmoles, 0.62 ml). The solution was stirred 30 min at 0°C, then diluted with ether (100 ml) and washed with sat. $NaHCO_3$ (100 ml). The organic phase was dried ($MgSO_4$), filtered and concentrated. Flash chromatography (2% ethyl acetate/hexanes) provided 0.69 g (68%) of a white solid.

### C. 2-(4-Ethylbenzyl)benzo[b]thiophene-3-sulfonyl chloride

2-(4-Ethylbenzyl)benzo[b]thiophene-3-sulfonyl chloride was prepared by the method of Reference Example 40B with DMF (5.4 mmoles, 0.42 ml), sulfuryl chloride (4.6 mmoles, 0.37 ml) and 2-(4-ethylbenzyl)benzo[b]thiophene (2.7 mmoles, 0.69 g). Flash chromatography (2% ethyl acetate/hexanes) provided 0.43 g (45%) of an orange solid.

### D. N-(4-bromo-3-methyl-5-isoxazolyl)-2-(4-ethylbenzyl)benzo[b]-thiophene-3-sulfonamide

N-(4-bromo-3-methyl-5-isoxazolyl)-2-(4-ethylbenzyl)benzo[b]-thiophene-3-sulfonamide was prepared by the method of Reference Example 41 using 4-bromo-3-methyl-5-aminoisoxazole (1.0 mmoles, 0.177 g), NaH (2.5 mmoles, 100 mg), 2-(4-ethylbenzyl)benzo[b]thiophene-3-sulfonyl chloride (1.2 mmoles, 0.42 g) and THF (6 ml). Flash chromatography (50% ethyl acetate/hexanes) followed by recrystallization from chloroform and hexanes provided 0.21 g (43%) of a tan solid, m.p. 128-130°C.

## INVENTIVE EXAMPLE 48

### N-(4-Bromo-3-methyl-5-isoxazolyl)-2-[3,4-(methylenedioxy)benzyl]-benzo[b]thiophene-3-sulfonamide

### A. α-(2-benzo[b]thienyl)-3,4-(methylenedioxy)benzyl alcohol

α-(2-Benzo[b]thienyl)-3,4-(methylenedioxy)benzyl alcohol was prepared by the method of Reference Example 40A using benzo[b]thiophene (7.5 mmoles, 1.0 g), t-BuLi (1.7 M, 9.7 mmoles, 5.7 ml), piperonal (8.9 mmoles, 1.0 g) and THF (20 ml). Flash chromatography (20% ethyl acetate/hexanes) provided 1.6 g (74%) of a yellow solid.

### B. 2-[3,4-(methylenedioxy)benzyl]benzo[b]thiophene

2-[3,4-(methylenedioxy)benzyl]benzo[b]thiophene was prepared by the method of Reference Example 47B using α-(2-benzo[b]thienyl)-3,4-(methylenedioxy)benzyl alcohol (6.2 mmoles, 1.8 g), triethylsilane (6.8 mmoles, 1.1 ml) $CH_2Cl_2$ (50 ml) and TFA (12.4 mmoles, 0.95 ml). Recrystallization from hexanes provided 1.2 g (73%) of a light orange solid.

### C. 2-[3,4-(methylenedioxy)benzyl]benzo[b]thiophene-3-sulfonyl chloride

2-[3,4-(methylenedioxy)benzyl]benzo[b]thiophene-3-sulfonyl chloride was prepared by the method of Reference Example 40B using DMF (9.1 mmoles, 0.70 ml), sulfuryl chloride (7.7 mmoles, 0.62 ml) and 2-[3,4-(methylenedioxy)benzyl]benzo[b]thiophene (4.6 mmoles, 1.2 g). Flash chromatography (5% ethyl acetate/hexanes) provided 0.71 g (42%) of a light yellow solid.

**D. N-(4-bromo-3-methyl-5-isoxazolyl)-2-[3,4-(methylenedioxy)benzyl]-benzo[b]thiophene-3-sulfonamide**

N-(4-bromo-3-methyl-5-isoxazolyl)-2-[3,4-(methylenedioxy)benzyl]-benzo[b]thiophene-3-sulfonamide was prepared by the method of Reference Example 41 using 4-bromo-3-methyl-5-aminoisoxazole (1.0 mmoles, 0.177 g), NaH (2.5 mmoles, 100 mg), 2-[3,4-(methylenedioxy)benzyl]benzo[b]thiophene-3-sulfonyl chloride (1.1 mmoles, 0.40 g) and THF (7 ml). Flash chromatography (50% ethyl acetate/hexanes) followed by recrystallization from chloroform and hexanes provided 0.23 g (45%) of a tan crystalline solid, m.p. 164-165°C.

**REFERENCE EXAMPLE 49**

**N-(4-Bromo-3-methyl-5-isoxazolyl)benzo-2,1,3-thiadiazole-4-sulfonamide**

N-(4-bromo-3-methyl-5-isoxazolyl)benzo-2,1,3-thiadiazole-4-sulfonamide was prepared from 5-amino-4-bromo-3-methylisoxazole and 2,1,3-thiadiazole-4-sulfonyl chloride according to the procedures described in Reference Example 39. The crude product was purified by recrystallization from ethyl acetate/hexanes to give a crystalline solid, m.p. 177-179° C, yield 34%.

**REFEREMCE EXAMPLE 50**

**N-(4-Bromo-3-methyl-5-isoxazolyl)-1-methylindole-2-sulfonamide**

**A. 2-Methylindole-2-sulfonyl chloride**

2-Methylindole-2-sulfonyl chloride was prepared by the method of Reference Example 34 with 1-methylindole (7.8 mmols, 1.0 ml), t-BuLi (1.7 m, 9.4 mmols, 5.5 ml), sulfur dioxide, NCS (8.6 mmols, 1.2 g) and THF (15 ml). Flash chromatography (2% ethyl acetate/hexanes) provided 0.66 g (36%) of a yellow solid.

**B. N-(4-bromo-3-methyl-5-isoxazolyl)-1-methylindole-2-sulfonamide**

N-(4-bromo-3-methyl-5-isoxazolyl)-1-methylindole-2-sulfonamide was prepared by the method of Reference Example 41 with 4-bromo-3-methyl-5-aminoisoxazole (1.0 mmols, 0.18 g), NaH (2.5 mmols, 60 mg), 1-methylindole-2-sulfonyl chloride (1.2 mmols, 0.26 g) and THF (7 ml). Recrystallization from chloroform and hexane provided 0.28 g (77%) of a brown solid, m.p. 132-134° C.

**REFERENCE EXAMPLE 51**

**N-(3,4-Dimethyl-5-isoxazolyl)-2-dibenzofuransulfonamide**

**A. 2-Dibenzofuransulfonyl chloride**

2-Dibenzofuransulfonic acid (12.8 mmol) was heated at 70° C with phosphorus oxychloride (1.30 ml, 14.0 mol) for 2 h. Excess phosphorus oxychloride was removed under reduced pressure. The residue was decomposed with ice water and extracted with ethyl acetate. The extract was washed with 5% sodium bicarbonate solution, dried over anhydrous magnesium sulfate and concentrated to yield 2.9 g crude 2-dibenzofuransulfonyl chloride.

**B. N-(3,4-Dimethyl-5-isoxazolyl)-2-dibenzofuransulfonamide**

The 2-benzofuransulfonyl chloride from step (a) was added to a solution of 5-amino-3,4-dimethylisoxazole (250 mg, 2.2 mmol) and 4-(dimethyl)aminopyridine (5 mg) in dry pyridine (2.0 ml). The reaction mixture was stirred at room temperature for 4 h. Pyridine was removed under reduced pressure and the residue was partitioned between water and ethyl acetate. The organic layer was washed with 1N HCl (2 X 25 ml), brine (25 ml) and dried over anhydrous magnesium sulfate. Evaporation of the solvents left an oily residue that, after purification by column chromatography over silica gel (1% methanol in chloroform as eluent), yielded white solid (32% yield). Purification was achieved by recrystallization from chloroform/hexanes to give a white "cotton-like" solid, m.p. 173-175° C (dec.).

**REFERENCE EXAMPLE 52**

**N-(4-chloro-3-methyl-5-isoxazolyl)-2-[2-(3,4-methylenedioxy)phenyl]-ethoxycarbonyl-3-sulfonamide**

N-(4-chloro-3-methyl-5-isoxazolyl)-2-[2-(3,4-methylenedioxy)-phenyl]ethoxycarbonyl-3-sulfonamide was prepared by the same method as described in Reference Example 97 with the exception that 2-(3,4-methylenedioxy) phenylethanol was used instead of sesamol. The final product was obtained by HPLC purification as a yellowish oil, (500mg, 25% yield).

**REFERENCE EXAMPLE 53**

**N-(4-Bromo-3-methyl-5-isoxazolyl)-2-phenylthiophene-3-sulfonamide**

**A. 3-bromo-2-phenyl-thiophene**

Tetrakis (triphenylphosphine) palladium (400 mg), $Na_2CO_3$ (4 M, 80 ml, 320 mmol) and phenylboric acid (3.81 g, 30.3 mmol) as a solution in ethanol (80 ml) were sequentially added to a solution of 2,3-dibromothiophene (7.33 g, 30.3 mmol) in benzene (100 ml). The mixture was heated at reflux for 12 hours. The aqueous layer of the crude mixture was removed and the organic layer was diluted with $Et_2O$ (200 ml), washed with 1N NaOH (2 x 150 ml) and was dried ($MgSO_2$), filtered and the soluent was evaporated. The residue was chromatographed using hexane as the eluent to give 3-bromo-2-phenylthiophene as a clear oil (3.31 g, 47% yield).

**B. 2-Phenylthiophene-3-sulfonylchloride**

n-BuLi (2.38 M, 11.5 ml, 27.28 mmol) was slowly added to a solution of 3-bromo-2-phenyl-thiophene (22.73 mmol) in ether (50 ml) at 0° C. The reaction was stirred at 0° C for 1 h. $SO_2$ was bubbled through the mixture for 15 minutes at 0 °C followed by the addition of NCS (3.95 g, 29.55 mmol) as a suspension in THF (20 ml). The crude products were purified by column chromatography (hexanes) to give 2-phenylthiophene-3-sulfonylchloride as a white solid (1.23 g, 34% yield).

**C. N-(4-bromo-3-methyl-5-isoxazolyl)-2-phenylthiophene-3-sulfonamide**

N-(4-bromo-3-methyl-5-isoxazolyl)-2-phenyl-thiophene-3-sulfonamide was prepared from 2-phenyl-3-thiophene sulfonylchloride using the method described in Reference Example 1. The product was purified by HPLC, 77% yield, reddish solid, mp 86-89° C.

**REFERENCE EXAMPLE 54**

**3-Phenoxy-N-(4-bromo-3-methyl-5-isoxazolyl)thiophene-2-sulfonamide**

**A. 3-Phenoxythiophene.**

Cuprous chloride (3.08 g, 31.1 mmol) and phenol (8.78 g, 93.3 mmol) were sequentially added to a solution of 3-bromothiophene (5.06 g, 31.1 mmol) in pyridine (150 ml). Sodium hydride (3.73 g, 93.3 mmol, 60% dispersion in mineral oil) was then slowly added. The reaction was heated at reflux for 20 hours under Argon. The pyridine was removed under reduced pressure. The residue was diluted with $Et_2O$ (200 ml) and washed with 1 N NaOH (3 x 100 ml), 1 N HCl (2 x 150 ml) and 1 N NaOH (150 ml). The organic layer was dried ($MgSO_4$), filtered, and the solvent was evaporated. The residue was chromatographed using hexanes to give 3-phenoxy-thiophene as a clear oil (4.0 g, 74% yield).

**B. 3-Phenoxythiophene-2-sulfonyl chloride**

BuLi (2.38 M, 11.5 ml, 27.28 mmol) was slowly added to a solution of 3-phenoxythiophene (4.0 g. 22.73 mmol) in ether (50 ml) at 0° C. The reaction was stirred at 0° C for 1 h. $SO_2$ was bubbled through the mixture for 15 minutes at 0 °C followed by the addition of NCS (3.95 g, 29.55 mmol) as a suspension in THF (20 ml). The mixture was allowed to warm up to 25° C and stirred for 2 more h. The precipitate was filtered, and the filtrate was concentrated and chromatographed (hexanes) to give 3-phenoxythiophene-2- sulfonyl chloride as a yellowish solid (1.03 g, 17% yield).

**C. N-(4-bromo-3-methyl-5-isoxazolyl)-3-phenoxythiophene-2-sulfonamide**

N-(4-bromo-3-methyl-5-isoxazolyl)-3-phenoxythiophene-2-sulfonamide was prepared from 3-phenoxythiophene-2-sulfonyl chloride and 5-amino-4-bromo-3-methylisoxazole using the method described in Reference Example 1. The product was recrystallized from acetonitrile/$H_2O$ to give a solid m.p. 121-123°C, 61% yield.

**REFERENCE EXAMPLE 55**

**N-(4-Bromo-3-methyl-5-isoxazolyl)-2-[N-(4-isopropylphenyl)aminocarbonyl]-thiophene-3-sulfonamide**

N-(4-bromo-3-methyl-5-isoxazolyl)-2-[N-(4-isopropylphenyl)-aminocarbonyl]thiophene-3-sulfonamide was prepared in the same manner as described in Reference Example 24 from N-(4-bromo-3-methyl-5-isoxazolyl)-2-(carboxyl)thiophene-3-sulfonamide and 4-isopropylaniline in 19% yield. The crude product was passed through silica gel column using ethyl acetate as eluent. This was further purified by HPLC (5% $CH_3CN$ to 100% $CH_3CN$ over 30 min.) to give a solid.

**REFERENCE EXAMPLE 56**

**N-(4-Bromo-3-methyl-5-isoxazolyl)-2-[N-(4-sec-butylphenyl)aminocarbonyl]thiophene-3-sulfonamide**

N-(4-bromo-3-methyl-5-isoxazolyl)-2-[N-(4-sec-butylphenyl)aminocarbonyl]thiophene-3-sulfonamide was prepared in the same manner as described in Reference Example 24 from N-(4-bromo-3-methyl-5-isoxazolyl)-2-(carboxyl)thiophene-3-sulfonamide and 4-sec-butylaniline in 25% yield. The crude product was passed through silica gel column using ethyl acetate as eluent. This was further purified by HPLC (5% $CH_3CN$ to 100% $CH_3CN$ over 30 min.) give a solid, m.p. 205 - 208°C.

**REFERENCE EXAMPLE 57**

**N-(4-Bromo-3-methyl-5-isoxazolyl)-2-[N-(4-*tert*-butylphenyl)aminocarbonyl]thiophene-3-sulfonamide**

N-(4-bromo-3-methyl-5-isoxazolyl)-2-[N-(4-*tert*-butylphenyl)-aminocarbonyl]thiophene-3-sulfonamide was prepared in the same manner as described in Reference Example 24 from N-(4-bromo-3-methyl-5-isoxazolyl)-2-(carboxyl)thiophene-3-sulfonamide and 4-*tert*-butylaniline in 28% yield. The crude product was passed through silica gel column using ethyl acetate as eluent. This was further purified by HPLC (5% $CH_3CN$ to 100% $CH_3CN$ over 30 min.) give a solid, m.p. 76 - 86°C.

**REFERENCE EXAMPLE 58**

**N-(4-Bromo-3-methyl-5-isoxazolyl)-2-[N-(4-butylphenyl)aminocarbonyl]-thiophene-3-sulfonamide**

N-(4-bromo-3-methyl-5-isoxazolyl)-2-[N-(4-butylphenyl)aminocarbonyl]thiophene-3-sulfonamide was prepared in the same manner as described in Reference Example 24 from N-(4-bromo-3-methyl-5-isoxazolyl)-2-(carboxyl)thiophene-3-sulfonamide and 4-butylaniline in 18% yield. The crude product was passed through silica gel column using ethyl acetate as eluent. This was further Purified by HPLC (5% $CH_3CN$ to 100% $CH_3CN$ over 30 min.) give a solid.

**REFERENCE EXAMPLE 59**

**N-(4-bromo-3-methyl-5-isoxazolyl)thiazole-2-sulfonamide**

**A. Thiazole-2-sulfonyl chloride**

Thiazole (0.51 g, 6 mmol) was dissolved in THF (5 ml) and cooled to -78°C under argon atmosphere. n-Butyllithium (2.5 M solution in hexane, 2.4 ml, 6 mmol) was added dropwise under constant stirring. The resultant reaction mixture was stirred at -78°C for 40 min. Sulfur dioxide was bubbled through the reaction mixture for 15 min at -78°C. The reaction mixture was allowed to attain ambient temperature slowly and stirred for 30 min. NCS was added and stirring was continued for 30 min. The reaction mixture was diluted with water (50 ml), extracted with ethyl acetate (2 X 50 ml) and the combined organic layers was dried over anhydrous $MgSO_4$. Removal of the solvent under reduced pressure gave crude product which was purified by column chromatography, using hexane as eluent, to give

thiazole-2-sulfonyl chloride as a liquid(0.6 g, 54% yield).

**B. N-(4-bromo-3-methyl-5-isoxazolyl)thiazole-2-sulfonamide**

N-(4-bromo-3-methyl-5-isoxazolyl)thiazole-2-sulfonamide was prepared in the same manner as described in Reference Example 2 from 5-amino-4-bromo-3-methylisoxazole and thiazole-2-sulfonyl chloride in 57% yield. This was purified by HPLC (5% $CH_3CN$ to 100% $CH_3CN$ over 30 min.) to give a solid., m.p. 175 - 177°C.

**REFERENCE EXAMPLE 60**

**N-(4-chloro-3-methyl-5-isoxazolyl)thiazole-2-sulfonamide**

N-(4-chloro-3-methyl-5-isoxazolyl)thiazole-2-sulfonamide was prepared in the same manner as described in Reference Example 2 from 5-amino-4-chloro-3-methylisoxazole and thiazole-2-sulfonyl chloride in 33% yield. This was purified by HPLC (5% $CH_3CN$ to 100% $CH_3CN$ over 30 min.) to give a solid, m.p. 171-173°C.

**REFERENCE EXAMPLE 61**

**N-(3,4-dimethyl-5-isoxazolyl)thiazole-2-sulfonamide**

N-(3,4-methyl-5-isoxazolyl)thioazole-2-sulfonamide was prepared in the same manner as described in Reference Example 14 from 5-amino-3,4-dimethylisoxazole and thiazole-2-sulfonyl chloride in 37% yield. This was purified by HPLC (5% $CH_3CN$ to 100% $CH_3CN$ over 30 min.) give a solid, m.p. 118 - 120°C.

**REFERENCE EXAMPLE 62**

**5-benzyl-N-(4-Bromo-3-methyl-5-isoxazolyl)thiophene-2-sulfonamide**

**A. 1-(2-Thienyl)benzyl alcohol**

Sodium borohydride (0.37 g, 10 mmol) was added to 2-benzoylthiophene (1.88 g, 10 mmol) dissolved in methanol/THF mixture (1:10 ratio, 11 ml). This was stirred at room temperature for 10 h. The reaction mixture was decomposed by addition of saturated ammonium chloride solution (50 ml) and was extracted with ethyl acetate (2 X 50 ml). The combined organic layers was dried over anhydrous $MgSO_4$. Removal of the solvent gave 1-(2-thienyl) benzyl alcohol as a solid (1.75 g, 92% yield).

**B. 2-Benzylthiophene**

Acetic anhydride (5 ml) was added to a solution of 1-(2-thienyl)benzyl alcohol in pyridine. The resultant solution was stirred at 70°C for 3h. Water (50 ml) was added and the reaction mixture was stirred at room temperature for 2h. This was extracted with ethyl acetate (2 X 50 ml) and the combined organic layers dried over anhydrous $MgSO_4$. Removal of the solvent gave crude product, which was purified by passing through silica gel using 3:1 hexane/ethyl acetate mixture to give 1-(2-thienyl)benzyl acetate.

A solution of 1-(2-thienyl)benzyl acetate in THF (5 ml) was added carefully to dry liquid ammonia (100 ml). Lithium metal was added in small portions until the blue color persisted. The resulting reaction mixture was stirred for 30 min, and the reaction was quenched by addition of solid ammonium chloride. The residue, after complete evaporation of liquid ammonia, was dissolved in water (50 ml) and was extracted with methylene chloride (2 X 50 ml). The combined organic layers was. dried over $MgSO_4$ and filtered. Removal of the solvent gave crude product, which was purified by column chromatography using hexane as eluentto give 2-benzylthiophene (1.2 g, 68 % yield).

**C. 5-Benzylthiophene-2-sulfonyl chloride**

To a solution of 2-benzylthiophene (0.875 g, 5 mmol) in chloroform (2 ml) at 0°C was added chlorosulfonic acid dropwise and the reaction was stirred at 0°C for 30 min. The reaction mixture was decomposed by pouring onto crushed ice (20 g). The mixture was extracted with ethyl acetate, dried over $MgSO_4$ and filtered. The solvent was removed under reduced pressure to give 5-benzylthiophene-2-sulfonic acid.

Phosphorous pentachloride (2.08 g, 40 mmol) was added to a solution of 5-benzylthiophene-2-sulfonic acid in phosphorous oxychloride (6.0 g, 40 mmol) at 0°C. The reaction mixture was kept at 50°C for 1 h, cooled to room

temperature, then poured onto crushed ice (50 g) and extracted with ethyl acetate (2 X 30 ml). Removal of the solvent under reduced pressure gave a crude product, which was purified by column chromatography using 3% ethyl acetate in hexane to give 2-benzylthiophene-5-sulfonyl chloride (0.6 g, 39 % yield).

**D. N-(4-bromo-3-methyl-5-isoxazolyl)-5-benzylthiophene-2-sulfonamide**

N-(4-Bromo-3-methyl-5-isoxazolyl)-5-benzylthiophene-2-sulfonamide was prepared in the same manner as described in Reference Example 2 from 5-amino-4-bromo-3-methylisoxazole and 5-benzyl-2-thiophenesulfonyl chloride in 22% yield. The product was purified by HPLC (5% $CH_3CN$ to 100% $CH_3CN$ over 30 min.) to give a solid, m.p. 49 - 50°C.

**REFERENCE EXAMPLE 63**

**N-(4-Bromo-3-methyl-5-isoxazolyl)-3-phenethylthiophene-2-sulfonamide**

**A. 1-(3-Thienyl)phenethyl alcohol**

Benzyl bromide (25.65 g, 150 mmol) was added dropwise over 8 h to a suspension of magnesium (3.6 g, 150 mmol) in ether (75 ml) dissolved in ether (30 ml). The resulting mixture was cooled to -10°C. 3-thiophenecarboxaldehyde in ether (45 ml) over 30 min was then added and the resultant reaction mixture was stirred at room temperature for 6 h. This was cooled to 0°C and the reaction mixture was decomposed by addition of 0.1 N HCl. The ether layer was separated and the aqueous phase was extracted with ethyl acetate (2 X 50 ml). The combined organic layers was dried over $MgSO_4$ and filtered. Removal of the solvent gave 1-(3-thienyl)phenethyl alcohol (16 g, 78% yield).

**B. 1-(3-Thienyl)phenethyl acetate**

1-(3-Thienyl)phenethyl alcohol (10 g, 49 mmol) was dissolved in a 2:1 pyridine and acetic anhydride mixture (50 ml). This was stirred at 80°C for 4 h. Excess of pyridine and acetic anhydride mixture was removed under reduced pressure and the residue was dissolved in water (100 ml). This was extracted with methylene chloride (3 X 75 ml) and the combined organic layers was dried over $MgSO_4$ and filtered. Removal of the solvent gave 1-(3-thienyl)phenethyl acetate (10.2 g, 84% yield).

**C. 3-Phenethylthiophene**

1-(3-thienyl)phenylethyl acetate dissolved in THF (20 ml) was added carefully to dry liquid ammonia (300 ml). Lithium metal was added in small portions until the blue color persisted. The resulting reaction mixture was stirred for 30 min and the reaction was quenched by addition of solid ammonium chloride. The residue, after the complete evaporation of liquid ammonia, was dissolved in water (100 ml) and was extracted with methylene chloride (4 X 50 ml). The combined organic layers was dried over $MgSO_4$ and filtered. Removal of the solvent gave a crude product, which was purified by column chromatography using hexane followed by mixture of ethyl acetate in hexane as eluent to give 3-phenethylthiophene (3.2 g, 34 % yield) and 1-(3-thienyl)phenethyl acetate (starting material, 7g).

**D. 3-Phenethylthiophene-2-sulfonyl chloride and 4-phenethylthiophene-2-sulfonyl chloride**

3-Phenethylthiophene (0.94 g, 5 mmol) was dissolved in THF (12 ml) and cooled to -78°C under argon atmosphere. n-Butyllithium (2.5 M solution in hexane, 4.4 ml, 5.5 mmol) was added dropwise with constant stirring under an argon atmosphere. The resultant reaction mixture was stirred at -10°C to 0°C for 3 h, cooled to -78°C and sulfur dioxide was bubbled through the reaction mixture for 15 min. The reaction mixture was allowed to attain ambient temperature slowly and stirring continued for 30 min. NCS (1 g) was added and stirring was continued for 1 h. The reaction mixture was diluted with water (50 ml), extracted with methylene chloride (2 X 50 ml) and the combined organic layers was dried over anhydrous $MgSO_4$. Removal of the solvent under reduced pressure gave a crude product which was purified by column chromatography, using 0.2% ethyl acetate in hexane as eluent, to give 3-phenethyl-2-thiophenesulfonyl chloride (0.06 g, 4% yield) and 4-phenethyl-2-thiophenesulfonyl chloride (0.72 g, 45% yield).

**E. N-(4-bromo-3-methyl-5-isoxazolyl)-3-phenethylthiophene-2-sulfonamide**

N-(4-bromo-3-methyl-5-isoxazolyl)-3-phenethylthiophene-2-sulfonamide was prepared in the same manner as described in Reference Example 2 from 5-amino-4-bromo-3-methylisoxazole and 3-phenethyl-2-thiophenesulfonyl

chloride in 48% yield. This was purified by HPLC (5% $CH_3CN$ to 100% $CH_3CN$ over 30 min.) to give a solid.

**REFERENCE EXAMPLE 64**

**N-(4-Bromo-3-methyl-5-isoxazolyl)-4-phenethylthiophene-2-sulfonamide**

N-(4-bromo-3-methyl-5-isoxazolyl)-4-phenethylthiophene-2-sulfonamide was prepared in the same manner as described in Reference Example 2 from 5-amino-4-bromo-3-methylisoxazole and 4-phenethyl-2-thiophenesulfonyl chloride in 32% yield. This was purified by HPLC (5% $CH_3CN$ to 100% $CH_3CN$ over 30 min.) to give a gum.

**REFERENCE EXAMPLE 65**

**N-(4-Bromo-3-methyl-5-isoxazolyl)-5-(3-methoxyphenyl)thiophene-2-sulfonamide**

**A. 5-bromothiophene-2-sulfonyl chloride**

Chlorosulfonic acid was added dropwise over 20 min. to a cold solution (-78°C) of 2-bromothiophene (16.3 g, 100 mmol) in methylene chloride (50 ml) was added After addition of chlorosulfonic acid was complete, the cold bath was removed. The reaction mixture was allowed to attain room temperature slowly (2 h), was added dropwise onto the crushed ice (1000 g) and was extracted with methylene chloride (4 X 100 ml). The combined organic layers was dried over $MgSO_4$, filtered and the solvent was removed under reduced pressure to give a crude product. This was purified by column chromatography using hexane as eluent to give 5-bromothiophene-2-sulfonyl chloride (22 g, 75% yield).

**B. N-(5-bromothiophene-2-sulfonyl)pyrrole**

N-(5-bromothiophene-2-sulfonyl)pyrrole was prepared in the same manner as described in Reference Example 33A from 5-bromothiophene-2-sulfonyl chloride and pyrrole in 88% yield. This was purified by recrystallization using hexane/ethyl acetate as a solvent.

**C. 3-Methoxyphenylboronic acid**

3-Methoxyphenylboronic acid was prepared in the same manner as described in Reference Example 33B from 3-bromoanisole and triisopropyl borate in 82% yield. This was used in the next step without any further purification.

**D. N-[5-(3-methoxyphenyl)thiophene-2-sulfonyl]pyrrole**

N-(5-(3-methoxyphenyl)thiophene-2-sulfonyl]pyrrole was prepared in the same manner as described in Reference Example 32C from 3-methoxyphenylboronic acid and N-(5-bromothiophene-2-sulfonyl)pyrrole in 93% yield. This was purified by recrystallization using hexane/ethyl acetate as solvent.

**E. 5-(3-Methoxyphenyl)thiophene-2-sulfonyl chloride**

To a suspension of N-[5-(3-methoxyphenyl)thiophene-2-sulfonyl]pyrrole (1.4 g, 4.5 mmol) in ethanol (15 ml) was added 6 N sodium hydroxide solution (15 ml) and the resultant reaction mixture refluxed for 14 h. The reaction mixture was cooled to room temperature. Ethanol was removed under reduced pressure and the resultant precipitate was filtered and dried under vacuum (1.1 g, 91 % yield).

Phosphorous pentachloride (2.08 g, 10 mmol) was added to the suspension of sodium slat of sulfonic acid (0.62 g, 2.5 mmol) (obtained from above step) in phosphorousoxy chloride (0.93 ml, 10 mmol) and the resultant reaction mixture stirred at room temperature for 3 h. This was decomposed by adding on to crushed ice and the product was extracted with methylene chloride (2 X 50 ml). The combined organic layers dried over $MgSO_4$ and filtered. Removal of the solvent gave the crude product which was purified by column chromatography using 2% ethyl acetate in hexane to give 5-(3-methoxyphenyl)thiophene-2-sulfonyl chloride (0.51 g, 75%).

**F. N-(4-bromo-3-methyl-5-isoxazolyl)-5-(3-methoxyphenyl)thiophene-2-sulfonamide**

N-(4-bromo-3-methyl-5-isoxazolyl)-5-(3-methoxyphenyl)thiophene-2-sulfonamide was prepared in the same manner as described in Reference Example 2 from 5-amino-4-bromo-3-methylisoxazole and 5-(3-methoxyphenyl)thiophene-2-sulfonyl chloride in 48% yield. This was purified by HPLC (5% $CH_3CN$ to 100% $CH_3CN$ over 30 min.) give a solid.

**REFERENCE EXAMPLE 66**

**N-(4-Bromo-3-methyl-5-isoxazolyl)-5-(2-methylfuranyl)thiophene-2-sulfonamide**

**A. N-(pyrrole)-5-(2-methylfuranyl)thiophene-2-sulfonamide**

t-BuLi (1.7 m solution in hexane, 7.9 ml, 14.6 mmol) was added dropwise under constant stirring under a nitrogen atmosphere to a solution of 2-methyl furan (1.0 g, 12 mmol) in THF (20 ml) at -78° C. The solution was then warmed to -10° C and stirring was continued for 45 min. The solution was then added to a solution of zinc chloride (27 ml of a 0.5 M solution in THF) at -30° C and then warmed to room temperature where stirring continued for 1 hr. resulting in a pale yellow clear solution. The solution was then transferred via a steel canula under nitrogen to a solution of N-(pyrrole)-5-bromothiophene-2-sulfonamide Reference Example 33A, 3.5 g, 12 mmol) and tetrakis(triphenylphosphine)-palladium (0) (693 mg, 0.6 mmol) in THF (15 ml) at -78° C. The solution was then warmed to room temperature and stirred for a period of 2 hours. Purification by column chromatography using 2% ethyl acetate gave 680 mg of N-(pyrrole)-5-(2-methylfuranyl)thiophene-2-sulfonamide as a pale yellow powder (19% yield).

**B. 2-(2-methylfuranyl)thiophene-5-sulfonyl chloride**

2-(2-methylfuranyl)thiophene-5-sulfonyl chloride was prepared in the same manner as described in Reference Example 33D from N-(pyrrole)-5-(2-methylfuranyl)thiophene-2-sulfonamide (300 mg, 1.02 mmol). Purification by column chromatography using 2% ethyl acetate/hexanes gave 145 mg (53%) of the sulfonyl chloride as a pale yellow solid.

**C. N-(4-bromo-3-methyl-5-isoxazolyl)-5-(2-methyl-furanyl)thiophene-2-sulfonamide**

N-(4-bromo-3-methyl-5-isoxazolyl)-5-(2-methyl-furanyl)thiophene-2-sulfonamide was prepared in the same manner as described in Reference Example 2. Reaction of 2-(2-methylfuranyl)thiophene-5-sulfonyl chloride (55 mg, 0.21 mmol) with 5-amino-4-bromo-3-methyl isoxazole (41 mg, 0.21 mmol), after purification by column chromatography using 10% $MeOH/CHCl_3$, gave 45 mg of the pure sulfonamide as a brown semisolid, 54% yield, m.p. 123-124° C.

**REFERENCE EXAMPLE 67**

**N-(4-Bromo-3-methyl-5-isoxazolyl)-5-(4-methoxyphenyl)thiophene-2-sulfonamide**

**A. N-[5-(4-methoxyphenyl)thiophene-2-sulfonyl]pyrrole**

N-[5-(4-methoxyphenyl)thiophene-2-sulfonyl]pyrrole was prepared, in the same manner as described in Reference Example 32C, from 4-methoxyphenylboronic acid and N-(5-bromothiophene-2-sulfonyl)pyrrole. Recrystallization using hexane/ethyl acetate gave a solid in quantative yield.

**B. 5-chlorosulfonyl-2-(4-methoxyphenyl)thiophene**

A solution of N-[5-(4-methoxyphenyl)thiophene-2-sulfonyl]pyrrole (1.4 g, 4.5 mmol) was suspended in ethanol (15 ml). A 6N sodium hydroxide solution was added, and the resulting suspension was refluxed for 14 hr. to give a clear solution. This was cooled to room temperature. Ethanol was removed under reduced pressure. A precipitate was formed on standing at room temperature which was filtered and washed with methylene chloride and dried under vacuum giving a solid (1.2 g, 91%).

The solid (0.67 g, 2.5 mmol) was suspended in phosphorous oxychloride (0.92 ml, 10 mmole) and phosphorous pentachloride (2.08 g, 10 mmole) was added. The resulting mixture was stirred at room temperature for 3 hr. The reaction mixture was decomposed by pouring onto crushed ice (50 g). The mixture was extracted with methylene chloride (2 x 50 ml) and the combined organic layers was dried over $MgSO_4$. Removal of solvent under reduced pressure gave a crude product which was purified by column chromatography using 2% EtOAc in hexane as solvent to give 5-chlorosulfonyl-2-(4-methoxyphenyl)-thiophene (530 mg, 86%).

### C. N-(4-bromo-3-methyl-5-isoxazolyl)-5-(4-methoxyphenyl)thiophene-2-sulfonamide

N-(4-bromo-3-methyl-5-isoxazolyl)-5-(4-methoxyphenyl)thiophene-2-sulfonamide was prepared in the same manner as described in Reference Example 1. Reaction of 5-chlorosulfonyl-2-(4-methoxyphenyl)thiophene with 5-amino-4-bromo-3-methylisoxazole gave N-(4-bromo-3-methyl-5-isoxazolyl)-5-(4-methoxyphenyl)thiophene-2-sulfonamide in 50% yield, m.p. 128-130° C.

## REFERENCE EXAMPLE 68

### N-(4-Bromo-3-methyl-5-isoxazolyl)-5-(3-thienyl)thiophene-2-sulfonamide

### A. 3-Thiopheneboric acid

n-Butyllithium (2.5 M solution in hexane, 20 ml, 50 mmol) was added dropwise to a solution of 3-bromothiophene (8.15 g, 50 mmol) in THF (20 ml) at -78°C under an argon atmosphere. The resulting solution was stirred at -78°C for 45 min, and then added to a solution of triisopropyl borate (9.4 g, 50 mmol) in THF at -78°C over 30 min through a steel cannula. The resulting reaction mixture was stirred at room temperature for 12 h and was decomposed by the addition of 100 ml 1 N HCl. The aqueous layer was extracted with ether (2 X 100 ml) and the combined organic layers was extracted with 1 M NaOH (3 X 30 ml), the aqueous extract was acidified with concentrated HCl to pH 2 and extracted with ether (3 X 50 ml). The combined ether extract was washed once with water, dried over $MgSO_4$ and filtered. Removal of the solvent gave 3-thenylboronic acid as a solid (5.2 g, 80% yield).

### B. N-[5-(3-thienyl)thiophene-2-sulfonyl]pyrrole

N-[5-(3-thienyl)thiophene-2-sulfonyl]pyrrole was prepared in the same manner as described in Reference Example 32C from 3-thienylboronic acid and N-(5-bromothiophene-2-sulfonyl)pyrrole in quantative yield. This was purified by recrystallization using hexane/ethyl acetate as solvent.

### C. 5-(3-Thienyl)thiophene-2-sulfonyl chloride

5-(3-thienyl)thiophene-2-sulfonyl chloride was prepared in the same manner as described in Reference Example 64E from N-[5-(4-methoxyphenyl)thiophene-2-sulfonyl]pyrrole in 74% yield.

### D. N-(4-bromo-3-methyl-5-isoxazolyl)-5-(3-thienyl)thiophene-2-sulfonamide

N-(4-bromo-3-methyl-5-isoxazolyl)-5-(3-thienyl)thiophene-2-sulfonamide was prepared in the same manner as described in Reference Example 2 from 5-amino-4-bromo-3-methylisoxazole and 5-(3-thienyl)thiophene-2-sulfonyl chloride in 40% yield. This was purified by HPLC (5% $CH_3CN$ to 100% $CH_3CN$ over 30 min.) give a solid.

## REFERENCE EXAMPLE 69

### N-(4-Bromo-3-methyl-5-isoxazolyl)furan-2-sulfonamide

N-(4-bromo-3-methyl-5-isoxazolyl)furan-2-sulfonamide was prepared by the method of Reference Example 1 with 5-amino-4-bromo-3-methylisoxazole (0.266 g, 1.5 mmol), NaH (60% oil dispersion) (0.15 g, 3.8 mmol) and furan-2-sulfonyl chloride (Example 36A) (0.30 mg, 1.8 mmol). Flash chromatography (50% EtOAc/hexane) and recrystallization from $CHCl_3$ and hexane provided 90 mg (20% yield) of light yellow crystals (m.p. 117-119°C).

## REFERENCE EXAMPLE 70

### N-(4-bromo-3-methyl-5-isoxazolyl)-5-(phenylthio)furan-2-sulfonamide

### A. 2-(phenylthio)furan

t-BuLi (1.7 m, 10 ml, 1.7 mmol) was added to a solution of furan (1.24 ml, 17 mmol) in 20 ml of THF at -60°C. Thirty minutes later diphenyldisulfide (3.7 g, 17 mmol) was added via cannula in 8 ml of THF. The reaction was warmed to ambient temperature for 30 minutes, then diluted with 150 ml of ether and washed with 3% NaOH (3 x 100 ml). The organic was dried ($MgSO_4$), filtered and concentrated to collect 2.92 g (97% yield) of a light yellow liquid.

**B. 5-phenylthiofuran-2-sulfonyl chloride**

5-phenylthiofuran-2-sulfonyl chloride was prepared by the method of Example 34A with 5-phenylthiofuran (1.5 g, 8.5 mmol), t-BuLi (1.2 m, 8.9 mmol, 5.3 ml) and NCS (1.14 g, 8.5 mmol). Flash chromatography (5% EtOAc/hexane) provided 1.61 g (69% yield) of a yellow-orange liquid.

**C. N-(4-bromo-3-methyl-5-isoxazolyl)-5-(phenylthio)furan-2-sulfonamide**

N-(4-bromo-3-methyl-5-isoxazolyl)-5-(phenylthio)furan-2-sulfonamide was prepared by the method of Reference Example 1 with 4-bromo-3-methyl-2-aminoisoxazole (0.354 g, 2.0 mmol), NaH (60% oil dispersion) (0.20 g, 5.0 mmol) and 5-phenylthiofuran-2-sulfonyl chloride (0.66 g, 2.4 mmol). Flash chromatography (50% EtOAc/hexane) and recrystallization from $CHCl_3$/hexane provided 82 mg (10% yield) of a tan solid (m.p. 90-91.5°C).

**REFERENCE EXAMPLE 71**

**N-(4-Bromo-3-methyl-5-isoxazolyl)-5-phenylfuran-2-sulfonamide**

**A. 2-Phenylfuran**

2-phenylfuran was prepared by the method of Example 32C from 2-bromofuran (0.93 g, 6.3 mmol), sodium carbonate (18 ml of 2 M aqueous solution), phenyl boric acid (0.93 g, 7.6 mmol) and tetrakis (triphenylphosphine) palladium (0) (0.36 g, 0.32 mmol). Flash chromatography with hexane provided 0.79 g (87% yield) of a colorless liquid.

**B. 5-phenylfuran-2-sulfonyl chloride**

5-phenylfuran-2-sulfonyl chloride was prepared by the method of Reference Example 34A with 2-phenylfuran (0.79 g, 5.5 mmol), t-BuLi (1.7 m, 6.0 mmol, 3.6 ml) and NCS (0.73 g, 5.5 mmol). Flash chromatography (5% EtOAc/hexane) provided 0.84 g (63% yield) of a light red solid.

**C. N-(4-bromo-3-methyl-5-isoxazolyl)-5-phenylfuran-2-sulfonamide**

N-(4-bromo-3-methyl-5-isoxazolyl)-5-phenylfuran-2-sulfonamide was prepared by the method of Example 1 with 4-bromo-3-methyl-2-amino isoxazole (0.354 g, 2.0 mmol), NaH (60% oil dispersion) (0.20 g, 5.0 mmol) and 5-phenylfuran-2-sulfonyl chloride (0.58 g, 2.4 mmol). Flash chromatography (50% EtOAc/hexane) and recrystallization from $CHCl_3$/hexane provided 0.23 g (29% yield) of light yellow crystals (m.p. 124-126°C).

**REFERENCE EXAMPLE 72**

**N-(4-Bromo-3-methyl-5-isoxazolyl)-5-(4-isopropylphenyl)thiophene-2-sulfonamide**

**A. 4-Isopropylphenyl boronic acid**

4-Isopropylphenyl boronic acid was prepared in the same manner as described in Reference Example 33B from 1-bromo-4-ethyl benzene. The boronic acid was isolated as a white powder in 63% yield, m.p. 133-135°C.

**B. N-[5-(4-isopropylphenyl)thiophene-2-sulfonyl]pyrrole**

N-[5-(4-isopropylphenyl)thiophene-2-sulfonyl]pyrrole was prepared in the same manner as described in Reference Example 33C, from 4-isopropylphenyl boronic acid and N-(5-bromothiophene sulfonyl)-pyrrole. Purification by column chromatography using 10% ethyl acetate/hexanes gave the pure sulfonamide as an off white colored solid in 84% yield, m.p. 112-114°C.

**C. 5-chlorosulfonyl-2-(4-ethylphenyl)thiophene**

5-chlorosulfonyl-2-(4-ethylphenyl)thiophene was prepared in the same manner as described in Reference Example 33D. Hydrolysis of 526 mg (1.59 mmol) of N-[5-(4-isopropylthiophene)-2-sulfonyl]pyrrole with 6N sodium hydroxide followed by chlorination using phosphorous oxychloride and phosphorous pentachloride gave the crude sulfonyl chloride as dark oil. Flash column chromatography over silica gel using 2% ethyl acetate/hexanes yielded 262

mg (55%) of the pure sulfonyl chloride as a light brown oil.

**D. N-(4-bromo-3-methyl-5-isoxazolyl)-5-(4-isopropylphenyl)thiophene-2-sulfonamide**

N-(4-bromo-3-methyl-5-isoxazolyl)-5-(4-isopropylphenyl)thiophene-2-sulfonamide was prepared in the same manner as described in Reference Example 2. Reaction of 5-chlorosulfonyl-2-(4-isopropyl)thiophene (260 mg, 0.87 mmol) with 5-amino-4-bromo-3-methylisoxazole (161 mg, 0.91 mmol) yielded after flash chromatography using 10% $MeOH/CHCl_3$ a pale brown solid (265 mg) which was further purified using preparative HPLC to give the pure sulfonamide as a light tan colored solid, m.p. 114-116°C.

## REFERENCE EXAMPLE 73

**N-(4-Bromo-3-methyl-5-isoxazolyl)-5-(4-propylphenyl)thiophene-2-sulfonamide**

**A. 1-bromo-4-propylbenzene**

A solution of 1-bromopropane (1.32 g, 0.6 mmol) was added dropwise at room temperature at a rate such that a gentle reflux was maintained to a suspension of magnesium (258 mg, 12 mmol) in dry tetrahydrofuran. The cloudy suspension was stored at room temperature for an additional 30 minutes to produce a gray solution that was then added dropwise over 15 minutes to a mixture of 1-iodo-4-bromobenzene (3.0 g, 10.6 mmol) and tetrakis (triphenylphosphine) palladium (0) in 50 mL of dry benzene at room temperature. The mixture was stirred for 2 hours, diluted with 50 mL of water, the organic layer was separated and the aqueous layer was extracted with ether (2 x 50 mL). The combined organic extracts was dried and evaporated to yield 1.69 g (80%) of a light brown oil, which was used in the next step without further purification.

**B. 4-propylphenyl boronic acid**

To a suspension of magnesium shavings (217 mg, 8.9 mmol) in 3 mL of dry tetrahydrofuran under argon, a crystal of iodine along with a solution of 4-bromopropylbenzene (1.69 g, 8.5 mmol) dissolved in 6 mL of tetrahydrofuran was added at such a rate that a gentle reflux was maintained. The solution was refluxed for an additional 0.5 h, cooled to room temperature and added in portions over 10 min to a solution of trimethylborate (924 mg, 8.9 mmol) previously dissolved in 4 mL of dry ether at -78°C. After 30 minutes, the solution was warmed to room temperature; and stirring was continued for 90 min. The reaction was then quenched by the addition of 2 mL of a 10% hydrochloric acid solution. The tetrahydrofuran was removed under reduced pressure and the remaining residue was extracted into diethyl ether (3 x 25 mL). The combined ether extracts was extracted with 1 M NaOH (3 x 25 mL) and the resulting aqueous layer was acidified to pH 2.0 using 6N HCI, then reextracted back into diethyl ether (3 x 25 mL). The combined organic layers was washed with water (1 x 25 mL), brine (1 x 25 mL) and dried over magnesium sulfate. Evaporation of solvent left a brown solid which was filtered through a small plug of silica gel using 10% $MeOH/CHCl_3$. Evaporation left 448 mg (32%) of a brown solid, m.p. 90-93°C.

**C. N-[5-(4-propylphenyl)thiophene-2-sulfonyl]pyrrole**

N-[5-(4-propylphenyl)thiophene-2-sulfonyl]pyrrole was prepared in the same manner as described in Reference Example 33C, from 4-propylphenyl boronic acid and N-(5-bromothiophenesulfonyl)pyrrole. Purification by column chromatography using 10% ethyl acetate/hexanes gave the pure sulfonamide as a white solid in 55% yield, m.p. 106-108°C.

**D. 5-chlorosulfonyl-2-(4-propylphenyl)thiophene**

5-chlorosulfonyl-2-(4-propylphenyl)thiophene was prepared in the same manner as described in Reference Example 33D. Hydrolysis of 240 mg (0.73 mmol) of N-[5-(4-propylphenylthiophene)-2-sulfonyl]pyrrole with 6N NaOH followed by chlorination using phosphorous oxychloride and phosphorous pentachloride gave the crude sulfonyl chloride as a greenish-brown oil. Flash chromatography over silica gel using 2% ethyl acetate/hexanes yielded 83 mg (81%) of the pure sulfonyl chloride as a pale yellow oil.

**E. N-(4-bromo-3-methyl-5-isoxazolyl)-5-(4-propylphenyl)thiopherie-2-sulfonamide**

N-(4-bromo-3-methyl-5-isoxazolyl)-5-(4-propylphenyl)thiophene-2-sulfonamide was prepared in the same

manner as described in Reference Example 2. Reaction of 5-chlorosulfonyl-2-(4-isopropyl)thiophene (260 mg, 0.87 mmol) with 5-amino-4-bromo-3-methylisoxazole (161 mg, 0.91 mmol) yielded after flash chromatography using 10% MeOH/$CHCl_3$ a brown solid (76.1 mg) which was further purified using preparative HPLC to give the pure sulfonamide as a tan colored oil.

**INVENTIVE EXAMPLE 74**

**N-(4-Bromo-3-methyl-5-isoxazolyl)-2-(3,4,5-trimethoxybenzyl)-benzo[b]-thiophene-3-sulfonamide**

**A. α-(2-benzo[b]thienyl)-3,4,5-trimethoxybenzyl alcohol**

α-(2-benzo[b]thienyl)-3,4,5-trimethoxybenzyl alcohol was prepared in the same manner as described in Reference Example 40A. Reaction of benzo[b]thiophene (7.5 mmoles, 1.0 g), t-BuLi (1.7m, 9.7 mmoles, 5.7 mls) and 3,4,5-trimethoxybenzaldehyde (8.9 mmoles, 1.8 g) in THF (20 ml) yielded, after flash chromatography using 50% ethyl acetate/hexanes, 2.4 g (97%) of a yellow-white solid.

**B. 2-(3,4,5-trimethoxybenzly)-benzo[b]thiophene**

2-(3,4,5-trimethoxybenzly)-benzo[b]thiophene was prepared in the same manner as described in Reference Example 47B. Reaction of α-(2-benzo[b]-thienyl)-3,4,5-trimethoxybenzyl alcohol (4.5 mmoles, 1.5 g), triethylsilane (5.0 mmoles, 0.80 mls), $CH_2Cl_2$ (50 ml) and TFA (9.1 mmoles, 0.70 mls) yielded, after flash chromatography using 20% ethyl acetate/hexanes, 0.77 g (54%) of a white solid.

**C. 2-(3,4,5-trimethoxybenzyl)-benzo[b]thiophene-3-sulfonylchloride**

2-(3,4,5-trimethoxybenzyl)-benzo[b]thiophene-3-sulfonylchloride was prepared in the same manner as described in Reference Example 40B. Reaction of dimethylformamide (DMF; 4.8 mmoles, 0.40 mis), sulfuryl chloride (4.1 mmoles, 0.33 mis) and 2-(3,4,5-trimethoxybenzyl)-benzo[b] thiophene (2.4 mmoles, 0.75 g) yielded, after flash chromatography using 20% ethyl acetate/hexanes, 0.29 g (30%) of a yellow-orange oil.

**D. N-(4-bromo-3-methyl-5-isoxazolyl)-2-(3,4,5-trimethoxybenzyl)-benzo[b]thiophene-3-sulfonamide**

N-(4-bromo-3-methyl-5-isoxazolyl)-2-(3,4,5-trimethoxybenzyl)-benzo[b]thiophene-3-sulfonamide was prepared in the same manner as described in Reference Example 41. Reaction of 4-bromo-3-methyl-5-aminoisoxazole (0.55 mmoles, 97 mg), NaH (1.4 mmoles, 55 mg), and 2-(3,4,5-trimethoxylbenzyl)-benzo[b]thiophene-3-sulfonyl chloride (0.66 mmoles, 0.27 g) in THF (2 ml) yielded, after flash chromatography using 50% ethyl acetate/haxanes and recrystallization from chloroform and hexanes, 94 mg of a tan solid, m.p. 154-156°C.

**REFERENCE EXAMPLE 75**

**N-(4-bromo-3-methyl-5-isoxazolyl) 2-ethyl-5-methylbenzo[b]thiophene -3-sulfonamide**

**A. 2-ethyl-5-methylbenzo[b]thiophene**

2-ethyl-5-methylbenzo[b]thiophene was prepared in the same manner as described in Reference Example 40A. Reaction of 5-methylbenzo[b]thiophene (3.4 mmoles, 0.50 g), t-BuLi (1.7 m, 5.1 mmoles, 3.0 ml) and ethyl iodide (6.8 mmoles, 0.54 ml) in THF (10 ml) yielded 0.58g (97%) of a light yellow liquid.

**B. 2-ethyl-5-methylbenzo[b]thiophene-3-sulfonylchloride**

2-ethyl-5-methylbenzo[b]thiophene-3-sulfonylchloride was prepared in the same manner as described in Reference Example 40B. Reaction of DMF (6.5 mmoles, 0.50 ml), sulfurylchloride (5.5 mmoles, 0.44 ml) and 2-ethyl-5-methylbenzo[b]thiophene (3.2 mmoles, 0.57 g) yielded, after flash chromatography, using 2% ethyl acetate/hexanes, 0.58 g (66%) of an orange solid.

**C. N-(4-bromo-3-methyl-5-isoxazolyl)-2-ethyl-5-methylbenzo[b]thiophene-3-sulfonamide**

N-(4-bromo-3-methyl-5-isoxazolyl)-2-ethyl-5-methylbenzo[b]thiophene-3-sulfonamide was prepared in the

same manner as described in Reference Example 41. Reaction of 4-bromo-3-methyl-5-aminoisoxazole (1.0 mmole, 0.18 g), NaH (2.5 mmoles, 0.10g), and 2-ethyl-5-methylbenzo[b]thiophene-3-sulfonylchloride (1.3 mmoles, 0.36 g) in THF (6 ml) yielded, after recrystallization from chloroform and hexanes, 0.25g (60%) of a light brown crystalline solid, m.p. 176-178° C.

## INVENTIVE EXAMPLE 76

### N-(4-chloro-3-methyl-5-isoxazolyl)-2-[3,4-(methylenedioxy)benzyl]-benzo[b]thiophene-3-sulfonamide

N-(4-chloro-3-methyl-5-isoxazolyl)-2-[3,4-(methylenedioxy)benzyl]-benzo[b]thiophene-3-sulfonamide was prepared in the same manner as described in Reference Example 41. Reaction of 4-chloro-3-methyl-5-aminoisoxazole (0.61 mmoles, 81 mg), NaH (1.5 mmoles), 61 mg), and 2-[3,4-(methylenedioxy)benzyl]benzo[b]thiophene-3-sulfonyl chloride (0.74 mmoles, 0.27 g) in THF (4ml) yielded, after flash chromatography using 50% ethyl acetate/hexanes followed by recrystallization from ethyl acetate and hexanes, 0.23 g (81%) of a light brown solid, m.p. 178-181° C.

## INVENTIVE EXAMPLE 77

### N-(4-Bromo-3-methyl-5-isoxazolyl)-2-(3,4-dimethoxybenzyl)-benzo[b]thiophene-3-sulfonamide

#### A. α-(2-benzo[b]thienyl)-3,4-dimethylbenzyl alcohol

α-(2-benzo[b]thienyl)-3,4-dimethylbenzyl alcohol was prepared in the same manner as described in Reference Example 40A. Reaction of benzo[b]-thiophene (7.5 mmoles, 1.0 g), t-BuLi (1.7 m, 97 mmoles, 5.7 ml) and 3,4-dimethoxybenzaldehyde (8.9 mmoles, 1.5 g) in THF (20 ml) yielded, after flash chromatography using 30% ethyl aceteate/hexanes, 2.25 g (≈ 100%) of a white gummy solid.

#### B. 2-(3,4-dimethoxybenzyl)benzo[b]thiophene

2-(3,4-dimethoxybenzyl)benzo[b]thiophene was prepared in the same manner as described in Reference Example 47B. Reaction of α-(2-benzo[b]thienyl)-3-4-dimethoxybenzyl alcohol (7.5 mmoles, 2.25 g), triethylsilane (8.2 mmoles, 1.3 ml) and $CH_2Cl_2$ (20 ml) in TFA (15 mmoles, 1.2 mls) yielded, after flash chromatography using 10% ethyl acetate/hexanes, 1.77g (84%) of a colorless oil.

#### C. 2-(3,4-dimethoxybenzyl)-benzo[b]thiophene-3-sulfonylchloride

2-(3,4-dimethoxybenzyl)-benzo[b]thiophene-3-sulfonylchloride was prepared in the same manner as described in Reference Example 40B. Reaction of DMF (90 mmoles, 7 ml) and 2-(3,4-dimethoxybenzyl)-benzo[b]thiophene (6.0 mmoles, 1.7 g) yielded, after flash chromatography using 15% ethyl acetate/hexanes, 1.24g (54%) of a green oil.

#### D. N-(4-bromo-3-methyl-5-isoxazolyl)-2-(3,4-dimethoxybenzyl)-benzo[b]thiophene-3-sulfonamide

N-(4-bromo-3-methyl-5-isoxazolyl)-2-(3,4-dimethoxy-benzyl)-benzo[b]thiophene-3-sulfonamide was prepared in the same manner as described in Reference Example 41. Reaction of 4-bromo-3-methyl-5-aminoisoxazole (1.0 mmoles, 0.18 g), NaH (2.5 mmoles, 60 mg), and 2-(3,4-dimethoxybenzyl)benzo[b]thiophene-3-sulfonylchloride (1.2 mmoles, 0.44g) in THF (6ml), after recrystallization from chloroform and hexanes, yielded 0.42 g (80%) of a brown solid, m.p. 151-153° C.

## REFERENCE EXAMPLE 78

### N-(3,4-dimethyl-5-isoxazolyl)-2-(3,4-methylenedioxy)benzo[b]thiophene-3-sulfonamide

N-(3,4-dimethyl-5-isoxazolyl)-2-(3,4-methylenedioxy)benzo[b]thiophene-3-sulfonamide was prepared in the same manner as described in Reference Example 41. Reaction of 3,4-dimethyl-5-aminoisoxazole (1.0 mmoles, 0.11 g), NaH (2.5 mmoles, 60 mg) and 2-[3,4-(methylenedioxy)benzyl]-benzo[b]thiophene-3-sulfonylchloride (1.1 mmoles, 0.40 g) in THF (6 ml) yielded, after flash chromatography using 50% ethyl acetate/hexanes followed by recrystallization from chloroform and hexanes, 0.35 g (79%) of a tan solid, m.p. 135-137° C.

**REFERENCE EXAMPLE 79**

**N-(4-Bromo-3-methyl-5-isoxazolyl)-2-(4-methoxybenzyl)benzo[b]-thiophene-3-sulfonamide**

**A. α-(2-benzo[b]thienyl)-4-methyoxybenzol alcohol**

α-(2-benzo[b]thienyl)-4-methyoxybenzol alcohol was prepared by the method of Reference Example 40A with benzo[b]thiophene (7.5 mmoles. 1.0 g), t-BuLi (1.7 m, 10.4 mmoles, 6.1 ml), 4-methoxy-benzaldehyde (8.9 mmoles, 1.1 ml) and THF (20 ml). Flash chromatography (20% ethyl acetate/hexanes) provided 1.75 g (87%) of a yellow solid.

**B. 2-(4-methyoxybenzyl)-benzo[b]thiophene**

2-(4-methyoxybenzyl)-benzo[b]thiophene was prepared by the method of Example Reference 47B with α-(2-benzo[b]thienyl)-4-methoxybenzyl alcohol (1.9 mmoles) 0.50 g), triethysilane (2.0 mmoles, 0.32 mls), $CH_2Cl_2$ (20 ml) and TFA (3.7 mmoles, 0.30 ml). Recrystallization with hexanes and chloroform provided 0.40 (85%) of a pink solid.

**C. 2-(4-methoxybenzyl)-benzo[b]thiophene-3-sulfonylchloride**

2-(4-methoxybenzyl)-behzb[b]thiophene-3-sulfonylchloride was prepared by the method of Reference Example 40B with DMF (3.2 mmoles, 0.24 ml), sulfurylchloride (2.7 mmoles, 0.22 ml) and 2-(4-methoyxybenzyl)-benzo[b] thiophene (1.6 mmoles. 0.4 g). Flash chromatography using 2% ethyl acetate/hexanes provided 0.19 g (33%) of a light yellow solid.

**D. N-(4-bromo-3-methyl-5-isoxazolyl)-2-(4-methoxybenzyl)-benzo[b]thiophene-3-sulfonamide**

N-(4-bromo-3-methyl-5-isoxazolyl)-2-(4-methoxybenzyl)-benzo[b]thiophene-3-sulfonamide was prepared by the method of Reference Example 41 with 4-bromo-3-methyl-5-aminoisoxazole (0.48 mmoles, 85 mg), NaH (1.2 mmoles, 48 mg), 2-(4-methoxybenzyl)-benzo[b]thiophene-3-sulfonylchloride (0.53 mmoles, 0.19 g) and THF (3 ml). Flash chromatography (50% ethyl acetate/hexanes) followed by recrystallization from methanol and water provided 46mg (20%) of a white crystalline solid, m.p. 120-122° C.

**REFERENCE EXAMPLE 80**

**N-(4-Bromo-3-methyl-5-isoxozofyl)-2-(2-methoxybenzyl)-benzo[b]thiophene-3-sulfonamide**

**A. α-(2-benzo[b]thiophene)-2-methoxybenzyl alcohol**

α-(2-Benzo[b]thiophene)-2-methoxybenzyl alcohol was prepared by the method of Reference Example 47A with benzo[b]thiophene (7.5 mmoles, 1.0 g) t-BuLi (1.7 m, 9.7 mmoles, 5.7 ml), 2-methoxybenzaldehyde (8.9 mmoles, 1.1 ml) and THF (20 ml). Flash chromatography (20% ethyl acetate/hexanes) provided 1.9 g (96%) of a yellow oil.

**B. 2-(2-Methoxybenzyl)-benzo[b]thiophene**

2-(2-Methoxybenzyl)-benzo[b]thiophene was prepared by the method of Example 47B α-(2-benzo[b]thiophene)-2-methoxybenzyl alcohol (7.1 mmoles, 1.9 g), triethylsilane (7.9 mmoles, 1.3 ml) and $CH_2Cl_2$ (30 ml) at 0°C was added TFA (14.3 mmoles, 1.1 ml). Flash chromatography (2% ethyl acetate/hexanes) provided 1.31g (72%) of a yellow solid.

**C. 2-(2-methoxybenzyl)-benzo[b]thiophene-3-sulfonyl chloride**

2-(2-methoxybenzyl)-benzo[b]thiophene-3-sulfonyl chloride was prepared by the method of Reference Example 40B with sulfuryl chloride (8.4 mmoles, 0.7 ml), DMF (9.8 mmoles, 0.8 ml) and 2-(2-methoxybenzyl)-benzo[b] thiophene (4.9 mmoles, 1.25 g). Flash chromatography (2% ethyl acetate/hexanes) provided 0.94 g (54%) of a yellow solid.

**D. N-(4-bromo-3-methyl-5-isoxozolyl)-2-(2-methoxybenzyl)-benzo[b]thiophene-3-sulfonamide**

N-(4-bromo-3-methyl-5-isoxozolyl)-2-(2-methoxybenzyl)-benzo[b]-thiophene-3-sulfonamide was prepared

by the method of Reference Example 41 with 5-amino-4-bromb-3-methylisoxazole (1.0 mmoles, 0.18 g), NaH (2.5 mmoles, 100 mg), 2-(2-methoxybenzyl)-benzo[b]thiophene-3-sulfonyl chloride (1.4 mmoles, 0.49 g) and THF (7ml). Flash chromatography (50% ethyl acetate/hexanes) followed by recrystallization from chloroform and hexanes provided 0.30 g (61%) of a brown solid, m.p. 80-84° C.

**REFERENCE EXAMPLE 81**

**N-(3,4-dimethyl-5-isoxazolyl)-2-(4-chlorobenzyl)-benzo[b]thiophene-3-sulfonamide**

**A. α-(2-benzo[b]thienyl)-4-chlorobenzyl alcohol**

α-(2-benzo[b]thienyl)-4-chlorobenzyl alcohol was prepared by the method of Reference Example 40A with benzo[b]thiophene (7.5 mmoles, 1.0 g), t-BuLi (1.7 m, 9.7 mmoles, 5.7 ml), 4-chlorobenzaldehyde (9.7 mmoles, 1.4 g) and THF (20 ml). The crude material (2.45 g) was taken forward without further purification.

**B. 2-(4-chlorobenzyl)benzo[b]thiophene**

2-(4-chlorobenzyl)benzo[b]thiophene was prepared by the method of Reference Example 47B with α-(2-benzo[b]thiophene)-4-chlorobenzyl alcohol (8.9 mmoles, 2.45 g), triethylsilane (9.8 mmoles, 1.6 ml), $CH_2Cl_2$ (40 ml) and TFA (13.4 mmoles, 1.0 ml). Flash chromatography (1 % ethyl acetate/hexanes) provided 1.3 g (67% - 2 steps) of an off-white solid.

**C. 2-(4-chlorobenzyl)benzo[b]thiophene-3-sulfonylchloride**

2-(4-chlorobenzyl)benzo[b]thiophene-3-sulfonylchloride was prepared by the method of Reference Example 40B with DMF (70 mmoles, 5.4 ml), sulfurylchloride (2.3 mmoles, 1.9 ml) and 2-(4-chlorobenzyl)-benzo[b]thiophene (4.6 mmoles, 1.2 g). Flash chromatography (2% ethyl acetate/hexanes) provided 0.51 g (31%) of an orange-yellow oil.

**D. N-(3,4-dimethyl-5-isoxazolyl)-2-(4-chlorobenzyl)-benzo[b]thiophene-3-sulfonamide**

N-(3,4-dimethyl-5-isoxazolyl)-2-(4-chlorobenzyl)-benzo[b]thiophene-3-sulfonamide was prepared by the method of Reference Example 41 with 3,4-dimethyl-5-aminoisoxazole (1.2 mmoles, 1.4 g), NaH (3.0-mmoles, 73 mg), 2-(4-chlorobenzyl)-benzo[b]thiophene-3-sulfonylchloride (1.4 mmoles, 0.50 g) and THF (8 ml). Flash chromatography (50% ethyl acetate/hexanes) followed by recrystallization from methanol and water provided 1.04 g (27%) of a yellow solid, m.p. 100-102° C.

**REFERENCE EXAMPLE 82**

**N-(4-chloro-3-methyl-5-isoxazolyl)-2-(4-dimethylaminobenzyl)benzo[b]thiophene-3-sulfonamide**

**A. α-(2-benzo[b]thienyl)-4-dimethylaminobenzyl alcohol**

α-(2-benzo[b]thienyl)-4-dimethylaminobenzyl alcohol was prepared by the method of Reference Example 40A with benzo[b]thiophene (7.5 mmoles, 1.0 g), t-BuLi (1.7 M, 8.9 mmoles, 5.3 ml), 4-dimethylaminobenzaldehyde (8.9 mmoles, 1.3 g) and THF (20 ml). The crude product (2.4 g) was carried forward without further purification.

**B. 2-(4-dimethyaminobenzyl)benzo[b]thiophene**

2-(4-dimethyaminobenzyl)benzo[b]thiophene was prepared by the method of Reference Example 47B with α-(2-benzo[b]thienyl)-4-dimethylaminobenzl alcohol (7.5 mmoles, 2.1 g), triethylsilane (2.8 mmoles, 1.3 ml), $CH_2Cl_2$ (50 ml) and TFA (11.2 mmoles, 0.9 ml). Flash chromatography (10% ethyl acetate/hexanes) provided 1.5 g (73% - for two steps) of a white solid.

**C. 2-(4-dimethylaminobenzyl)-benzo[b]thiophene-3-sulfonylchloride**

Chlorosulfonic acid (9.4 mmoles, 0.6ml) was added to 2-(4-dimethylaminobenzyl)-benzo[b]thiophene (3.7 mmoles, 1.0 g) in $CH_2Cl_2$ (100 ml) at -78° C. The solution was stirred 20 min. at -78° C. Phosphorous oxychloride (11.2 mmoles, 1.0 ml) and phosphorous pentachloride (11.2 mmoles, 2.3 g) were added to the reaction mixture. The reaction

mixture was warmed to ambient temperature and stirring was continued an additional 1.5 hr. followed by dilution with ice (= 200 ml) and extraction with ethyl acetate (200 ml). The organic layer was washed carefully with sat. $NaHCO_3$ (3 x 100 ml), then dried ($MgSO_4$), filtered and concentrated. Flash chromatography (5% ethyl acetate/hexanes) provided 0.61 g (45%) of a yellow solid.

**D. N-(4-chloro-3-methyl-5-isoxazolyl)-2-(4-dimethylaminobenzyl)-benzo[b]thiophene-3-sulfonamide**

N-(4-chloro-3-methyl-5-isoxazolyl)-2-(4-dimethylaminobenzyl)-benzo[b]thiophene-3-sulfonamide was prepared by the method of Reference Example 41 with 4-chloro-3-methyl-5-aminoisoxazole (0.52 mmoles, 69 mg), NaH (1.3 mmoles, 31 mg), 2-(4-dimethylaminobenzyl)-benzo[b]thiophene-3-sulfonylchloride (0.58 mmoles, 0.21g) and THF (4 ml). Flash chromatography (5% methanol/chloroform) provided 0.16 g (66%) of a yellow gummy solid, m.p. 105-110° C.

**REFERENCE EXAMPLE 83**

**N-(4-bromo-3-methyl-5-isoxazolyl)-2,5-dimethylfuran-3-sulfonamide**

**A. 2,5-dimethyl-furan-3-sulfonylchloride**

2,5-dimethyl-furan-3-sulfonylchloride was prepared by the method of Reference Example 40B with DMF (28 mmoles, 2.2 ml), sulfurylchloride (24 mmoles, 1.9 ml) and 2,5-dimethyl-furan (14 mmoles, 1.5 ml). Flash chromatography (5% ethyl acetate/hexanes) provided 0.61 g (22%) of a yellow liquid.

**B. N-(4-bromo-3-methyl-5-isoxazolyl)-2,5-dimethyl-furan-3-sulfonamide**

N-(4-bromo-3-methyl-5-isoxazolyl)-2,5-dimethyl-furan-3-sulfonamide was prepared by the method of Reference Example 41 with 4-bromo-3-methyl-5-amino-isoxazole (2.0 mmoles, 0.35 g), NaH (5.0 mmoles, 200 mg), 2,5-dimethyl-furan-3-sulfonylchloride (2.4 mmoles, 0.47 g) and THF (9 ml). Flash chromatography (5% methanol/chloroform) followed by recrystallization from chloroform and hexanes provided 0.21 g (31%) of a light brown solid, m.p. 85.5-87° C.

**REFERENCE EXAMPLE 84**

**N-(4-Bromo-3-methyl-5-isoxazolyl)-2,5-dimethyl-4-phenylthiophene-3-sulfonamide**

**A. 2,5-dimethyl-3,4-dibromothiophene**

NBS (13.2 mmoles, 2.4 g) was added to 2,5-dimethylthiopene (5.3 mmoles, 0.59 g) in $CHCl_3$ (30 ml). The reaction mixture was stirred at ambient temperature for 1.5 hr., then diluted with ether (50 ml) and washed with water (3 x 50 ml). The organic was dried ($MgSO_4$), filtered and concentrated. Flash chromatography (hexanes) provided 1.2 g (84%) of a white solid.

**B. 2,5-dimethyl-3-bromo-4-phenylthiophene**

Phenyl boronic acid (5.0 mmoles, 0.61 g) was added to 2,5-dimethyl-3,4-bromothiophene (4.5 mmoles, 1.2 g), tetrakis (triphenylphosphine) palladium(0) (0.23 mmoles, 0.26 g) and $Na_2CO_3$ (2 M, 26 mmoles, 13 ml) in benzene (20 ml). The biphasic reaction mixture was heated to reflux for 24 hr. then cooled to ambient temperature and diluted with ether (100 ml) and washed with water (100 ml). The organic later was dried ($MgSO_4$), filtered and concentreated. Flash chromatography (hexanes) provided 0.60 g (49%) of a yellow solid.

**C. 2,5-dimethyl-4-phenyl-3-sulfonylchloridethiophene**

t-BuLi (1.7 M, 2.7 mmoles, 1.6 ml) was added to 2,5-dimethyl-3-bromo-4-phenyl thiophene (2.2 mmoles, 0.59 g) in THF (8 mis.) at -30° C. The solution was stirred 20 min. at -30°C, then the flask was evacuated with sulfur dioxide, and warmed to -20° C upon which NCS (2.2 mmoles, 0.30 g) was added. The reaction mixture was warmed to ambient temperature for 30 min., then diluted with ethyl acetae (50 ml) and washed with water (2 x 50 ml). The organic layer was dried ($MgSO_4$), filtered and concentrated. Flash chromatography (2% ethyl acetate/hexanes) provided 0.26 g (41 %) of a light yellow solid.

**D. N-(4-bromo-3-methyl-5-isoxazole)-2,5-dimethyl-4-phenylthiophene-3-sulfonamide**

N-(4-bromo-3-methyl-5-isoxazole)-2,5-dimethyl-4-phenylthiophene-3-sulfonamide was prepared by the method of Reference Example 41 with 4-bromo-3-methyl-5-amino isoxazole (0.79 mmoles, 0.14 g), NaH (2.0 mmoles, 80 mg), 2.5-dimethyl-4-phenylthiophene-3-sulfonylchloride (0.91 mmoles, 0.26 g) and THF (3 ml). Recrystallization twice from chloroform and hexanes provided 0.15g (45%) of a white crystalline solid, m.p. 166 - 168° C.

**REFERENCE EXAMPLE 85**

**N-(4-Bromo-3-methyl-5-isoxazolyl)-2-(hydroxymethyl)thiophene-3-sulfonamide**

$BH_3$·THF (3.62 ml, 1 M in THF) was added to a solution of N-(4-bromo-3-methyl-5-isoxazolyl)-2-carboxylthiophene-3-sulfonamide (1.09, 2.72 mmol) in dry THF (15 ml) at room temperature. After stirring at room temperature for 10 minutes, the mixture was refluxed for 1 hour. The reaction was cooled with an ice-bath and 1N HCl (10 ml) was added. The resulting mixture was concentrated. The aqueous residue was then partitioned between 1 N HCl and EtOAc. The organic layer was dried ($MgSO_4$). The solid was filtered and the filtrate concentrated. The residue was treated with MeOH and concentrated again. This process was repeated 3 more times to give N-(4-bromo-3-methyl-5-isoxazolyl)-2-(hydroxymethyl)thiophene-3-sulfonamide (680 mg, 71% yield) as a yellow oil.

**REFERENCE EXAMPLE 86**

**N-(4-Bromo-3-methyl-5-isoxazolyl)-2-[(3-methoxyphenyl)aminomethyl]-thiophene-3-sulfonamide**

$BH_3$·THF (15 ml, 1 M in THF) was added to a solution of N-(4-bromo-3-methyl-5-isoxazolyl)-2-[N-(3-methoxyphenyl)aminocarbonyl]thiophene-3-sulfonamide (Reference Example 22) (1.0 g, 2.12 mmol) in dry THF (15 ml). The mixture was refluxed for 8 hours and cooled. THF was evaporated on a rotavap and MeOH was added to the residue. The resulting solution was concentrated. The final residue was purified by HPLC to give N-(4-bromo-3-methyl-5-isoxazolyl)-2-[(3-methoxyphenyl)-aminomethyl]thiophene-3-sulfonamide (113 mg., 12% yield) as a grey powder, m.p. 70-73°C.

**REFERENCE EXAMPLE 87**

**N-(4-bromo-3-methyl-5-isoxazolyl)-2-[N-(3-carboxyphenylaminocarbonyl]-thiophene-3-sulfonamide**

$Et_3N$ (2.27 ml. 16. mmol), ethyl 3-aminobenzoate (836 ml, 5.44 mmol) and phosphonitrilic chloride trimer (1.89 g, 5.44 mmol) were sequentially added to a solution of N-(4-bromo-3-methyl-5-isoxazolyl)-2-(carbonyl)thiophene-3-sulfonamide (Reference Example 17) (1g, 2.27 mmol) in dry THF (20 ml). The reaction was stirred at room temperature for 1 hour and cooled. Water (5 ml) was added to quench the reaction. The resulting solution was concentrated on a rotavap. The residue was diluted with EtOAc and washed with 2N HCl (2 x 150 ml). The organic layer was dried ($MgSO_4$). The solid was filtered off and the filtrate was concentrated. The residue was treated with 1 N NaOH (200 ml) and stirred at 0° C for 15 minutes. The mixture was then acidified with conc. HCl to pH ~ 1. The resulting yellow precipitate was filtered off and recrystalized from $CH_3CN/H_2O$ to give N-(4-bromo-3-methyl-5-isoxazolyl)-2-[N-(3-carboxyphenyl)aminocarbonyl]thiophene-3-sulfonamide (153 mg., 11.6%) as a yellowish powder, m.p. 183-185° C.

**REFERENCE EXAMPLE 88**

**N-(4-Bromo-3-methyl-5-isoxazolyl)-2-[N-(2-carboxylphenyl)aminocarbonyl]thiophene-3-sulfonamide**

N-(4-bromo-3-methyl-5-isoxazolyl)-2-[N-(2-carboxylphenyl)aminocarbonyl]thiophene-3-sulfonamide was prepared by the same method as described in Reference Example 87, with the exception of using ethyl-2-aminobenzoate instead of ethyl-3-aminobenzoate.

**REFERENCE EXAMPLE 89**

**N-(4-bromo-3-methyl-5-isoxazolyl)-2-(aminocarbonyl)thiophene-3-sulfonamide**

Carbonydiimidazole (485 mg, 2.99 mmol) was added to a solution of N-(4-bromo-3-methyl-5-isoxazolyl)-2-carboxylthiophene-3-sulfonamide (1 g, 2.72 mmol) in THF (10 ml) at room temperature. The mixture was stirred for 15

minutes. Aqueous $NH_3$ (5 ml) was then added, and the mixture was stirred at room temperature for 30 minutes. The solvent was evaporated and the residue was partitioned between EtOAc and 1 N HCl. The organic layer was dried ($MgSO_4$). The solid was filtered and the filtrate concentrated. The oily residue was recrystalized from EtOAc to give N-(4-bromo-3-methyl-5-isoxazolyl)-2-(aminocarbonyl)thiophene-3-sulfonamide (946 mg, 95% yield) as a white solid, m.p. 168-170° C.

**REFERENCE EXAMPLE 90**

**N-(4-Bromo-3-methyl-5-isoxazolyl)-2-[(5-dimethylamino-1-naphthyl)-sulfonylaminocarbonyl]thiophene-3-sulfonamide**

Dansylchloride (90.2 mg, 0.328 mmol) was added to a solution of N-(4-bromo-3-methyl-5-isoxazolyl)-2-(aminocarbonyl)thiophene-3-sulfonamide (Reference Example 89) (100 mg, 0.273 mmol) and NaH (43.7 mg, 60% dispersion in mineral oil, 1.10 mmol). The reaction was stirred at room temperature for 1 hour. Water was added to quench the reaction and THF was stripped off on a rotavap. The aqueous residue was partitioned between 1N HCl and EtOAc. The organic layer was dried ($MgSO_4$). The solid was filtered and the filtrate concentrated. The residue was recrystalized from EtOAc to give N-(4-bromo-3-methyl-5-isoxazolyl)-2-[(5-dimethylamino-1-naphthyl)sulfonylaminocarbonyl]thiophene-3-sulfonamide (55 mg., 34% yield) as a white powder (m.p. 184-186° C).

**REFERENCE EXAMPLE 91**

**N-(4-Bromo-3-methyl-5-isoxazolyl)-2-[(3,4-methylenedioxyphenyl)aminocarbonyl]thiophene-3-sulfonamide**

N-(4-bromo-3-methyl-5-isoxazolyl)-2-[(3,4-methylenedioxyphenyl)-aminocarbonyl]thiophene-3-sulfonamide was prepared by the same method as described in Reference Example 89 with the exception that 3,4-methylenedioxyaniline was used in place of ammonium hydroxide. N-(4-bromo-3-methyl-5-isoxazolyl)-2-[(3,4-methylenedioxyphenyl)aminocarbonyll]thiophene-3-sulfonamide was purified by HPLC to give 15% yield of the desired product as a dark grey powder, m.p. 138-140° C.

**REFERENCE EXAMPLE 92**

**N-(4-Bromo-3-methyl-5-isoxazolyl)-2-[(3,4-methylenedioxy)phenoxycarbonyl]thiophene-3-sulfonamide**

Carbonyldiimidazole (530 mg., 3.26 mmol.) was added to a solution of N-(4-bromo-3-methyl-5-isoxazolyl)-2-carboxylthiophene-3-sulfonamide (Reference Example 17) (1.0g, 2.72 mmol) in dry THF (10 ml). The mixture was stirred at room temperature for 15 minutes. Sesamol (767 mg., 5.44 mmol) and imidazole (185 mg, 2.72 mmol) were added simultaneously. The resulting mixture was refluxed for 1 hour and allowed to cool to room temperature. The solvent was evaporated. The residue was partitioned between 1N HCl and EtOAc. The organic layer was dried ($MgSO_4$). The solid was filtered and the filtrate concentrated to give a yellow oil which was recrystalized from EtOAc/$Et_2O$/Hexane. N-(4-bromo-3-methyl-5-isoxazolyl)-2-[(3,4-methylenedioxy)phenoxycarbonyl]thiophene-3-sulfonamide was obtained as a white powder (494 mg, 37% yield), m.p. 174-176° C.

**REFERENCE EXAMPLE 93**

**N-(4-Bromo-3-methyl-5-isoxazolyl)-2-[(3,4-methylenedioxy)benzoyl]-thiophene-3-sulfonamide**

**A. N-(4-bromo-3-methyl-5-isoxazolyl)-2-[(N-methoxy-N-methyl)aminocarbonyl]thiophene-3-sulfonamide**

N-(4-bromo-3-methyl-5-isoxazolyl)-2-[(N-methoxy-N-methyl)carboxamide]thiophene-3-sulfonamide was prepared by the same method as described in Reference Example 89 with the exception that N,0-dimethylhydroxylamine was used in place of ammonium hydroxide. The yield was 90%.

**B. N-(4-Bromo-3-methyl-5-isoxazolyl)-2-[(3,4-methylenedioxy)-benzoyl]thiophene-3-sulfonamide**

Freshly prepared (3,4-methylenedioxy)phenyl magnesium bromide (1.28 g of (3,4-methylenedioxy)bromobenzene and 172 mg Mg turnings) was added to a solution of N-(4-bromo-3-methyl-5-isoxazolyl)-2-[(N-methoxy-N-methyl)aminocarbonyl]thiophene-3-sulfonamide (A) (652 mg, 1.59 mmol) in THF (10 ml) at room temperature. The resulting mixture was refluxed for 30 minutes. To workup, the mixture was allowed to cool to room temperature and

was quenched with 1N HCl (10 ml). THF was then evaporated. The aqueous residue was partitioned between 1 N HCl and EtOAc. The organic layer was concentrated and the residue was purified by HPLC to give N-(4-bromo-3-methyl-5-isoxazolyl)-2-[(3,4-methylenedioxy)benzoyl]thiophene-3-sulfonamide (90 mg, 12% yield) as a dark yellow powder, m.p. 47-49° C.

**REFERENCE EXAMPLE 94**

**N-(4-Bromo-3-methyl-5-isoxazolyl)-2-[(2-hydroxyphenyl)aminocarbonyl]-thiophene-3-sulfonamide**

N-(4-bromo-3-methyl-5-isoxazolyl)-2-[(2-hydroxyphenyl)aminocarbonyl]thiophene-3-sulfonamide was prepared by the same method as described in Reference Example 89 with the exception that 3-aminophenol was used in place of ammonium hydroxide. The product was purified by HPLC to give N-(4-bromo-3-methyl-5-isoxazolyl)-2-[(2-hydroxyphenyl)aminocarbonyllthiophene-3-sulfonamide (50 mg, 18% yield) as a dull yellow solid, m.p. 42-44° C.

**REFERENCE EXAMPLE 95**

**N-(3,4-dimethyl-5-isoxazolyl)-2[(3,4-methylenedioxy)phenoxycarbonyl]thiophene-3-sulfonamide**

N-(3,4-dimethyl-5-isoxazolyl)-2[3,4-(methylenedioxy)phenoxycarbonyl]thiophene-3-sulfonamide was prepared by the same method as described in Reference Example 92 with the exception that N-(3,4-dimethyl-5-isoxazolyl)-2-carboxylthiophene-3-sulfonamide was used instead of N-(3-bromo-4-methyl-5-isoxazolyl)-2-carboxylthiophene-3-sulfonamide. N-(3,4-dimethyl-5-isoxazolyl)-2[(3,4-methylenedioxy)phenoxycarbonyl]thiophene-3-sulfonamide was purified by HPLC and was obtained as an orange oil (200 mg., 15% yield).

**REFERENCE EXAMPLE 96**

**N-(4-Bromo-3-methyl-5-isoxazolyl)-2-{[(3,4-methylenedioxy)benzoyl]-aminocarbonyl}thiophene-3-sulfonamide**

Carbonyldiimidazole (213 mg. 1.31 mmol) was added to a solution of piperonylic acid (181.5 mg., 1.09 mmol) in dry THF (10 ml). The resulting mixture was stirred for 15 minutes. N-(4-bromo-3-methyl-5-isoxazolyl)-2-aminocarbonylthiophene-3-sulfonamide (Reference Example 89) (400 mg, 1.09 mmol) and NaH (175 mg, 60% in mineral oil, 4.37 mmol) were added sequentially. The mixture was stirred at room temperature for 8 hours. Water was added to destroy the excess NaH. The solvent was then evaporated and the residue was partitioned between 1 N HCl and EtOAc. The organic layer was dried ($MgSO_4$), the solid filtered and the filtrate concentrated. The residue was recrystalized from EtOAc to give N-(4-bromo-3-methyl-5-isoxazolyl)-2-{[(3,4-methylenedioxy)benzoyl]-aminocarbonyl}thiophene-3-sulfonamide (20 mg, 3.6% yield) as a yellowish powder (m.p. 90-93° C).

**REFERENCE EXAMPLE 97**

**N-(4-chloro-3-methyl-5-isoxazolyl)-2-[(3,4-methylenedioxy)phenoxycarbonyl]thiophene-3-sulfonamide**

N-(4-chloro-3-methyl-5-isoxazolyl)-2-[(3,4-methylenedioxy)phenoxycarbonyl]thiophene-3-sulfonamide was prepared by the same method as described in Reference Example 93 with the exception that N-(4-chloro-3-methyl-5-isoxazolyl)-2-carboxylthiophene-3-sulfonamide was used instead of N-(4-bromo-3-methyl-5-isoxazolyl)-2-carboxylthiophene-3-sulfonamide. N-(4-chloro-3-methyl-5-isoxazolyl)-2-[(3,4-methylenedioxy)phenoxycarbonyl]-thiophene-3-sulfonamide was recrystalized from EtOAc (49% mg, 20% yield) as a white solid, m.p. 189-191° C.

**REFERENCE EXAMPLE 98**

**N-(4-Bromo-3-methyl-5-isoxazolyl)-2-[(3,4-methylenedioxy)phenylacetyl]thiophene-3-sulfonamide**

N-(4-bromo-3-methyl-5-isoxazolyl)-2-[(3,4-methylene dioxy)phenylacetyl]thiophene-3-sulfonamide was prepared by the same method as described in Reference Example 93 with the exception that piperonylmagnesium chloride was used instead of (3,4-methylenedioxy)-phenylmagnesium bromide and the reaction mixture was stirred overnight at room temperature instead of refluxing for 30 minutes. The crude mixture was purified by HPLC to give N-(4-bromo-3-methyl-5-isoxazolyl)-2-[3,4-(methylenedioxy)phenylacetyl]thiophene-3-sulfonamide (20 mg, 40% yield) as a yellow oil.

**REFERENCE EXAMPLE 99**

**N-(4-Bromo-3-methyl-5-isoxazolyl)-2-[(3,4-methylenedioxy)phenoxycarbonylamino]thiophene-3-sulfonamide**

Triethylamine (2.28 ml, 16.35 mmol) and diphenylphosphorylazide (773 mg., 2.72 mmol) were sequentially added to a solution of N-(4-bromo-3-methyl-5-isoxazolyl)-2-carboxylthiophene-3-sulfonamide (Example 17) (1.0 g, 2.72 mmol) in dry THF (40 ml). The mixture was stirred for 8 hours. Sesamol (1.54 g, 10.9 mmol) was added and the mixture was refluxed for 2 hr. The mixture was allowed to cool to room temperature. The solvent was stripped off on a rotavap and the residue was partitioned between EtOAc and 1 N HCl. The organic layer was dried ($MgSO_4$). The solid was filtered and the filtrate concentrated. The residue was purified by HPLC to afford N-(4-bromo-3-methyl-5-isoxazolyl)-2-[3,4-(methylenedioxy)phenoxycarbonylamino]thiophene-3-sulfonamide (400 mg, 29% yield) as a beige powder, m.p. 39-43° C.

**REFERENCE EXAMPLE 100**

**N-(4-Bromo-3-methyl-5-isoxazolyl)-2-[(3,4-methylenedioxy)phenylureido]-thiophene-3-sulfonamide**

N-(4-bromo-3-methyl-5-isoxazolyl)-2-[(3,4-methylenedioxy)phenylureido]thiophene-3-sulfonamide was prepared by the same method as described in Reference Example 99 with the exception that 3,4-methylenedioxyaniline was used instead of sesamol. N-(4-bromo-3-methyl-5-isoxazolyl)-2-[3,4-(methylenedioxy)phenylureido]thiophene-3-sulfonamide (157 mg, 12% yield) was obtained via HPLC purification as a brownish-greyish powder, m.p. 62-65° C.

**REFERENCE EXAMPLE 101**

**N-(4-chloro-3-methyl-5-isoxazolyl)-2-[(3,4-methylenedioxy)benzyloxycarbonyl]thiophene-3-sulfonamide**

N-(4-chloro-3-methyl-5-isoxazolyl)-2-[(3,4-methylenedioxy)benzyloxycarbonyl]thiophene-3-sulfonamide was prepared by the same method as described in Reference Example 97 with the exception that piperonyl alcohol was used instead of sesamol. N-(4-chloro-3-methyl-5-isoxazolyl)-2-[(3,4-(methylenedioxy)benzyloxycarbonyl]thiophene-3-sulfonamide (210 mg, 15% yield) was obtained via HPLC purification as a yellowish powder, m.p. 35-38° C.

**REFERENCE EXAMPLE 102**

**N-(4-chloro-3-methyl-5-isoxazolyl)-2-[(3,4-methylenedioxy)phenylacetyl]-thiophene-3-sulfonamide**

N-(4-chloro-3-methyl-5-isoxazolyl)-2-[(3,4-methylenedioxy)phenylacetyl]thiophene-3-sulfonamide was prepared by the same method as described in Reference Example 98 with the exception that N-(4-chloro-3-methyl-5-isoxazolyl)-2-carboxylthiophene-3-sulfonamide was used instead of N-(4-bromo-3-methyl-5-isoxazolyl)-2-carboxylthiophene-3-sulfonamide. N-(4-chloro-3-methyl-5-isoxazolyl)-2-[(3,4-methylenedioxy)phenylacetyl]thiophene-3-sulfonamide (3g, 50% yield) was obtained via HPLC purification as a yellow solid, m.p. 35-38° C.

**REFERENCE EXAMPLE 103**

**N-(4-chloro-3-methyl-5-isoxazolyl)-2-[(3,4-methylenedioxy)phenethyloxycarbonyl]thiophene-3-sulfonamide**

N-(4-chloro-3-methyl-5-isoxazolyl)-2-[(3,4-methylenedioxy)-phenethyloxycarbonyl]thiophene-3-sulfonamide was prepared by the same method as described in Reference Example 97 with the exception that (3,4-methylenedioxy) phenethyl alcohol was used instead of sesamol. N-(4-chloro-3-methyl-5-isoxazolyl)-2-[3,4-(methylenedioxy)phenethyloxycarbonyl]thiophene-3-sulfonamide (500 mg, 34% yield) was obtained via HPLC purification as a yellowish oil.

**REFERENCE EXAMPLE 104**

**N-(4-chloro-3-methyl-5-isoxazolyl)-2-{[4-(3,4-methylenedioxybenzyl)-piperazin-1-yl]carbonyl}thiophene-3-sulfonamide**

N-(4-chloro-3-methyl-5-isoxazolyl)-2-{[4-(3,4-methylenedioxybenzyl)piperazin-1-yl]carbonyl}thiophene-3-sulfonamide was prepared by the same method as described in Reference Example 89 with the exception that N-(4-chloro-3-methyl-5-isoxazolyl)-2-carboxylthiophene-3-sulfonamide was used instead of N-(4-bromo-3-methyl-5-isoxazolyl)-2-carboxylthiophene-3-sulfonamide, and 1-piperonylpiperazine was used instead of ammonium hydroxide. N-(4-chloro-3-methyl-5-isoxazolyl)-2-{(4-(3,4-methylenedioxybenzyl)piperazin-1-yl]carbonyl}thiophene-3-sulfonamide (872 mg, 54% yield) was obtained via HPLC purification as a white powder, m.p. 221-223° C.

**REFERENCE EXAMPLE 105**

**N-(4-chloro-3-methyl-5-isoxazolyl)-2-aminothiophene-3-sulfonamide**

N-(4-chloro-3-methyl-5-isoxazolyl)-2-aminothiophene-3-sulfonamide was prepared by the same method as described in Reference Example 99 except that the mixture was refluxed without the addition of sesamol. N-(4-chloro-3-methyl-5-isoxazolyl)-2-aminothiophene-3-sulfonamide (298 mg, 31 % yield) was obtained via HPLC as a yellow solid, m.p. 39-42° C.

**REFERENCE EXAMPLE 106**

**N-(4-chloro-3-methyl-5-isoxazolyl)-2-{1-cyano-1-[(3,4-methylenedioxy)-phenyl]acetyl}thiophene-3-sulfonamide**

Carbonyldiimidazole (603 mg, 3.72 mmol) was added to a solution of N-(4-chloro-3-methyl-5-isoxazolyl)-2-carboxylthiophene-3-sulfonamide (Reference Example 17) (1.0g, 3.1 mmol) in dry THF (40 ml). The mixture (I) was stirred at room temperature for 15 minutes.

NaH (868 mg, 60% in mineral oil, 21.7 mmol) was added to a solution of (3,4-methylenedioxy)phenylacetonitrile in THF (100 ml). The mixture (II) was refluxed for 30 minutes and then allowed to warm to room temperature.

The mixture (I) was then cannulated into mixture (II) while cooled by ice-bath and the resulting mixture was allowed to warm to room temperature. Water was added to quench excess NaH. THF was then stripped off on a rotavap. The residue was partitioned between 1 N NaOH and $Et_2O$. The aqueous layer was acidified with concentrated HCl with cooling to pH ~ 1 and extracted with EtOAc. The organic layer was dried ($MgSO_4$), the solid filtered and the filtrate concentrated. The residue was purified by HPLC to give N-(4-chloro-3-methyl-5-isoxazolyl)-2-{1-cyano-1-[3,4-(methylenedioxy)phenyl]acetyl}thiophene-3-sulfonamide (277 mg, 19% yield) as a yellowish powder, m.p. 142-142°C.

**REFERENCE EXAMPLE 107**

**N-(4-chloro-3-methyl-5-isoxazolyl)-2-[3-(dimethylamino)phenoxycarbonyl]thiophene-3-sulfonamide**

N-(4-chloro-3-methyl-5-isoxazolyl)-2-[3-(dimethylamino)phenoxycarbonyl]thiophene-3-sulfonamide was prepared by the same method as described in Reference Example 97 with the exception that 3-dimethylaminophenol was used instead of sesamol. N-(4-chloro-3-methyl-5-isoxazolyl)-2-[3-(dimethylamino)phenoxycarbonyl]thiophene-3-sulfonamide (50 mg, 7.3% yield) was obtained via HPLC purification as a dark brown oil.

**REFERENCE EXAMPLE 108**

**N-(4-chloro-3-methyl-5-isoxazolyl)-2-(cyclohexyloxycarbonyl)thiophene-3-sulfonamide**

N-(4-chloro-3-methyl-5-isoxazolyl)-2-(cyclohexyloxycarbonyl)-thiophene-3-sulfonamide was prepared by the same method as described in Reference Example 97 with the exception that cyclohexanol was used instead of sesamol. N-(4-chloro-3-methyl-5-isoxazolyl)-2-(cyclohexyloxycarbonyl)-thiophene-3-sulfonamide (29 mg, 5% yield) was obtained via HPLC purification as an off-white solid, m.p. 134-137° C.

**REFERENCE EXAMPLE 109**

**N-(4-chloro-3-methyl-5-isoxazolyl)-2-[β-hydroxy(3,4-methylenedioxy)phenylethyl]thiophene-3-sulfonamide**

$LiBH_4$ (36.6 mg, 1.68 mmol) was added slowly to a solution of N-(4-chloro-3-methyl-5-isoxazolyl)-2-[3,4-methylenedioxy)phenylacetyl]thiophene-3-sulfonamide (Reference Example 102) (74 mg, 0.168 mmol) in the THF (10 ml). The resulting mixture was stirred for 8 hours. Saturated $NH_4Cl$ (aq) was added to quench the excess $LiBH_4$. The resulting mixture was concentrated on a rotavap. The residue was partitioned between EtOAc and 1 N HCl. The organic layer was dried ($MgSO_4$), and the solid was filtered.

**REFERENCE EXAMPLE 110**

**N,N'-bis{3-[(3,4-dimethyl-5-isoxazolyl)aminosulfonyl]thien-2-yl}urea**

Triethylamine (1.4 ml, 9.93 mmol) and diphenylphosphorylazide (9.39 mg., 3.31 mmol) were sequentially added to a solution of N-(3,4-dimethyl-5-isoxazolyl)-2-(carboxyl)thiophene-3-sulfonamide (Reference Example 17) (1.0 g, 3.31 mmol) in THF (50 ml). The resulting mixture was stirred for 30 minutes at room temperature and then refluxed for 1 hour. The mixture was allowed to cool to room temperature. THF was stripped off by use of a rotavap. The residue was partitioned between EtOAc and 1N HCl. The organic layer was dried ($MgSO_4$), the solid filtered, and the filtrate concentrated. N,N'-bis{3-[(3,4-dimethyl-5-isoxazolyl)aminosulfonyl]thien-2-yl}urea (140 mg, 14% yield) was obtained via HPLC purification as a pale powder, m.p. 112-114°C.

**REFERENCE EXAMPLE 111**

**N,N'-bis{3-[(4-bromo-3-methyl-5-isoxazolyl)aminosulfonyl]thien-2-yl}urea**

N,N'-bis{3-[(4-bromo-3-methyl-5-isoxazolyl)aminosulfonyl]thien-2-yl}urea was prepared by the same method as described in Reference Example 110 with the exception that N-(4-bromo-3-methyl-5-isoxazolyl)-2-carboxythiophene-3-sulfonamide was used instead of N-(3,4-dimethyl-5-isoxazolyl)-2-carboxylthiopehene-3-sulfonamide. N,N'-bis{3-[(4-bromo-3-methyl-5-isoxazolyl)aminosulfonyl]thien-2-yl)urea (80 mg, 15.5% yield) was obtained via HPLC purification as an off-white solid, m.p. 127-129° C.

**REFERENCE EXAMPLE 112**

**N-(4-Bromo-3-methyl-5-isoxazolyl)-5-(benzyloxymethyl)thiophene-2-sulfonamide**

**A. 2-(benzyloxymethyl)thiophene**

Sodium hydride (0.41 mg, 20 mmol) was added to a solution of 2-thiophene methanol (2.0g, 0.18mmol) in THF (20 ml) at -40°C. The reaction was stirred at -40° C for 25 min., then neat benzylbromide (3.6 g. 20 mmol) was added by syringe. The solution was stirred at -40° C for 0.5 hr, then at room temperature for 1 hr. The THF was evaporated off and the remaining residue was taken up in ether (~ 50 ml). The organic solution was washed with water (1 x 10 ml), brine (1 x 10 ml) and dried over $MgSO_4$. Evaporation of solvents left an oil which was purified by column chromatography using 1% ether-hexanes to give 2.6 g of the thiophene as a pale yellow oil (78% yield).

**B. 2-chlorosulfonyl-5-(benzyloxymethyl)thiophene**

2-chlorosulfonyl-5-(benzyloxymethyl)thiophene was prepared in the same manner as described in Reference Example 132A from 2-(benzyloxymethyl)-thiophene (1.0 g, 5.25 mmol). Purification by column chromatography using 2.5% ethyl acetate/hexanes gave 520 mg of the pure thiophene as a brown oil (32% yield).

**C. N-(4-bromo-3-methyl-5-isoxazolyl)-5-(benzyloxymethyl)thiophene-2-sulfonamide**

N-(4-bromo-3-methyl-5-isoxazolyl)-5-(benzyloxymethyl)thiophene-2-sulfonamide was prepared as described in Reference Example 2 from 2-chlorosulfonyl-5-(benzyloxymethyl)thiophene (520 mg, 1.72 mmol) and 5-amino-4-bromo-3-methyl isoxazole (319 mg, 1.8 mmol). Purification by column chromatography using 10% MeOH/$CHCl_3$) gave 238 mg of pure N-(4-bromo-3-methyl-5-isoxazolyl)-5-(benzyloxymethyl)thiophene-2-sulfonamide as brown semisolid (31% yield, m.p. 92° C).

**REFERENCE EXAMPLE 113**

**N-(4-Chloro-3-methyl-5-isoxazolyl)-2-ethylbenzo[b]furan-3-sulfonamide**

**A. 2-Ethylbenzo[b]furan**

2-Ethylbenzo[b]furan was prepared by the method of Reference Example 40A with benzo[b]furan (7.3 mmols), 0.86 g) t-BuLi (1.7 m, 9.4 mmols) iodoethane (4 mmols, 0.9 mls) and THF (15 ml). 1.0 g (95%) of a yellow liquid was isolated.

**B. 2-Ethylbenzo[b]furan-3-sulfonyl chloride**

2-Ethylbenzo[b]furan-3-sulfonyl chloride was prepared by the method of Reference Example 82C with 2-ethylbenzo[b]furan (6.9 mmols, 1.0 g), chlorosulfonic acid (8.9 mmols, 0.6 ml) phosphorous oxychloride (21 mmols, 1.9 ml), phosphorous pentachloride (6.9 mmols, 1.4 g) and $CH_2Cl_2$ (10 ml). Flash chromatography (2% ethyl acetate/hexanes) provided 0.71 g (42%) of an orange solid.

**C. N-(4-chloro-3-methyl-5-isoxazolyl)-2-ethylbenzo[b]furan-3-sulfonamide**

N-(4-chloro-3-methyl-5-isoxazolyl)-2-ethylbenzo[b]furan-3-sulfonamide compound was prepared by the method of Reference Example 41 with 4-chloro-3-methyl-5-amino-isoxazole (1.0 mmols, 0.13 g), NaH (2.5 mmols, 60 mg), 2-ethylbenzo[b]furan-3-sulfonyl chloride (1.2 mmols, 0.28 g) and THF (7 ml). Flash chromatography (20% ethyl acetate/hexanes) followed by recrystallization from chloroform and hexane provided 97 mg (28%) of a white solid, m. p. 132-133.5° C.

**REFERENCE EXAMPLE 114**

**N-(4-Bromo-3-methyl-5-isoxazolyl)-2-[(3,4-methylenedioxy)benzyl])thiophene-3-sulfonamide**

**A. N-(2-carbomethoxythiophene-3-sulfonyl)pyrrole**

N-(2-carbomethoxythiophene-3-sulfonyl)pyrrole was prepared in the same manner as described in Reference Example 33A by reacting 2-carbomethoxythiophene-3-sulfonyl chloride with pyrrole, 50% yield.

**B. N-[(2-hydroxymethyl)thiophene-3-sulfonyl]pyrrole**

To a stirred solution of N-(2-carbomethoxythiophene-3-sulfonyl)pyrrole (3.0 g, 11.02 mmole) in a mixture of THF and methanol (3:1 mixture, 40 ml) was added sodium borohydride (1.2 g) in six lots over a 30 min. period (exothermic reaction) with constant stirring. The solvent was removed under reduced pressure and the residue was dissolved in saturated ammonium chloride solution (50 ml). The crude product was extracted with ethyl acetate, and the combined organic layers was dried over $MgSO_4$, and evaporated to give a crude product (2.4 g, 90%), which was used directly in the next step.

**C. N-[2-(bromomethyl)thiophene-3-sulfonyl]pyrrole**

Bromine was added to a stirred mixture of triphenylphosphine (3.1 g, 12 mmole) in methylene chloride (50 ml) at 0° C under nitrogen atmosphere with stirring until the yellow color persisted. A few crystals of triphenylphosphine were added to consume excess bromine followed by pyridine (1.21 ml), and N-[(2-hydroxymethyl)-3-sulfonyl]pyrrole dissolved in methylene chloride (10 ml) was added. The reaction was stirred at 0° C for 1 hr and concentrated to give a crude product. The crude product was purified by flash chromatography on silica gel using 10:1 hexane in ethyl acetate to give N-[2-(bromomethyl)thiophene-3-sulfonyl]pyrrole (2.7 g, 80% yield).

**D. N-{2-[(3,4-methylenedioxy)benzyl]thiophene-3-sulfonyl]}pyrrole**

N-{2-[(3,4-methylenedioxy)benzyl]thiophene-3-sulfonyl]}pyrrole was prepared in the same manner as described in Reference Example 32C using N-[2-(bromomethyl)thiophene-3-sulfonyl]pyrrole and 3,4-methylenedioxyphenylboronic acid, 53% yield.

**E. 3-chlorosulfonyl-2-[(3,4-methylenedioxy)benzyl]thiophene**

3-chlorosulfonyl-2-(3,4-methylenedioxybenzyl)thiophene was prepared in the same manner as described in Reference Example 65E from N-{2-[(3,4-methylenedioxy)benzyl]-3-sulfonyl}pyrrole by basic hydrolysis of the sulfonamide to the sodium salt of sulfonic acid and by conversion of this salt to the corresponding sulfonyl chloride, 54% yield.

**F. N-(4-bromo-3-methyl-5-isoxazolyl)-2-[(3,4-methylenedioxybenzyl]-thiophene-3-sulfonamide**

N-(4-bromo-3-methyl-5-isoxazolyl)-2-[(3,4-methylenedioxy)benzyl]-thiophene-3-sulfonamide was prepared in the same manner as described in Reference Example 2 by reacting 5-amino-4-bromo-3-methylisoxazole and 3-chlorosulfonyl-2-[(3,4-methylenedioxy)benzyl]thiophene. The crude product was purified by HPLC, 37% yield.

**REFERENCE EXAMPLE 115**

**N-(4-Bromo-3-methyl-5-isoxazolyl)-5-(2-methoxyphenyl)thiophene-2-sulfonamide**

**A. N-[5-(2-methoxyphenyl)thiophene-2-sulfonyl]pyrrole**

N-[5-(2-methoxyphenyl)thiophene-2-sulfonyl]pyrrole was prepared in the same manner as described in Reference Example 32C using 2-methoxyphenylboronic acid and N-(5-bromothiophenesulfonyl)pyrrole, 74% yield.

**B. 5-chlorosulfonyl-2-(2-methoxyphenyl)thiophene**

5-chlorosulfonyl-2-(2-methoxyphenyl)thiophene was prepared in the same manner as described in Reference Example 65E from N-[5-(2-methoxyphenyl)-thiophene-2-sulfonyl]pyrrole by hydrolysis of the sulfonamide to the sodium salt of sulfonic acid (83%) followed by conversion of the salt to the corresponding sulfonyl chloride, resulting in a 24% yield.

**C. N-(4-bromo-3-methyl-5-isoxazolyl)-5-(2-methoxyphenyl)thiophene-2-sulfonamide**

N-(4-bromo-3-methyl-5-isoxazolyl)-5-(2-methoxyphenyl)thiophene-2-sulfonamide was prepared in the same manner as described in Reference Example 1. Reaction of 2-chlorosulfonyl-5-(2-methoxyphenyl)thiophene with 5-amino-4-bromo-3-methylisoxazole gave N-(4-bromo-3-methyl-5-isoxazolyl)-5-(2-methyoxyphenyl)thiophene-2-sulfonamide in 32% yield, m.p. 114-117° C.

**REFERENCE EXAMPLE 116**

**N-(4-Bromo-3-methyl-5-isoxazolyl)-5-(2-tolyl)thiophene-2-sulfonamide**

**A. 2-(2-tolyl)thiophene**

Sodium carbonate (5 ml., 2M aqueous solution) followed by 2-methylphenylboronic acid (0.294 g, 2.4 mmol) were added to a solution of 2-bromothiophene (0.542 g, 2 mmol) and tetrakis(triphenylphosphine)-palladium(0) (100 mg) in toluene (5 ml) and ethanol (5 ml) under nitrogen. The mixture was refluxed for 2 hours, cooled to room temperature and extracted with ethyl acetate (2 x 50 ml). The combined organic layers was dried over $MgSO_4$ and evaporated. The residue was flash chromatographed on silica gel using hexane as eluent to afford 1.2 g of 2-(2-tolyl)thiophene as a colorless gum.

**B. 2-chlorosulfonyl-5-(2-tolyl)thiophene**

To the cold (-5 to 0° C) solution of 2-(2-tolyl)thiophene (0.87 g, 5 mmole) was added chlorosulfonic acid (0.33 ml, 5 mmole) over a 15 min. period with constant stirring. After 10 min., phosphorous oxychloride (2 ml) and phosphorous pentachloride were added. The reaction mixture was slowly allowed to attain ambient temperature and stirred for 3 hours. The mixture was then poured onto crushed ice (50 g) and was extracted with ethyl acetate (2 x 50 ml). The combined organic layers was dried over $MgSO_4$ and evaporated. The residue was purified by flash column chromatography on silica gel using 2% ethyl acetate in hexane to give 2-chlorosulfonyl-5-(2-tolyl)thiophene, (1.1 g, 72% yield).

**C. N-(4-bromo-3-methyl-5-isoxazolyl)-5-(2-tolyl)thiophene-2-sulfonamide**

N-(4-bromo-3-methyl-5-isoxazolyl)-5-(2-tolyl)thiophene-2-sulfonamide was prepared in the same manner as described in Reference Example 2. Reaction of 5-chlorosulfonyl-2-(2-tolyl)thiophene with 5-amino-4-bromo-3-methylisoxazole gave the crude product which was purified by column chromatography giving the pure product (gum). This gum was dissolved in 5 ml of $NH_4OH$, concentrated and dried under high vacuum to get the ammonium salt of N-(4-bromo-3-methyl-5-isoxazolyl)-5-(2-tolyl)thiophene-2-sulfonamide in 67% yield, m.p. 180-184° C ($NH_3^+$ salt).

**REFERENCE EXAMPLE 117**

**N-(4-Bromo-3-methyl-5-isoxazolyl)-5-(3-tolyl)thiophene-2-sulfonamide**

**A. 2-(3-tolyl)thiophene**

2-(3-tolyl)thiophene was prepared in the same manner as described in Reference Example 116A using 2-bromothiophene and 3-methylphenylboronic acid. The crude product was purified by flash chromatography on silica gel using hexane as the eluent (86% yield).

**B. 2-chlorosulfonyl-5-(3-tolyl)thiophene**

2-chlorosulfonyl-5-(3-tolyl)thiophene was prepared in the same manner as described in Example 117B from 2-(3-tolyl)thiophene, 22% yield.

**C. N-(4-bromo-3-methyl-5-isoxazolyl)-5-(3-tolyl)thiophene-2-sulfonamide**

N-(4-bromo-3-methyl-5-isoxazolyl)-5-(3-tolyl)thiophene-2-sulfonamide was prepared in the same manner as described in the Reference Example 116C using 2-chlorosulfonyl-5-(3-tolyl)thiophene and 5-amino-4-bromo-3-methylisoxazole. To obtain the ammonium salt of the final product, aqueous $NH_4OH$ was used (31% yield; hygroscopic).

**REFERENCE EXAMPLE 118**

**N-(4-Bromo-3-methyl-5-isoxazolyl)-3-benzylthiophene-2-sulfonamide**

**A. 3-benzylthiophene**

3-benzylthiophene was prepared in the same manner as described in Reference Example 32C using 3-thienylboronic acid and benzyl bromide, 74% yield.

**B. 2-chlorosulfonyl-3-benzylthiophene**

2-chlorosulfonyl-3-benzylthiophene was prepared in the same manner as described in Reference Example 117B using 3-benzylthiophene, 78% yield.

**C. N-(4-bromo-3-methyl-5-isoxazolyl)-3-benzylthiophene-2-sulfonamide**

N-(4-bromo-3-methyl-5-isoxazolyl)-3-benzylthiophene-2-sulfonamide was prepared in the same manner as described in Reference Example 2 by reacting 2-chlorosulfonyl-3-benzylthiophene with 5-amino-4-bromo-3-methylisoxazole resulting in a 24% yield, m.p. 180-183° C.

**REFERENCE EXAMPLE 119**

**N-(4-Bromo-3-methyl-5-isoxazolyl)-5-(2-methylfuranyl)thiophene-2-sulfonamide**

**A. N-[5-(2-methyl-5-furyl)thiophene-2-sulfonyl]pyrrole**

*t*-BuLi (1.7 m solution in hexane, 7.9 ml, 14.6 mmol) was added dropwise under constant stirring under a nitrogen atmosphere to a solution of 2-methylfuran (1.0 g, 12 mmol) in THE (20 ml) at -78° C. The solution was then warmed to -10° C and stirring continued for 45 min. The solution was then added to a solution of zinc chloride (27 ml of a 0.5 M solution in THF) at -30° C and then warmed to room temperature where stirring continued for 1 hr. resulting in a pale yellow clear solution. The solution was then transferred via a steel canula under nitrogen to a solution of N-(5-bromothiophene-2-sulfonyl)pyrrole Reference Example 33A, 3.5 g, 12 mmol) and tetrakis(triphenylphosphine)-palladium (0) (693 mg, 0.6 mmol) in THF (15 ml) at -78° C. The solution was then warmed to room temperature and stirred for a period of 2 hours. Purification by column chromatography using 2% ethyl acetate gave 680 mg of N-[5-(2-methyl-5-furyl)thlophene-2-sulfonyl]pyrrole as a pale yellow powder (19% yield).

**B. 2-(2-methyl-5-furyl)thiophene-5-sulfonyl chloride**

2-(2-methyl-5-furyl)thiophene-5-sulfonyl chloride was prepared in the same manner as described in Reference Example 33D from N-[5-(2-methyl-5-furyl)thiophene-2-sulfonyl]pyrrole (300 mg, 1.02 mmol). Purification by column chromatography using 2% ethyl acetate/hexanes gave 145 mg (53%) of the sulfonyl chloride as a pale yellow solid.

**C. N-(4-bromo-3-methyl-5-isoxazolyl)-5-(2-methyl-5-furyl)thiophene-2-sulfonamide**

N-(4-bromo-3-methyl-5-isoxazolyl)-5-(2-methyl-5-furyl)thiophene-2-sulfonamide was prepared in the same manner as described in Reference Example 2. Reaction of 2-(2-methyl-5-furyl)thiophene-5-sulfonyl chloride (55 mg, 0.21 mmol) with 5-amino-4-bromo-3-methylisoxazole (41 mg, 0.21 mmol), after purification by column chromatography using 10% $MeOH/CHCl_3$, gave 45 mg of the pure sulfonamide as a brown semisolid ( 54% yield, m.p. 123-124° C).

**REFERENCE EXAMPLE 120**

**N-(4-Bromo-3-methyl-5-isoxazolyl)-4-(phenethyl)thiophene-2-sulfonamide**

N-(4-bromo-3-methyl-5-isoxazolyl)-4-(phenethyl)thiophene-2-sulfonamide was prepared in the same manner as described in Reference Example 2 using 5-amino-4-bromo-3-methyl-isoxazole (132.75 mg, 0.75 mmole) and 4-(phenethyl)thiophene-2-sulfonyl chloride (Example 119D; 225.62 mg, 0.75 mmol). The product was purified by HPLC (5-95% acetonitrile with 0.1% TFA over 30 min.) to give N-(4-bromo-3-methyl-5-isoxazolyl)-4-(phenethyl)thiophene-2-sulfonamide as a brownish oil 72.3mg, 32% yield.

**REFERENCE EXAMPLE 121**

**N-(3,4-dimethyl-5-isoxazolyl)-2-[(4-methylphenyl)aminocarbonyl]-thiophene-3-sulfonamide**

Phosphonitrilic chloride trimer dissolved in THF (5 ml) was added to a suspension of N-(3,4-dimethyl-5-isoxazolyl)-2-(carboxyl)thiophene-3-sulfonamide (2.0 g, 6.6 mmol) (Reference Example 28) in THF (5 ml) and $Et_3N$ at 0° C. The cold bath was removed and the reaction mixture stirred at room temperature for 2 hours. The mixture was diluted with water (150 ml) and acidified to pH 2 using concentrated hydrochloric acid. The reaction mixture was then extracted with methylene chloride (2 x 100 ml), and the combined organic layers was washed with 2N hydrochloric acid (3 x 100 ml), dried over $MgSO_4$, and concentrated be get crude product. The crude product was dissolved in ether and allowed to stand at room temperature to give a precipitate which was filtered and washed with cold ether giving N-(3,4-dimethyl-5-isoxazolyl)-2-[(4-methylphenyl)aminocarbonyl]thiophene-3-sulfonamide (1.6 g, 61% yield).

**REFERENCE EXAMPLE 122**

**N-(4-Bromo-3-methyl-5-isoxazolyl)-5-[(4-tolyl)aminocarbonyl]thiophene-2-sulfonamide**

N-(4-bromo-3-methyl-5-isoxazolyl)-5-[(4-tolyl)aminocarbonyl]-thiophene-2-sulfonamide was prepared in the same manner as described in Reference Example 24 from N-(4-bromo-3-methyl-5-isoxazolyl)-5-(4-carboxybenzene) thiophene-2-sulfonamide (Reference Example 148), 110 mg, 0.25 mmol) and 4-methylaniline (53 mg, 0.49 mmol). Purification by recrystallization from methanol/water gave 91 mg of the pure sulfonamide as a light tan powder (61% yield, m.p. 188° C).

**REFERENCE EXAMPLE 123**

**N-(4-chloro-3-methyl-5-isoxazolyl)-2-[(4-tolyl)aminocarbonyl]thiophene-3-sulfonamide**

**A. N-(4-chloro-3-methyl-5-isoxazolyl)-2-(carboxyl)thiophene-3-sulfonamide**

N-(4-chloro-3-methyl-5-isoxazolyl)-2-(carboxyl)thiophene-3-sulfonamide was prepared in the same manner as described in Reference Example 17 using N-(4-chloro-3-methyl-5-isoxazolyl)-2-(carbomethoxy)thiophene-3-sulfomanide (Example 123), 78% yield.

**B. N-(4-chloro-3-methyl-5-isoxazolyl)-2-[(4-tolyl)aminocarbonyl]-thiophene-3-sulfonamide**

N-(4-chloro-3-methyl-5-isoxazolyl)-2-[(4-tolyl)aminocarbonyl]-thiophene-3-sulfonamide was prepared in the same manner as described in Reference Example 122 using N-(4-chloro-3-methyl-5-isoxazolyl)-2-(carboxyl)thiophene-3-sulfonamide. The crude product was dissolved in a small quantity of EtOAc (2 ml) and ether (15 ml) was added. The resulting precipitate was filtered and washed with cold ether (50 ml) giving N-(4-chloro-3-methyl-5-isoxazolyl)-2-[(4-tolyl)aminocarbonyl]-thiophene-3-sulfonamide in 53% yield (m.p. 177-179°).

**REFERENCE EXAMPLE 124**

**N-(4-Bromo-3-methyl-5-isoxazolyl)-2-[(N-methyl)-N-phenylaminocarbonyl]-thiophene-3-sulfonamide**

A solution of N-(4-bromo-3-methyl-5-isoxazolyl)-2-(carboxyl)-thiophene-3-sulfonamide (183.6 mg, 0.5 mmol) in dry THF (I ml) was added to a solution of N-methyl-4-methylaniline (0.066 ml, 0.6 mmol) in THF (0.5 ml). Triethylamine (0.63 ml, 4.2 mmol) was added to the mixture and, after 10 min., a solution of phosphonitrilic chloride trimer (210.7 mg, 0.6 mmol) was added. The mixture was stirred for 1 hr. at 50°C, cooled, neutralized with 10 ml 1N HCI (pH 3) and extracted with ethyl acetate. The extract was dried over anhydrous magnesium sulfate and the solvent was removed under reduced pressure to give a crude product which was purified by HPLC (5-95% acetonitrile with 0.1 % TFA over 30 min.) resulting in N-(4-bromo-3-methyl-5-isoxazolyl)-2-[(N-methyl)-N-phenylaminocarbonyl]thiophene-3-sulfonamide as a white solid (92.1 mg, 39.4% yield, m.p. 51-55°C).

**REFERENCE EXAMPLE 125**

**N-(4-Bromo-3-methyl-5-isoxazolyl)-3-[3,4-(methylenedioxy)phenyl]-thiophene-2-sulfonamide**

**A. 3-bromothiophene-2-sulfonyl chloride**

Chlorosulfonic acid (20 ml, 300 mmol) was added to a solution of 3-bromothiophene (8.15 g, 50 mmol) in methylene chloride (50 ml) at -78° C over a 20 min. period. After the completion of addition, the cold bath was removed and stirring continued at ambient temperature for 1 hr. The reaction mixture was carefully added, dropwise, to crushed ice (100 g). The mixture was extracted with methylene chloride (2 x 100 ml). The combined organic layers was dried over $MgSO_4$ and evaporated. The crude product was purified by flash. chromatography on silica gel using hexane as the eluent resulting in 3-bromothiophene-2-sulfonyl chloride (4 g, 30% yield) and 4-bromothiophene-2-sulfonyl chloride (200 mg, ≤ 1%).

**B. N-(3-bromothiophene-2-sulfonyl)pyrrole**

N-(3-bromothiophene-2-sulfonyl)pyrrole was prepared in the same manner as described in Reference Example 33A by reacting 3-bromothiophene-2-sulfonylchloride with pyrrole (for 16 hr.). N-(3-bromothiophene-2-sulfonyl) pyrrole was obtained in 54% yield.

**C. N-{[3-(3,4-methylenedioxy)phenyl]thiophene-2-sulfonyl}pyrrole**

N-{[3-(3,4-methylenedioxy)phenyl]thiophene-2-sulfonyl}pyrrole was prepared in the same manner as described in Reference Example 32C using 3,4-methylenedioxyphenylboronic acid and N-(3-bromothiophene-2-sulfonyl) pyrrole. The crude product was purified by flash column chromatography on silica gel using 2% EtOAc in hexane as the eluent resulting in N-{[3-(3,4-methylenedioxy)phenyl]thiophene-2-sulfonyl}-pyrrole in a 90% yield.

**D. 2-chlorosulfonyl-3-[3,4-(methylenedioxy)phenyl]thiophene**

2-chlorosulfonyl-3-[3,4-(methylenedioxy)phenyl]hiophene was prepared in the same manner as described in Reference Example 112B using N-{[3-(3,4-methylenedioxy)phenyl]thiophene-2-sulfonyl}pyrrole by basic hydrolysis of the sulfonamide to the sodium sulfonate (100% yield) followed by conversion of the salt to the corresponding sulfonyl chloride resulting in a 34% yield of the final product.

**E. N-(4-bromo-3-methyl-5-isoxazolyl)-3-[3,4-(methylenedioxy)phenyl]-thiophene-2-sulfonamide**

N-(4-bromo-3-methyl-5-isoxazolyl)-3-[3,4-(methylenedioxy)phenyl]-thiophene-2-sulfonamide was prepared

in the same manner as described in Reference Example 1 by reaction of 2-chlorosulfonyl-3-[3,4-(methylenedioxy)-phenyl]thiophene with 5-amino-4-bromo-3-methylisoxazole resulting in a 60% yield, m.p. 183-186° C.

**REFERENCE EXAMPLE 126**

**N-(4-Bromo-3-methyl-5-isoxazolyl)-2-[(2-chloro-3,4-methylenedioxy)-phenoxymethyllthiophene-3-sulfonamide**

**A. N-{2-[(3,4-methylenedioxy)phenoxymethyl]thiophene-3-sulfonyl}-pyrrole**

Sodium hydride (100 mg, 5 mmole) was added to a stirred solution of 3,4-methylenedioxyphenol (0.607 g, 4.5 mmol) in DMF (dry, 5 ml) at 0° C under a nitrogen atmosphere with stirring. The reaction mixture was permitted to attain room temp and stirring continued for 1 hr. The reaction mixture was cooled to 0° C and N-[(2-bromomethyl) thiophene-3-sulfonyl]pyrrole was added. Stirring was continued at ambient temperature for 16 hr. The reaction mixture was diluted with water (100 ml), extracted with ethyl acetate (2 x 50 ml) and washed with 1N NaOH (2 x 25 ml) to remove phenol derivative. The mixture was dried over $MgSO_4$ and concentrated resulting in N-{2-[(3,4-methylenedioxy) phenoxymethyl]thiophene-3-sulfonyl}pyrrole, which was recrystallized using hexane/EtOAc (1.0 g, 92% yield).

**B. 3-chlorosulfonyl-2-[(2-chloro-3,4-methylenedioxy)phenoxymethyl]-thiophene**

3-chlorosulfonyl-2-[(2-chloro-3,4-methylenedioxy)phenoxymethyl]-thiophene was prepared in the same manner as described in Reference Example 64E using N-{2-[(3,4-methylenedioxy)phenoxymethyl]thiophene-3-sulfonyl}-pyrrole by conducting a basic hydrolysis (using potassium hydroxide in iso-propanol) to the potassium sulfonate followed by conversion of the salt to the corresponding sulfonyl chloride in an overall yield of 50%.

**C. N-(4-bromo-3-methyl-5-isoxazolyl)-2-[(2-chloro-3,4-methylenedioxy)phenoxymethyl]thiophene-3-sulfonamide**

N-(4-bromo-3-methyl-5-isoxazolyl)-2-[(2-chloro-3,4-methylenedioxyphenoxy)methyl]thiophene-3-sulfonamide was prepared in the same manner as described in Reference Example 1 by reaction of 3-chlorosulfonyl-2-[(2-chloro-3,4-methylenedioxyphenoxy)methyl]thiophene with 5-amino-4-bromo-3-methylisoxazole, 47% yield, m.p. 152-154°C.

**REFERENCE EXAMPLE 127**

**N-(4-Bromo-3-methyl-5-isoxazolyl)-2-[*trans*-3,4-(methylenedioxy)cinnamyl]-thiophene-3-sulfonamide**

**A. Diethyl 2-{3-[(N-pyrrolyl)sulfonyl]thienylmethyl}phosphonate**

N-[2-bromomethyl)thiophene-3-sulfonyl]pyrrole (0.915 g, 3 mmol) was suspended in triethylphosphite (5 ml) and was heated to 140° C for 1 hr. with stirring under nitrogen atmosphere. Excess triethylphosphate was removed under reduced pressure and the residue was dried under vacuum resulting in 0.9g, 83% yield of diethyl 2-{3-[(N-pyrrolyl) sulfonyl]-thienylmethyl}phosphonate.

**B. N-{2-[*trans*-3,4-(methylenedioxy)cinnamyl]thiophene-3-sulfonyl}-pyrrole**

Sodium hydride (200 mg, 60% dispersion) was added in two lots to the stirred solution of diethyl 2-{3-[(N-pyrrolyl)sulfonyl]thienylmethyl}-phosphonate (900 mg, 2.48 mmol) in dry THF (10 ml) at 0° C. The mixture was stirred at room temperature for 1 hr. then piperonal (600 mg) was added. Stirring was continued for 12 hours. The mixture was diluted with water (100 ml) and extracted with methylene chloride (2 x 50 ml). The combined organic layers was dried over $MgSO_4$, evaporated, and the residue was flash chromatographed on silica gel using 0.5% ethyl acetate in hexane to give N-{2-[*trans*-(3,4-methylenedioxy)cinnamyl]-thiophene-3-sulfonyl}pyrrole (750 mg, 84% yield).

**C. 3-chlorosulfonyl-2-[*trans*-3,4-(methylenedioxy)cinnamyl]thiophene**

3-chlorosulfonyl-2-[*trans*-3,4-(methylenedioxy)cinnamyl]thiophene was prepared in the same manner as described in Reference Example 64E from N-{2-[*trans*-3,4-(methylenedioxy)cinnamyl]thiophene-3-sulfonyl}pyrrole by basic hydrolysis (using isopropanol and potassium hydroxide) to the corresponding potassium sulfonate (100%) followed

by conversion of the salt to the corresponding sulfonyl chloride in a 31% overall yield.

**D. N-(4-bromo-3-methyl-5-isoxazolyl)-2-[*trans*-3,4-(methylenedioxy)cinnamyl]thiophene-3-sulfonamide**

N-(4-bromo-3-methyl-5-isoxazolyl)-2-[*trans*-3,4-(methylenedioxy)-cinnamyl]thiophene-3-sulfonamide was prepared in the same manner as described in Reference Example 1 by reaction of 3-chlorosulfonyl-2-[*trans*-3,4-(methylenedioxy)cinnamyl]thiophene with 5-amino-4-bromo-3-methylisoxazole. The crude product was purified by HPLC resulting in a 33% yield, m.p. 147-149°C.

## REFERENCE EXAMPLE 128

### N-(4-Bromo-3-methyl-5-isoxazoltyl)-2-[3,4-(methylenedioxy)phenethyl]-thiophene-3-sulfonamide

**A. N-{2-[3,4-(methylenedioxy)phenethyl]thiophene-3-sulfonyl}pyrrole**

An ethyl acetate (15 ml) solution of N-{2-[*trans*-3,4-(methylenedioxy)cinnamyl]thiophene-3-sulfonyl}pyrrole (Reference Example 127B, 0.6 g, 1.67 mmol) was subjected to catalytic hydrogenation using 10% Pd-C (100 mg) at 55 psi for 14 hr. The catalyst was filtered and the filtrate concentrated to resulting in N-{2-[3,4-(methylenedioxy)phenethyl]thiophene-3-sulfonyl}pyrrole (0.55 g, 91% yield).

**B. 3-chlorosulfonyl-2-[3,4-(methylenedioxy)phenethyl]thiophene**

3-chlorosulfonyl-2-[3,4-(methylenedioxy)phenethyl]thiophene was prepared in the same manner as described in the Reference Example 64E using N-{2-[3,4-(methylenedioxy)phenethyl]thiophene-3-sulfonyl}pyrrole by conducting basic hydrolysis (iso-propanol and potassium hydroxide) of the sulfonamide to the potassium salt of sulfonic acid (93%) followed by conversion of the salt to the corresponding sulfonyl chloride in a 42% yield.

**C. N-(4-bromo-3-methyl-5-isoxezolyl)-2-[3,4-(methylenedioxy)-phenethyl]thiophene-3-sulfonamide**

N-(4-bromo-3-methyl-5-isoxazolyl)-2-[3,4-(methylenedioxy)-phenethyl]thiophene-3-sulfonamide was prepared in the same manner as described in Reference Example 2. By reacting 3-chlorosulfonyl-2-[3,4-(methylenedioxy) phenethyl]thiophene with 5-amino-4-bromo-3-methylisoxazole and purifying the crude product by HPLC, N-(4-bromo-3-methyl-5-isoxazolyl)-2-[3,4-(methylenedioxy)phenethyl]thiophene-3-sulfonamide was obtained in a 30% yield, m.p. 180° (dec.).

## REFERENCE EXAMPLE 129

### N-(4-Bromo-3-methyl-5-isoxazolyl)-3-(phenylthio)thiophene-2-sulfonamide

**A. 3-phenylthiothiophene-2-sulfonyl chloride**

A stirred solution of 3-(phenylthio)thiophene (1.0 g, 5.2 mmol) in 5 ml of dry THF was placed under an argon atmosphere and cooled to -78° C. n-butyl lithium (2.78 ml of 2.3M solution) was added over 20 min. and stirring was continued at this temperature for an additional 20 min. Sulfur dioxide gas was then bubbled in at -78° C for a period of 30 min., resulting in the formation of a yellow percipitate. This was immediately followed by dropwise addition of N-chlorosuccinimide (764 mg, 5.72 mmol) dissolved in THF. The mixture was warmed to room temperature and stirring continued for an additional 1.5 hr. The mixture was then concentrated and the residue dissolved in ether. The organic layer was washed with water, brine solution and dried over magnesium sulfate. Evaporation of solvents left a light brown oil which was subjected to flash chromatography. Elution with 2% ethylacetate-hexanes gave 840 mg (56%) of a pale yellow solid.

**B. N-(4-bromo-3-methyl-6-isoxazolyl)-3-(phenylthio)thiophene-2-sulfonamide**

N-(4-bromo-3-methyl-6-isoxazolyl)-3-(phenylthio)thiophene-2-sulfonamide was prepared in the same manner as described in Reference Example 2 using 5-amino-4-bromo-3-methylisoxazole (192 mg, 1.1 mmol) and 3-phenylthiothiophene-2-sulfonyl chloride (300 mg, 1.0 mmol). Purification by column chromatography using 10% MeOH/ $CHCl_3$ yielded 358 mg (83%) of the pure sulfonamide as a brown oil.

**REFERENCE EXAMPLE 130**

**N-(3,4 dimethyl-5-isoxazolyl)-3-(phenylaminocarbonyl)thiophene-2-sulfonamide.**

**A. N-(3,4-dimethyl-5-isoxazolyl)thiophene-2-sulfonamide**

N-(3,4-dimethyl-5-isoxazolyl)thiophene-2-sulfonamide was prepared in the same manner as described in Reference Example 14 using thiophene-2-sulfonyl chloride and 3,4-dimethylaminoisoxazole. Purification by column chromatography using 3% MeOH/$CHCl_3$ yielded 48% N-(3,4-dimethyl-5-isoxazolyl)thiophene-2-sulfonamide.

**B. N-(methyoxyethoxymethyl)-N-(3,4-dimethyl-5-isoxazolyl)thiophene-2-sulfonamide**

N-(methyoxyethoxymethyl)-N-(3,4-dimethyl-5-isoxazolyl)thiophene-2-sulfonamide was prepared in the same manner as described in Reference Example 32B using N-(3,4-dimethyl-5-isoxazolyl)thiophene-2-sulfonamide and methoxyethoxymethyl chloride resulting in 34% yield. HPLC analysis of the crude oil obtained after workup showed that the oil was approximately 96% pure and so was used in the next step without further purification.

**C. N-(methyoxyethoxymethyl)-N-(3,4-dimethyl-5-isoxazolyl)-5-trimethylsilyl)thiophene-2-sulfonamide**

A stirred solution of N-(methyoxyethoxymethyl)-N-(3,4-dimethyl-5-isoxazolyl)thiophene-2-sulfonamide (300 mg, 0.87 mmol) in 5.0 ml of dry THF was placed under an argon atmosphere and cooled to -78° C. Over the course of 20 min, a solution of t-BuLi in hexanes (461 ml of a 2.25 M solution) was added dropwise and stirring was continued at this temperature for about 25 min. Then neat trimethylsilychloride (135 ml, 1 mmol) was added dropwise and the solution was stirred at -78° C for 15 min. then at room temp. for 1.5 hr. TLC (1 % $CHCl_3$ in MeOH) showed complete reaction of starting material at this time thus, the reaction was quenched by addition of 2.0 ml of water. After evaporation of solvents the remaining residue was extracted into ethyl acetate, washed with brine solution and dried over magnesium sulfate. Purification by column chromatography using 20% ethyl acetate/hexanes gave the pure sulfonamide as a clear oil (52% yield).

**D. N-(methyoxyethoxymethyl)-N-(3,4-dimethyliisoxazolyl)-3-(phenylaminocarbonyl)-5-(trimethylsilyl) thiophene-2-sulfonamide**

A stirred solution of N-(methyoxyethoxymethyl)-N-(3,4-dimethyl-5-isoxazolyl)-5-trimethylsilyl)thiophene-2-sulfonamide (180 mg, 0.43 mmol) in 4 ml of dry THF was placed under an argon atmosphere and cooled to -78° C. At this temperature a solution of t-BuLi in hexanes (215 μl at a 2.55 M solution) was added dropwise and stirring was continued at -78° C for 0.5 hr. resulting in a clear yellow solution. Phenylisocyanate (77 μl, 0.65 mmol) was added dropwise at -78° C and the solution allowed to reach room temperature. The solution was then worked up as above in part C. Purification of the final product was achieved by column chromatography using 30% ethyl acetate/hexanes to give 108 mg of the sulfonamide a 54% yield.

**E. N-(3,4 dimethyl-5-isoxazolyl)-3-(phenylaminocarbonyl)thiophene-2-sulfonamide**

N-(3,4 dimethyl-5-isoxazolyl)-3-(phenylaminocarbonyl)thiophene-2-sulfonamide was prepared in the same manner as described in Reference Example 32D using N-(methyoxyethoxymethyl)-N-(3,4-dimethyliisoxazolyl)-3-(N-phenylcarboxamide-5-trimethylsilyl)thiophene-2-sulfonamide (108 mg, 0.23 mmol). Purification was achieved by recrystallization from acetonitrile/water to give 62 mg (71% yield) N-(3,4 dimethyl-5-isoxazolyl)-3-(phenylaminocarbonyl) thiophene-2-sulfonamide as a brown powder, m.p. 152°C.

**REFERENCE EXAMPLE 131**

**N-(3,4-dimethyl-5-isoxazolyl)-2-(α-hydroxybenzyl)thiophene-3-sulfonamide**

**A. Thiophene-3-sulfonyl chloride**

n-BuLi (2.38M, 17ml) was slowly added to a solution of 3-bromothiophene (6.5 g, 40 mmol) in ether (30 ml) at -78° C. The reaction was stirred at -78° C for 45 min. $SO_2$ was bubbled through the mixture for 15 minutes at -78° C followed by the addition of NCS (6.4 g, 48 mmol) as a suspension in THF (40 ml). The crude product was purified by column chromatography using 5% ethyl acetate/hexanes to give 3.92 g thiophene-3-sulfonyl chloride as a pale

yellow solid (54% yield).

**B. N-(3,4-dimethyl-5-isoxazolyl)thiophene-3-sulfonamide**

N-(3,4-dimethyl-5-isoxazolyl)thiophene-3-sulfonamide was prepared in the same manner as described in Reference Example 14 using thiophene-3-sulfonyl chloride and 3,4-dimethylaminoisoxazole resulting in 66% yield as a pale brown solid.

**C. 2-[2-(trimethylsilyl)ethoxymethyl]-N-(3,4-dimethyl-5-isoxazolyl)thiophene-3-sulfonamide**

N,N-diisoproplyethylamine (222 μl, 128 mmol) was added to a solution of N-(3,4-dimethyl-5-isoxazolyl)thiophene-3-sulfonamide (300 mg, 1.16 mmol) in methylene chloride (5 ml), and the mixture was stirred at room temperature for 15 min. The mixture was then cooled to 0° C and 2-(trimethysilyl)ethoxy methyl chloride (SEM chloride) (226 μ1, 1.28 mmol) was added dropwise via syringe, and the resultant yellow solution was stirred at room temperature for 5 hours. Evaporation of solvents left an oil which was extracted into ethyl acetate, washed with water and brine solution and dried over magnesium sulfate. Flash chromatography of the residue using 10% ethyl acetate/hexanes yielded 321 mg 2-[2-(trimethylsilyl)ethoxymethyl]-N-(3,4-dimethyl-5-isoxazolyl)thiophene-3-sulfonamide as a clear colorless oil which solidified into a white solid upon standing (71% yield).

**D. 2-[2-(trimethylsilyl)ethoxymethyl]-N-(3,4-dimethyl-5-isoxazolyl)-2-(α-hydroxybenzyl)thiophene-3-sulfonamide**

n-BuLi (2.39 M, 177 μl) was slowly added to a solution of (2-trimethylsilylethoxymethyl)-N-(3,4-dimethyl-5-isoxazolyl)thiophene-3-sulfonamide (156 mg, 0.38 mmol) in THF at -78° C under nitrogen. The reaction was stirred at -78° C for 45 min., then benzaldehyde (45 μl, 0.42 mmol) was added in one lot at -78° C and the solution was allowed to come to room temperature. Stirring was continued for 1 hr. Purification was achieved by column chromatography using 10% ethyl acetate/hexanes to give 179 mg 2-[2-(trimethylsilyl)ethoxymethyl]-N-(3,4-dimethyl-5-isoxazolyl)-2-(α-hydroxybenzyl)thiophene-3-sulfonamide as a yellow viscous oil (90% yield).

**E. N-(3,4-dimethyl-5-isoxazolyl)-2-(α-hydroxybenzyl)thiophene-3-sulfonamide**

To a solution of 2-[2-(trimethylsilyl)ethoxymethyl]-N-(3,4-dimethyl-5-isoxazolyl)-2-(α-hydroxybenzyl)thiophene-3-sulfonamide (70 mg, 0-14 mmol) in DMF (2 ml) was added cesium fluoride (26 mg, 0.17 mmole) in one portion. The resulting mixture was heated to 100° C for 10 hours. The solvents were removed by evaporation under vacuum and the remaining residue was extracted into ethyl acetate, washed with water, brine, and dried over $MgSO_4$. The product was then purified by chromatography using 50-70% ethyl acetate/hexanes to give 26.2 mg N-(3,4-dimethyl-5-isoxazolyl)-2-(α-hydroxybenzyl)thiophene-3-sulfonamide as a pale white semisolid (51% yield).

**REFERENCE EXAMPLE 132**

**N-(4-Bromo-5-methyl-3-isoxazolyl)-5-(4-methylphenyl)thiophene-2-sulfonamide**

**A. N-[5-(4-methylphenyl)thiophene-2-sulfonyl]pyrrole**

N-[5-(4-methylphenyl)thiophene-2-sulfonyl]pyrrole was prepared in the same manner as described in Reference Example 32C using 4-methyl-phenylboronic acid and N-(5-bromothiophenesulfonyl)pyrrole. Purification by column chromatography using 2% ethyl acetate/hexanes gave N-[5-(4-methylphenyl)thiophene-2-sulfonyl]pyrrole as a pale yellow solid in 77% yield.

**B. 2-chlorosulfonyl-5-(4-methylphenyl)thiophene**

2-chlorosulfonyl-5-(4-methylphenyl)thiophene was prepared in the same manner as described in Reference Example 33D using N-[5-(4-methylphenyl)-thiophene-2-sulfonyl]pyrrole. Purification by column chromatography using 2% ethyl acetate/hexanes gave 2-chlorosulfonyl-5-(4-methylphenyl)thiophene as a pale yellow powder (61% yield).

**C. N-(4-bromo-3-methyl-5-isoxazolyl)-5-(4-methylphenyl)thiophene-2-sulfonamide**

N-(4-bromo-3-methyl-5-isoxazolyl)-5-(4-methylphenyl)thiophene-2-sulfonamide was prepared in the same

manner as described in Reference Example 2. Reaction of 2-chlorosulfonyl-5-(4-methylphenyl)thiophene (100 mg, 0.37 mmol) with 5-amino-4-bromo-3-methylisoxazole (65 mg, 0.37 mmol) yielded, after column chromatography using 10% MeOH/$CHCl_3$, 96 mg final product as a pale yellow solid, (63% yield, m.p. 175° C).

**REFERENCE EXAMPLE 133**

**N-(4-Bromo-3-methyl-5-isoxazolyl)-5-(4-phenyl)thiophene-2-sulfonamide**

**A. N-(pyrrole)-5-(4-phenyl)thiophene-2-sulfonamide**

N-(pyrrole)-5-(4-phenyl)thiophene-2-sulfonamide was prepared in the same manner as described in Reference Example 32C using phenyl boronic acid and N-(5-bromothiophenesulfonyl)pyrrole. Purification by column chromatography using 2% ethyl acetate/hexanes gave the pure sulfonamide as a yellow powder in 67% yield.

**B. 2-chlorosulfonyl-5-(4-phenyl)thiophene**

2-chlorosulfonyl-5-(4-phenyl)thiophene was prepared in the same manner as in Reference Example 33D from N-(pyrrole)-5-(4-phenyl)thiophene-2-sulfonamide. Purification by column chromatography using 2% ethyl acetate/hexanes gave the pure thiophene in 77% yield as a yellow powder.

**C. N-(4-bromo-3-methyl-5-isoxazolyl)-5-(4-phenyl)thiophene-2-sulfonamide**

N-(4-bromo-3-methyl-5-isoxazolyl)-5-(4-phenyl)thiophene-2-sulfonamide was prepared in the same manner as described in Reference Example 2. Reaction of 2-chlorosulfonyl-5-(4-phenyl)thiophene (94 mg, 0.36 mmol) with 5-amino-4-bromo-3-methyl isoxazole (64 mg, 0.36 mmol) yielded. after column chromatography using 10% MeOH/$CHCl_3$, 85 mg of N-(4-bromo-3-methyl-5-isoxazolyl)-5-(4-phenyl)thiophene-2-sulfonamide as a light brown solid, (59% yield, m.p. 132 ° C).

**REFERENCE EXAMPLE 134**

**N-(4-bromo-3-methyl-5-isoxazolyl)-5-[4-(trifluoromethyl)phenyl]thiophene-2-sulfonamide**

**A. N-{5-[4-(trifluoromethyl)phenyl]thiophene-2-sulfonyl}pyrrole**

N-{5-[4-(trifluoromethyl)phenyl]thiophene-2-sulfonyl}pyrrole was prepared in the same manner as described in Reference Example 32C using 4-trifluoromethylbenzene boronic acid and N-(5-bromothiophene sulfonyl)-pyrrole. Purification by column chromatography using 2% ethyl acetate/hexanes gave the pure sulfonamide as a white powder in 75% yield.

**B. 2-chlorosulfonyl-5-[4-(trifluoromethyl)phenyl]thiophene**

2-chlorosulfonyl-5-[4-(trifluoromethyl)phenyl]thiophene was prepared in the same manner as Reference Example 33D from N-{5-[4-(trifluoromethyl)phenyl]thiophene-2-sulfonyl)pyrrole. Purification by column chromatography using 2% ethyl acetate/hexanes gave the pure thiophene as a white powder in 41 % yield.

**C. N-(4-bromo-3-methyl-5-isoxazolyl)-5-[4-(trifluoromethyl)phenyl]-thiophene-2-sulfonamide**

N-(4-bromo-3-methyl-5-isoxazolyl)-5-[4-(trifluoromethyl)phenyl]-thiophene-2-sulfonamide was prepared in the same manner as described in Reference Example 2. Reaction of 2-chlorosulfonyl-5-[4-(trifluoromethyl)-phenyl] thiophene (100 mg, 0.31 mmol) with 5-amino-4-bromo-3-methyl isoxazole (54 mg, 0.31 mmol) yielded, after column chromatography, 39 mg of N-(4-bromo-3-methyl-5-isoxazolyl)-5-[4-(trifluoromethyl)phenyl]-thiophene-2-sulfonamide as a pale yellow powder, (27% yield, m.p, 132° C).

## REFERENCE EXAMPLE 135

### N-(4-Bromo-3-methyl-5-isoxazolyl)-5-(2-formylphenyl)thiophene-2-sulfonamide

### A. N-(3-methyl-5-isoxazolyl)-5-bromothiophene-2-sulfonamide

N-(3-methyl-5-isoxazolyl)-5-bromothiophene-2-sulfonamide was prepared in the same manner as described in Reference Example 14 from 5-bromothiophene-2-sulfonyl chloride and 5-amino-3-methyl isoxazole. Purification was achieved by extraction of the crude sulfonamide into aqueous 2N NaOH, washing of the aqueous layer with ethyl acetate and acidification, using concentrated HCl, to pH - 2. Re-extraction into ethyl acetate was followed by washing of the organic material with water, brine and drying over magnesium sulfate. After evaporation of solvents, a brownish solid remained which was sufficiently pure to use in the next step.

### B. N-(3-methyl-5-isoxazolyl)-5-(2-formylphenyl)thiophene-2-sulfonamide

N-(3-methyl-5-isoxazolyl)-5-(2-formylphenyl)thiophene-2-sulfonamide was prepared in the same manner as described in Reference Example 32C from 2-formylbenzeneboronic acid (281 mg, 1.87 mmol) and N-(3-methyl-5-isoxazolyl)-5-bromothiophene-2-sulfonamide (550 mg, 1.7 mmol). Purification by column chromatography using 15% MeOH/$CHCl_3$ gave 163 mg (28%) of the pure sulfonamide as a brown oil.

### C. N-(4-bromo-3-methyl-5-isoxazolyl)-5-(2-formylphenyl)thiophene-2-sulfonamide

N-bromosuccinamide (81 mg, 0.45 mmol) was added to a solution of N-(3-methyl-5-isoxazolyl)-5-(2-formylphenyl)thiophene-2-sulfonamide (155 mg, 0.45 mmol) in $CHCl_3$ (5 ml). The resulting brownish solution was stirred at room temperature for 3 hours. The solvent was stripped off and the material extracted into ethyl acetate and washed with brine solution. Evaporation of solvents gave 85 mg of the product (45% yield). A portion of this was further purified by preparative HPLC. N-(4-bromo-3-methyl-5-isoxazolyl)-5-(2-formylphenyl)thiophene-2-sulfonamide was isolated as a pale brown oil.

## REFERENCE EXAMPLE 136

### N-(4-Bromo-3-methyl-5-isoxazolyl)-5-(3-aminophenyl)thiophene-2-sulfonamide

### A. N-(3-methyl-5-isoxazolyl)-5-(3-aminophenyl)thiophene-2-sulfonamide

N-(3-methyl-5-isoxazolyl)-5-(3-aminophenyl)thiophene-2-sulfonamide was prepared in the same manner as described in Reference Example 32C from 3-amino benzene boronic acid (256 mg, 1.87 mmol) and N-(3-methyl-5-isoxazolyl)-5-bromothiophene-2-sulfonamide (55 mg, 1.7 mmol). Purification by column chromatography using 15% MeOH/$CHCl_3$ gave 318 mg (56%) of the product.

### B. N-(4-bromo-3-methyl-5-isoxazolyl)-5-(3-aminophenyl)thiophene-2-sulfonamide

N-(4-bromo-3-methyl-5-isoxazolyl)-5-(3-aminophenyl)thiophene-2-sulfonamide was prepared in the same manner as described in Reference Example 30A (without acetic acid) using N-(3-methyl-5-isoxazolyl)-5-(3-aminophenyl)thiophene-2-sulfonamide resulting in a 33% yield. Further purification was achieved using preparative HPLC giving pure N-(4-bromo-3-methyl-5-isoxazolyl)-5-(3-aminophenyl)thiophene-2-sulfonamide as a clear colorless oil.

## REFERENCE EXAMPLE 137

### N-(4-Bromo-3-methyl-5-isoxazolyl)-5-(3,3-dimethylbutyn-1-yl)thiophene-2-sulfonamide

### A. N-[5-(3,3-dimethylbutyn-1-yl)thiophene-2-sulfonyl]pyrrole

A mixture of N-(5-bromothiophene-2-sulfonyl)pyrrole (600 mg, 2.05 mmol), 3,3-dimethyl-1-butyne (338 mg, 4.1 mmol), copper iodide (39 mg, 0.21 mmol), tetrakistriphenylphosphine palladium [$Pd(PPh_3)_4$] (118 mg, 0.1 mmol) and piperidine (5 ml) was stirred at room temperature for a period of 24 hours under a nitrogen, atmosphere. The mixture was then diluted with water (10 ml) and extracted with 3 x 25 ml portions of ether. The combined ether extracts are washed with brine and dried over $MgSO_4$. The solvent was removed under reduced pressure and crude product

purified by column chromatography using 2% ethyl acetate/hexanes to give 423 mg N-[5-(3,3-dimethylbutyn-1-yl)thiophene-2-sulfonyl]pyrrole as a yellow powder (70% yield).

**B. 2-chlorosulfonyl-5-(3,3-dimethylbutyn-1-yl)thiophene**

2-chlorosulfonyl-5-(t-butylethynyl)thiophene was prepared in the same manner as described in Reference Example 33D from N[5-(3,3-dimethylbutyn-1-yl)thiophene-2-sulfonamide. Purification by column chromatography using 2% ethyl acetate/hexanes gave the pure sulfonamide in 33% yield.

**C. N-(4-bromo-3-methyl-5-isoxazolyl)-5-(3,3-dimethylbutyn-1-yl)thiophene-2-sulfonamide**

N-(4-bromo-3-methyl-5-isoxazolyl)-5-(3,3-dimethylbutyn-1-yl)thiophene-2-sulfonamide was prepared in the same manner as described in Reference Example 2. Reaction of 2-chlorosulfonyl-5-(3,3-dimethylbutyn-1-yl)thiophene (120 mg. 0.46 mmol) with 5-amino-4-bromo-3-methylisoxazole (85 mg, 0.48 mmol) yielded, after column chromatography using 10% MeOH/$CHCl_3$, 116 mg N-(4-bromo-3-methyl-5-isoxazolyl)-5-(3,3-dimethylbutyn-1-yl)thiophene-2-sulfonamide as a viscous clear oil (63%).

**REFERENCE EXAMPLE 138**

**N-(4-Bromo-3-methyl-5-isoxazolyl)-5-[3,5-bis(trifluoromethyl)phenyl]-thiophene-2-sulfonamide**

**A. N-{5-[3,5-bis(trifluoromethyl)phenyl]thiophene-2-sulfonyl}pyrrole**

N-{5-[3,5-bis(trifluoromethyl)phenyl]thiophene-2-sulfonyl}pyrrole was prepared in the same manner as described in Reference Example 32C from 3,5-bis(trifluoromethyl)benzeneboronic acid (619 mg, 2.26 mmol) and N-[5-bromothiophene-2-sulfonyl]pyrrole (60 mg, 2.05 mmol). Purification by column chromatography using 2% ethyl acetate/hexanes gave the pure sulfonamide as a white solid in 93% yield.

**B. 2-chlorosulfonyl-5-[3,5-bis(trifluoromethyl)phenyl]thiophene**

2-chlorosulfonyl-5-[3,5-bis(trifluoromethyl)phenyl]thiophene was prepared in the same manner as described in Reference Example 33D from N-{5-[3,5-bis(trifluoromethyl)phenyl]thiophene-2-sulfonyl}pyrrole. Purification by column chromatography using 2% ethyl acetate/hexanes gave the pure thiophene in 73% yield as a brownish clear oil.

**C. N-(4-bromo-3-methyl-5-isoxazolyl)-5-[3,5 bis(trifluoromethyl)-phenyl]thiophene-2-sulfonamide**

N-(4-bromo-3-methyl-5-isoxazolyl)-5-[3,5 bis(trifluoromethyl)-phenyl]thiophene-2-sulfonamide was prepared in the same manner as in Reference Example 2. Reaction of 2-chlorosulfonyl-5-[3,5-bis(trifluoromethyl)-phenyl]thiophene (250 mg, 0.63 mmol) with 5-amino-4-bromo-3-methylisoxazole (118 mg, 0.67 mmol) yielded, after column chromatography using 5% MeOH/$CHCl_3$, 115.2 mg N-(4-bromo-3-methyl-5-isoxazolyl)-5-[3,5 bis(trifluoromethyl)phenyl]thiophene-2-sulfonamide as a white powder (34% yield). A portion of this sample was further purified by preparative HPLC, m.p. 140° C.

**REFERENCE EXAMPLE 139**

**N-(4-Bromo-3-methyl-5-isoxazolyl)-5-(5-methyl-2-thienyl)thiophene-2-sulfonamide**

**A. 2-methylthiophene-5-boronic acid**

n-BuLi (2.38 M, 16 ml) was slowly added to a solution of 2-methyl thiophene (3.0 g, 31 mmol) in THF (20 ml) at -78° C. The solution was kept at -78° C for 10 min. then warmed to 0° C for an additional 0.5 hr. The solution was then transferred by steel canula under nitrogen into a vessel containing triisopropylborate (6.3 g, 33 mmol) in ether (15 ml) at -78° C. The resulting milky white solution was stirred at -78° C for 20 min. then at room temperature for 2 hours. The reaction was quenched by the addition of 10% aqueous HCl (5.0 ml) and the solution was extracted into ether. The combined ether extracts were extracted with 10 M NaOH (2 x 30 ml), the aqueous extracts were acidified with dilute HCl to pH 2 and extracted into ether (3 x 25 ml). The combined ether extracts were washed once with water (10 ml), once with brine (10 ml) and dried and evaporated to give 3.91 g 2-methylthiophene-5-boronic acid as a light brown solid. This was used in the next step with no further purification.

**B. N-[5-(5-methyl-2-thienyl)thiophene-2-sulfonyl]pyrrole**

N-[5-(5-methyl-2-thienyl)thiophene-2-sulfonyl]pyrrole was prepared in the same manner as described in Reference Example 32C from 2-methylthiophene-5-boronic acid and N-(5-bromothiophene-2-sulfonyl)pyrrole. Purification by column chromatography using 2% ether/hexanes gave the pure sulfonamide in 72% yield as a white solid.

**C. 2-chlorosulfonyi-5-(5-methyl-2-thienyl)thiophene**

2-chlorosulfonyl-5-(5-methyl-2-thienyl)thiophene was prepared in the same manner as described in Reference Example 33D from N-[5-(5-methyl-2-thienyl)thiophene-2-sulfonyl]pyrrole (570 mg, 1.84 mmol). Purification by column chromatography using 2% ethyl acetate/hexanes gave 258 mg (50%) of the sulfonyl chloride as a light green solid.

**D. N-(4-bromo-3-methyl-5-isoxazolyl)-5-(5-methyl-2-thienyl)thiophene-2-sulfonamide**

N-(4-bromo-3-methyl-5-isoxazolyl)-5-(5-methyl-2-thienyl)thiophene-2-sulfonamide was prepared in the same manner as described in Reference Example 2. Reaction of 2-chlorosulfonyl-5-(5-methyl-2-thienyl)thiophene (200 mg, 0.72 mmol) with 5-amino-4-bromo-3-methylisoxazole (127 mg, 0.72 mmol) yielded 273 mg (90%) of the crude sulfonamide. After passing through a small plug of silica gel, a portion of the product was further purified by preparative HPLC to give the pure sulfonamide as a white powder, m.p. 161-162° C.

**REFERENCE EXAMPLE 140**

**N-(4-Bromo-3-methyl-5-isoxazolyl)-5-(5-ethyl-2-thienyl)thiophene-2-sulfonamide**

**A. 2-ethylthiophene-5-boronic acid**

2-ethylthiophene-5-boronic acid was prepared in the same manner as described in Reference Example 139A from 2-ethylthiophene (2.0 g, 18 mmol). Evaporation of solvents after workup gave 2.16g (78%) of 2-ethylthiophene-5-boronic acid as a white sold which was used on the next step with no further purification.

**B. N-[5-(5-ethyl-2-thienyl)thiophene-2-sulfonyl]pyrrole**

N-[5-(5-ethyl-2-thienyl)thiophene-2-sulfonyl]pyrrole was prepared in the same manner as described in Reference Example 32C from 2-ethylthiophene-5-boronic acid (411 mg, 2.64 mmol) and N-(5-bromothiophene-2-sulfonyl)-pyrrole (700 mg, 2.39 mmol). Purification by column chromatography using 2% ethyl acetate/hexanes gave 6.30 mg of the pure product as a dark brown solid (90% yield).

**C. 2-chlorosulfonyl-5-(5-ethyl-2-thienyl)thiophene**

2-chlorosulfonyl-5-(5-ethyl-2-thienyl)thiophene was prepared in the same manner as described in Example 33D from N-(pyrrole)-5-(5-ethyl-2-thienyl)thiophene-2-sulfonamide (630mg, 2.16 mmol). Purification by column chromatography using 1 % ethyl acetate/hexanes gave 400.3 mg of the pure sulfonyl chloride as a bright yellow solid (57% yield).

**D. N-(4-bromo-3-methyl-5-isoxazolyl)-5-(5-ethyl-2-thienyl)thiophene-2-sulfonamide**

N-(4-bromo-3-methyl-5-isoxazolyl)-5-(5-ethyl-2-thienyl)thiophene-2-sulfonamide was prepared in the same manner as described in Reference Example 2. Reaction of 2-chlorosulfonyl-5-(5-ethyl-2-thienyl)thiophene (200 mg, 0.68 mmol) with 5-amino-4-bromo-3-methyl isoxazole (121 mg, 0.68 mmol) yielded 174 mg N-(4-bromo-3-methyl-5-isoxazolyl)-5-(5-ethyl-2-thienyl)-thiophene-2-sulfonamide (59% yield). After passing through a small plug of silica gel with elution using 10% MeOH/$CHCl_3$, a small fraction of the product was further purified using preparative HPLC to give the sulfonamide as a light tan colored powder, m.p. 126°C.

**REFERENCE EXAMPLE 141**

**N-(4-chloro-3-methyl-5-isoxazolyl)-5-(5-ethyl-2-thienyl)thiophene-2-sulfonamide**

N-(4-chloro-3-methyl-5-isoxazolyl)-5-(5-ethyl-2-thienyl)thiophene-2-sulfonamide was prepared in the same manner as in Reference Example 2. Reaction of 2-chlorosulfonyl-5-(5-ethyl-2-thienyl)thiophene (Example 141C, 200 mg, 0.68 mmol) with 5-amino-4-chloro-3-methylisoxazole (91 mg, 0.68 mmol) yielded 188 mg of the final product (71% yield). A small portion of the product was further purified by preparative HPLC to give the pure sulfonamide as a tan colored solid.

**REFERENCE EXAMPLE 142**

**N-(4-Bromo-3-methyl-5-isoxazolyl)-5-(benzo[b]thien-2-yl)thiophene-2-sulfonamide**

**A. Benzo[b]thiophene-2-boronic acid**

Benzo[b]thiophene-2-boronic acid was prepared in the same manner as described in Reference Example 140A from benzo[b]thiophene except that t-BuLi was used as the base in place of n-BuLi resulting in a tan solid in 78% yield.

**B. N-[5-(benzo[b]thien-2-yl)thiophene-2-sulfonyl]pyrrole**

N-[5-(benzo[b]thien-2-yl)thiophene-2-sulfonyl]pyrrole was prepared in the same manner as described in Reference Example 32C from benzo[b]-thiophene-2-boronic acid (426 mg, 2.39 mmol) and N-(5-bromothiophene-2-sulfonyl)pyrrole (700 mg, 2.39 mmol). Purification by column chromatography using 2% ethyl acetate/hexanes gave the pure sulfonamide in 68% yield as a brownish-red solid.

**C. 2-chlorosulfonyl-5-(benzo[b]thien-2-yl)thiophene**

2-chlorosulfonyl-5-(benzo[b]thien-2-yl)thiophene was prepared in the same manner as in Reference Example 33D from N-[5-(benzo[b]thien-2-yl)thiophene-2-sulfonyl]pyrrole (520 mg, 1.5 mmol), Purification by column chromatography using 2% ethyl acetate/hexanes gave 153 mg (32% yield) of the pure sulfonyl chloride as a white solid.

**D. N-(4-bromo-3-methyl-5-isoxazolyl)-5-(benzo[b]thien-2-yl)thiophene-2-sulfonamide**

N-(4-bromo-3-methyl-5-isoxazolyl)-5-(benzo[b]thien-2-yl)thiophene2-sulfonamide was prepared in the same manner as in Reference Example 2. Reaction of 2-chlorosulfonyl-5-(benzo[b]thien-2-yl)thiophene (150 mg, 0.48 mmol) with 5-amino-4-bromo-3-methyl isoxazole (84 mg, 0.48 mmol) resulted in 97 mg of pure N-(4-bromo-3-methyl-5-isoxazolyl)-5-(benzo[b]thien-2-yl)thiophene-2-sulfonamide as a light tan powder, (45% yield, m.p. 164° C).

**REFERENCE EXAMPLE 143**

**N-(Bromo-3-methyl-5-isoxazolyl)-5-(1-pentynyl)thiophene-2-sulfonamide**

**A. N-[5-(1-pentynyl)thiophene-2-sulfonyl)pyrrole**

N-[5-(1-pentynyl)thiophene-2-sulfonyl)pyrrole was prepared as in Reference Example 138A from N-(5-bromothiophene-2-sulfonyl)pyrrole (600 mg, 2.05 mmol) and 1-pentynel (280 mg, 4.1 mmol). Purification by column chromatography using 2% ethyl acetate/hexanes gave 424 mg of the pure sulfonamide as a brown oil (74% yield).

**B. 2-chlorosulfonyl-5-(1-pentynyl)thiophene**

2-chlorosulfonyl-5-(1-pentynyl)thiophene was prepared in the same manner as described in Reference Example 33D from N-[5-(1-pentynyl)thiophene-2-sulfonyl]pyrrole (420 mg, 1.5 mmol). Purification by column chromatography using 1% ethyl acetate/hexanes gave 55 mg of the pure thiophene as a brown oil (15% yield).

**C. N-(bromo-3-methyl-5-isoxazolyl)-5-(1-pentynyl)thiophene-2-sulfonamide**

N-(bromo-3-methyl-5-isoxazolyl)-5-(1-pentynyl)thiophene-2-sulfonamide was prepared in the same manner as described in Reference Example 2. Reaction of 2-chlorosulfonyl-5-(1-pentynyl)thiophene (55 mg. 0.22 mmol) with 5-amino-4-bromo-3-methylisoxazole (43 mg, 0.22 mmol) yielded 75 mg of N-(bromo-3-methyl-5-isoxazolyl)-5-(1-pentynyl)thiophene-2-sulfonamide (87% yield). A portion of the product was further purified by preparative HPLC to give the pure sulfonamide as a light brown oil.

**REFERENCE EXAMPLE 144**

**N-(4-Bromo-3-methyl-5-isoxazolyl)-5-(1-napthyl)thiophene-2-sulfonamide**

**A. N-[5-(1-napthyl)thiophene-2-sulfonyl]pyrrole**

N-[5-(1-napthyl)thiophene-2-sulfonyl]pyrrole was prepared in the same manner as described in Reference Example 32C from 1-napthaleneboronic acid (353 mg, 2.05 mmol) and N-(5-bromothiophene-2-sulfonyl)pyrrole (600 mg. 2.05 mmol). Purification by column chromatography using 2% ethyl acetate/hexanes gave the pure sulfonamide in 87% yield as a pale yellow clear oil.

**B. 2-chlorosulfonyl-5-(1-napthyl)thiophene**

2-chlorosulfonyl-5-(1-napthyl)thiophene was prepared as described in Reference Example 33D from N-[5-(1-napthyl)thiophene-2-sulfonyl]pyrrole (604 mg, 1.28 mmol). Purification by column chromatography using 2% ethyl acetate/hexanes gave 376 mg of the pure thiophene in 68% yield.

**C. N-(4-bromo-3-methyl-5-isoxazolyl)-5-(1-napthyl)thiophene-sulfonamide**

N-(4-bromo-3-methyl-5-isoxazolyl)-5-(1-napthyl)thiophene-sulfonamide was prepared in the same manner as described in Reference Example 2. Reaction of 2-chlorosulfonyl-5-(1-napthyl)thiophene (200 mg, 0.65 mmol) with 5-amino-4-bromo-3-methylisoxazole (0.65 mmol), after purification by column chromatography using 1 % MeOH/$CHCl_3$, gave 65.3 mg of pure N-(4-bromo-3-methyl-5-isoxazolyl)-5-(1-napthyl)thiophene-sulfonamide as a brown solid, (22% yield, m.p. 118° C).

**REFERENCE EXAMPLE 145**

**N-(4-Bromo-3-methyl-5-isoxazolyl)-5-(3-nitrophenyl)thiophene-2-sulfonamide**

**A. N-(3-methyl-5-isoxazolyl)-5-(3-nitrophenyl)thiophene-2-sulfonamide**

**N-(3-methyl-5-isoxazolyl)-5-(3-nitrophenyl)thiophene-2-sulfonamide** was prepared in the same manner as described in Reference Example 32C from 3-nitrobenzene boronic acid (362 mg, 2.17 mmol) and N-(3-methyl-5-isoxazolyl)-5-bromothiophene-2-sulfonamide(700 mg, 2.17 mmol). Purification by column chromatography using 10% MeOH/$CHCl_3$ gave 166 mg of the pure sulfonamide (21 % yield).

**B. N-(4-bromo-3-methyl-5-isoxazolyl)-5-(3-nitrophenyl)thiophene-2-sulfonamide**

N-(4-bromo-3-methyl-5-isoxazolyl)-5-(3-nitrophenyl)thiophene-2-sulfonamide was prepared in the same manner as described in Reference Example 136C. Reaction of N-(3-methyl-5-isoxazolyl)-5-(3-nitrophenyl)thiophene-2-sulfonamide (328 g, 0.90 mmol) with N-bromosuccinamide (160 mg, 0.90 mmol) gave the final product. A fraction of this material was further purified by preparative HPLC to give the pure sulfonamide as a brown solid, m.p. 132°C.

**REFERENCE EXAMPLE 146**

**N-(4-Bromo-3-methyl-5-isoxazolyl)-5-(4-methyoxycarbonylphenyl)-thiophene-2-sulfonamide**

**A. 2-(4-methoxycarbonylphenyl)thiophene**

2-(4-methoxycarbonylphenyl)thiophene was prepared in the same manner as described in Reference Example 32C from thiophene-2-boronic acid (1.0 g, 7.81 mmol) and methyl-4-bromobenzoate (1.68 g, 7.81 mmol). Purification by column chromatography using 2% ethyl acetate/hexanes gave 1.1 g of 2-(4-methoxycarbonylphenyl)thiophene as a white solid (65% yield).

**B. 2-chlorosulfonyl-5-(4-methoxycarbonylphenyl)thiophene**

Chlorosulfonic acid (1.06 g, 9.16 mmol) was slowly added to a solution of 2-(4-methoxycarbonylphenyl)thiophene (500 mg, 2.29 mmol) in $CH_2Cl_2$ (10 ml) at -78° C. The resulting solution was stirred at -78° C for 1 hr. by which time the sulfonic acid had completely formed as judged by TLC using 10% ethyl acetate/hexanes. Phosphorous oxychloride (2 ml) was then added at -78° C followed immediately by addition of phosphorous pentachloride (954 mg, 4.58 mmol). The resulting solution was stirred at -78° C for 0.5 hr. and then at room temperature for 25 min. The solution was then carefully poured onto crushed ice (100 g) and extracted into diethyl ether (100 ml). The combined organic layers was washed with brine (1 x 25 ml) and dried over $MgSO_4$. After filtration, removal of solvents left a light greenish solid which was further purified by column chromatography using 2% ethyl acetate/hexanes to give 620 mg of pure 2-chlorosulfonyl-5-(4-methoxycarbonylphenyl)-thiophene as a pale yellow solid (85% yield).

**C. N-(4-bromo-3-methyl-5-isoxazolyl)-5-(4-methoxycarbonylphenyl)-thiophene-2-sulfonamide**

N-(4-bromo-3-methyl-5-isoxazolyl)-5-(4-methoxycarbonylphenyl)-thiophene-2-sulfonamide was prepared in the same manner as described in Reference Example 2 from 2-chlorosulfonyl-5-(4-methoxycarbonylphenyl)thiophene (646 mg, 2.04 mmol) and 5-amino-4-bromo-3-methylisoxazole (361 mg, 2.04 mmol). Purification by column chromatography using 10% MeOH/$CHCl_3$ gave 384 mg of N-(4-bromo-3-methyl-5-isoxazolyl)-5-(4-methoxycarbonylphenyl) thiophene-2-sulfonamide as a brown oil (41 % yield).

**REFERENCE EXAMPLE 147**

**N-(4-Bromo-3-methyl-5-isoxazolyl)-5-(4-carboxyphenyl)thiophene-2-sulfonamide**

Lithium hydroxide (13.3 mg, 0.32 mmol) in methanol (2 ml) was added to a solution of N-(4-bromo-3-methyl-5-isoxazolyl)-5-(4-methoxycarbonylphenyl)thiophene-2-sulfonamide (Reference Example 146C, 121 mg, 0.27 mmol) previously dissolved in methanol (5 ml). The solution was stirred at room temperature for a period of 18 hours. The methanol was removed *in vacuo* and the remaining residue dissolved in water. 4N HCl was added until pH 2.0 was reached, and then the aqueous solution was extracted with ethyl acetate (3 x 25ml). The combined organic layers was washed with water (1 x 10 ml), brine (1 x 10ml) and dried over $MgSO_4$. Evaporation left 50 mg (43% yield) of a pale yellow residue which was further purified by preparative HPLC to give N-(4-bromo-3-methyl-5-isoxazolyl)-5-(4-carboxyphenyl)thiophene-2-sulfonamide as a white solid, m.p. 219-228° C.

**REFERENCE EXAMPLE 148**

**N-(4-chloro-3-methyl-5-isoxazolyl)2-{[2-acetyl-4,5-(methylenedioxy) phenyl]aminocarbonyl}thiophene-3-sulfonamide**

Carbonyldiimidazole (553 mg, 3.41 mmol) was added to a solution of N-(4-chloro-3-methyl-5-isoxazolyl)-2-carboxylthiophene-3-sulfonamide (1.0 g, 3.1 mmol) in dry DMF (10 ml). The mixture was stirred at room temperature for 15 minutes to give mixture (I).

NaH (60% dispersion in mineral oil, 521 mg, 13.02 mmol) was added to a solution of 2'-amino-4',5'-(methylenedioxy)acetophenone (1.13 g, 6.2 mmol) in dry DMF (10 ml) at 0°C. The mixture was stirred at 0°C for 15 minutes to give mixture (II). Mixture (I) was slowly cannulated into mixture (II) at 0°C. The resulting mixture was stirred at 0°C for 4 hours. The reaction mixture was poured into 2 N HCl (aq., 200 ml) and the resulting precipitate was filtered. The solid was washed with water (2x10 ml) and ethyl ether (2x10 ml) to give N-(4-chloro-3-methyl-5-isoxazolyl)2-{[2-acetyl-4,5-(methylenedioxy)phenyl]aminocarbonyl}thiophene-3-sulfonamide (730 mg, 49% yield) as a dull yellow powder, m. p. 191-193° C.

## RERERENCE EXAMPLE 149

### N-(4-chloro-3-methyl-5-isoxazolyl)-2-[(4,5-dimethoxy-2-methoxycarbonylphenyl)aminocarbonyl]thiophene-3-sulfonamide and N-(4-chloro-3-methyl-5-isoxazolyl)2-{[4,5-dimethoxy-2]4,5-dimethoxy-2-methoxycarbonyl)phenyl]phenylaminocarbonyl}thiophene-3-sulfonamide

The title compounds were prepared by the method set forth for N-(4-chloro-3-methyl-5-isoxazolyl)2-{[2-acetyl-4,5-(methylenedioxy)phenyl]-aminocarbonyl}thiophene-3-sulfonamide (REFERENCE EXAMPLE 148) except that methyl-2-amino-4,5-dimethoxybenzoate was used instead of 2'-amino-4',5'-(methylenedioxy)acetophenone. The crude product was purified via HPLC to give N-(4-chloro-3-methyl-5-isoxazolyl)-2-[(4,5-dimethoxy-2-methoxycarbonylphenyl)aminocarbonyl]thiophene-3-sulfonamide as a yellow powder (13% yield, m.p. 167-168° C) and N-(4-chloro-3-methyl-5-isoxazolyl) 2-{[4,5-dimethoxy-2,4,5-dimethoxy-2-methoxycarbonyl)-phenyl]phenylaminocarbonyl}thiophene-3-sulfonamide as a dull yellow solid (1% yield, m.p. 228-230° C).

## REFERENCE EXAMPLE 150

### N-(4-chloro-3-methyl-5-isoxazolyl)-2-[(2-methyl-1,3,4-thiadiazol-5-yl)eminocarbonyl]thiophene-3-sulfonamide

Carbonyldiimidazole (553 mg, 3.4immol) was added to a solution of N-(4-chloro-3-methyl-5-isoxazolyl)-2-carboxyl-thiophene-3-sulfonamide (10 g, 3.1 mmol) in dry DMF (10 ml). The mixture was stirred at room temperature for 15 minutes before the sequential addition of 2-amino-5-methyl-1,3,4-thiadiazole (736 mg, 6.2 mmol) and pyridine (10 ml). The resulting mixture was stirred at room temperature overnight. To work up, the reaction mixture was poured into 1 N HCl (150 ml) and extracted with EtOAc. The organic layer was dried (MgSO4), the solid was filtered and the filtrate was concentrated. The residue was purified via HPLC to give N-(4-chloro-3-methyl-5-isoxazolyl)-2-[(2-methyl-1,3,4-thiadiazol-5-yl)aminocarbonyl]thiophene-3-sulfonamide as a white powder (15% yield, m.p. 192-194° C).

## REFERENCE EXAMPLE 151

### N-(4-chloro-3-methyl-5-isoxazolyl)2-{[2-carboxyl-4,5-(methylenedioxy)phenyl]aminocarbonyl}thiophene-3-sulfonamide

NaOH (1.5 N, 250 ml) was added to N-(4-chloro-3-methyl-5-isoxazolyl)-2-[(4,5-dimethoxy-2-methoxycarbonylphenyl)aminocarbonyl]thiophene-3-sulfonamide (REFERENCE EXAMPLE 149); 410 mg). The resulting suspension was stirred at room temperature overnight to give a clear solution. The mixture was acidified using concentrated HCl with cooling. The resulting precipitate was filtered, washed with water (3x50 ml) and dried on a lyophilyzer to give N-(4-chloro-3-methyl-5-isoxazolyl)2-{[2-carboxyl-4,5-(methylenedioxy]phenyl]aminocarbonyl}thiophene-3-sulfonamide as a yellow powder (87% yield, m.p. 192-195° C).

## REFERENCE EXAMPLE 152

### N-(3,4-dimethyl-5-isoxazolyl)-2-{[2-acetyl-4,5-(methylenedioxy)phenyl]aminocarbonyl}thiophene-3-sulfonamide

N-(3,4-dimethyl-5-isoxazolyl)-2-{[2-acetyl-4,5-(methylenedioxy)phenyl]aminocarbonyl}thiophene-3-sulfonamide was prepared by the method set forth for N-(4-chloro-3-methyl-5-isoxazolyl)2-{[2-acetyl-4,5-(methylenedioxy)phenyl]aminocarbonyl}thiophene-3-sulfonamide (REFERENCE EXAMPLE 148) except that N-(3,4-dimethyl-5-isoxazolyl)-2-carboxylthiophene-3-sulfonamide was used instead of N-(4-chloro-3-methyl-5-isoxazolyl)-2-carboxylthiophene-3-sulfonamide. N-(3,4-dimethyl-5-isoxazolyl)-2-{[2-acetyl-4,5-(methylenedioxy)phenyl]aminocarbonyl}thiophene-3-sulfonamide was obtained as a yellow powder (8% yield, m.p. 228-231° C).

## INVENTIVE EXAMPLE 153

### N-(4-chloro-3-methyl-5-isoxazolyl)-2-[(4-methoxy-2-methylphenyl)amino-carbonyl]thiophene-3-sulfonamide

N-(4-chloro-3-methyl-5-isoxazolyl)-2-[(4-methoxy-2-methylphenyl)-aminoarbonyl]thiophene-3-sulfonamide was prepared by the method set forth for N-(4-chloro-3-methyl-5-isoxazolyl)-2-[(2-methyl-1,3,4-thiadiazol-5-yl)aminocarbonyl]thiophene-3-sulfonamide (REFERENCE EXAMPLE 150), except that 4-methoxy-2-methylaniline was used instead of 2-amino-5-methyl-1,3,4-thiadiazole and that pyridine was not used. The title compound was obtained via

HPLC purification as a dull yellow powder (66% yield, m.p. 58-62° C).

**REFERENCE EXAMPLE 154**

**N-(4-chloro-3-methyl-5-isoxazolyl)-2-[(2-cyano-4,5-dimethoxyphenyl)aminocarbonyl]thiophene-3-sulfonamide**

N-(4-chloro-3-methyl-5-isoxazolyl)-2-[(2-cyano-4,5-dimethoxyphenyl)aminocarbonyl]thiophene-3-sulfonamide was prepared by the method set forth for N-(4-chloro-3-methyl-5-isoxazolyl)2-{[2-acetyl-4,5-(methylenedioxy) phenyl]aminocarbonyl}thiophene-3-sulfonamide (REFERENCE EXAMPLE 148) except that 2-amino-4,5-dimethoxybenzonitrile was used in stead of 2'-amino-4',5'-(methylenedioxy)acetophenone. N-(4-chloro-3-methyl-5-isoxazolyl)-2-[{2-cyano-4,5-dimethoxyphenyl)aminocarbonyl]-thiophene-3-sulfonamide was obtained via HPLC purification as a light brown powder (36% yield, m.p. 53-56° C).

**INVENTITE EXAMPLE 155**

**N-(4-chloro-3-methyl-5-isoxazolyl)-2-[(2,4-dimethoxyphenyl)aminocarbonyl]thiophene-3-sulfonamide**

N-(4-chloro-3-methyl-5-isoxazolyl)-2-[(2,4-dimethoxyphenyl)aminocarbonyl]thiophene-3-sulfonamide was prepared by the method set forth for N-(4-chloro-3-methyl-5-isoxazolyl)-2-(4-methoxy-2-methylphenyl)-aminocarbonyl] thiophene-3-sulfonamide (INVENTIVE EXAMPLE 153), except that 2,4-dimethoxyaniline was used in stead of 4-methoxy-2-methylaniline. N-(4-chloro-3-methyl-5-isoxazolyl)-2-[(2,4-dimethoxyphenyl)aminocarbonyl]-thiophene-3-sulfonamide was obtained via recrystalization (CH3CN/H2O) as yellow crystals (16% yield, m.p. 162-164° C).

**REFERENCE EXAMPLE 156**

**N-(4-chloro-3-methyl-5-isoxazolyl)-2-[(3-methyl-6-pyridyl)aminocarbonyl]thiophene-3-sulfonamide**

N-(4-chloro-3-methyl-5-isoxazolyl)-2-[(3-methyl-6-pyridyl)aminocarbonyl]thiophene-3-sulfonamide was prepared by the method set forth for N-(4-chloro-3-methyl-5-isoxazolyl)2-{(2-acetyl-4,5-(methylenedioxy)-phenyl]aminocarbonyl}thiophene-3-sulfonamide (REFERENCE EXAMPLE 148), except that 2-amino-5-picoline was used in stead of 2'-amino-4',5'-(methylenedioxy)acetophenone, N-(4-chloro-3-methyl-5-isoxazolyl)-2-[(3-methyl-6-pyridyl)aminocarbonyl]thiophene-3-sulfonamide was obtained, via HPLC purification of the crude reaction mixture, as a bright yellow powder (17% yield, m.p. 158-160° C).

**REFERENCE EXAMPLE 157**

**N-(4-bromo-3-methyl-5-isoxazolyl)-2-[(4-methylphenyl)acetyl]thiophene-3-sulfonamide**

N-(4-bromo-3-methyl-5-isoxazolyl)-2-[(4-methylphenyl)-acetyl]thiophene-3-sulfonamide was prepared in the same manner as described in Reference Example 93B by reacting 4-methylbenzylmagnesium chloride with N-(4-bromo-3-methyl-5-isoxazolyl)-2-[N-methoxy(methylaminocarbonyl)]thiophene-3-sulfonamide in THF (see Example 93A) at -78° C to room temperature, resulting in 78% yield. m.p. 146-150° C.8

**REFERENCE EXAMPLE 158**

**N-(4-bromo-3-methyl-5-isoxazolyl)-2-[(4-methyl)(cinnamyl)] thiophene-3-sulfonamide**

**A. N-[2-(4-methyl-*trans*-styryl)-3-sulfonyl}pyrrole**

N-[2-(4-methyl-*trans*-styryl)-3-sulfonyl}pyrrole was prepared in the same manner as described in Reference Example 127B using diethyl{3-[(N-pyrrolylsulfonyl)thien-2-[yl]methyphosphonate and 4-methylbenzaldehyde in 30% yield.

**B. 2-(4-methyl-*trans*-styryl)thiophene-3-sulfonyl chloride**

2-(4-methyl-*trans*-styryl)thiophene-3-sulfonyl chloride was prepared in the same manner as described in Reference Example 64E from N-[2-(4-methyl-*trans*-styryl)-3-sulfonyl}pyrrole by basic hydrolysis (using ethanol and sodium

hydroxide) to the corresponding sodium sulfonate followed by conversion to the corresponding sulfonyl chloride in 13% yield.

**C. N-(4-bromo-3-methyl-5-isoxazolyl)-2-(4-methyl-*trans*-styryl)thiophene-3-sulfonamide**

N-(4-bromo-3-methyl-5-isoxazolyl)-2-(4-methyl-*trans*-styryl)thiophene-3-sulfonamide was prepared in the same manner as described in Reference Example 2 by reaction of 2-(4-methyl-*trans*-styryl)thiophene-3-sulfonyl chloride with 5-amino-4-bromo-3-methylisoxazole. The crude product was purified by HPLC followed by crystallization resulting in a 34% yield, m.p. 101-105°C.

**REFERENCE EXAMPLE 159**

**N-(4-bromo-3-methyl-5-isoxazolyl)-2-[(4-methyl)phenethyl]thiophene-3-sulfonamide**

**A. N-{2-[(4-methyl)phenethyl]thiophene-3-sulfonyl}pyrrole**

N-{2-[(4-methyl)phenethyl]thiophene-3-sulfanyl)pyrrole was prepared as described in Reference Example 128A by the catalytic hydrogenation of N-[2-(4-methyl-*trans*-styryl)-3-sulfonyl}pyrrole in 80% yield.

**B. 2-[(4-methyl)phenethyl]thiophene- 3-sulfonylchloride**

2-[(4-methyl)phenethyl]thiophene- 3-sulfonylchloride was prepared, as described in Reference Example 64E, using N-{2-[(4-methyl)phenethyl]thiophene-3-sulfonyl}pyrrole by basic hydrolysis (KOH/ethanol) of the sulfonamide to this potassium salt followed by conversion of salt to the corresponding sulfonyl chloride in 51% yield.

**C. N-(4-bromo-3-methyl-5-isoxazolyl)-2-[(4-methyl)phenethyl]thiophene-3-sulfonamide**

N-(4-bromo-3-methyl-5-isoxazolyl)-2-[(4-methyl)phenethyl]thiophene-3-sulfonamide was prepared, as described in Reference Example 2, using 2-[(4-methyl)phenethyl]thiophene-3-sulfonylchloride and 5-amino-4-bromo-3-methylisoxazole in 52% yield.

**REFERENCE EXAMPLE 160**

**N-(4-bromo-3-methyl-5-isoxazolyl)-2-[(4-methylphenoxy)methyl]thiophene-3-sulfonamide**

**A. N-{2-[(4-methylphenoxy)methyl]thiophene-3-sulfonyl}pyrrole**

N-{2-[(4-methylphenoxy)methyl]thiophene-3-sulfonyl}pyrrole was prepared, as described in Reference Example 126A, by reacting N-[2-bromomethyl)thiophene-3-sulfonyl]pyrrole with 4-methylphenol, in 81% yield.

**B. 2-[(4-methylphenoxy)methyl]thiophene-3-sulfonyl chloride**

2-[(4-methylphenoxy)methyl]thiophene-3-sulfonyl chloride was prepared, as described in Reference Example 64E, using N-{2-[(4-methylphenoxymethyl]thiophene-3-sulfonyl}pyrrole by basic hydrolysis (NaOH/EtOH) followed by conversion to the corresponding sulfonyl chloride, in 46% yield.

**C. N-(4-bromo-3-methyl-5-isoxazolyl)-2-[(4-methylphenoxy)methyl]-thiophene-3-sulfonamide**

N-(4-bromo-3-methyl-5-isoxazolyl)-2-[(4-methylphenoxy)methyl]-thiophene-3-sulfonamide was prepared, as described in Reference Example 2, by reacting 3-chlorosulfonyl-2-[(4-methylphenoxy)methyl]thiophene with 5-amino-4-bromo-3-methylisoxazole,resulting in a 64% yield, m.p. 128-130° C.

**REFERENCE EXAMPLE 161**

**N-(3,4-dimethyl-5-isoxazolyl)-2-(4-tolylacetylphenyl)thiophene-3-sulfonamide**

**A. N-(3,4-dimethyl-5-isoxazolyl)-2-[N-methoxy(methylaminocarbonyl)-thiophene-3-sulfonamide**

N-(3,4-dimethyl-5-isoxazolyl)-2-[N-methoxy(methylaminocarbonyl)-thiophene-3-sulfonamide was prepared, as described in Reference Example 93A, by reacting N-(3,4-dimethyl-5-isoxazolyl)-2-(carboxyl)thiophene-3-sulfonamide with N-O-dimethylhydroxylamine hydrochloride using triethylamine as base and carbonyldiimidazole,resulting in a 23% yield. Crude product purified by column chromatography using 1:1 hexanes/EtOAc as the eluent.

**B. N-(3,4-dimethyl-5-isoxazolyl)-2-(4-tolylacetyl)thiophene-3-sulfonamide**

N-(3,4-dimethyl-5-isoxazolyl)-2-(4-tolylacetyl)thiophene-3-sulfonamide was prepared, as described in Reference Example 93B, by reacting N-(3,4-dimethyl-5-isoxazolyl)-2-[N-methoxy(methylaminocarbonyl)]thiophene-3-sulfonamide with 4-tolylmagnesium chloride,resulting in a 65% yield, m.p. 95-100° C.

**REFERENCE EXAMPLE 162**

**N-(3,4-dimethyl-5-isoxazolyl)-2-[3,4-(methylenedioxy)phenylacetyl]-thiophene-3-sulfonamide**

N-(3,4-dimethyl-5-isoxazolyl)-2-[3,4-(methylenedioxy)phenylacetyl]-thiophene-3-sulfonamide was prepared, as described in Reference Example 93B, by reacting N-(3,4-dimethyl-5-isoxazolyl)-2-[N-methoxy(methylaminocarbonyl)]thiophene-3-sulfonamide with 3,4-methylenedioxy)phenylmagnesium chloride, resulting in a 65% yield, m.p. 95-100° C.

**REFERENCE EXAMPLE 163**

**N-(4-chloro-3-methyl-5-isoxazolyl)-2-{[2-cyano-4,5-(methylenedioxy)-phenyl]aminocarbonyl}thiophene-3-sulfonamide**

N-(4-chloro-3-methyl-5-isoxazolyl)-2-{[2-cyano-4,5-(methylenedioxy)phenyl]aminocarbonyl}thiophene-3-sulfonamide was prepared as described in Reference EXAMPLE 148, except that 2'-amino-4',5'-(methylenedioxy) benzonitrile was used instead of 2'-amino-4',5'-(methylenedioxy)-acetophenone. It was obtained via HPLC purification as a yellowish solid in ~40% yield, m.p. 167-168° C.

**INVENTIVE EXAMPLE 164**

**N-(4-chloro-3-methyl-5-isoxazolyl)-2-(3-methoxycarbonyl-2,4,6-trimethyl)phenylaminocarbonyl-3-thiophenesulfonamide**

**A. Methyl 3-amino-2,4,6-trimethylbenzoate**

Methyl 3-amino-2,4,6-trimethylbenzoate was synthesized in the same manner as (3,4-methylenedioxy)-6-methylaniline (see Inventive Example 177, below).

**B. N-(4-chloro-3-methyl-5-isoxazolyl)-2-(3-methoxycarbonyl-2,4,6-trimethyl)phenylaminocarbonyl-3-thiophenesulfonamide**

N-(4-chloro-3-methyl-5-isoxazolyl)-2-(3-methoxycarbonyl-2,4,6-trimethyl)phenylaminocarbonyl-3-thiophenesulfonamide was synthesized in the same manner as for Reference Example 94 except that DMF was used instead of THF and the reaction was heated at 80°C for 5 hours. The crude product was purified via preparative HPLC to give N-(4-chloro-3-methyl-5-isoxazolyl)-2-(3-methoxycarbonyl-2,4,6-trimethyl)phenylaminocarbonyl-3-thiophenesulfonamide as an off-white powder (48 mg, 1% yield, m.p. 66-70°C).

**INVENTIVE EXAMPLE 165**

**N-(4-chloro-3-methyl-5-isoxazolyl)-2-(2,4,6-trimethyl)phenylacetyl-3-thiophenesulfonamide**

N-(4-chloro-3-methyl-5-isoxazolyl)-2-(2,4,6-trimethyl)phenylacetyl-3-thiophenesulfonamide was synthesized in the same manner as for Reference Example 102 using 2,4,6-trimethylbenzyl chloride and N-(4-chloro-3-methyl-5-isoxazolyl)-2-(N-methyl-N'-methoxy)aminocarbonyl-3-thiophenesulfonamide. The crude product was purified by flash column chromatography (eluent 1 % methanol in $CH_2Cl_2$) to give N-(4-chloro-3-methyl-5-isoxazolyl)-2-(2,4,6-trimethyl)phenylacetyl-3-thiophenesulfonamide as a solid (31% yield, m.p. 42-46°C).

**INVENTIVE EXAMPLE 166**

**N-(4-chloro-3-methyl-5-isoxazolyl)-2-(2,4,6-trimethyl)phenylaminocarbonyl-3-thiophenesulfonamide**

N-(4-chloro-3-methyl-5-isoxazolyl)-2-(2,4,6-trimethyl)phenylaminocarbonyl-3-thiophenesulfonamide was synthesized in the same manner as Reference Example 94. The crude product was purified via preparative HPLC to give N-(4-chloro-3-methyl-5-isoxazolyl)-2-(2,4,6-trimethyl)phenylaminocarbonyl-3-thiophenesulfonamide as a yellowish-brownish powder (410 mg, 30% yield, m.p. 45-48°C).

**INVENTIVE EXAMPLE 167**

**N-(3,4-dimethyl-5-isoxazolyl)-2-(2,4-dimethyl)phenylacetyl-3-thiophenesulfonamide**

N-(3,4-dimethyl-5-isoxazolyl)-2-{2,4-dimethyl)phenylacetyl-3-thiophenesulfonamide was synthesized by the same method as described for Reference Example 102 using 2,4-dimethylbenzyl chloride and N-(3,4-dimethyl-5-isoxazolyl)-2-(N-methyl-N'-methoxy)aminocarbonyl-3-thiophenesulfonamide. The crude product was purified by flash column chromatography (eluent 1% methanol in $CH_2Cl_2$) and further by preparative HPLC to give N-(3,4-dimethyl-5-isoxazolyl)-2-(2,4-dimethyl)phenylacetyl-3-thiophenesulfonamide as a semi-solid (34% yield).

**INVENTIVE EXAMPLE 168**

**N-(4-chloro-3-methyl-5-isoxazolyl)-2-(2,4-dimethyl)phenylacetyl-3-thiophenesulfonamide**

N-(4-chloro-3-methyl-5-isoxazolyl)-2-(2,4-dimethyl)phenylacetyl-3-thiophenesulfonamide was synthesized in the same manner as for Reference Example 102 using 2,4-dimethylbenzyl chloride and N-(4-chloro-3-methyl-5-isoxazolyl)-2-(N-methyl-N'-methoxy)aminocarbonyl-3-thiophenesulfonamide. The crude product was purified by flash column chromatography (eluent 1% methanol in $CH_2Cl_2$) to give N-(4-chloro-3-methyl-5-isoxazolyl)-2-(2,4-dimethyl)phenylacetyl-3-thiophenesulfonamide as a solid (52% yield, m.p. 48-54°C).

**INVENTIVE EXAMPLE 169**

**N-(4-bromo-3-methyl-5-isoxazolyl)-2-(2,4-dimethyl)phenylacetyl-3-thiophenesulfonamide**

N-(4-bromo-3-methyl-5-isoxazolyl)-2-(2,4-dimethyl)phenylacetyl-3-thiophenesulfonamide was synthesized in the same manner as for Reference Example 102 using 2,4-dimethylbenzyl chloride and N-(4-bromo-3-methyl-5-isoxazolyl)-2-(N-methyl-N'-methoxy)aminocarbonyl-3-thiophenesulfonamide. The crude product was purified by flash column chromatography (eluent 1 % methanol in $CH_2Cl_2$) and further by preparative HPLC to give N-(4-bromo-3-methyl-5-isoxazolyl)-2-(2,4-dimethyl)phenylacetyl-3-thiophenesulfonamide as a solid (28% yield, m.p. 58-63°C).

**REFERENCE EXAMPLE 170**

**N-(4-chloro-3-methyl-5-isoxazolyl)-2-(3,5-dimethyl)phenylacetyl-3-thiophenesulfonamide**

N-(4-chloro-3-methyl-5-isoxazolyl)-2-(3,5-dimethyl)phenylacetyl-3-thiophenesulfonamide was synthesized in the same manner as for Reference Example 102 using 3,5-dimethylbenzyl bromide and N-(4-chloro-3-methyl-5-isoxazolyl)-2-(N-methyl-N'-methoxy)aminocarbonyl-3-thiophenesulfonamide. The crude product was purified by flash column chromatography (eluent 2% methanol in $CH_2Cl_2$) to give N-(4-chloro-3-methyl-5-isoxazolyl)-2-(3,5-dimethyl)phenylacetyl-3-thiophenesulfonamide as a solid (57% yield, m.p. 45-50°C).

## REFERENCE EXAMPLE 171

### N-(4-chloro-3-methyl-5-isoxazolyl)-2-(2,5-dimethyl)phenylacetyl-3-thiophenesulfonamide

N-(4-chloro-3-methyl-5-isoxazolyl)-2-(2,5-dimethyl)phenylacetyl-3-thiophenesulfonamide was synthesized in the same manner as for Reference Example 102 using 2,5-dimethylbenzyl chloride and N-(4-chloro-3-methyl-5-isoxazolyl)-2-(N-methyl-N'-methoxy)aminocarbonyl-3-thiophenesulfonamide. The crude product was purified by flash column chromatography (eluent 2% methanol in $CH_2Cl_2$) to give N-(4-chloro-3-methyl-5-isoxazolyl)-2-(2,5-dimethyl)phenylacetyl-3-thiophenesulfonamide as a solid (33% yield, m.p. 72-76°C).

## REFERENCE EXAMPLE 172

### N-(4-chloro-3-methyl-5-isoxazolyl)-2-[3,4-(methylenedioxy)-6-(2-acetoxyethyl)]phenylaminocarbonyl-3-thiophenesulfonamide

### A. 2-(3,4-methylenedioxy)phenyl-1-ethanol

To a solution of 2-(3,4-methylenedioxy)phenylacetic acid (5 g, 25.75 mmol) in anhydrous THF (20 ml) at 0°C was added $BH_3 \cdot THF$ (40 ml, 1.0 M in THF). The mixture was stirred at room temperature for 1h. To work up, THF was evaporated on a rotavap. The residue was treated with water (100 ml) Acidified and extracted with ether (2 x 100ml). Removal of the solvent under reduced pressure gave 2-(3,4-methylenedloxy)phenyl-1-ethanol as an oil (4.7g, 98% yield).

### B. 1-acetoxy-2-[(3,4-methylenedioxy)phenyl]ethane

To a stirred solution of 2-(3,4-methylenedioxy)phenyl-1-ethanol (1.68 g, 10 mmol) in dry pyridine was added acetic anhydride and the resultant reaction mixture was stirred at 80°C for 1h. The reaction mixture was poured into ice-water and was extracted with ether (2 x 75 ml). The combined ether extract was washed with water (2 x 50 ml), 5% Hcl (2 x 50 ml) and then with 5% $NaHCO_3$ (2 x 50 ml). The organic layer was dried over magnesium sulfate and the solvent was removed under reduced pressure to give 1-acetoxy-2-[(3,4-methylenedioxy)phenyl]ethane as a solid (1.7 g, 81% yield).

### C. 1-acetoxy-2-[(3,4-methylenedioxy)-6-nitrophenyl]ethane

To a stirred solution of 1-acetoxy-2-[(3,4-methylenedioxy)-phenyl]ethane (1.7 g, 8.09 mmol) in acetic acid (10 ml) was added, dropwise, concentrated $HNO_3$ (4.5 ml). This was stirred at room temperature for 30 min. The reaction mixture was poured into water (100 ml). The precipeated solid was filtered, washed with water and dried under high vacuum to get 1-acetoxy-2-[(3,4-methylenedioxy)-6-nitrophenyl]ethane (1.8 g, 88% yield).

### D. 1-acetoxy-2-[(3,4-methylenedioxy)-6-aminophenyl]ethane

The solution of 1-acetoxy-2-[(3,4-methylenedioxy)-6-nitrophenyl]ethane (0.8 g, 3.13 mmol) in ethyl acetate (25 ml) was subjected to catalytic hydrogenation using 10% palladium on carbon (100 mg) at 50 psi for 30 min. The catalyst was filtered and the solvent was removed under reduced pressure to give 1-acetoxy-2-[(3,4-methylenedioxy)-6-aminophenyl]ethane as a solid (0.69 g, 98% yield).

### E. N-(4-chloro-3-methyl-5-isoxazolyl)-2-[3,4-(methylenedioxy)-6-(2-acetoxyethyl)]phenylaminocarbonyl-3-thiophenesulfonamide

N-(4-chloro-3-methyl-5-isoxazolyl)-2-[3,4-(methylenedioxy)-6-(2-acetoxyethyl)lphenylaminocarbonyl-3-thiophenesulfonamide was synthesized in the same manner as Reference Example 87. The crude product was purified by preparative HPLC to give N-(4-chloro-3-methyl-5-isoxazolyl)-2-[3,4-(methylenedioxy)-6-(2-acetoxyethyl)]phenylaminocarbonyl-3-thiophenesulfonamide as a dull yellow powder (12% yield, m.p. 78-82°C).

**REFERENCE EXAMPLE 173**

**N-(4-chloro-3-methyl-5-isoxazolyl)-2-[3,4-(methylenedioxy)-6-(2-hydroxyethyl)]phenylaminocarbonyl-3-thiophenesulfonamide**

To a stirred solution of N-(4-chloro-3-methyl-5-isoxazolyl)-2-[3,4-(methylenedioxy)-6-(2-acetoxyethyl)]phenylaminocarbonyl-3-thiophenesulfonamide (35 mg, 0.066 mmol) in methanol was added NaOH powder (40 mg) and stirred at room temperature for 30 min. HPLC analysis showed complete consumption of starting material. The reaction mixture was diluted with water and acidified to pH 2 -3. This was extracted with ethyl acetate (2 x 25 ml). The combined organic layer was dried over magnesium sulfate and the solvent removed under reduced pressure to give N-(4-chloro-3-methyl-5-isoxazolyl)-2-[3,4-(methylenedioxy)-6-(2-hydroxyethyl)]phenylaminocarbonyl-3-thiophenesulfonamide as a solid (84% yield, m.p. 47-52°C).

**REFERENCE EXAMPLE 174**

**N-(4-chloro-3-methyl-5-isoxazolyl)-2-[3,4-(methylenedioxy)-6-(2-acetoxyethoxy)]phenylaminocarbonyl-3-thiophenesulfonamide**

**A. Methyl 2-[3,4-(methylenedioxy)phenoxy]acetate and 2-[3,4-(methylenedioxy)phenoxy]acetic acid**

The mixture of sesamol (13.8 g, 100 mmol, methyl bromoacetate (15.3 g, 100 mmol) and potassium carbonate in acetone (200 ml) was refluxed for 24h with stirring. Acetone was removed under reduced pressure. The residue was dissolved in water (200ml) and was extracted with ether (2 x 100 ml). the combined organic layer was dried over magnesium sulfate and the solvent removed under reduced pressure to give methyl 2-[3,4-(methylenedioxy)phenoxy] acetate as an oil (12 g, 57% yield). The aqueous phase was neutralized to pH 2 - 3 with concentrated HCl and the precipitated solid was filtered to get 2-[3,4-(methylenedioxy)phenoxy]acetic acid as a solid (6 g, 31% yield).

**B. 2-3,4-(methylenedioxy)phenoxy-1-ethanol**

2-(3,4-methylenedioxy)phenoxy-1-ethanol was synthesized in the same manner as for Reference Example 172(A) using 2-[3,4-(methylenedioxy)-phenoxy]acetic acid and $BH_3$•THF complex. The reaction was carried out for 12 hours at room temperature (98% yield).

**C. 1-acetoxy-2-[3,4-(methylenedioxy)phenoxy]ethane**

1-Acetoxy-2-[3,4-(methylenedioxy)phenoxy]ethane was synthesized in the same manner as for Reference Example 172(B) by acetylation of 2-(3,4-methylenedioxy)phenoxy-1-ethanol using acetic anhydride and pyridine (92% yield).

**D. 1-acetoxy-2-[3,4-(methylenedioxy)-6-nitrophenoxy]ethane**

1-acetoxy-2-(3,4-(methylenedioxy)-6-nitrophenoxy]ethane was synthesized in the same manner as for Reference Example 172(C) by nitration of 1-acetoxy-2-[3,4-(methylendioxy)phenoxy]ethane. The reaction was carried out between 0°C and 5°C for 30 min (78 % yield).

**E. 1-acetoxy-2-[3,4-(methylenedioxy)-6-aminophenoxy]ethane**

1-acetoxy-2-[3,4-(methylenedioxy)-6-aminophenoxy]ethane was synthesized in the same manner as for Reference Example 172(D) by reduction of 1-acetoxy-2-[3,4-(methylenedioxyl-6-nitrophenoxylethane (100% yield).

**F. N-(4-chloro-3-methyl-5-isoxazolyl)-2-[3,4-(methylenedioxy)-6-(2-acetoxyethoxy)]phenylaminocarbonyl-3-thiophenesulfonamide**

N-(4-chloro-3-methyl-5-isoxazolyl)-2-[3,4-(methylenedioxy)-6-(2-acetoxyethoxy)]phenylaminocarbonyl-3-thiophenesulfonamide was synthesized in the same manner as Reference Example 87 using 1-acetoxy-2-[3,4-(methylenedioxy)-6-aminophenoxy]ethane and N-(4-chloro-3-methyl-5-isoxazolyl)-2-carboxylthiophene-3-sulfonamide. The crude product was purified by preparative HPLC to give N-(4-chloro-3-methyl-5-isoxazolyl)-2-[3,4-(methylenedioxy)-6-(2-acetoxyethoxy)]phenylaminocarbonyl-3-thiophenesulfonamide as a dull yellow powder (21% yield, m.p.

117-119°C).

**REFERENCE EXAMPLE 175**

**N-(4-chloro-3-methyl-5-isoxazolyl)-2-[3,4-(methylenedioxy)-6-(2-hydroxyethoxy)]phenylaminocarbonyl-3-thiophenesulfonamide**

N-(4-chloro-3-methyl-5-isoxazolyl)-2-(3,4-(methylenedioxy)-6-(2-hydroxyethoxy)]phenylaminocarbonyl-3-thiophenesulfonamide was synthesized in the same manner as Reference Example 173 by basic hydrolysis of N-(4-chloro-3-methyl-5-isoxazolyl)-2-[3,4-(methylenedioxy)-6-(2-acetoxyethoxy)]phenylaminocarbonyl-3-thiophenesulfonamide (86% yield, m.p. 158-161 °C).

**INVENTIVE EXAMPLE 176**

**N-(4-chloro-3-methyl-5-isoxazolyl)-2-(4-methoxycarbonyl-2,6-dimethyl)phenylaminocarbonyl-3-thiophenesulfonamide**

**A. N-(4-chloro-3-methyl-5-isoxazolyl)-N-(methoxyethoxymethyl)-2-carboxylthiophene-3-sulfonamide**

To a mixture of N-(4-chloro-3-methyl-5-isoxazolyl)-2-carboxylthiophene-3-sulfonamide (3.23 g, 100 mmol) and diisopropylethyl amine (3 ml) in ethyl acetate (20 ml) was added methoxyethoxymethyl chloride and the resultant reaction mixture was stirred at room temperature for 12 hours. This was diluted with ethyl acetate (100 ml) and washed with 1 N HCl (2 x 50 ml). The organic layer was dried over magnesium sulfate and the solvent removed under reduced pressure to give N-(4-chloro-3-methyl-5-isoxazolyl)-N-(methoxyethoxymethyl)-2-(carbomethoxy)-thiophene-3-sulfonamide as light brown oil.

The crude N-(4-chloro-3-methyl-5-isoxazolyl)-N-(methoxyethoxymethyl)-2-(carbomethoxy)thiophene-3-sulfonamide was dissolved in methanol (50 ml) and potassium hydroxide (5 g) and water (5 ml) were added. The reaction mixture was stirred at room temperature for 12 hours and extracted with ethyl acetate (2 x 50 ml). The aqueous phase was neutralized to pH 2 - 3 and was extracted with ethyl acetate (2 x 50 ml). The combined organic layer was dried over magnesim sulfate and solvent removed to give N-(4-chloro-3-methyl-5-isoxazolyl)-N-(methoxyethoxymethyl)-2-carboxylthiophene-3-sulfonamide as light brown solid (3.5 g, 85% yield).

**B. 4-Carbomethoxy-2,6-dimethylaniline**

To a warm solution of 3,5-dimethyl benzoic acid (5 g, 33.33 mmol) in acetic acid (30 ml) was added fuming nitric acid (30 ml), dropwise. After completion of the addition the reaction mixture was warmed with a heat gun. This was stirred for an additional 2 hours during which period a solid was precipitated. The reaction mixture was diluted with water (200 ml) and filtered. The solid was dried under reduced pressure.

To the above solid was added 20 ml of oxalyl chloride and a catalytic amount of DMF (2 drops). This was stirred at room temperature for 3 hours during which period a clear solution was formed. Excess oxalyl chloride was removed under reduced pressure to give yellow solid.

To the yellow solid was added dry methanol (100 ml) and the mixture stirred at room temperature for 1 hour. Excess methanol was removed under reduced pressure and the residue dissolved in ether (200 ml). This was washed with water (100 ml) followed by saturated $NaHCO_3$ solution (100 ml). The oganic layer was dried over magnesium sulfate and the solvent removed giving 4-carbomethoxy-2,6-dimethylnitrobenzene as a yellow solid (5.8 g, 83% yield).

4-Carbomethoxy-2,6-dimethylnitrobenzene (2 g, 9.5 mmol) was dissolved in in ethyl acetate (20 ml) and subjected catalytic hydrogenation using 10% palladium on carbon (300 mg) at 55 psi for 30 min. The catalyst was filtered and solvent removed to give 4-carbomethoxy-2,6-dimethylaniline as a solid (1.7 g, 100% yield).

**C. N-(4-chloro-3-methyl-5-isoxazolyl)-2-(4-methoxycarbonyl-2,6-dimethyl)phenylaminocarbonyl-3-thiophenesulfonamide**

N-(4-chloro-3-methyl-5-isoxazolyl)-N-(methoxyethoxymethyl)-2-carboxylthiophene-3-sulfonamide (3.5 g, 8.5 mmol) (from step A) was dissolved in oxalyl chloride (5 ml) and a drop of DMF was added. This was stirred at room temperature for 6 hours. Excess oxalyl chloride was removed under reduced pressure and the mixture dried under high vacuum.

To a solution of 4-Carbomethoxy-2,6-dimethylaniline (0.9 g) (from step B) and triethyl amine (2 ml) in methylene chloride (20 ml) at 0°C was added the acid chloride in 10 ml of methylene chloride (2.4 g, 4.98 mmol) prepared

in the above step. The reaction mixture was permitted to warm to room temperture, was diluted with methylene chloride (50 ml) and was washed with 1 N HCI followed by saturated $NaHCO_3$ solution. The organic layer was dried over magnesium sulfate and the solvent removed giving the crude product. This was purified by column chromatography using 4:6 ethyl acetate and hexane as a eluent to give N-(4-chloro-3-methyl-5-isoxazolyl)-N-(methoxyethoxymethyl) 2-(4-methoxycarbonyl-2,6-dimethyl)phenylaminocarbonyl-3-thiophenesulfonamide an oil (0.6 g, 20% yield).

N-(4-chloro-3-methyl-5-isoxazolyl)-N-(methoxyethoxymethyl)2-(4-methoxycarbonyl-2,6-dimethyl)phenylaminocarbonyl-3-thiophenesulfonamide (0.6g) was dissolved in a mixture of methanol (8 ml) and concentrated HCI (1.5 ml) and the reultant reaction mixture was refluxed under stirring for 8 hours. Excess methanol was removed under reduced pressure and the residue dissolved in ethyl acetate (50 ml). This was washed with saturated sodium chloride solution. The organic layer was dried over magnesium sulfate and the solvent removed giving N-(4-chloro-3-methyl-5-isoxazolyl)-2-(4-methoxycarbonyl-2,6-dimethyl)phenylaminocarbonyl-3-thiophenesulfonamide which was crystallized using methylene chloride and hexane (0.23 g, 47% yield, m.p. 152-154°C).

**INVENTIVE EXAMPLE 177**

**N-(4-chloro-3-methyl-5-isoxazolyl)-2-[3,4-(methylenedioxy)-6-methyl]phenylaminocarbonyl-3-thiophenesulfonamide**

**A. (3,4-methylenedioxy)-6-methylaniline**

To a solution of (3,4-methylenedioxy)toluene (5 ml) in acetic acid (20 ml) cooled with a cold water bath was added, dropwise, nitric acid (70%, 5 ml). The mixture was stirred for 45 min. To work up, water (100 ml) was added and the resulting yellow precipitate was filtered and washed with water until the aqueous filtrate was colorless. The yellow solid was dissolved in EtOAc (250 ml) and dried ($MgSO_4$), and the solid was filtered off. The filtrate was subjected to catalytic hydrogenation (10% Pd/C, 1 atm) for 12 hours. The reaction mixture was then filtered off the catalyst and the filtrate was concentrated on a rotavap to give (3,4-methylenedioxy)-6-methylaniline as a brownish grey solid (5.49g, 87% yield).

**B. N-(4-chloro-3-methyl-5-isoxazolyl)-2-[3,4-(methylenedioxy)-6-methyl]phenylaminocarbonyl-3-thiophenesulfonamide**

N-(4-chloro-3-methyl-5-isoxazolyl)-2-[3,4-(methylenedioxy)-6-methyl]phenylaminocarbonyl-3-thiophenesulfonamide was synthesized in the same manner as Reference Example 94 using (3,4-methylenedioxy)-6-methylaniline. The crude product was purified by preparative HPLC to give N-(4-chloro-3-methyl-5-isoxazolyl)-2-[3,4-(methylenedioxy)-6-methyl]phenylaminocarbonyl-3-thiophenesulfonamide as a yellow solid (45% yield, m.p. 60-62°C).

**REFERENCE EXAMPLE 178**

**N-(4-chloro-3-methyl-5-isoxazolyl)-2-(3,4-dimethoxy-6-amino carbonyl)phenylaminocarbonyl-3-thiophenesulfonamide**

N-(4-chloro-3-methyl-5-isoxazolyl)-2-(3,4-dimethoxy-6-aminocarbonyl)phenylaminocarbonyl-3-thiophenesulfonamide was synthesized in the same manner as for Reference Example 94 using N-(4-chloro-3-methyl-5-isoxazolyl)2-{[2-carboxyl-4,5-(methylenedioxy)phenyl]aminocarbonyl}thiophene-3-sulfonamide (Example 151) and ammonium hydroxide. The crude product was purified by preparative HPLC to give N-(4-chloro-3-methyl-5-isoxazolyl)-2-(3,4-dimethoxy-6-aminocarbonyl)phenylaminocarbonyl-3-thiophenesulfonamide as yellow powder (66% yield, m.p. 189-192°C).

**INVENTIVE EXAMPLE 179**

**N-(4-chloro-3-methyl-5-isoxazolyl)-2-[3,4-(methylenedioxy)-6-methyl]-phenylacetyl-3-thiophenesulfonamide**

**A. (3,4-methylenedioxy)-6-methylbenzyl chloride**

To a 1:1 mixture of ethyl ether (100 ml) and conc. HCl (100 ml) at 0°C was added (3,4-methylenedioxy)toluene (10 ml). Formaldehyde (20 ml, 37% in water) was then added dropwise. The reaction was stirred at 0°C for 2 hours and at room temperature for an additional 10 hours. The reaction mixture was then diluted with ethyl ether (100 ml) and the two layers were separated. The organic layer was dried ($MgSO_4$), the solid was filtered and the filtrate was

concentrated. The residue was then heated with hexane (200 ml) and the insolubles were filtered off the hot solution. The filtrate was concentrated to give a mixture of (3,4-methylenedioxy)-6-methylbenzyl chloride (9.4 g, 63% yield) and bis[(3,4-methylenedioxy)-6-methyl]phenylmethane (3.6 g) as a white solid. This mixture was carried on to the next step without further purification.

**B. N-(4-chloro-3-methyl-5-isoxazolyl)-2-[3,4-(methylenedioxy)-6-methyl]phenylacetyl-3-thiophenesulfonamide**

N-(4-chloro-3-methyl-5-isoxazolyl)-2-[3,4-(methylenedioxy)-6-methyl]phenylacetyl-3-thiophenesulfonamide was synthesized in the same manner as for Reference Example 102 using (3,4-methylenedioxy)-6-methylbenzyl chloride instead of (3,4-methylenedioxy)benzyl chloride. The crude product was purified by preparative HPLC to give N-(4-chloro-3-methyl-5-isoxazolyl)-2-[3,4-(methylenedioxy)-6-methyl]phenylacetyl-3-thiophenesulfonamide as a yellow powder (71% yield, m.p. 42-45°C)

**REFERENCE EXAMPLE 180**

**N-(4-chloro-3-methyl-5-isoxazolyl)-2-[3,4-(methylenedioxy)-6-methanesulfonylaminomethyl]phenylaminocarbonyl-3-thiophenesulfonamide**

A. N-(3,4-methylenedioxy)benzyl-methanesulfonamide

To a solution of piperonylamine (6.07 g, 38.95 mmol) and triethylamine (5.37 g, 53.12 mmol) in dichloromethane (100 ml) at 0°C was added methanesulfonyl chloride (4.14 g, 35.41 mmol). The reaction was stirred at 0°C for 1 hour. The mixture was then diluted with dichloromethane (100 ml) and washed with 1 N HCl (2 x 100 ml). The organic layer was dried ($MgSO_4$), the solid was filtered and the filtrate was concentrated to give N-(3,4-methylenedioxy)benzyl-methanesulfonamide as a grey solid (8.4 g, 92% yield).

**B. N-[3,4-(methylenedioxy)-6-amino]benzyl-methanesulfonamide**

N-[3,4-(methylenedioxy)-6-aminolbenzyl-methanesulfonamide was synthesized in the same manner as for (3,4-methylenedioxy)-6-methylaniline (Inventive Example 177).

**C. N-(4-chloro-3-methyl-5-isoxazolyl)-2-[3,4-(methylenedioxy)-6-methanesulfonylaminomethyl]phenylaminocarbonyl-3-thiophenesulfonamide**

N-(4-chloro-3-methyl-5-isoxazolyl)-2-[3,4-(methylenedioxy)-6-methanesulfonylaminomethyl]phenylaminocarbonyl-3-thiophenesulfonamide was synthesized in the same manner as Reference Example 94. The crude product was recrystalized from acetonitrile and water to give N-(4-chloro-3-methyl-5-isoxazolyl)-2-[3,4-(methylenedioxy)-6-methanesulfonylaminomethyllphenylaminocarbonyl-3-thiophenesulfonamide as an off-white solid (13% yield, m.p. 147-150°C).

**REFERENCE EXAMPLE 181**

**N-(4-chloro-3-methyl-5-isoxazolyl)-2-[3,4-(methylenedioxy)-6-cyanomethyl]phenylaminocarbonyl-3-thiophenesulfonamide**

**A. [3,4-(methylenedioxy)-6-amino]phenylacetonitrile**

[3,4-(methylenedioxy)-6-amino]phenylacetonitrile was synthesized in the same manner as for (3,4-methylenedioxy)-6-methylaniline (Inventive Example 177).

**B. N-(4-chloro-3-methyl-5-isoxazolyl)-2-[3,4-(methylenedioxy)-6-cyanomethyl]phenylaminocarbonyl-3-thiophenesulfonamide**

N-(4-chloro-3-methyl-5-isoxazolyl)-2-[3,4-(methylenedioxy)-6-cyanomethyl]phenylaminocarbonyl-3-thiophenesulfonamide was synthesized in the same manner as in Reference Example 94. The crude product was recrystalized from acetonitrile/water to give N-(4-chloro-3-methyl-5-isoxazolyl)-2-[3,4-(methylenedioxy)-6-cyanomethyl]phenylaminocarbonyl-3-thiophenesulfonamide as a red-brownish powder (15% yield, m.p. 190-193°C).

## REFERENCE EXAMPLE 182

### N-(4-chloro-3-methyl-5-isoxazolyl)-2-[3,4-(methylenedioxy)-6-(3-hydroxypropyl)]phenylaminocarbonyl-3-thiophenesulfonamide

### A. 3-(3,4-methylenedioxy)phenyl-1-propanol

To a solution of 3-(3,4-methylenedioxy)phenylpropanoic acid (5 g, 25.75 mmol) in anhydrous THF (20 ml) at 0°C was added $BH_3$•THF (51.5 ml, 1.0 M in THF, 51.5 mmol). The mixture was refluxed for 1 hour. Then the THF was evaporated on a rotavap. The residue was treated with methanol (20 ml) and the solution was concentrated. This process was repeated 6 times to give 3-(3,4-methylenedioxy)phenyl-1-propanol as an oil (4.7g, ~100 yield).

### B. 3-[3,4-(methylenedioxy)-6-amino]phenyl-1-propanol

3-[3,4-(methylenedioxy)-6-amino]phenyl-1-propanol was synthesized in the same manner as for (3,4-methylenedioxy)-6-methylaniline (Inventive Example 177).

### C. N-(4-chloro-3-methyl-5-isoxazolyl)-2-[3,4-(methylenedioxy)-6-(3-hydroxypropyl)]phenylaminocarbonyl-3-thiophenesulfonamide

N-(4-chloro-3-methyl-5-isoxazolyl)-2-[3,4-(methylenedioxy)-6-(3-hydroxypropyl)]phenylaminocarbonyl-3-thiophenesulfonamide was synthesized in the same manner as Reference Example 94. The crude product was purified by preparative HPLC to give N-(4-chloro-3-methyl-5-isoxazolyl)-2-[3,4-(methylenedioxy)-6-(3-hydroxypropyl)]phenylaminocarbonyl-3-thiophenesulfonamide as a dull yellow powder (18% yield, m.p. 66-69°C).

## REFERENCE EXAMPLE 183

### N-(4-chloro-3-methyl-5-isoxazolyl)-2-[3,4-(methylenedioxy)-6-cyano]phenylacetyl-3-thiophenesulfonamide

### A. Methyl (3,4-methylenedioxy)phenylacetate

Methyl (3,4-methylenedioxy)phenylacetate was prepared as described by known methods (see, Rachele (1963) Journal of Organic Chemistry 28:2898).

### B. Methyl 6-bromo-(3,4-methylenedioxy)phenylacetate

To a solution of methyl (3,4-methylenedioxy)phenylacetate (5 g, 25.8 mmol) in acetic acid (15 ml) was added, dropwise, bromine until a red-brown color persisted. After stirring at RT for 30 minutes, the reaction mixture was partitioned between water (200 ml) and ether (200 ml). The organic layer was washed with water (3 x 200 ml), dried ($MgSO_4$), the solid was filtered off and the filtrate was concentrated to give methyl 6-bromo-(3,4-methylenedioxy)phenylacetate as an oil (5.9 g, 84% yield).

### C. Methyl (3,4-methylenedioxy)-6-cyanophenylacetate

Methyl (3,4-methylenedioxy)-6-cyanophenylacetate was prepared as described by L. Friedman and H. Shechter in the Journal of Organic Chemistry 26:2522 (1961).

### D. t-Butyl (3,4-methylenedioxy)-6-cyanophenylacetate

To a solution of methyl (3,4-methylenedioxy)-6-cyanophenylacetate (5 g, 18.32 mmol) in methanol (100 ml) was added 1 N NaOH (50 ml). The reaction was stirred at room temperature for 1.5 hours. Methanol was then stripped off on a rotavap. The aqueous residue was acidified with conc. HCl to pH ~ 1 and extracted with ethyl acetate. The organic layer was dried ($MgSO_4$), the solid was filtered and the filtrate was concentrated to give a solid. The solid was treated with thionyl chloride (50 ml) and the mixture was refluxed for 10 min before the volatiles were stripped off on a rotavap. The residue was dissolved in dichloromethane (15 ml) and the solution was added dropwise to a solution of 2-methyl-2-propanol (6.8 g, 91.6 mmol) and triethylamine (9.3 g, 91.6 mmol) in dichloromethane (100 ml) at 0°C. The mixture was stirred at 0°C for 1 hour and at room temperature for 2 hours. The mixture was then washed with water (3x150 ml). The organic layer was dried ($MgSO_4$), the solid was filtered and the filtrate was concentrated to give

t-butyl (3,4-methylenedioxy)-6-cyanophenylacetate as a solid (335 mg, 7% yield).

**E. N-(4-chloro-3-methyl-5-isoxazolyl)-2-[3,4-(methylenedioxy)-6-cyano]phenylacetyl-3-thiophenesulfonamide**

To a solution of N-(4-chloro-3-methyl-5-isoxazolyl)-2-carboxyl-3-thiophenesulfonamide (2.78 g, 8.63 mmol) in anhydrous DMF (30 ml) was added carbonyldiimidazole (1.40 g, 8.63 mmol). The mixture was stirred at room temperature for 20 min to give mixture I.

To a solution of t-butyl (3,4-methylenedioxy)-6-cyanophenylacetate (1.5 g, 5.75 mmol) in anhydrous DMF (15 ml) was added NaH (1.2 g, 60% dispersion in mineral oil, 29.9 mmol) at 0°C. The mixture was stirred at room temperature for 30 min to give mixture II. Mixture I was syringed to mixture II at 0°C and the resulting mixture was stirred at 0°C for 1 hour and at RT for 10 hours. The crude mixture was poured to a 2:2:1 mixture of acetonitrile/water/conc. HCl and the resulting mixture was heated at 40°C for 12 hours. Acetonitrile was then removed on a rotavap and the aqueous residue was partitioned between ethyl acetate (200 ml) and 1 N HCl (150 ml). The organic layer was washed with 1 N HCl (3 x 150 ml) and dried ($MgSO_4$), the solid was filtered off and the filtrate was concentrated. The residue was purified by preparative HPLC to give N-(4-chloro-3-methyl-5-isoxazolyl)-2-[3,4-(methylenedioxy)-6-cyano]phenylacetyl-3-thiophenesulfonamide as a light dull yellow powder (450 mg, 17% yield, m.p. 105-108°C).

**REFERENCE EXAMPLE 184**

**N-(4-chloro-3-methyl-5-isoxazolyl)-2-[3,4-(methylenedioxy)-6-dimethylaminocarbonylmethyl] phenylaminocarbonyl-3-thiophenesulfonamide**

**A. N,N-dimethyl (3,4-methylenedioxy)phenylacetamide**

N,N-dimethyl (3,4-methylenedioxy)phenylacetamide was synthesized in the same manner as described in Reference Example 94.

**B. N,N-dimethyl (3,4-methylenedioxy)-6-aminophenylacetamide**

N,N-dimethyl (3,4-methylenedioxy)-6-aminophenylacetamide was synthesized in the same manner as (3,4-methylenedioxy)-6-methylaniline (see Inventive Example 177).

**C. N-(4-chloro-3-methyl-5-isoxazolyl)-2-[3,4-(methylenedioxy)-6-dimethylaminocarbonylmethyl] phenylaminocarbonyl-3-thiophenesulfonamide**

N-(4-chloro-3-methyl-5-isoxazolyl)-2-[3,4-(methylenedioxy)-6-dimethylaminocarbonylmethyl]phenylaminocarbonyl-3-thiophenesulfonamide was synthesized in the same manner as Reference Example 94. The crude product was recrystalized from acetonitrile/water to give N-(4-chloro-3-methyl-5-isoxazolyl)-2-[3,4-(methylenedioxy)-6-dimethylaminocarbonylmethyl]phenylaminocarbonyl-3-thiophenesulfonamide as a greyish powder ( 400 mg, 19% yield, m.p. 190-193°C).

**INVENTIVE EXAMPLE 185**

**N-(4-chloro-3-methyl-5-isoxazolyl)-2-[3,4-(methylenedioxy)-6-methyl]phenylhydroxyimino-3-thiophenesulfonamide**

To N-(4-chloro-3-methyl-5-isoxazolyl)-2-[3,4-(methylenedioxy)-6-methyl]phenylacetyl-3-thiophenesulfonamide (100 mg) was added $NH_2OH$•HCl (300 mg) and water (15 ml). After stirring for 5 min, NaOH pellet (300 mg) and methanol (2 ml) were added. The warm mixture was heated at 80°C for 20 min and was cooled to 0°C. It was then poured into a dilute HCl solution (~30 ml). The resulting white precipitate was filtered to give N-(4-chloro-3-methyl-5-isoxazolyl)-2-[3,4-(methylenedioxy)-6-methyl]phenylhydroxyimino-3-thiophenesulfonamide as a white solid ( 72 mg, 70% yield, m.p. 154-156°C).

**INVENTIVE EXAMPLE 186**

**N-(4-chloro-3-methyl-5-isoxazolyl)-2-{1-acetoxy-2-*cis*-[3,4-(methylenedioxy)-6-methyl]phenyl}vinyl-3-thiophenesulfonamide**

To a solution of N-(4-chloro-3-methyl-5-isoxazolyl)-2-[3,4-(methylenedioxy)-6-methyl]phenylacetyl-3-thiophenesulfonamide (50 mg, 0.11 mmol) in anhydrous DMF (1 ml) was added NaH (11 mg, 60% dispersion in mineral oil, 0.275 mmol). After stirring at room temperature for 5 min, acetic anhydride (16.8 mg, 0.165 mmol) was added. After stirring at room temperature for an additional 10 min., the mixture was poured into dilute HCl solution and the resulting precipitate was filtered to give N-(4-chloro-3-methyl-5-isoxazolyl)-2-{1-acetoxy-2-*cis*-[3,4-(methylenedioxy)-6-methyl] phenyl}vinyl-3-thiophenesulfonamide as a yellowish powder (40 mg, 73%, m.p. 55-58°C).

**REFERENCE EXAMPLE 187**

**N-(4-chloro-3-methyl-5-isoxazolyl)-2-(1,2,3-trimethoxy-6-cyano)phenylaminocarbonyl-3-thiophenesulfonamide**

**A. 2-amino-3,4,5-trimethoxybenzonitrile**

2-amino-3,4,5-trimethoxybenzonitrile was synthesized in the same manner as (3,4-methylenedioxy)-6-methylaniline (see Inventive Example 177), and the crude product was recrystalized from methanol/water to give a yellow powder (13% yield).

**B. N-(4-chloro-3-methyl-5-isoxazolyl)-2-(1,2,3-trimethoxy-6-cyano)phenylaminocarbonyl-3-thiophenesulfonamide**

N-(4-chloro-3-methyl-5-isoxazolyl)-2-(1,2,3-trimethoxy-6-cyano)phenylaminocarbonyl-3-thiophenesulfonamide was synthesized in the same manner as Reference Example 148. The crude product was purified via preparative HPLC to give N-(4-chloro-3-methyl-5-isoxazolyl)-2-(1,2,3-trimethoxy-6-cyano)phenylaminocarbonyl-3-thiophenesulfonamide as a yellow powder (180 mg, 10% yield, m.p. 88-90°C).

**INVENTIVE EXAMPLE 188**

**N-(3,4-dimethyl-5-isoxazolyl)-2-[3,4-(methylenedioxy)-6-methyl]-phenylacetyl-3-thiophenesulfonamide**

N-(3,4-dimethyl-5-isoxazolyl)-2-[3,4-(methylenedioxy)-6-methyl]phenylacetyl-3-thiophenesulfonamide was synthesized in the same manner as Inventive Example 179. The crude product was purified via preparative HPLC to give N-(3,4-dimethyl-5-isoxazolyl)-2-[3,4-(methylenedioxy)-6-methyl]phenylacetyl-3-thiophenesulfonamide as a yellow powder (417 mg, 14% yield, m.p. 45-50°C).

**INVENTIVE EXAMPLE 189**

**N-(4-chloro-5-methyl-3-isoxazolyl)-2-[3,4-(methylenedioxy)-6-methyl]-phenylacetyl-3-thiophenesulfonamide**

N-(4-chloro-5-methyl-3-isoxazolyl)-2-[3,4-(methylenedioxy)-6-methyl]phenylacetyl-3-thiophenesulfonamide was synthesized in the same manner as Inventive Example 179. The crude product was purified via preparative HPLC to give N-(4-chloro-5-methyl-3-isoxazolyl)-2-[3,4-(methylenedioxy)-6-methyl]phenylacetyl-3-thiophenesulfonamide as a yellowish powder (330 mg, 16% yield, m.p. 46-50°C).

**INVENTIVE EXAMPLE 190**

Other Inventive and reference compounds that have been prepared by the above methods or routine modifications thereof, include, but are not limited to: N-(4-chloro-3-methyl-5-isoxazolyl)-2-[(4-methoxyphenoxy)carbonyl] thiophene-3-sulfonamide, N-(4-bromo-3-methyl-5-isoxazolyl)-2-[(4-methylphenoxy)carbonyl]thiophene-3-sulfonamide, N-(4-bromo-3-methyl-5-isoxazolyl)-3-[(4-methylphenoxy)methyl]thiophene-2-sulfonamide, N-(4-bromo-3-methyl-5-isoxazolyl)-2-[(4-methylphenoxy)methyl]thiophene-3-sulfonamide, N-(4-bromo-3-methyl-5-isoxazolyl)-3-(4-methyl-*trans*-styryl)-thiophene-2-sulfonamide, N-(4-bromo-3-methyl-5-isoxazolyl)-3-(4-methylphenethyl)thiophene-2-sulfonamide, N-(4-bromo-3-methyl-5-isoxazolyl)-2-[(4-methylphenyl)acetyl]thiophene-3-sulfonamide, N-(4-bromo-3-methyl-5-isoxazolyl)-2-[(3-methoxyphenyl)acetyl]thiophene-3-sulfonamide, N-(4-bromo-3-methyl-5-isoxazolyl)-3-(4-methylphenethyl)-5-(4-tolyl)thiophene-2-sulfonamide, N-(4-bromo-3-methyl-5-isoxazolyl)-3-(4-methylbenzyl)-5-(4-tolyl) thiophene-2-sulfonamide, N-(4-bromo-3-methyl-5-isoxazolyl)-3-(4-methyl-*trans*-styryl)-5-(4-tolyl)thiophene-2-sulfonamide, N-(4-chloro-3-methyl-5-isoxazolyl)-2-[3,4-(methylenedioxy)benzyl]thiophene-3-sulfonamide, N-(4-chloro-3-methyl-5-isoxazolyl)-2-[(5-methyl-3-isoxazolyl)aminocarbonyl]thiophene-3-sulfonamide, N-(4-bromo-3-methyl-5-isoxazolyl)-2-[(3-hydroxyl-6-pyridazinyl)aminocarbonyl]thiophene-3-sulfonamide, N-(4-bromo-3-methyl-5-isoxazolyl)-3-{[3, 4-(methylenedioxy)phenoxy]methyl}-thiophene-2-sulfonamide, N-(4-bromo-3-methyl-5-isoxazolyl)-2-[(4-methyl)(cinnamyl)] thiophene-3-sulfonamide, N-(4-bromo-3-methyl-5-isoxazolyl)-3-[3,4-(methylenedioxy)phenethyl]thiophene-2-sulfonamide, N-(4-bromo-3-methyl-5-isoxazolyl)-3-[3,4-(methylenedioxy)-*trans*-styryl]thiophene-2-sulfonamide, N-(4-bromo-3-methyl-5-isoxazolyl)-2-[(4-methyl)phenethyl]thiophene-3-sulfonamide, N-(3,4-dimethyl-5-isoxazolyl)-2-(4-tolylacetylphenyl)thiophene-3-sulfonamide, N-(3,4-dimethyl-5-isoxazolyl)-2-[3,4-(methylenedioxy)phenylacetyl]thiophene-3-sulfonamide, N-(4-chloro-3-methyl-5-isoxazolyl)-2-[2-hydroxy-4-methylphenyl)aminocarbonyl]thiophene-3-sulfonamide and others, including those set forth in TABLE 1 that are not specifically exemplified herein.

For example, N-(4-bromo-3-methyl-5-isoxazolyl)-3-[2-methyl-4,5-(methylenedioxy)cinnamyl]thiophene-2-sulfonamide, N-(4-bromo-3-methyl-5-isoxazolyl)-3-[2-(hydroxymethyl)-4,5-(methylenedioxy)-cinnamyllthiophene-2-sulfonamide, N-(4-bromo-3-methyl-5-isoxazolyl)-3-{2-[(tetrahydro-4H-pyran-2-ylxoy)methyl]-4, 5-(methylenedioxy) cinnamyl}-thiophene-2-sulfonamide and N-(4-bromo-3-methyl-5-isoxazolyl)-3-(2,4-dimethylcinnamyl)thiophene-2-sulfonamide have been prepared in the same manner as N-[4-bromo-3-methyl-5-isoxazolyl)-3-[3,4-(methylenedioxy)-*trans*-styryl]thiophene-2-sulfonamide. N-(4-bromo-3-methyl-5-isoxazolyl)-3-[2-methyl-4,5-(methylenedioxy)phenethyl]thiophene-2-sulfonamide and N-(4-bromo-3-methyl-5-isoxazolyl)-2-(2,4,6-trimethylphenethyl)thiophene-3-sulfonamide have been prepared in the same manner as N-(4-bromo-3-methyl-5-isoxazolyl)-2-[(4-methyl)-phenethyl]thiophene-3-sulfonamide (see Reference Example 159). N-(4-bromo-3-methyl-5-isoxazolyl)-3-{[2-propyl-4,5-(methylenedioxy)-phenoxylmethyl}thiophene-2-sulfonamide has been prepared in the same manner as N-(4-bromo-3-methyl-5-isoxazolyl)-3-[(4-methylphenoxy)-methyl]thiophene-2-sulfonamide and N-(4-bromo-3-methyl-5-isoxazolyl)-3-{[3,4-(methylenedioxy)phenoxy]methyl}thiophene-2-sulfonamide. N-(4-bromo-3-methyl-5-isoxazolyl)-2-[2-methyl-4,5-(methylenedioxy)phenethyl]thiophene-3-sulfonamide has been prepared in the same manner as N-(4-bromo-3-methyl-5-isoxazolyl)-2-[(3,4-methylenedioxy)phenethyl]thiophene-3-sulfonamide. Compounds, such as N-(4-bromo-3-methyl-5-isoxazolyl)-3-(2-tolyl)thiophene-2-sulfonamide, N-(4-bromo-3-methyl-5-isoxazolyl)-3-(3-tolyl)thiophene-2-sulfonamide, N-(4-bromo-3-methyl-5-isoxazolyl)-3-(2-tolyl)thiophene-2-sulfonamide, N-(4-bromo-3-methyl-5-isoxazolyl)-3-(3-methoxyphenyl)thiophene-2-sulfonamide, N-(4-bromo-3-methyl-5-isoxazolyl)-3-(3-methoxyphenyl)thiophene-2-sulfonamide, N-(4-bromo-3-methyl-5-isoxazolyl)-3-(2-methoxyphenyl)thiophene-2-sulfonamide, N-(4-bromo-3-methyl-5-isoxazolyl)-3-(4-ethylphenyl)thiophene-2-sulfonamide, N-(4-bromo-3-methyl-5-isoxazolyl)-3-(4-propylphenyl) thiophene-2-sulfonamide, N-(4-bromo-3-methyl-5-isoxazolyl)-3-(4-*iso*-propylphenyl)thiophene-2-sulfonamide,N-(4-bromo-3-methyl-5-isoxazolyl)-3-(4-butylphenyl)-thiophene-2-sulfonamide, N-(4-bromo-3-methyl-5-isoxazolyl)-3-(2, 4-dimethylphenyl)thiophene-2-sulfonamide, N-(4-bromo-3-methyl-5-isoxazolyl)-3-(4-*iso*-butylphenyl)thiophene-2-sulfonamide, N-(4-bromo-3-methyl-5-isoxazolyl)-3-(4-*iso*-pentylphenyl)thiophene-2-sulfonamide, N-(4-bromo-3-methyl-5-isoxazolyl)-3-(2-methyl-4-propylphenyl)thiophene-2-sulfonamide, N-(4-bromo-3-methyl-5-isoxazolyl)-3-(4-*iso*-butyl-2-methylphenyl)-thiophene-2-sulfonamide and N-(4-bromo-3-methyl-5-isoxazolyl)-3-(4-*iso*-pentyl-2-methylphenyl)thiophene-2-sulfonamide have been prepared in the same manner as N-(4-bromo-3-methyl-5-isoxazolyl)-3-[(3,4-methylenedioxy)phenyl]thiophene-2-sulfonamide (see Reference Example 125).

N-(4-bromo-3-methyl-5-isoxazolyl)-2-(2-methyl-4,5-(methylenedioxy)phenethyl]thiophene-3-sulfonamide has been prepared in the same manner as N-(4-Bromo-3-methyl-5-isoxazolyl)-2-[3,4-(methylenedioxy)phenethyl]thiophene-3-sulfonamide (Example 128). N-(4-bromo-3-methyl-5-isoxazolyl)-2-[2-methyl-4,5-(methylenedioxy)cinnamyl] thiophene-3-sulfonamide has been prepared in the same manner as N-(4-bromo-3-methyl-5-isoxazolyl)-2-[(4-methyl) (cinnamyl)] thiophene-3-sulfonamide Reference Example 158).

N-(4-bromo-3-methyl-5-isoxazolyl)-2-{[3,4-(methylenedioxy)-phenoxylmethyl}thiophene-3-sulfonamide, N-(4-bromo-3-methyl-5-isoxazolyl)-2-[(2,4,6-trimethylphenoxy)methyl)thiophene-3-sulfonamide, N-(4-bromo-3-methyl-5-isoxazolyl)-2-{[4,5-(methylenedioxy)-2-propylphenoxy)methyl}thiophene-3-sulfonamide have been prepared in the same manner as N-(4-bromo-3-methyl-5-isoxazolyl)-2-[(4-methylphenoxy)-methyl]thiophene-3-sulfonamide (Reference Example 160).

Any corresponding N-(4-halo-3-methyl-5-isoxazolyl), N-(4-halo-5-methyl-3-isoxazolyl), N-(3,4-dimethyl-5-isoxazolyl}, N-(4-halo-5-methyl-3-isoxazolyl), N-(4-halo-3-methyl-5-isoxazolyl), N-(4,5-dimethyl-3-isoxazolyl) derivative of any of these compounds or any compound dislosed herein may also be prepared and used as described herein.

## INVENTIVE EXAMPLE 191

Other inventive and reference compounds that can be prepared by the above methods or routine modifications thereof, include, but are not limited to:

N-(4-chloro-3-methyl-5-isoxazolyl)-2-(2,3,4-trimethoxy-6-methylphenylaminocarbonyl)thiophene-3-sulfonamide,
N-(4-chloro-3-methyl-5-isoxazolyl)-2-(2,3,4-trimethoxy-6-acetylphenylaminocarbonyl)thiophene-3-sulfonamide,
N-(4-chloro-3-methyl-5-isoxazolyl)-2-(2,3,4-trimethoxy-6-methoxycarbonylphenylaminocarbonyl)thiophene-3-sulfonamide,
N-(4-chloro-3-methyl-5-isoxazolyl)-2-(2,3,4-trimethoxy-6-carboxylphenylaminocarbonyl)thiophene-3-sulfonamide,
N-(4-chloro-3-methyl-5-isoxazolyl)-2-(2,3,4-trimethoxy-6-methanesulfonylphenylaminocarbonyl)thiophene-3-sulfonamide,
N-(4-chloro-3-methyl-5-isoxazolyl)-2-[2,3,4-trimethoxy-6-(cyanomethyl)phenylaminocarbonyl]thiophene-3-sulfonamide,
N-(4-chloro-3-methyl-5-isoxazolyl)-2-[2,3,4-trimethoxy-6-(2-hydroxyethyl)phenylaminocarbonyl]thiophene-3-sulfonamide,
N-(4-chloro-3-methyl-5-isoxazolyl)-2-[3,4-(methylenedioxy)-2-methoxy-6-methylphenylaminocarbonyl]thiophene-3-sulfonamide,
N-(4-chloro-3-methyl-5-isoxazolyl)-2-[3,4-(methylenedioxy)-2-methoxy-6-acetylphenylaminocarbonyl]thiophene-3-sulfonamide,
N-(4-chloro-3-methyl-5-isoxazolyl)-2-[3,4-(methylenedioxy)-2-methoxy-6-methoxycarbonylphenylaminocarbonyl]thiophene-3-sulfonamide,
N-(4-chloro-3-methyl-5-isoxazolyl)-2-[3,4-(methylenedioxy)-2-methoxy-6-carboxylphenylaminocarbonyl]thiophene-3-sulfonamide,
N-(4-chloro-3-methyl-5-isoxazolyl)-2-[3,4-(methylenedioxy)-2-methoxy-6-methanesulfonylphenylaminocarbonyl]thiophene-3-sulfonamide,
N-(4-chloro-3-methyl-5-isoxazolyl)-2-[3,4-(methylenedioxy)-2-methoxy-6-cyanophenylaminocarbonyl]thiophene-3-sulfonamide,
N-(4-chloro-3-methyl-5-isoxazolyl)-2-[3,4-(methylenedioxy)-2-methoxy-6-cyanomethylphenylaminocarbonyl]thiophene-3-sulfonamide,
N-(4-chloro-3-methyl-5-isoxazolyl)-2-[3,4-(methylenedioxy)-2-methoxy-6-(2-hydroxyethyl)phenylaminocarbonyl]thiophene-3-sulfonamide,
N-(4-chloro-3-methyl-5-isoxazolyl)-2-[3,4-(methylenedioxy)-2,6-dimethylphenylaminocarbonyl]thiophene-3-sulfonamide,
N-(4-chloro-3-methyl-5-isoxazolyl)-2-[3,4-(methylenedioxy)-6-acetyl-2-methylphenylaminocarbonyllthiophene-3-sulfonamide,
N-(4-chloro-3-methyl-5-isoxazolyl)-2-[3,4-(methylenedioxy)-6-methoxycarbonyl-2-methylphenylaminocarbonyl]thiophene-3-sulfonamide,
N-(4-chloro-3-methyl-5-isoxazolyl)-2-[3,4-(methylenedioxy)-6-carboxyl-2-methylphenylaminocarbonyl]thiophene-3-sulfonamide, N-(4-chloro-3-methyl-5-isoxazolyl)-2-[3,4-(methylenedioxy)-6-methoxy-2-methylphenylaminocarbonyl]thiophene-3-sulfonamide,
N-(4-chloro-3-methyl-5-isoxazolyl)-2-[3,4-(methylenedioxy)-6-methanesulfonyl-2-methylphenylaminocarbonyl]thiophene-3-sulfonamide,
N-(4-chloro-3-methyl-5-isoxazolyl)-2-[3,4-(methylenedioxy)-6-cyano-2-methylphenylaminocarbonyllthiophene-3-sulfonamide,
N-(4-chloro-3-methyl-5-isoxazolyl)-2-[3,4-(methylenedioxy)-6-(cyanomethyl)-2-methylphenylaminocarbonyl]thiophene-3-sulfonamide,
N-(4-chloro-3-methyl-5-isoxazolyl)-2-[3,4-(methylenedioxy)-6-(2-hydroxyethyl)-2-methylphenylaminocarbonyl]thiophene-3-sulfonamide,
N-(4-chloro-3-methyl-5-isoxazolyl)-2-[3,4-(methylenedioxy)-2-cyano-6-methylphenylaminocarbonyl]thiophene-3-sulfonamide,
N-(4-chloro-3-methyl-5-isoxazolyl)-2-[3,4-(methylenedioxy)-6-methoxy-2-cyanophenylaminocarbonyl]thiophene-3-sulfonamide,
N-(4-chloro-3-methyl-5-isoxazolyl)-2-[3,4-(methylenedioxy)-2-acetyl-6-methylphenylaminocarbonyl]thiophene-3-sulfonamide,
N-(4-chloro-3-methyl-5-isoxazolyl)-2-[3,4-(methylenedioxy)-6-methoxy-2-acetylphenylaminocarbonyl]thiophene-3-sulfonamide,
N-(4-chloro-3-methyl-5-isoxazolyl)-2-(3-cyano-2,4,6-trimethylphenylaminocarbonyl]thiophene-3-sulfonamide,
N-(4-chloro-3-methyl-5-isoxazolyl)-2-(3-carboxyl-2,4,6-trimethylphenylaminocarbonyl]thiophene-3-sulfonamide,
N-(4-chloro-3-methyl-5-isoxazolyl)-2-(3-hydroxymethyl-2,4,6-trimethylphenylaminocarbonyl]thiophene-3-sulfonamide,
N-(4-chloro-3-methyl-5-isoxazolyl)-2-(3-methanesulfonyl-2,4,6-trimethylphenylaminocarbonyl]thiophene-3-sulfonamide,
N-(4-chloro-3-methyl-5-isoxazolyl)-2-(3-cyanomethyl-2,4,6-trimethylphenylaminocarbonyl]thiophene-3-sulfonamide,
N-(4-chloro-3-methyl-5-isoxazolyl)-2-[3-(2-hydroxyethyl)-2,4,6-trimethylphenylaminocarbonyl]thiophene-3-sulfonamide,
N-(4-chloro-3-methyl-5-isoxazolyl)-2-[3-(carboxylmethyl)-2,4,6-trimethylphenylaminocarbonyl]thiophene-3-sulfonamide,
N-(4-chloro-3-methyl-5-isoxazolyl)-2-(4-cyano-2,6-dimethylphenylaminocarbonyl)thiophene-3-sulfonamide,
N-(4-chloro-3-methyl-5-isoxazolyl)-2-(4-carboxyl-2,6-dimethylphenylaminocarbonyl)thiophene-3-sulfonamide,
N-(4-chloro-3-methyl-5-isoxazolyl)-2-[4-(hydroxymethyl)-2,6-dimethylphenylaminocarbonyl]thiophene-3-sulfonamide,
N-(4-chloro-3-methyl-5-isoxazolyl)-2-[4-(2-hydroxyethyl)-2,6-dimethylphenylaminocarbonyllthiophene-3-sulfonamide,
N-(4-chloro-3-methyl-5-isoxazolyl)-2-[4-(cyanomethyl)-2,6-dimethylphenylaminocarbonyl]thiophene-3-sulfonamide,
N-(4-chloro-3-methyl-5-isoxazolyl)-2-[4-(carboxylmethyl)-2,6-dimethylphenylaminocarbonyl]thiophene-3-sulfonamide,
N-(4-chloro-3-methyl-5-isoxazolyl)-2-(4-methanesulfonyl-2,6-dimethylphenylaminocarbonyl)thiophene-3-sulfonamide,
N-(4-chloro-3-methyl-5-isoxazolyl)-2-(2,3,4-trimethoxy-6-methylphenylacetyl)thiophene-3-sulfonamide,
N-(4-chloro-3-methyl-5-isoxazolyl)-2-(2,3,4-trimethoxy-6-acetylphenylacetyl)thiophene-3-sulfonamide,
N-(4-chloro-3-methyl-5-isoxazolyl)-2-(2,3,4-trimethoxy-6-methoxycarbonylphenylacetyl)thiophene-3-sulfonamide,
N-(4-chloro-3-methyl-5-isoxazolyl)-2-(2,3,4-trimethoxy-6-carboxylphenylacetyl)thiophene-3-sulfonamide,
N-(4-chloro-3-methyl-5-isoxazolyl)-2-(2,3,4-trimethoxy-6-methanesulfonylphenylacetyl)thiophene-3-sulfonamide,
N-(4-chloro-3-methyl-5-isoxazolyl)-2-[2,3,4-trimethoxy-6-(cyanomethyl)phenylacetyl]thiophene-3-sulfonamide,
N-(4-chloro-3-methyl-5-isoxazolyl)-2-[2,3,4-trimethoxy-6-(2-hydroxyethyl)phenylacetyl]thiophene-3-sulfonamide,
N-(4-chloro-3-methyl-5-isoxazolyl)-2-[3,4-(methylenedioxy)-2-methoxy-6-methylphenylacetyl]thiophene-3-sulfonamide,
N-(4-chloro-3-methyl-5-isoxazolyl)-2-[3,4-(methylenedioxy)-2-methoxy-6-acetylphenylacetyl]thiophene-3-sulfonamide,
N-(4-chloro-3-methyl-5-isoxazolyl)-2-[3,4-(methylenedioxy)-2-methoxy-6-methoxycarbonylphenylacetyl]thiophene-3-sulfonamide,
N-(4-chloro-3-methyl-5-isoxazolyl)-2-[3,4-(methylenedioxy)-2-methoxy-6-carboxylphenylacetyl]thiophene-3-sulfonamide,
N-(4-chloro-3-methyl-5-isoxazolyl)-2-[3,4-(methylenedioxy-2-methoxy-6-methanesulfonyl)phenylacetyl]thiophene-3-sulfonamide,
N-(4-chloro-3-methyl-5-isoxazolyl)-2-[3,4-(methylenedioxy)-2-methoxy-6-(cyano)phenylacetyl]thiophene-3-sulfonamide,
N-(4-chloro-3-methyl-5-isoxazolyl)-2-(3,4-(methylenedioxy)-2-methoxy-6-(cyanomethylphenylacetyl]thiophene-3-sulfonamide,
N-(4-chloro-3-methyl-5-isoxazolyl)-2-[3,4-(methylenedioxy)-2-methoxy-6-(2-hydroxyethyl)phenylacetyl]thiophene-3-sulfonamide,
N-(4-chloro-3-methyl-5-isoxazolyl)-2-[3,4-(methylenedioxy)-2,6-dimethylphenylacetyl]thiophene-3-sulfonamide,
N-(4-chloro-3-methyl-5-isoxazolyl)-2-[3,4-(methylenedioxy)-6-acetyl-2-methylphenylacetyl]thiophene-3-sulfonamide,
N-(4-chloro-3-methyl-5-isoxazolyl)-2-[3,4-(methylenedioxy)-6-methoxycarbonyl-2-methylphenylacetyl]thiophene-3-sulfonamide,
N-(4-chloro-3-methyl-5-isoxazolyl)-2-[3,4-(methylenedioxy)-6-carboxyl-2-methylphenylacetyl]thiophene-3-sulfonamide,
N-(4-chloro-3-methyl-5-isoxazolyl)-2-[3,4-(methylenedioxy)-6-methoxy-2-methylphenylacetyl]thiophene-3-sulfonamide,
N-(4-chloro-3-methyl-5-isoxazolyl)-2-[3,4-(methylenedioxy)-6-methanesulfonyl-2-methylphenylacetyl]thiophene-3-sulfonamide,
N-(4-chloro-3-methyl-5-isoxazolyl)-2-[3,4-(methylenedioxy)-6-cyano-2-methylphenylacetyl]thiophene-3-sulfonamide,
N-(4-chloro-3-methyl-5-isoxazolyl)-2-[3,4-(methylenedioxy)-6-(cyanomethyl)-2-methylphenylacetyl]thiophene-3-sulfonamide,

N-(4-chloro-3-methyl-5-isoxazolyl)-2-[3,4-(methylenedioxy)-6-(2-hydroxyethyl)-2-methylphenylacetyl]thiophene-3-sulfonamide,
N-(4-chloro-3-methyl-5-isoxazolyl)-2-[3,4-(methylenedioxy)-2-cyano-6-methylphenylacetyl]thiophene-3-sulfonamide,
N-(4-chloro-3-methyl-5-isoxazolyl)-2-[3,4-(methylenedioxy)-6-methoxy-2-cyanophenylacetyl]thiophene-3-sulfonamide,
N-(4-chloro-3-methyl-5-isoxazolyl)-2-[3,4-(methylenedioxy)-2-acetyl-6-methylphenylacetyl]thiophene-3-sulfonamide,
N-(4-chloro-3-methyl-5-isoxazolyl)-2-[3,4-(methylenedioxy)-6-methoxy-2-acetylphenylacetyl]thiophene-3-sulfonamide,
N-(4-chloro-3-methyl-5-isoxazolyl)-2-(3-cyano-2,4,6-trimethylphenylacetyl)thiophene-3-sulfonamide,
N-(4-chloro-3-methyl-5-isoxazolyl)-2-(3-carboxyl-2,4,6-trimethylphenylacetyl)thiophene-3-sulfonamide,
N-(4-chloro-3-methyl-5-isoxazolyl)-2-(3-hydroxymethyl-2,4,6-trimethylphenylacetyl)thiophene-3-sulfonamide,
N-(4-chloro-3-methyl-5-isoxazolyl)-2-(3-methanesulfonyl-2,4,6-trimethylphenylacetyl)thiophene-3-sulfonamide,
N-(4-chloro-3-methyl-5-isoxazolyl)-2-[3-(cyanomethyl)-2,4,6-trimethylphenylacetyl]thiophene-3-sulfonamide,
N-(4-chloro-3-methyl-5-isoxazolyl)-2-[3-(2-hydroxyethyl)-2,4,6-trimethylphenylacetyl]thiophene-3-sulfonamide,
N-(4-chloro-3-methyl-5-isoxazolyl)-2-[3-(carboxylmethyl)-2,4,6-trimethylphenylacetyl]thiophene-3-sulfonamide,
N-(4-chloro-3-methyl-5-isoxazolyl)-2-(4-cyano-2,6-dimethylphenylacetyl)thiophene-3-sulfonamide,
N-(4-chloro-3-methyl-5-isoxazolyl)-2-(4-carboxyl-2,6-dimethylphenylacetyl)thiophene-3-sulfonamide,
N-(4-chloro-3-methyl-5-isoxazolyl)-2-(4-hydroxymethyl-2,6-dimethylphenylacetyl)thiophene-3-sulfonamide,
N-(4-chloro-3-methyl-5-isoxazolyl)-2-[4-(2-hydroxyethyl)-2,6-(dimethyl)phenylacetyl]thiophene-3-sulfonamide,
N-(4-chloro-3-methyl-5-isoxazolyl)-2-[4-cyanomethyl-2,6-(dimethyl)phenylacetyl]thiophene-3-sulfonamide,
N-(4-chloro-3-methyl-5-isoxazolyl)-2-[4-(carboxylmethyl)-2,6-dimethylphenylacetyl]thiophene-3-sulfonamide,
and
N-(4-chloro-3-methyl-5-isoxazolyl)-2-(4-methanesulfonyl-2,6-dimethylphenylacetyl)thiophene-3-sulfonamide.

**INVENTIVE EXAMPLE 192**

Other inventive and reference compounds, having activity generally at $IC_{50}$ concentrations of 10 μM or substantially less for $ET_A$ or $ET_B$ receptors, in which $Ar^2$ contains a heterocyclic ring, such as thienyl-, furyl- and pyrrolesulfonamides of interest herein, can be or have been prepared (see, e.g., TABLE 1) by methods analogous to those set forth in the above Examples. Such compounds include, but are not limited to the following compounds: N-(4-bromo-3-methyl-5-isoxazolyl)-2-carboxyl-1-methylindole-3-sulfonamide, N-(4-chloro-3-methyl-5-isoxazolyl)-2-[(4-oxacyclohexyl)oxycarbonyl]thiophene-3-sulfonamide, 2-[3,4-(methylenedioxy)phenylacetyl]thiophene-3-sulfonamide, N-(4-chloro-3-methyl-5-isoxazolyl)-{2-[3,4-(methylenedioxy)-phenyl]acetyl}thiophene-3-sulfonamide oxime, N-(4-chloro-3-methyl-5-isoxazolyl)-2-phenylbenzo[b]thiophene sulfonamide, N-(4-chloro-3-methyl-5-isoxazolyl)-2-[(4-tolyl)aminocarbonyl]-1-methylindole-3-sulfonamide, N-(4-chloro-3-methyl-5-isoxazolyl)-2-[(4-methoxyphenoxy)carbonyl]thiophene-3-sulfonamide, N-(4-bromo-3-methyl-5-isoxazolyl)-1-[3,4-(methylenedioxy)benzyl]indole-2-sulfonamide, N-(4-bromo-3-methyl-5-isoxazolyl)-2-[(4-methylphenoxy)carbonyl]thiophene-3-sulfonamide, N-(4-chloro-3-methyl-5-isoxazolyl)-2-[(4-methoxyphenyl)acetyl]thiophene-3-sulfonamide, N-(4-chloro-3-methyl-5-isoxazolyl)-6-methoxy-2-[3,4-(methylenedioxy)-benzyl]benzo[b]thiophene-3-sulfonamide, N-(4-bromo-3-methyl-5-isoxazolyl)-3-[(4-methylphenoxy)methyl]thiophene-2-sulfonamide, N-(4-bromo-3-methyl-5-isoxazolyl)-2-[(4-methylphenoxy)methyl]thiophene-3-sulfonamide, N-(4-bromo-3-methyl-5-isoxazolyl)-3-(4-methyl-*trans*-styryl)thiophene-2-sulfonamide, N-(4-bromo-3-methyl-5-isoxazolyl)-3-(4-methylphenethyl)thiophene-2-sulfonamide,N-(4-bromo-3-methyl-5-isoxazolyl)-2-[(4-methylphenyl)acetyl]thiophene-3-sulfonamide, N-(4-chloro-3-methyl-5-isoxazolyl)-2-[(3-methoxyphenyl)acetyl]thiophene-3-sulfonamide, N-[4-chloro-3-methyl-5-isoxazolyl)-2-{1-hydroxy-1-[3,4-(methylenedioxy)-benzyl]ethyl}thiophene-3-sulfonamide, N-4-(bromo-3-methyl-5-isoxazolyl)-3-(4-methylphenrthyl)(4-tolyl)thiophene-2-sulfonamide, N-(4-bromo-3-methyl-5-isoxazolyl)-3-(4-methylbenzyl)-5-(4-tolyl)thiophene-2-sulfonamide, N-(4-bromo-3-methyl-5-isoxazolyl)-3-(4-methyl-*trans*-styryl)-5-(4-tolyl)thiophene-2-sulfonamide, N-(4-chloro-3-methyl-5-isoxazolyl)-2-[β,β-(ethylenedioxy)3,4-(methylenedioxy)phenethyl]thiophene-3-sulfonamide, N-(4-chloro-3-methyl-5-isoxazolyl)-2-[β-(dimethylamino)-3,4-(methylenedioxy)phenethy]thiophene-3-sulfonamide, N-(4-chloro-3-methyl-5-isoxazolyl)-2-{α-hydroxy-[3,4-(methylenedioxy)phenyl]acetyl}thiophene-3-sulfonamide; N-(4-chloro-5-methyl-3-isoxazolyl)-2-[3,4-(methylenedioxy)-benzyl]benzo[b]thiophene-3-sulfonamide; N-(4-bromo-3-methyl-5-isoxazolyl)-3-styrylthiophene-2-sulfonamide; N-(4-bromo-3-methyl-5-isoxazolyl)-2-styrylthiophene-3-sulfonamide; N-(4-bromo-3-methyl-5-isoxazolyl)-2-(benzoylamino)thiophene-3-suifonamide; N-(4-bromo-3-methyl-5-isoxazolyl)-2-[(phenyl)methylaminocarbonyl]thiophene-3-sulfonamide; N-(4-bromo-3-methyl-5-isoxazolyl)-5-(phenylthio)furan-2-sulfonamide; N-(4-bromo-3-methyl-5-isoxazolyl)-5-(hydroxymethyl)furan-2-sulfonamide; N-(4-bromo-3-methyl-5-isoxazolyl)-5-(carbomethoxy)furan-2-sulfonamide; N-(4-bromo-3-methyl-5-isoxazolyl)-2,5-dimethylfuran-3-sulfonamide; N-(4-bromo-3-methyl-5-isoxazolyl)-2-(diisopropylaminocarbonyl)-thiophene-3-sulfonamide; N-(4-bromo-3-methyl-5-isoxazolyl)-2-(diethylaminocarbonyl)thiophene-3-sulfonamide; N-(4-bromo-3-methyl-5-isoxazolyl)-5-styrylfuran-2-sulfonamide; N-(4-bromo-3-methyl-5-isoxazolyl)-5-styrylthiophene-2-sulfonamide; N-(4-chloro-3-methyl-5-isoxazolyl)-2-[3,4-(methylenedioxy)benzyl]-5-(dimethylamino)benzo[b]thiophene-3-sulfonamide; N-(4-chloro-3-methyl-5-isoxazolyl)-2-[3,4-(methylenedioxy)benzyl]-7-methoxybenzo[b]thiophene-3-sulfonamide; N-(4-chloro-3-methyl-5-isoxazolyl)-2-[3,4-(methylenedioxy)benzyl]-7-phenoxybenzo[b]thiophene-3-sulfonamide; N-(4-chloro-3-methyl-5-isoxazolyl)-2-[3,4-(methylenedioxy)benzyl]-5-methoxybenzo[b]thiophene-3-sulfonamide; N-(4-chloro-3-methyl-5-isoxazolyl)-2-[3,4-(methylenedioxy)benzyl]-5-isobutylaminobenzo[b]thiophene-3-sulfonamide; N-(4-chloro-3-methyl-5-isoxazolyl)-2-[3,4-(methylenedioxy)benzyl]-5-benzylaminobenzo[b]thiophene-3-sulfonamide; N-(4-chloro-3-methyl-5-isoxazolyl)-2-[3,4-(methylenedioxy)-phenoxy] benzo[b]thiophene-3-sulfonamide; N-(4-chloro-3-methyl-5-isoxazolyl)-2-[3,4-(methylenedioxy)phenoxy]-5-dimethylaminobenzo[b]thiophene-3-sulfonamide; N-(4-chloro-3-methyl-5-isoxazolyl)-2-[(3,4-methylenedioxy)phenyl]acetyl-5-dimethylaminobenzo[b]thiophene-3-sulfonamide; N-(4-chloro-3-methyl-5-isoxazolyl)-2-[3,4-(methylenedioxy)-benzylcarbonyl]-N-methylindole-3-sulfonamide; N-(4-chloro-3-methyl-5-isoxazolyl)-2-[3,4-(methylenedioxy)phenoxycarbonyl]indole-3-sulfonamide; N-(4-chloro-3-methyl-5-isoxazolyl)-2-[3,4-(methylenedioxy)phenoxycarbonyl]-N-methylindole-3-sulfonamide; N-(4-chloro-3-methyl-5-isoxazolyl)-2-[3,4-(methylenedioxy)phenoxycarbonyl]indole-3-sulfonamide; N-(4-chloro-3-methyl-5-isoxazolyl)-2-[3,4-(methylenedioxy)benzyl]-N-methylindole-3-sulfonamide; N-(4-chloro-3-methyl-5-isoxazolyl)-2-[3,4-(methylenedioxy)benzyl]indole-3-sulfonamide; N-(4-chloro-3-methyl-5-isoxazolyl)-2-[3,4-(methylenedioxy)benzyloxycarbonyl]-7-(N,N-dimethylamino)benzo[b]thiophene-3-sulfonamide; N-(4-chloro-3-methyl-5-isoxazolyl)-2-[3,4-(methylenedioxy)benzyl]-7-(N,N-dimethylamino)benzo[b]thiophene-3-sulfonamide;N-(4-chloro-3-methyl-5-isoxazolyl)-2-[3,4-(methylenedioxy)benzoyl]-7-(N,N-dimethyl)amino)benzo[b]thiophene-3-sulfonamide; N-(4-chloro-3-methyl-5-isoxazolyl)-7-(N,N-dimethylamino)benzo[b]thiophene-3-sulfonamide; N-(4-chloro-3-methyl-5-isoxazolyl)-7-(methoxycarbonyl)benzo[b]thiophene-3-sulfonamide; N-(4-chloro-3-methyl-5-isoxazolyl)-2-[3,4-(methylenedioxy)benzyl]-7-(methoxy)-benzo[b]thiophene-3-sulfonamide; N-(4-chloro-3-methyl-5-isoxazolyl)-7-(methoxy)benzo[b]thiophene-3-sulfonamide; N-(4-bromo-3-methyl-5-isoxazolyl)-2-(4-methylphenethyl)thiophene-3-sulfonamide; N-(4-bromo-3-methyl-5-isoxazolyl)-2-(*trans*-4-methylcinnamyl)thiophene-3-sulfonamide; N-(4-bromo-3-methyl-5-isoxazolyl)-3-(4-methylphenethyl)thiophene-2-sulfonamide; N-(4-bromo-3-methyl-5-isoxazolyl)-3-(3-methylphenethyl)thiophene-2-sulfonamide; N-(4-bromo-3-methyl-5-isoxazolyl)-3-(2-methylphenethyl)thiophene-2-sulfonamide; N-(4-bromo-3-methyl-5-isoxazolyl)-3-(*trans*-4-methylcinnamyl)thiophene-2-sulfonamide; N-(4-bromo-3-methyl-5-isoxazolyl)-3-(*trans*-3-methylcinnamyl)thiophene-2-sulfonamide; N-(4-bromo-3-methyl-5-isoxazolyl)-3-(*trans*-2-methylcinnamyl)thiophene-2-sulfonamide; N-(4-bromo-3-methyl-5-isoxazolyl)-3-[(4-methylphenoxy)-methyl]thiophene-2-sulfonamide; N-(4-bromo-3-methyl-5-isoxazolyl)-3-[3,4-(methylenedioxy)phenethyl]thiophene-2-sulfonamide; N-(4-chloro-3-methyl-5-isoxazolyl)-2-{(3,4-(dimethoxy)phenyl]acetyl)}thiophene-3-sulfonamide; N-(4-chloro-3-methyl-5-isoxazolyl)-2-[(3,5-dimethoxyphenyl)-acetyl]thiophene-3-sulfonamide; N-(4-chloro-3-methyl-5-isoxazolyl)-2-[(3,4,5-trimethoxyphenyl)acetyl]thiophene-3-sulfonamide; N-(4-chloro-3-methyl-5-isoxazolyl)-2-[3,4-(methylenedioxy)benzylsulfonyl]thiophene-3-sulfonamide; N-(4-chloro-3-methyl-5-isoxazolyl)-2-[3,4-(methylenedioxy)-benzylsulfinyl]thiophene-3-sulfonamide; N-(4-chloro-3-methyl-5-isoxazolyl)-2-[3,4-(methylenedioxy)benzylsulfenyl]thiophene-3-sulfonamide; N-(4-chloro-3-methyl-5-isoxazolyl)-2-{1-(dimethylamino)-2-[3,4-(methylenedioxy)phenyl}ethylthiophene-3-sulfonamide; N-(4-chloro-3-methyl-5-isoxazolyl)-2-{ 1-methylamino)-2-[3,4-(methylenedioxy)phenyl]ethyl}thiophene-3-sulfonamide; N-(4-chloro-3-methyl-5-isoxazolyl)-2-{1-(methoxylimino)-2-[3,4-(methylenedioxy)phenyl]ethyl}thiophene-3-sulfonamide; N-(4-chloro-3-methyl-5-isoxazolyl)-2-{1-(carboxyl)-2-[3,4-(methylenedioxy)phenyl]-ethyl}thiophene-3-sulfonamide; N-(4-chloro-3-methyl-5-isoxazolyl)-2-{2-(carboxyl)-1-[3,4-(methylenedioxy)benzyl]vinyl}thiophene-3-sulfonamide; N-(4-chloro-3-methyl-5-isoxazolyl)-2-{3-[3,4-(methylenedioxy)phenyll-1,2,4-oxadiazol-5-lyl} thiophene-3-sulfonamide; and N-(4-chloro-3-methyl-5-isoxazolyl-2-{3-[3,4-(methylenedioxy)benzyl}-1,2,4-oxadiazol-5-lyl}thiophene-3-sulfonamide.

Additional compounds include, but are not limited to: N-(4-chloro-3-methyl-5-isoxazolyl)-2-{[2-(methanesulfonyl)-4,5-(methylenedioxy)-phenyl]aminocarbonyl}thiophene-3-sulfonamide; N-(4-chloro-3-methyl-5-isoxazolyl)-2-{[3,4-(methylenedioxy)-6-carboxylphenyl]aminocarbonyl}thiophene-3-sulfonamide; N-(4-chloro-3-methyl-5-isoxazolyl)-2-{[4,5-(methylenedioxy)-2-(methoxycarbonyl]phenyl}aminocarbonyl}thiophene-3-sulfonamide; N-(4-chloro-3-methyl-5-isoxazolyl)-2-{[2-cyano-4,5-(methylenedioxy)phenyl]aminocarbonyl}thiophene-3-sulfonamide; N-(4-chloro-3-methyl-5-isoxazolyl)-2-{[4,5-(methylenedioxy)-2-hydroxymethyl)phenyl]aminocarbonylthiophene-3-sulfonamide; N-(4-chloro-3-methyl-5-isoxazolyl)-2-[(2-acetyl-4-methylphenyl]aminocarbonyl]thiophene-3-sulfonamide; N-(4-chloro-3-methyl-5-isoxazolyl)-2-{[2-(methanesulfonyl)-4-methylphenyl]aminocarbonyl}thiophene-3-sulfonamide; N-(4-chloro-3-methyl-5-isoxazolyl)-2-[(2-carboxyl-4-methylphenyl)aminocarbonyl]thiophene-3-sulfonamide; N-(4-chloro-3-methyl-5-isoxazolyl)-2-[(2-methoxycarbonyl-4-methylphenyl)aminocarbonyl]thiophene-3-sulfonamide; N-(4-chloro-3-methyl-5-isoxazolyl)-2-[(2-cyano-4-methylphenyl)aminocarbonyl]thiophene-3-sulfonamide; N-(4-chloro-3-methyl-5-isoxazolyl)-2-{[2-(hydroxymethyl)-4-methylphenyl]aminocarbonyl}thiophene-3-sulfonamide; N-(4-chloro-3-methyl-5-isoxazolyl)-2-[(3,4-dimethoxy-6-acetylphenyl)aminocarbonyl]thiophene-3-sulfonamide;N-(4-chloro-3-methyl-5-isoxazolyl)-2-{[2-(methanesulfonyl)-4,5-dimethoxyphenyl]aminocarbonyl}thiophene-3-sulfonamide; N-(4-chloro-3-methyl-5-isoxazolyl)-2-[(4,5-dimethoxy-2-carboxylphenyl)aminocarbonyl]thiophene-3-sulfonamide; N-(4-chloro-3-methyl-5-isoxazolyl)-2-[(4,5-dimethoxy-2-methoxycarboxyl)phenyl)aminocarbonyl]thiophene-3-sulfonamide; N-(4-chloro-3-methyl-5-isoxazolyl)-2-[2-cyano(4,5-dimethoxyphenyl)aminocarbonyl]thiophene-3-sulfonamide; N-(4-chloro-3-methyl-5-isoxazolyl)-2-(4,5-dimethoxy-2-hydroxymethyl)phenylaminocarbonylthiophene-3-sulfonamide; N-(4-chloro-3-methyl-5-isoxazolyl)-2-{[2-acetyl-4,5-(methylenedioxy)phenyl]acetyl}thiophene-3-sulfonamide; N-(4-chloro-3-methyl-5-isoxazolyl)-2-{[2-(methanesulfonyl)-4,5-(methylenedioxy)phenyl]-acetyl)thiophene-3-sulfonamide; N-(4-chloro-3-methyl-5-isoxazolyl)-2-{[carboxyl 4,5-(methylenedioxy)-2-phenylacetylthiophene-3-sulfonamide; N-(4-chloro-3-methyl-5-isoxazolyl)-2-{[4,5-(methylenedioxy)-2-methoxycarbonylphenyl]acetylthiophene-3-sulfonamide; N-(4-chloro-3-methyl-5-isoxazolyl)-2-{2-cyano[4,5-(methylenedioxy)-phenyl]acetyl}thiophene-3-sulfonamide; N-(4-chloro-3-methyl-5-isoxazolyl)-2-{2-hydroxymethyl[4,5-(methylenedioxy)-phenyl]acetyl}thiophene-3-sulfonamide; N-(4-chloro-3-methyl-5-isoxazolyl)-2-[(2,4-dimethoxy)phenyl)aminocarbonyl]thiophene-3sulfonamide; N-(4-chloro-3-methyl-5-isoxazolyl)-2-[(4-methoxy-2-methylphenyl)aminocarbonyl]thiophene-3-sulfonamide; N-(4-chloro-3-methyl-5-isoxazolyl)-2-[(2,3-dimethylphenyl) aminocarbonyl]thiophene-3-sulfonamide; N-(4-chloro-3-methyl-5-isoxazolyl)-2-[(2,4-dimethylphenyl)aminocarbonyl] thiophene-3-sulfonamide; N-(4-chloro-3-methyl-5-isoxazolyl)-2-[(2,5-dimethylphenyl)aminocarbonyl]thiophene-3-sulfonamide; N-(4-chloro-3-methyl-5-isoxazolyl)-2-[(2,6-dimethylphenyl)aminocarbonyl]thiophene-3-sulfonamide; N-(4-chloro-3-methyl-5-isoxazolyl)-2-[(3,4-dimethylphenyl)aminocarbonyl]thiophene-3-sulfonamide; N-(4-chloro-3-methyl-5-isoxazolyl)-2-[(2,5-dimethylphenyl)aminocarbonyl]thiophene-3-sulfonamide; N-(4-chloro-3-methyl-5-isoxazolyl)-2-(3,5-dimethyl)phenylaminocarbonylthiophene-3-sulfonamide; N-(4-chloro-3-methyl-5-isoxazolyl)-2-[(2-methoxy-6-methylphenyl)aminocarbonyl]thiophene-3-sulfonamide; N-(4-chloro-3-methyl-5-isoxazolyl)-2-[(2,4,6-trimethylphenyl)aminocarbonyl]-thiophene-3-sulfonamide; N-(4-chloro-3-methyl-5-isoxazolyl)-2-[(4-methoxy-2-methylphenyl)aminocarbonyl]-thiophene-3-sulfonamide; N-(4-chloro-3-methyl-5-isoxazolyl)-2-[2-ethyl(4-methoxy-)phenyl)aminocarbonyl)thiophene-3-sulfonamide; N-(4-chloro-3-methyl-5-isoxazolyl)-2-[(2-isopropyl-4-methoxy-phenyl)aminocarbonyl] thiophene-3-sulfonamide; N-(4-chloro-3-methyl-5-isoxazolyl)-2-[(2-propyl-4-methoxy-phenyl)aminocarbonyl]-thiophene-3-sulfonamide; N-(4-chloro-3-methyl-5-isoxazolyl)-2-[(4-methoxy-2-biphenylaminocarbonyl]-thiophene-3-sulfonamide; N-(4-chloro-3-methyl-5-isoxazolyl)-2-{[3,4-(methylenedioxy)-6-methylphenyl)acetyll-thiophene-3-sulfonamide; N-(4-chloro-3-methyl-5-isoxazolyl)-2-{[3,4-(methylenedioxy)-6-ethylphenyl)-acetyl}thiophene-3-sulfonamide; N-(4-chloro-3-methyl-5-isoxazolyl)-2-{[3,4-(methylenedioxy)-6-methoxyphenyl]acetyl}thiophene-3-sulfonamide.

### INVENTIVE EXAMPLE 217

### Assays for identifying compounds that exhibit endothelin antagonistic and/or agonist activity

Compounds that are potential endothelin antagonists are identified by testing their ability to compete with $^{125}$I-labeled ET-1 for binding to human $ET_A$ receptors or $ET_B$ receptors present on isolated cell membranes. The effectiveness of the test compound as an antagonist or agonist of the biological tissue response of endothelin can also be assessed by measuring the effect on endothelin induced contraction of isolated rat thoracic aortic rings. The ability of the compounds to act as antagonists or agonists for $ET_B$ receptors can be assess by testing the ability of the compounds are to inhibit endothelin-1 induced prostacyclin release from cultured bovine aortic endothelial cells.

### A. Endothelin binding inhibition - Binding Test #1: Inhibition of binding to $ET_A$ receptors

TE 671 cells (ATCC Accession No. HTB 139) express $ET_A$ receptors. These cells were grown to confluence in T-175 flasks. Cells from multiple flasks were collected by scraping, pooled and centrifuged for 10 min at 190 X g. The cells were resuspended in phosphate buffered saline (PBS) containing 10 mM EDTA using a Tenbroeck homogenizer. The suspension was centrifuged at 4° C at 57,800 X g for 15 min, the pellet was resuspended in 5 ml of buffer A (5mM HEPES buffer, pH 7.4 containing aprotinin (100 KIU/ml)) and then frozen and thawed once. 5 ml of Buffer B (5 mM HEPES Buffer, pH 7.4 containing 10 mM $MnCl_2$ and 0.001% deoxyribonuclease Type 1) was added, the suspension mixed by inversion and then incubated at 37° C for 30 minutes. The mixture was centrifuged at 57,800 X g as described above, the pellet washed twice with buffer A and then resuspended in buffer C (30 mM HEPES buffer, pH 7.4 containing aprotinin (100 KIU/ml) to give a final protein concentration of 2 mg/ml and stored at -70° C until use.

The membrane suspension was diluted with binding buffer (30 mM HEPES buffer, pH 7.4 containing 150 mM NaCl, 5mM $MgCl_2$, 0.5% Bacitracin) to a concentration of 8 μg/50 μl. $^{125}$I-endothelin-1 (3,000 cpm, 50 mL) was added to 50 μL of either: (A) endothelin-1 (for non specific binding) to give a final concentration 80 nM); (B) binding buffer (for total binding); or (C) a test compound (final concentration 1 nM to 100 μM). The membrane suspension (50 μL), containing up to 8 μg of membrane protein, was added to each of (A), (B), or (C). Mixtures were shaken, and incubated at 4° C for 16-18 hours, and then centrifuged at 4° C for 25 min at 2,500 X g. Alternatively, the incubation was conducted at 24° C. When incubated at 24° C, the $IC_{50}$ concentrations are 2- to 10-fold higher than when the incubation is conducted at 4° C. This, must be kept in mind when comparing $IC_{50}$ concentrations among compounds provided herein.

The supernatant, containing unbound radioactivity, was decanted and the pellet counted on a Genesys multiwell gamma counter. The degree of inhibition of binding (D) was calculated according to the following equation:

$$\% \text{ D} = 100 - \frac{(\text{C}) - (\text{A})}{(\text{B}) - (\text{A})} \text{ X } 100$$

Each test was generally performed in triplicate.

**B. Endothelin binding inhibition - Binding Test #2: Inhibition of binding to $ET_B$ receptors**

COS7 cells were transfected with DNA encoding the $ET_B$ receptor, The resulting cells, which express the human $ET_B$ receptor, were grown to confluence in T-150 flasks. Membrane was prepared as described above. The binding assay was performed as described above using the membrane preparation diluted with binding buffer to a concentration of 1 µg/50 µl.

Briefly, the COS7 cells, described above, that had been transfected with DNA encoding the $ET_B$ receptor and express the human $ET_B$ receptor on their surfaces were grown to confluence in T-175 flasks. Cells from multiple flasks were collected by scraping, pooled and centrifuged for 10 min at 190 X g. The cells were resuspended in phosphate buffered saline (PBS) containing 10 mM EDTA using a Tenbroeck homogenizer. The suspension was centrifuged at 4° C 57,800 X g for 15 min, the pellet was resuspended in 5 ml of buffer A (5mM HEPES buffer, pH 7.4 containing aprotinin (100 KIU/ml)) and then frozen and thawed once. Five ml of Buffer B (5 mM HEPES Buffer, pH 7.4 containing 10 mM $MnCl_2$ and 0.001 % deoxyribonuclease Type 1) was added, the suspension mixed by inversion and then incubated at 37° C for 30 minutes. The mixture was centrifuged at 57,800 X g as described above, the pellet washed-twice with buffer A and then resuspended in buffer C (30 mM HEPES buffer, pH 7.4 containing aprotinin (100 KIU/ml) to give a final protein concentration of 2 mg/ml.

The binding assay was performed as described above using the membrane preparation diluted to give 1 µg/50 µl of binding buffer.

**C. Test for activity against endothelin-induced contraction of isolated rat thoracic aortic rings**

The effectiveness of the test compound as an antagonist or agonist of the biological tissue response of endothelin also is assessed by measuring the effect on endothelin induced contraction of isolated rat thoracic aortic rings (see, e.g., Borges et al. (1989) Eur. J. Pharmacol. 165:223-230) or by measuring the ability to contract the tissue when added alone.

Compounds to be tested are prepared as 100 µM stocks. If necessary to effect dissolution, the compounds are first dissolved in a minimum amount of DMSO and diluted with 150 mM NaCl. Because DMSO can cause relaxation of the aortic ring, control solutions containing varying concentrations of DMSO were tested.

The thoracic portion of the adult rat aorta is excised, the endothelium abraded by gentle rubbing and then cut into 3 mm ring segments. Segments are suspended under a 2 g preload in a 10 ml organ bath filled with Krebs'-Henseleit solution saturated with a gas mixture of 95% 02 and 5% $CO_2$ (118 mM NaCl, 4.7 mM KCl, 1.2 mM $MgSO_4$, 1.2 mM $KH_2PO_4$, 25 mM $NaHCO_3$, 2.5 mM $CaCl_2$, 10 mM D-glucose).

There is a correlation between activity as an antagonist of endothelin-induced thoracic aortic ring contraction and activity as an inhibitor of binding of endothelin to endothelin receptors. The $pA_2$ is a linear function of the log of the $IC_{50}$.

**D. Assay for identifying compounds that have agonist and/or antagonistic activity against $ET_B$ receptors**

**1. Stimulation of prostacyclin release**

Since endothelin-1 stimulates the release of prostacyclin from cultured bovine aortic endothelial cells, the compounds that have agonist or antagnoist activity are identified by their ability to inhibit endothelin-1 induced prostacyclin release from such endothelial cells by measuring 6-keto $PGF_{1\alpha}$ substantially as described by (Filep et al. (1991) Biochem. Biophys. Res. Commun. 177 171-176. Bovine aortic cells are obtained from collagenase-treated bovine aorta, seeded into culture plates, grown in Medium 199 supplemented with heat inactivated 15% fetal calf serum, and L-glutamine (2 mM), penicillin, streptomycin and fungizone, and subcultured at least four times. The cells are then seeded in six-well plates in the same medium. Eight hours before the assay, after the cells reach confluence, the medium is replaced. The cells are then incubated with a) medium alone, b) medium containing endothelin-1 (10 nM), c) test compound alone, and d) test compound + endothelin-1 (10 nM).

After a 15 min incubation, the medium is removed from each well and the concentrations of 6-keto $PGF_{1\alpha}$

are measured by a direct immunoassay. Prostacyclin production is calculated as the difference between the amount of 6-keto $PGF_{1\alpha}$ released by the cells challenged with the endothelin-1 minus the amount released by identically treated unchallenged cells. Compounds that stimulate 6-keto $PGF_{1\alpha}$ release possess agonist activity and those which inhibit endothelin-1 6-keto $PGF_{1\alpha}$ release possess antagonist activity.

### 2. Inhibition of sarafotoxin 6c induced contraction

Sarafotoxin 6c is a specific $ET_B$ antagonist that contracts rat fundal stomach strips. The effectiveness of tests compounds to inhibit this sarafotoxin 6c-induced contraction of rat fundal stomach strips is used as a measure $ET_B$ antagonist activity. Two isolated rat fundal stomach strips are suspended under a 1 g load in a 10 ml organ bath filled with Krebs'-Henseleit solution containing 10 μM cyclo(D-Asp-Pro-D-Val-Leu-D-Trp) (BQ-123; see, U.S. Patent No. 5,114,918 to Ishikawa et al.), 5 μM indomethacin, and saturated with a gas mixture of 95% $O_2$/5% $CO_2$. Changes in tension are measured isometrically and recorded using a Grass Polygraph coupled to a force transducer. Sarafotoxin 6c is added cumulatively to one strip while the second strip is preincubated for 15 min with a test compound prior to addition of cumulative doses of sarafotoxin 6c. The effects of the test compounds on the concentration-response curve for sarafotoxin 6c are examined.

### E. Deoxycorticosterone acetate (DOCA)-salt hypertensive rat model for assessing in vivo activity of selected compounds

Selected compounds disclosed herein have been tested for activity in the deoxycorticosterone acetate (DOCA)-salt hypertensive rat model. To perform these tests, silastic MDX4-4210 elastomer implants containing 47 mg (DOCA) were prepared according to the method of Ornmsbee et al. ((1973) the J. Pharm. Sci. 62:255-257). Briefly, DOCA is incorporated into silicon rubber implants for sustained release. To prepare the implants the DOCA is incorporated into unpolymerized silicone rubber, catalyst is added and the mixture is cast in a hemicylindrical shape.

Sprague Dawley rats (7-8 weeks old) were unilaterally nephrectomized under ketamine anesthesia and a DOCA-implant was placed on the left lateral dorsal abdomen of the animal. The rats were allowed to recover for three weeks. During recovery they were permitted free access to normal rat chow and 0.9% NaCl drinking solution in place of drinking water. The rats develop hypertension within 3 weeks.

All animals were used in the tests between 21 and 30 days post surgery. The mean arterial blood presure in these animals ranged from 165-200 mm Hg.

On the day of experimentation, catheters were inserted under brevital anesthesia into the right femoral artery for measurement of blood pressure, and into the right femoral vein for administration of a selected compound. The animals were placed in a restrainer and allowed to recover for a minimum of 60 min or until a steady mean arterial blood pressure was recorded. At that time, the selected compound or control vehicle was administered either intravenously, as a 60 minute infusion, or orally by oral gavage. Blood pressure was recorded continuously for a fruther 10 hrs.

### F. Effect of Intravenous administration on ET-1-induced pressor responses in conscious, autonomically blocked rats; a model for assessing in vivo activity of selected compounds

Male Sprague Dawley rats (250-450 g) were anesthetized (Brevital 50 mg/kg, IP) and cannulae were placed in the femoral artery to measure mean arterial pressure (MAP) and in the femoral vein for intravenous drug administration. Animals were placed in a restrainer and allowed to regain consciousness. Thirty minutes later autonomic blockade was administered (atropine methyl nitrate, 3 mg/kg, IV, followed by propranalol, 2 mg/kg, IV). An hour later animals received a bolus injection of vehicle (0.5 ml) followed thirty minutes later by intravenous bolus administration of ET-1 (Control, 1 μg/kg). Following recovery from this challenge, test -compounds were administered by intravenous bolus administration (0.5 ml) and then re-challenged with ET-1 thirty minutes later. Results are expressed as the percent inhibition of the ET-1-induced pressor response after administration of the test compound compared to the pressor response induced by the control ET-1 challenge. In some cases a third ET-1 challenge was administered ninety minutes after administration of the test compound.

### G. Results

### 1. In vitro

The $IC_{50}$ for each of the compounds of the preceding Examples for $ET_A$ and $ET_B$ receptors has been measured. Almost all of the compounds have an $IC_{50}$ of less than 10 μM for either or both of the $ET_A$ and $ET_B$ receptors. Many of the compounds have an $IC_{50}$ less than about 10 μM, others have an $IC_{50}$ less than about 1 μM and some of

the compounds have an $IC_{50}$ less than about 0.1 μM. A number of the compounds have an $IC_{50}$ for $ET_A$ receptors that is substantially less (10 to 100-fold or more) than for $ET_B$ receptors, and, thus are selective for $ET_A$ receptors. Others of the compounds are $ET_B$ selective.

**2. In vivo**

**a.** Selected compounds, such as N-(4-chloro-3-methyl-5-isoxazolyl)-2-(N-(4-methyl-phenyl)aminocarbonyl)thiophene-3-sulfonamide, N-(4-bromo-3-methyl-5-isoxazolyl)-2-[3,4-(methylenedioxy)-benzyl]benzo[b]thiophene-3-sulfonamide, N-(4-chloro-3-methyl-5-isoxazolyl)-2-(3,4,-methylenedioxy)benzyl)benzo[b]thiophene-3-sulfonamide, N-(4-chloro-3-methyl-5-isoxazolyl)-2-[β-hydroxy(3,4-methylenedioxy)phenylethyl]thiophene-3-sulfonamide, N-(4-chloro-3-methyl-5-isoxazolyl)-2-(3,4-methylenedioxybenzylcarbonyl)thiophene-3-sulfonamide, have been tested in the hypertensive rat model, and were effective in decreasing blood pressure.

**b.** Selected compounds, such as N-(4-chloro-3-methyl-5-isoxazolyl)-2-{[3,4-(methylenedioxy)phenyl)acetyl}thiophene-3-sulfonamide, N-(4-chloro-3-methyl-5-isoxazolyl)2-{[2-acetyl-4,5-(methylenedioxy)phenyl]aminocarbonyl}thiophene-3-sulfonamide, N-(4-chloro-3-methyl-5-isoxazolyl)-2-[(4-methoxy-2-methylphenyl)aminocarbonyl] thiophene-3-sulfonamide and N-(4-chloro-3-methyl-5-isoxazolyl)-2-[2-cyano-4,5-dimethoxyphenyl)aminocarbonyl]thiophene-3-sulfonamide, and -(4-chloro-3-methyl-5-isoxazolyl)-2-[2-methyl-4,5-(methylenedioxy)-phenylacetyl]thiophene-3-sulfonamide have been tested in the autonomically blocked, normotensive rat model and shown to have substantial activity, reducing pressure about 30% in 30 min at dosages as low as 30 mg/kg, and more than 50% at dosages of 60 mg/kg. On the average dosages of 30-60 mg/kg of the test compound resulted in a 40-60% inhibition of pressor response.

Since modifications will be apparent to those of skill in this art, it is intended that this invention be limited only by the scope of the appended claims.

## Claims

**1.** A compound that has formula I:

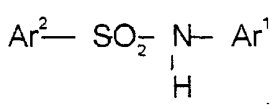

or a pharmaceutically acceptable salt, acid or ester thereof, wherein:

$Ar^1$ is:

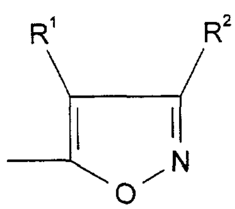

or

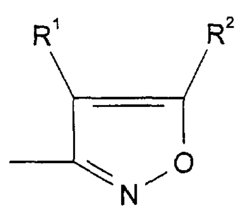

where $R^1$ is selected from halide, $CH_3$, $C_2H_5$, $CF_3$, $C_2F_5$, n-$C_3H_7$ and cyclo-$C_3H_7$, and $R^2$ is selected from H, $CH_3$, $C_2H_5$, $CF_3$, $C_2F_5$, n-$C_3H_7$ and cyclo-$C_3H_7$; and
$Ar^2$ has formula VI:

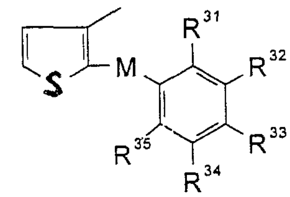

,

wherein:

M is selected from

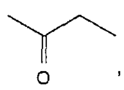 , 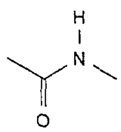 , 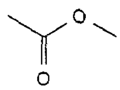 , 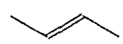 ,

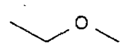 , 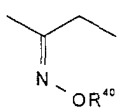 , 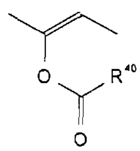 and 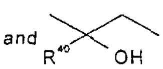

where $R^{40}$ is methyl, ethyl or hydrogen;

$R^{31}$, $R^{32}$, $R^{33}$, $R^{34}$ and $R^{35}$ are each independently selected from (i) or (ii) as follows:

(i) $R^{31}$, $R^{32}$ and $R^{34}$ are each independently selected from hydrogen, $C_{1-6}$alkyl, halo$C_{1-6}$alkyl, phenyl, $C_{1-6}$alkoxy, $C_{1-6}$alkylsulfonylamino$C_{1-6}$alkyl, cyano$C_{1-6}$alkyl, acetyl, $C_{1-6}$alkoxycarbonyl, cyano, OH, acetoxy$C_{1-6}$alkyl, hydroxy $C_{1-6}$allkyl, acetoxy $C_{1-6}$alkoxy or $C_{1-6}$alkoxycarbonyl; $R^{33}$ and $R^{35}$ are other than hydrogen and are selected from $C_{1-6}$alkyl and $C_{1-6}$ alkoxy; or
(ii) $R^{35}$ is $C_{1-6}$alkyl or $C_{1-6}$ alkoxy, and $R^{32}$ and $R^{33}$ or $R^{33}$ and $R^{34}$ form methylenedioxy or ethylenedioxy, and the others of $R^{31}$, $R^{32}$, $R^{33}$ and $R^{34}$ are selected as in (i), with the proviso that when M is $(CH_2)_mC(O)NH(CH_2)_r$, then at least two of $R^{31}$, $R^{32}$, $R^{33}$, $R^{34}$ and $R^{35}$ are not hydrogen.

**2.** A compound of claim 1 or a pharmaceutically acceptable salt, acid or ester thereof, wherein $Ar^2$ has formula VII:

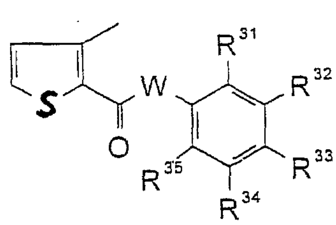

in which W is $CH_2$ or NH.

**3.** A compound of claim 1 or a pharmaceutically acceptable salt, acid or ester thereof, wherein M is selected from

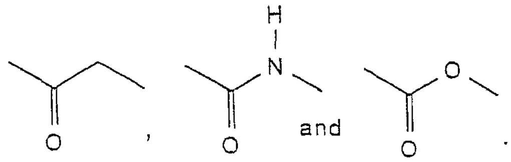

4. A compound or a pharmaceutically acceptable salt, acid, or ester thereof, that is selected from N-(4-bromo-3-methyl-5-isoxazolyl)-2-[3,4-(methylenedioxy)benzyl]benzo[b]thiophene-3-sulfonamide; N-(4-bromo-3 -methyl-5-isoxazolyl)-2-(3,4,5-trimethoxybenzyl)-benzo[b]-thiophene-3-sulfonamide; N-(4-chloro-3-methyl-5-isoxazolyl)-2-[(3,4-methylenedioxy)benzyl]benzo[b]thiophene-3-sulfonamide; N-(4-bromo-3-methyl-5-isoxazolyl)-2-(3,4-dimethoxybenzyl)-benzo[b]thiophene-3-sulfonamide;N-(4-chloro-3-methyl-5-isoxazolyl)-6-methoxy-2-[3,4-(methylenedioxy)benzyl]benzo[b]thiophene-3-sulfonamide; and N-(4-chloro-5-methyl-3-isoxazolyl)-2-[3,4-(methylenedioxy) benzyl]benzo[b]thiophene-3-sulfonamide.

5. A compound of any of claims 1-3, or a pharmaceutically acceptable salt, acid, or ester thereof, wherein R' is halide or $CH_3$, and $R^2$ is H, $CH_3$, $C_2H_5$, or $CF_3$.

6. A compound of any of claims 1-3 and 5 or a pharmaceutically acceptable salt, acid, or ester thereof that is a (phenylacetyl)thiophenesulfonamide.

7. A compound of any of claims 1-3 and 5 or a pharmaceutically acceptable salt, acid, or ester thereof that is a (phenyoxy)thiophenesulfonamide.

8. A compound of claim 1 or a pharmaceutically acceptable salt, acid, or ester thereof that is selected from N-(4-bromo-3-methyl-5-isoxazolyl)-2-[(3,4-methylenedioxy)phenoxycarbonyl]thiophene-3-sulfonamide, N-(4-chloro-3-methyl-5-isoxazolyl)-2-[3,4-(methylenedioxy)phenoxycarbonyl]thiophene-3-sulfonamide, N-(4-bromo-3-methyl-5-isoxazolyl)-2-[(3,4-methylenedioxy)phenylacetyl]thiophene-3-sulfonamide, N-(4-chloro-3-methyl-5-isoxazolyl)-{2-[3,4-(methylenedioxy)-phenyl]acetyl}thiophene-3-sulfonamide oxime, N-(4-chloro-3-methyl-5-isoxazolyl)-2-[{2-cyano-4,5-dimethoxyphenyl)aminocarbonyl]thiophene-3-sulfonamide,N-(4-chloro-3-methyl-5-isoxazolyl)-2-[2-hydroxy-4-methylphenyl)aminocarbonyl]thiophene-3-sulfonamide, N-(4-chloro-3-methyl-5-isoxazolyl)-2-{[2-cyano-4,5-(methylenedioxy)phenyl]aminocarbonyl}thiophene-3-sulfonamide, N-(4-chloro-3-methyl-5-isoxazolyl)-2-[2-methyl-4,5-(methylenedioxy)phenylaminocarbonyl]-thiophene-3-sulfonamide, N-(3,4-dimethyl-5-isoxazolyl)-2-(2,4-dimethylphenylacetyl)thiophene-3-sulfonamide, N-(4-chloro-3-methyl-5-isoxazolyl)-2-(2,4-dimethylphenylacetyl)thiophene-3-sulfonamide, N-(4-bromo-3-methyl-5-isoxazolyl)-2-(2,4-dimethylphenylacetyl)thiophene-3-sulfonamide, N-(4-chloro-3-methyl-5-isoxazolyl)-2-[3,4-(methylenedioxy)]phenylaminocarbonyl-3-thiophenesulfonamide, N-(4-chloro-3-methyl-5-isoxazolyl)-2-[2-methyl-4,5-(methylenedioxy)phenylacetyl]thiophene-3-sulfonamide, N-(4-chloro-3-methyl-5-isoxazolyl)-2-[3,4-(methylenedioxy)-6-(2-acetoxyethyl)phenylaminocarbonyl]thiophene-3-sulfonamide, N-(4-chloro-3-methyl-5-isoxazolyl)-2-[3,4-(methylenedioxy)-6-(2-hydroxyethyl)-phenylaminocarbonyl]thiophene-3-sulfonamide, N-(4-chloro-3-methyl-5-isoxazolyl)-2-(2,5-dimethylphenylacetyl)thiophene-3-sulfonamide, N-(4-chloro-3-methyl-5-isoxazolyl)-2-[2-methanesulfonylaminomethyl)-4,5-(methylenedioxy)phenylaminocarbonyl]thiophene-3-sulfonamide, N-(4-chloro-3-methyl-5-isoxazolyl)-2-[2-cyanomethyl-4,5-(methylenedioxy)-6-cyanomethyl]phenylaminocarbonyl-3-thiophenesulfonamide, N-(4-chloro-3-methyl-5-isoxazolyl)-2-[2-hyroxyproyl-4,5-(methylenedioxy)phenylaminocarbonyl]thiophene-3-sulfonamide, N-(4-chloro-3-methyl-5-isoxazolyl)-2-{2-[(dimethylamino)carbonylmethyl]-4,5-(methylenedioxy)-phenylaminocarbonyl}thiophene-3-sulfonamide, N-(4-chloro-3-methyl-5-isoxazolyl)-2-[β-acetoxy-2-methyl-4,5-(methylenedioxy)styryl]thiophene-3-sulfonamide, N-(4-chloro-3-methyl-5-isoxazolyl)-2-[(2,3,4-trimethoxy-6-cyano)phenylaminocarbonyl]thiophene-3-sulfonamide, N-(3,4-dimethyl-5-isoxazolyl)-2-[2-methyl-4,5-(methylenedioxy)phenylacetyl]thiophene-3-sulfonamide, N-(4-chloro-3-methyl-5-isoxazolyl)-2-(2,4,6-trimethylphenylaminocarbonyl)thiophene-3-sulfonamide, N-(4-chloro-3-methyl-5-isoxazolyl)-2-(2,4,6-trimethylphenylacetyl)thiophene-3-sulfonamide, N-(4-chloro-5-methyl-3-isoxazolyl)-2-[2-methyl-4,5-(methylenedioxy)phenylacetyl]thiophene-3-sulfonamide, N-(4-bromo-3-methyl-5-isoxazolyl)-2-[2-methyl-4,5-(methylenedioxy)cinnamyl]thiophene-3-sulfonamide, N-(4-bromo-3-methyl-5-isoxazolyl)-2-(2,4-dimethylphenethyl)-thiophene-3-sulfonamide and N-(4-chloro-3-methyl-5-isoxazolyl)-2-[(4-methoxycarbonyl-2,6-dimethyl)phenylaminocarbonyl]thiophene-3-sulfonamide or any corresponding N-(4-halo-3-methyl-5-isoxazolyl), N-(4-halo-5-methyl-3-isoxazolyl), N-(3,4-dimethyl-5-isoxazolyl), N-(4-halo-5-methyl-3-isoxazolyl), N-(4-halo-3-methyl-5-isoxazolyl), N-(4,5-dimethyl-3-isoxazolyl) derivative of any of these compounds.

**9.** A compound of claim 1 or a pharmaceutically acceptable salt, acid, or ester thereof selected from N-(4-chloro-3-methyl-5-isoxazolyl)-2-(2,3,4-trimethoxy-6-methylphenylaminocarbonyl)thiophene-3-sulfonamide, N-(4-chloro-3-methyl-5-isoxazolyl)-2-(2,3,4-trimethoxy-6-acetylphenylaminocarbonyl)thiophene-3-sulfonamide, N-(4-chloro-3-methyl-5-isoxazolyl)-2-(2,3,4-trimethoxy-6-methoxycarbonylphenylaminocarbonyl)thiophene-3-sulfonamide, N-(4-chloro-3-methyl-5-isoxazolyl)-2-(2,3,4-trimethoxy-6-carboxylphenylaminocarbonyl)thiophene-3-sulfonamide, N-(4-chloro-3-methyl-5-isoxazolyl)-2-(2,3,4-trimethoxy-6-methanesulfonylphenylaminocarbonyl)thiophene-3-sulfonamide, N-(4-chloro-3-methyl-5-isoxazolyl)-2-[2,3,4-trimethoxy-6-(cyanomethyl)phenylaminocarbonyl]thiophene-3-sulfonamide, N-(4-chloro-3-methyl-5-isoxazolyl)-2-[2,3,4-trimethoxy-6-(2-hydroxyethyl)phenylaminocarbonyl]thiophene-3-sulfonamide, N-(4-chloro-3-methyl-5-isoxazolyl)-2-[3,4-(methylenedioxy)-2-methoxy-6-methylphenylaminocarbonyl]thiophene-3-sulfonamide, N-(4-chloro-3-methyl-5-isoxazolyl)-2-[3,4-(methylenedioxy)-2-methoxy-6-acetylphenylaminocarbonyl]thiophene-3-sulfonamide, N-(4-chloro-3-methyl-5-isoxazolyl)-2-[3,4-(methylenedioxy)-2-methoxy-6-methoxycarbonylphenylaminocarbonyl]thiophene-3-sulfonamide, N-(4-chloro-3-methyl-5-isoxazolyl)-2-[3,4-(methylenedioxy)-2-methoxy-6-carboxylphenylaminocarbonyl]thiophene-3-sulfonamide, N-(4-chloro-3-methyl-5-isoxazolyl)-2-[3,4-(methylenedioxy)-2-methoxy-6-methanesulfonylphenylaminocarbonyl]thiophene-3-sulfonamide, N-(4-chloro-3-methyl-5-isoxazolyl)-2-[3,4-(methylenedioxy)-2-methoxy-6-cyanophenylaminocarbonyl]thiophene-3-sulfonamide, N-(4-chloro-3-methyl-5-isoxazolyl)-2-[3,4-(methylenedioxy)-2-methoxy-6-cyanomethylphenylaminocarbonyl]thiophene-3-sulfonamide, N-(4-chloro-3-methyl-5-isoxazolyl)-2-[3,4-(methylenedioxy)-2-methoxy-6-(2-hydroxyethyl)phenylaminocarbonyl]thiophene-3-sulfonamide, N-(4-chloro-3-methyl-5-isoxazolyl)-2-[3,4-(methylenedioxy)-2,6-dimethylphenylaminocarbonyl]thiophene-3-sulfonamide, N-(4-chloro-3-methyl-5-isoxazolyl)-2-[3,4-(methylenedioxy)-6-acetyl-2-methylphenylaminocarbonyl]thiophene-3-sulfonamide, N-(4-chloro-3-methyl-5-isoxazolyl)-2-[3,4-(methylenedioxy)-6-methoxycarbonyl-2-methylphenylaminocarbonyl]thiophene-3-sulfonamide, N-(4-chloro-3-methyl-5-isoxazolyl)-2-[3,4-(methylenedioxy)-6-carboxyl-2-methylphenylaminocarbonyl]thiophene-3-sulfonamide, N-(4-chloro-3-methyl-5-isoxazolyl)-2-[3,4-(methylenedioxy)-6-methoxy-2-methylphenylaminocarbonyl]thiophene-3-sulfonamide, N-(4-chloro-3-methyl-5-isoxazolyl)-2-[3,4-(methylenedioxy)-6-methanesulfonyl-2-methylphenylaminocarbonyl]thiophene-3-sulfonamide, N-(4-chloro-3 -methyl-5-isoxazolyl)-2-[3,4-(methylenedioxy)-6-cyano-2-methylphenylaminocarbonyl]thiophene-3-sulfonamide, N-(4-chloro-3-methyl-5-isoxazolyl)-2-[3,4-(methylenedioxy)-6-(cyanomethyl)-2-methylphenylaminocarbonyl]thiophene-3-sulfonamide, N-(4-chloro-3-methyl-5-isoxazolyl)-2-[3,4-(methylenedioxy)-6-(2-hydroxyethyl)-2-methylphenylaminocarbonyl]thiophene-3-sulfonamide, N-(4-chloro-3-methyl-5-isoxazolyl)-2-[3,4-(methylenedioxy)-2-cyano-6-methylphenylaminocarbonyl]thiophene-3-sulfonamide, N-(4-chloro-3-methyl-5-isoxazolyl)-2-[3,4-(methylenedioxy)-6-methoxy-2-cyanophenylaminocarbonyl]thiophene-3-sulfonamide, N-(4-chloro-3-methyl-5-isoxazolyl)-2-[3,4-(methylenedioxy)-2-acetyl-6-methylphenylaminocarbonyl]thiophene-3-sulfonamide, N-(4-chloro-3-methyl-5-isoxazolyl)-2-[3,4-(methylenedioxy)-6-methoxy-2-acetylphenylaminocarbonyl]thiophene-3-sulfonamide, N-(4-chloro-3-methyl-5-isoxazolyl)-2-(3-cyano-2,4,6-trimethylphenylaminocarbonyl]thiophene-3-sulfonamide, N-(4-chloro-3-methyl-5-isoxazolyl)-2-(3-carboxyl-2,4,6-trimethylphenylaminocarbonyl]thiophene-3-sulfonamide, N-(4-chloro-3-methyl-5-isoxazolyl)-2-(3-hydroxymethyl-2,4,6-trimethylphenylaminocarbonyl]thiophene-3-sulfonamide, N-(4-chloro-3-methyl-5-isoxazolyl)-2-(3-methanesulfonyl-2,4,6-trimethylphenylaminocarbonyl]thiophene-3-sulfonamide, N-(4-chloro-3-methyl-5-isoxazolyl)-2-(3-cyanomethyl-2,4,6-trimethylphenylaminocarbonyl]thiophene-3-sulfonamide, N-(4-chloro-3-methyl-5-isoxazolyl)-2-[3-(2-hydroxyethyl)-2,4,6-trimethylphenylaminocarbonyl]thiophene-3-sulfonamide, N-(4-chloro-3-methyl-5-isoxazolyl)-2-[3-(carboxylmethyl)-2,4,6-trimethylphenylaminocarbonyl]thiophene-3-sulfonamide, N-(4-chloro-3-methyl-5-isoxazolyl)-2-(4-cyano-2,6-dimethylphenylaminocarbonyl)thiophene-3-sulfonamide, N-(4-chloro-3-methyl-5-isoxazolyl)-2-(4-carboxyl-2,6-dimethylphenylaminocarbonyl)thiophene-3-sulfonamide, N-(4-chloro-3-methyl-5-isoxazolyl)-2-[4-(hydroxymethyl)-2,6-dimethylphenylaminocarbonyl]thiophene-3-sulfonamide, N-(4-chloro-3-methyl-5-isoxazolyl)-2-[4-(2-hydroxyethyl)-2,6-dimethylphenylaminocarbonyl]thiophene-3-sulfonamide, N-(4-chloro-3-methyl-5-isoxazolyl)-2-[4-(cyanomethyl)-2,6-dimethylphenylaminocarbonyl]thiophene-3-sulfonamide, N-(4-chloro-3-methyl-5-isoxazolyl)-2-[4-(carboxylmethyl)-2,6-dimethylphenylaminocarbonyl]thiophene-3-sulfonamide, N-(4-chloro-3-methyl-5-isoxazolyl)-2-(4-methanesulfonyl-2,6-dimethylphenylaminocarbonyl)thiophene-3-sulfonamide, N-(4-chloro-3-methyl-5-isoxazolyl)-2-(2,3,4-trimethoxy-6-methylphenylacetyl)thiophene-3-sulfonamide, N-(4-chloro-3-methyl-5-isoxazolyl)-2-(2,3,4-trimethoxy-6-acetylphenylacetyl)thiophene-3-sulfonamide, N-(4-chloro-3-methyl-5-isoxazolyl)-2-(2,3,4-trimethoxy-6-methoxycarbonylphenylacetyl)thiophene-3-sulfonamide, N-(4-chloro-3-methyl-5-isoxazolyl)-2-(2,3,4-trimethoxy-6-carboxylphenylacetyl)thiophene-3-sulfonamide, N-(4-chloro-3-methyl-5-isoxazolyl)-2-(2,3,4-trimethoxy-6-methanesulfonylphenylacetyl)thiophene-3-sulfonamide, N-(4-chloro-3 -methyl-5-isoxazolyl)-2-[2,3,4-trimethoxy-6-(cyanomethyl)phenylacetyl]thiophene-3-sulfonamide, N-(4-chloro-3-methyl-5-isoxazolyl)-2-[2,3,4-trimethoxy-6-(2-hydroxyethyl)phenylacetyl]thiophene-3-sulfonamide, N-(4-chloro-3-methyl-5-isoxazolyl)-2-[3,4-(methylenedioxy)-2-methoxy-6-methylphenylacetyl]thiophene-3-sulfonamide, N-(4-chloro-3-methyl-5-isoxazolyl)-2-[3,4-(methylenedioxy)-2-methoxy-6-acetylphenylacetyl]thiophene-3-sulfonamide, N-(4-chloro-3-methyl-5-isoxazolyl)-2-[3,4-(methylenedioxy)-2-methoxy-6-methoxycarbonylphenylacetyl]thiophene-3-sulfonamide, N-(4-chloro-3-methyl-5-isoxazolyl)-2-[3,4-(methylenedioxy)-2-methoxy-6-carboxylphenylacetyl]thiophene-3-sulfonamide, N-(4-chloro-3-methyl-5-isoxazolyl)-2-[3,4-(methylenedioxy-2-methoxy-6-methanesulfonyl)phenylacetyl]thiophene-3-sulfonamide, N-(4-chloro-3-methyl-5-isoxazolyl)-2-[3,4-(methylenedioxy)-2-methoxy-6-(cyano)phenylacetyl]thiophene-3-sulfonamide, N-(4-chloro-3-methyl-5-isoxazolyl)-2-[3,4-(methylenedioxy)-2-methoxy-6-(cyanomethylphenylacetyl]thiophene-3-sulfonamide, N-(4-chloro-3-methyl-5-isoxazolyl)-2-[3,4-(methylenedioxy)-2-methoxy-6-(2-hydroxyethyl)phenylacetyl]thiophene-3-sulfonamide, N-(4-chloro-3-methyl-5-isoxazolyl)-2-[3,4-(methylenedioxy)-2,6-dimethylphenylacetyl]thiophene-3-sulfonamide, N-(4-chloro-3-methyl-5-isoxazolyl)-2-[3,4-(methylenedioxy)-6-acetyl-2-methylphenylacetyl]thiophene-3-sulfonamide, N-(4-chloro-3-methyl-5-isoxazolyl)-2-[3,4-(methylenedioxy)-6-methoxycarbonyl-2-methylphenylacetyl]thiophene-3-sulfonamide, N-(4-chloro-3-methyl-5-isoxazolyl)-2-[3,4-(methylenedioxy)-6-carboxyl-2-methylphenylacetyl]thiophene-3-sulfonamide, N-(4-chloro-3-methyl-5-isoxazolyl)-2-[3,4-(methylenedioxy)-6-methoxy-2-methylphenylacetyl]thiophene-3-sulfonamide, N-(4-chloro-3-methyl-5-isoxazolyl)-2-[3,4-(methylenedioxy)-6-methanesulfonyl-2-methylphenylacetyl]thiophene-3-sulfonamide, N-(4-chloro-3-methyl-5-isoxazolyl)-2-[3,4-(methylenedioxy)-6-cyano-2-methylphenylacetyl]thiophene-3-sulfonamide, N-(4-chloro-3-methyl-5-isoxazolyl)-2-[3,4-(methylenedioxy)-6-(cyanomethyl)-2-methylphenylacetyl]thiophene-3-sulfonamide, N-(4-chloro-3-methyl-5-isoxazolyl)-2-[3,4-(methylenedioxy)-6-(2-hydroxyethyl)-2-methylphenylacetyl]thiophene-3-sulfonamide, N-(4-chloro-3-methyl-5-isoxazolyl)-2-[3,4-(methylenedioxy)-2-cyano-6-methylphenylacetyl]thiophene-3-sulfonamide, N-(4-chloro-3-methyl-5-isoxazolyl)-2-[3,4-(methylenedioxy)-6-methoxy-2-cyanophenylacetyl]thiophene-3-sulfonamide, N-(4-chloro-3-methyl-5-isoxazolyl)-2-[3,4-(methylenedioxy)-2-acetyl-6-methylphenylacetyl]thiophene-3-sulfonamide, N-(4-chloro-3-methyl-5-isoxazolyl)-2-[3,4-(methylenedioxy)-6-methoxy-2-acetylphenylacetyl]thiophene-3-sulfonamide, N-(4-chloro-3-methyl-5-isoxazolyl)-2-(3-cyano-2,4,6-trimethylphenylacetyl)thiophene-3-sulfonamide, N-(4-chloro-3-methyl-5-isoxazolyl)-2-(3-carboxyl-2,4,6-trimethylphenylacetyl)thiophene-3-sulfonamide, N-(4-chloro-3-methyl-5-isoxazolyl)-2-(3-hydroxymethyl-2,4,6-trimethylphenylacetyl)thiophene-3-sulfonamide, N-(4-chloro-3-methyl-5-isoxazolyl)-2-(3-methanesulfonyl-2,4,6-trimethylphenylacetyl)thiophene-3-sulfonamide, N-(4-chloro-3-methyl-5-isoxazolyl)-2-[3-(cyanomethyl)-2,4,6-trimethylphenylacetyl]thiophene-3-sulfonamide, N-(4-chloro-3-methyl-5-isoxazolyl)-2-[3-(2-hydroxyethyl)-2,4,6-trimethylphenylacetyl]thiophene-3-sulfonamide, N-(4-chloro-3-methyl-5-isoxazolyl)-2-[3-(carboxylmethyl)-2,4,6-trimethylphenylacetyl]thiophene-3-sulfonamide, N-(4-chloro-3-methyl-5-isoxazolyl)-2-(4-cyano-2,6-dimethylphenylacetyl)thiophene-3-sulfonamide, N-(4-chloro-3-methyl-5-isoxazolyl)-2-(4-carboxyl-2,6-dimethylphenylacetyl)thiophene-3-sulfonamide, N-(4-chloro-3-methyl-5-isoxazolyl)-2-(4-hydroxymethyl-2,6-dimethylphenylacetyl)thiophene-3-sulfonamide, N-(4-chloro-3-methyl-5-isoxazolyl)-2-[4-(2-hydroxyethyl)-2,6-(dimethyl) phenylacetyl]thiophene-3-sulfonamide, N-(4-chloro-3-methyl-5-isoxazolyl)-2-[4-cyanomethyl-2,6-(dimethyl)phenylacetyl]thiophene-3-sulfonamide, N-(4-chloro-3-methyl-5-isoxazolyl)-2-[4-(carboxylmethyl)-2,6-dimethylphenylacetyl]thiophene-3-sulfonamide, and N-(4-chloro-3-methyl-5-isoxazolyl)-2-(4-methanesulfonyl-2,6-dimethylphenylacetyl)thiophene-3-sulfonamide or any corresponding N-(4-halo-3-methyl-5-isoxazolyl), N-(4-halo-5-methyl-3-isoxazolyl), N-(3,4-dimethyl-5-isoxazolyl), N-(4-halo-5-methyl-3-isoxazolyl), N-(4-halo-3-methyl-5-isoxazolyl), N-(4,5-dimethyl-3-isoxazolyl) derivative of any of these compounds.

**10.** A compound of claim 1 or a pharmaceutically acceptable salt, acid, or ester thereof selected from N-(4-bromo-3-methyl-5-isoxazolyl)-2-[3,4-(methylenedioxy)benzyl]benzo[b]thiophene-3-sulfonamide, N-(4-chloro-3-methyl-5-isoxazolyl)-2-(3,4,-methylenedioxy)benzyl)benzo[b]thiophene-3-sulfonamide, N-(4-chloro-3-methyl-5-isoxazolyl)-2-[β-hydroxy(3,4-methylenedioxy)phenylethyl]-thiophene-3-sulfonamide and N-(4-chloro-3-methyl-5-isoxazolyl)-2-(3,4-methylenedioxybenzylcarbonyl)thiophene-3-sulfonamide or any corresponding N-(4-halo-3-methyl-5-isoxazolyl), N-(4-halo-5-methyl-3-isoxazolyl), N-(3,4-dimethyl-5-isoxazolyl), N-(4-halo-3-methyl-5-isoxazolyl), N-(4,5-dimethyl-3-isoxazolyl) derivative thereof.

**11.** The compound of claim 1 or a pharmaceutically acceptable salt, acid, or ester thereof that is N-(4-chloro-3-methyl-5-isoxazolyl)-2-[2-hydroxy-4-methylphenyl)aminocarbonyl]thiophene-3-sulfonamide.

**12.** The compound of claim 1 or a pharmaceutically acceptable salt, acid, or ester thereof that is N-(4-chloro-3-methyl-5-isoxazolyl)-2-{[2-cyano-4,5-(methylenedioxy)phenyl]aminocarbonyl}thiophene-3-sulfonamide.

**13.** The compound of claim 1 or a pharmaceutically acceptable salt, acid, or ester thereof that is N-(4-chloro-3-methyl-5-isoxazolyl)-2-[2-methyl-4,5-(methylenedioxy)phenylaminocarbonyl]thiophene-3-sulfonamide.

**14.** The compound of claim 1 or a pharmaceutically acceptable salt, acid, or ester thereof that is N-(4-chloro-3-methyl-5-isoxazolyl)-2-(2-carboxamido-4,5-dimethoxyphenylaminocarbonyl)thiophene-3-sulfonamide.

**15.** The compound of claim 1 or a pharmaceutically acceptable salt, acid, or ester thereofthat is N-(3,4-dimethyl-5-isoxazolyl)-2-(2,4-dimethylphenylacetyl)thiophene-3-sulfonamide.

**16.** The compound of claim 1 or a pharmaceutically acceptable salt, acid, or ester thereof that is N-(4-chloro-3-methyl-5-isoxazolyl)-2-(2,4-dimethylphenylacetyl)thiophene-3-sulfonamide.

**17.** The compound of claim 1 or a pharmaceutically acceptable salt, acid, or ester thereof that is N-(4-bromo-3-methyl-5-isoxazolyl)-2-(2,4-dimethylphenylacetyl)thiophene-3-sulfonamide.

**18.** The compound of claim 1 or a pharmaceutically acceptable salt, acid, or ester thereof that is N-(4-chloro-3-methyl-5-isoxazolyl)-2-[3,4-(methylenedioxy)]-phenylaminocarbonyl-3-thiophenesulfonamide.

**19.** The compound of claim 1 or a pharmaceutically acceptable salt, acid, or ester thereof that is N-(4-chloro-3-methyl-5-isoxazolyl)-2-[2-methyl-4,5-(methylenedioxy)phenylacetyl]thiophene-3-sulfonamide.

**20.** The compound of claim 1 or a pharmaceutically acceptable salt, acid, or ester thereof that is N-(4-chloro-3-methyl-5-isoxazolyl)-2-[3,4-(methylenedioxy)-6-(2-acetoxyethyl)phenylaminocarbonyl]thiophene-3-sulfonamide.

**21.** The compound of claim 1 or a pharmaceutically acceptable salt, acid, or ester thereof that is N-(4-chloro-3-methyl-5-isoxazolyl)-2-[3,4-(methylenedioxy)-6-(2-hydroxyethyl)phenylaminocarbonyl]thiophene-3-sulfonamide.

**22.** The compound of claim 1 or a pharmaceutically acceptable salt, acid, or ester thereof that is N-(4-chloro-3-methyl-5-isoxazolyl)-2-(2,5-dimethylphenylacetyl)thiophene-3-sulfonamide.

**23.** The compound of claim 1 or a pharmaceutically acceptable salt, acid, or ester thereof that is N-(4-chloro-3-methyl-5-isoxazolyl)-2-[2-methanesulfonylaminomethyl)-4,5-(methylenedioxy)phenylaminocarbonyl]thiophene-3-sulfonamide.

**24.** The compound of claim 1 or a pharmaceutically acceptable salt, acid, or ester thereof that is N-(4-chloro-3-methyl-5-isoxazolyl)-2-[2-cyanomethyl-4,5-(methylenedioxy)-6-cyanomethyl]phenylaminocarbonyl-3-thiophenesulfonamide.

**25.** The compound of claim 1 or a pharmaceutically acceptable salt, acid, or ester thereof that is N-(4-bromo-3-methyl-5-isoxazolyl)-3-[2-methyl-4,5-(methylenedioxy)cinnamyl]thiophene-2-sulfonamide.

**26.** The compound of claim 1 or a pharmaceutically acceptable salt, acid, or ester thereof that is N-(4-chloro-3-methyl-5-isoxazolyl)-2-{2-[(dimethylamino)-carbonylmethyl]-4,5-(methylenedioxy)phenylaminocarbonyl}thiophene-3-sulfonamide.

**27.** The compound of claim 1 or a pharmaceutically acceptable salt, acid, or ester thereof that is N-(4-chloro-3-methyl-5-isoxazolyl)-2-[β-acetoxy-2-methyl-4,5-(methylenedioxy)styryl]thiophene-3-sulfonamide.

**28.** The compound of claim 1 or a pharmaceutically acceptable salt, acid, or ester thereof that is N-(4-chloro-3-methyl-5-isoxazolyl)-2-[(2,3,4-trimethoxy-6-cyano)phenylaminocarbonyl]thiophene-3-sulfonamide.

**29.** The compound of claim 1 or a pharmaceutically acceptable salt, acid, or ester thereof that is N-(4-chloro-3-methyl-5-isoxazolyl)-2-(2,4,6-trimethylphenylaminocarbonyl)thiophene-3-sulfonamide.

**30.** The compound of claim 1 or a pharmaceutically acceptable salt, acid, or ester thereof that is N-(4-chloro-3-methyl-5-isoxazolyl)-2-(3-methoxycarbonyl-2,4,6-trimethylphenylaminocarbonyl)thiophene-3-sulfonamide.

**31.** The compound of claim 1 or a pharmaceutically acceptable salt, acid, or ester thereof that is N-(4-chloro-3-methyl-5-isoxazolyl)-2-(2,4,6-trimethylphenylacetyl)thiophene-3-sulfonamide.

**32.** The compound of claim 1 or a pharmaceutically acceptable salt, acid, or ester thereof that is N-(4-chloro-5-methyl-3-isoxazolyl)-2-[2-methyl-4,5-(methylenedioxy)phenylacetyl]thiophene-3-sulfonamide.

**33.** The compound of claim 1 or a pharmaceutically acceptable salt, acid, or ester thereof that is N-(4-chloro-3-methyl-5-isoxazolyl)-2-[(4-methoxycarbonyl-2,6-dimethyl)phenylaminocarbonyl]thiophene-3-sulfonamide.

**34.** A pharmaceutical composition, comprising a compound of any of claims 1 to 33 or a pharmaceutically acceptable salt, acid, or ester thereof together with a pharmaceutically acceptable carrier.

**35.** A pharmaceutical composition, comprising the compound of claim 19, or a pharmaceutically acceptable salt, acid, or ester thereof, together with a pharmaceutically acceptable carrier.

**36.** A compound of any of claims 1 to 33, or a pharmaceutically acceptable salt, acid, or ester thereof, for use in the treatment of an endothelin-mediated disorder.

**37.** The compound of claim 19, or a pharmaceutically acceptable salt, acid, or ester thereof, for use in the treatment of an endothelin-mediated disorder.

**38.** Use of one or more compounds of any of claims 1 to 33, or a pharmaceutically acceptable salt, acid, or ester thereof, for the preparation of a medicament for the treatment of an endothelin-mediated disorder.

**39.** Use of the compound of claim 19, or a pharmaceutically acceptable salt, acid, or ester thereof, for the preparation of a medicament for the treatment of an endothelin-mediated disorder.

**40.** The use of claim 38 or claim 39, wherein the disorder is selected from hypertension, cardiovascular disease, asthma, pulmonary hypertension, inflammatory diseases, ophthalmologic disease, menstrual disorders, obstetric conditions, wounds, gastroenteric disease, renal failure, immunosuppressant-mediated renal vasoconstriction, erythropoietin-mediated vasoconstriction, endotoxin shock, anaphylactic shock and hemorrhagic shock.

**41.** The use of claim 40, wherein the disorder is selected from hypertension, cardiovascular disease, pulmonary hypertension, erythropoietin-mediated vasoconstriction, endotoxin shock, anaphylactic shock and hemorrhagic shock.

**42.** The use of claim 40, wherein the disorder is selected from asthma and inflammatory diseases.

**43.** Use of one or more compounds of any of claims 1 to 33 or a pharmaceutically acceptable salt, ester or acid thereof in the preparation of a medicament for inhibiting the binding of an endothelin peptide to an endothelin$_A$ ($ET_A$) or endothelin$_B$ ($ET_B$) receptor.

**44.** Use of the compound of claim 19 or a pharmaceutically acceptable salt, ester or acid thereof in the preparation of a medicament for inhibiting the binding of an endothelin peptide to an endothelin$_A$ ($ET_A$) or endothelin$_B$ ($ET_B$) receptor.

**45.** Use of one or more compounds of any of claims 1 to 33, or a pharmaceutically acceptable salt, ester or acid thereof, in the preparation of a medicament for altering endothelin receptor-mediated activity.

**46.** Use of the compound of claim 19, or a pharmaceutically acceptable salt, ester or acid thereof, in the preparation of a medicament for altering endothelin receptor-mediated activity.

**47.** A pharmaceutical composition formulated for single dosage administration, comprising an effective amount of one or more compounds of any of claims 1 to 33 or a pharmaceutically acceptable salt, ester or acid thereof, wherein the amount is effective for ameliorating the symptoms of an endothelin-mediated disease.

**48.** A pharmaceutical composition formulated for single dosage administration, comprising an effective amount of the compound of claim 19 or a pharmaceutically acceptable salt, ester or acid thereof, wherein the amount is effective for ameliorating the symptoms of an endothelin-mediated disease.

**49.** An article of manufacture, comprising packaging material and one or more compounds of any of claims 1 to 33 or a pharmaceutically acceptable salt, ester or acid thereof contained within the packaging material, wherein the compound is effective for antagonizing the effects of endothelin, ameliorating the symptoms of an endothelin-mediated disorder, or inhibiting the binding of an endothelin peptide to an ET receptor with an $IC_{50}$ of less than about 10 µM, and the packaging material includes a label that indicates that the sulfonamide or salt thereof is used for antagonizing the effects of endothelin, inhibiting the binding of endothelin to an endothelin receptor or treating an endothelin-mediated disorder.

**50.** An article of manufacture, comprising packaging material and a compound of claim 19 or a pharmaceutically acceptable salt, ester or acid thereof contained within the packaging material, wherein the compound is effective for antagonizing the effects of endothelin, ameliorating the symptoms of an endothelin-mediated disorder, or inhibiting the binding of an endothelin peptide to an ET receptor with an $IC_{50}$ of less than about 10 μM, and the packaging material includes a label that indicates that the sulfonamide or salt thereof is used for antagonizing the effects of endothelin, inhibiting the binding of endothelin to an endothelin receptor or treating an endothelin-mediated disorder.

**51.** The use of claim 38 or claim 39, wherein the disorder is glaucoma.

**Patentansprüche**

**1.** Verbindung mit der Formel I:

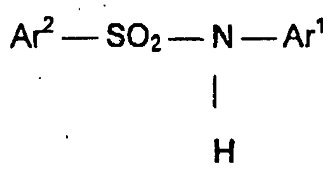

oder ein pharmazeutisch geeignetes Salz, Säure oder Ester davon, wobei:

$Ar^1$ ist:

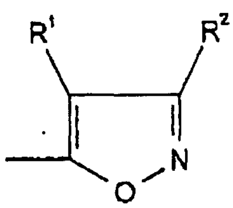

oder

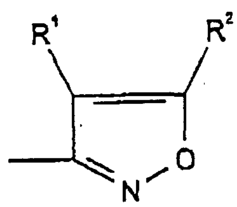

worin $R^1$ gewählt ist aus Halid, $CH_3$, $C_2H_5$, $CF_3$, $C_2F_5$, n-$C_3H_7$ und Cyclo-$C_3H_7$, und $R^2$ gewählt ist aus H, $CH_3$, $C_2H_5$, $CF_3$, $C_2F_5$, n-$C_3H_7$ und Cyclo-$C_3H_7$; und

$Ar^2$ die Formel VI aufweist:

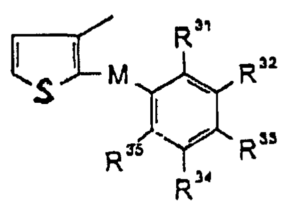

worin:

M gewählt ist aus

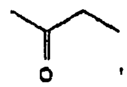, 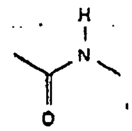, 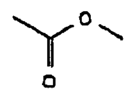, 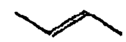,

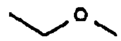, 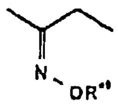, 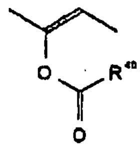 und 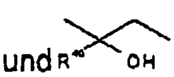

worin $R^{40}$ Methyl, Ethyl oder Wasserstoff ist;
$R^{31}$, $R^{32}$, $R^{33}$, $R^{34}$ und $R^{35}$ jeweils unabhängig voneinander gewählt sind aus (i) oder (ii) wie folgt:

(i) $R^{31}$, $R^{32}$ und $R^{34}$ jeweils unabhängig voneinander gewählt sind aus Wasserstoff, $C_{1-6}$-Alkyl, Halo-$C_{1-6}$-alkyl, Phenyl, $C_{1-6}$-Alkoxy, $C_{1-6}$-Alkylsulfonylamino-$C_{1-6}$-alkyl, Cyano-$C_{1-6}$-alkyl, Acetyl, $C_{1-6}$Alkoxycarbonyl, Cyano, OH, Acetoxy-$C_{1-6}$-alkyl, Hydroxy-$C_{1-6}$-alkyl, Acetoxy-$C_{1-6}$-alkoxy oder $C_{1-6}$-Alkoxycarbonyl; $R^{33}$ und $R^{35}$ andere als Wasserstoff sind und gewählt sind aus $C_{1-6}$-Alkyl und $C_{1-6}$-Alkoxy; oder

(ii) $R^{35}$ ist $C_{1-6}$-Alkyl oder $C_{1-6}$-Alkoxy, und $R^{32}$ und $R^{33}$ oder $R^{33}$ und $R^{34}$ Methylendioxy oder Ethylendioxy bilden, und die anderen von $R^{31}$, $R^{32}$, $R^{33}$ und $R^{34}$ gewählt sind wie bei (i), unter der Voraussetzung, dass dann, wenn M $(CH_2)_mC(O)NH(CH_2)_r$ ist, mindestens zwei von $R^{31}$, $R^{32}$, $R^{33}$, $R^{34}$ und $R^{35}$ nicht Wasserstoff sind.

2. Verbindung nach Anspruch 1 oder ein pharmazeutisch geeignetes Salz, Säure oder Ester davon, worin $Ar^2$ die Formel VII aufweist:

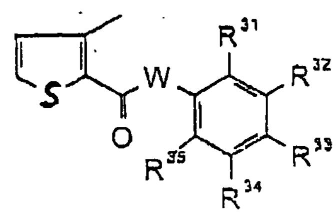

worin W $CH_2$ oder NH ist.

3. Verbindung nach Anspruch 1 oder ein pharmazeutisch geeignetes Salz, Säure oder Ester davon, worin M gewählt ist aus

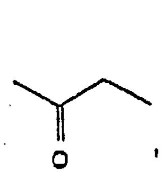, 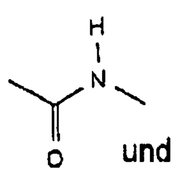 und 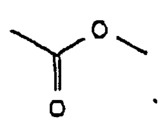.

4. Verbindung oder ein pharmazeutisch geeignetes Salz, Säure oder Ester davon, die gewählt ist aus N-(4-Bromo-3-methyl-5-isoxazolyl)-2-[3,4-(methylendioxy)-benzyl]benzo[b]thiophen-3-sulfonamid; N-(4-Bromo-3-methyl-5-isoxazolyl)-2-(3,4,5-trimethoxybenzyl)-benzo[b]-thiophen-3-sulfonamid; N-(4-Chloro-3-methyl-5-isoxazolyl)-2-[(3,4-methylendioxy)benzyl]benzo[b]thiophen-3-suffonamid; N-(4-Bromo-3-methyl-5-isoxazolyl)-2-(3,4-dimethoxybenzyl]benzo[b]thiophen-3-sulfonamid; N-(4-Chloro-3-methyl-5-isoxazolyl)-6-methoxy-2-[3,4-(methylendioxy)benzyl]benzo[b]thiophen-3-sulfonamid; und N-(4-Chloro-5-methyl-3-isoxazolyl)-2-[3,4-(methylendioxy)benzyl]benzo[b]thiophen-3-sulfonamid.

5. Verbindung nach einem der Ansprüche 1-3, oder ein pharmazeutisch geeignetes Salz, Säure oder Ester davon, worin $R^1$ Halid oder $CH_3$ ist, und $R^2$ H, $CH_3$, $C_2H_5$ oder $CF_3$ ist.

6. Verbindung nach einem der Ansprüche 1-3 und 5, oder ein pharmazeutisch geeignetes Salz, Säure oder Ester davon, das ein (Phenylacetyl)thiophensulfonamid ist.

7. Verbindung nach einem der Ansprüche 1-3 und 5, oder ein pharmazeutisch geeignetes Salz, Säure oder Ester davon, das ein (Phenyloxy)thiophensulfonamid ist.

8. Verbindung nach Anspruch 1 oder ein pharmazeutisch geeignetes Salz, Säure oder Ester davon, das gewählt ist aus N-(4-Bromo-3-methyl-5-isoxazolyl)-2-[3,4-(methylendioxy)phenoxycarbonyl]thiophen-3-sulfonamid, N-(4-Chloro-3-methyl-5-isoxazolyl)-2-[3,4-(methylendioxy)phenoxycarbonyl]thiophen-3-sulfonamid, N-(4-Bromo-3-methyl-5-isoxazolyl)-2-[3,4-(methylendioxy)phenylacetyl]thiophen-3-sulfonamid; N-(4-Chloro-3-methyl-5-isoxazolyl)-{2-[3,4-(methylendioxy)phenyl]-acetyl}thiophen-3-sulfonamidoxim, N-(4-Chloro-3-methyl-5-isoxazolyl)-2-[(2-cyano-4,5-dimethoxyphenyl)aminocarbonyl]thiophen-3-sulfonamid, N-(4-Chloro-3-methyl-5-isoxazolyl)-2-[2-hydroxy-4-methylphenyl)aminocarbonyl]thiophen-3-sulfonamid, N-(4-Chloro-3-methyl-5-isoxazolyl)-2-{[2-cyano-4,5-(methylendioxy)-phenyl]aminocarbonyl}thiophen-3-sulfonamid, N-(4-Chloro-3-methyl-5-isoxazolyl)-2-[2-methyl-4,5-(methylendioxy)phenylaminocarbonyl]thiophen-3-sulfonamid, N-(3,4-Dimethyl-5-isoxazolyl)-2-(2,4-dimethylphenylacetyl)thiophen-3-sulfonamid, N-(4-Chloro-3-methyl-5-isoxazolyl)-2-(2,4-dimethylphenylacetyl)-thiophen-3-sulfonamid, N-(4-Bromo-3-methyl-5-isoxazolyl)-2-(2,4-dimethylphenylacetyl)thiophen-3-sulfonamid, N-(4-Chloro-3-methyl-5-isoxazolyl)-2-[3,4-(methylendioxy)]phenylaminocarbonyl-3-thiophensulfonamid, N-(4-Chloro-3-methyl-5-isoxazolyl)-242-methyl-4,5-(methylendioxy)phenylacetyl]thiophen-3-sulfonamid, N-(4-Chloro-3-methyl-5-isoxazolyl)-2-[3,4-(methylendioxy)-6-(2-acetoxyethyl)phenylaminocarbonyl]thiophen-3-sulfonamid, N-(4-Chloro-3-methyl-5-isoxazolyl)-2-[3,4-(methylendioxy)-6-(2-hydroxyethyl)phenylaminocarbonyl]-thiophen-3-sulfonamid, N-(4-Chloro-3-methyl-5-isoxazolyl)-2-(2,5-dimethylphenylacetyl)thiophen-3-sulfonamid, N-(4-Chloro-3-methyl-5-isoxazolyl)-2-[2-methansulfonylaminomethyl)-4,5-(methylendioxy)phenylaminocarbonyl]thiophen-3-sulfonamid, N-(4-Chloro-3-methyl-5-isoxazolyl)-2-[2-cyanomethyl-4,5-(methylendioxy)-6-cyanomethyl]phenylaminocarbonyl-3-thiophensulfonamid, N-(4-Chloro-3-methyl-5-isoxazolyl)-2-[2-hydroxypropyl-4,5-(methylendioxy)phenylaminocarbonyl]thiophen-3-sulfonamid, N-(4-Chloro-3-methyl-5-isoxazolyl)-2-{2-[(dimethylamino)carbonylmethyl]-4,5-(methylendioxy)phenylaminocarbonyl}thiophen-3-sulfonamid, N-(4-Chloro-3-methyl-5-isoxazolyl)-2-[β-acetoxy-2-methyl-4,5-(methylendioxy)styryl]thiophen-3-sulfonamid, N-(4-Chloro-3-methyl-5-isoxazolyl)-2-[(2,3,4-trimethoxy-6-cyano)phenylaminocarbonyl]thiophen-3-sulfonamid, N-(3,4-Dimethyl-5-isoxazolyl)-2-[2-methyl-4,5-(methylendioxy)-phenylacetyl]thiophen-3-sulfonamid, N-(4-Chloro-3-methyl-5-isoxazolyl)-2-(2,4,6-trimethylphenylaminocarbonyl)thiophen-3-sulfonamid, N-(4-Chloro-3-methyl-5-isoxazolyl)-2-(2,4,6-trimethylphenylacetyl)thiophen-3-sulfonamid, N-(4-Chloro-5-methyl-3-isoxazolyl)-2-[2-methyl-4,5-(methylendioxy)phenylacetyl]thiophen-3-sulfonamid, N-(4-Bromo-3-methyl-5-isoxazolyl)-2-[2-methyl-4,5-(methylendioxy)-cinnamyl]thiophen-3-sulfonamid, N-(4-Bromo-3-methyl-5-isoxazolyl)-2-(2,4-dimethylphenethyl)thiophen-3-sulfonamid und N-(4-Chloro-3-methyl-5-isoxazolyl)-2-[(4-methoxycarbonyl-2,6-dimethyl)phenylaminocarbonyl)thiophen-3-sulfonamid oder jegliches entsprechende N-(4-Halo-3-methyl-5-isoxazolyl)-, N-(4-Halo-5-methyl-3-isoxazolyl)-, N-(3,4-Dimethyl-5-isoxazolyl)-, N-(4-Halo-5-methyl-3-isoxazolyl)-, N-(4-Halo-3-methyl-5-isoxazolyl)-, N-(4,5-Dimethyl-3-isoxazolyl)-Derivat einer beliebigen dieser Verbindungen.

9. Verbindung nach Anspruch 1 oder ein pharmazeutisch geeignetes Salz, Säure oder Ester davon, gewählt aus N-(4-Chloro-3-methyl-5-isoxazolyl)-2-(2,3,4-trimethoxy-6-methylphenylaminocarbonyl)thiophen-3-sulfonamid, N-(4-Chloro-3-methyl-5-isoxazolyl)-2-(2,3,4-trimethoxy-6-acetylphenylaminocarbonyl)thiophen-3-sulfonamid, N-(4-Chloro-3-methyl-5-isoxazolyl)-2-(2,3,4-trimethoxy-6-methoxycarbonylphenylaminocarbonyl)thiophen-3-sulfonamid. N-(4-Chloro-3-methyl-5-isoxazolyl)-2-(2,3,4-trimethoxy-6-carboxylphenylaminocarbonyl)-thiophen-3-sulfonamid, N-(4-Chloro-3-methyl-5-isoxazolyl)-2-(2,3,4-trimethoxy-6-methansulfonylphenylaminocarbonyl)thiophen-3-sulfonamid, N-(4-Chloro-3-methyl-5-isoxazolyl)-2-[2,3,4-trimethoxy-6-(cyanomethyl)phenylaminocarbonyl]-thiophen-3-sulfonamid, N-(4-Chloro-3-methyl-5-isoxazolyl)-2-[2,3,4-trimethoxy-6-(2-hydroxyethyl)phenylaminocarbonyl]thiophen-3-sulfonamid, N-(4-Chloro-3-methyl-5-isoxazolyl)-2-[3,4-(methylendioxy)-2-methoxy-6-methylphenylaminocarbonyl]thiophen-3-sulfonamid, N-(4-Chloro-3-methyl-5-isoxazolyl)-2-[3,4-(methylendioxy)-2-methoxy-6-acetylphenylaminocarbonyl]thiophen-3-sulfonamid, N-(4-Chloro-3-methyl-5-isoxazolyl)-2-[3,4-(methylendioxy)-2-methoxy-6-methoxycarbonylphenylaminocarbonyl]thiophen-3-sulfonamid, N-(4-Chloro-3-methyl-5-isoxazolyl)-2-[3,4-(methylendioxy)-2-methoxy-6-carboxylphenylaminocarbonyl]thiophen-3-sulfonamid,N-(4-Chloro-3-methyl-5-isoxazolyl)-2-[3,4-(methylendioxy)-2-methoxy-6-methansulfonylphenylaminocarbonyl]thiophen-3-sulfonamid, N-(4-Chloro-3-methyl-5-isoxazolyl)-2-[3,4-(methylendioxy)-2-methoxy-6- cyanophenylaminocarbonyl]thiophen-3-sulfonamid, N-(4-Chloro-3-methyl-5-isoxazolyl)-2-[3,4-(methylendioxy)-2-methoxy-6- cyanomethylphenylaminocarbonyl]thiophen-3-sulfonamid, N-(4-Chloro-3-methyl-5-isoxazolyl)-2-[3,4-(methylendioxy)-2-methoxy-6-(2-hydroxyethyl)phenylaminocarbonyl]thiophen-3-sulfonamid, N-(4-Chloro-3-methyl-5-isoxazolyl)-2-[3,4-(methylendioxy)-2,6-dimethylphenylaminocarbonyl]thiophen-3-sulfonamid, N-(4-Chloro-3-methyl-5-isoxazolyl)-2-[3,4-(methylendioxy)-6-acetyl-2-methylphenylaminocarbonyl]-thiophen-3-sulfonamid, N-(4-Chloro-3-methyl-5-isoxazolyl)-2-[3,4-(methylendioxy)-6-methoxycarbonyl-2-methylphenylaminocarbonyl]thiophen-3-sulfonamid, N-(4-Chloro-3-methyl-5-isoxazolyl)-2-[3,4-(methylendioxy)-6-carboxyl-2-methylphenylaminocarbonyl]thiophen-3-sulfonamid, N-(4-Chloro-3-methyl-5-isoxazolyl)-2-[3,4-(methylendioxy)-6-methoxy-2-methylphenylaminocarbonyl]thiophen-3-sulfonamid, N-(4-Chloro-3-methyl-5-isoxazolyl)-2-[3,4-(methylendioxy)-6-methansulfonyl-2-methylphenylaminocarbonyl]thiophen-3-sulfonamid, N-(4-Chloro-3-methyl-5-isoxazolyl)-2-[3,4-(methylendioxy)-6-cyano-2-methylphenylaminocarbonyl]thiophen-3-sulfonamid,N-(4-Chloro-3-methyl-5-isoxazolyl)-2-[3,4-(methylendioxy)-6-(cyanomethyl)-2-methylphenylaminocarbonyl]thiophen-3-sulfonamid, N-(4-Chloro-3-methyl-5-isoxazolyl)-2-[3,4-(methylendioxy)-6-(2-hydroxyethyl)-2-methylphenylaminocarbonyl]thiophen-3-sulfonamidN-(4-Chloro-3-methyl-5-isoxazolyl)-2-[3,4-(methylendioxy)-2-cyano-6-methylphenylaminocarbonyl]thiophen-3-sulfonamid, N-(4-Chloro-3-methyl-5-isoxazolyl)-2-[3,4-(methylendioxy)-6-methoxy-2-cyanophenylaminocarbonyl]-thiophen-3-sulfonamid, N-(4-Chloro-3-methyl-5-isoxazolyl)-2-[3,4-(methylendioxy)-2-acetyl-6-methylphenylaminocarbonyl] thiophen-3-sulfonamid, N-(4-Chloro-3-methyl-5-isoxazolyl)-2-[3,4-(methylendioxy)-6-methoxy-2-acetylphenylaminocarbonyl]thiophen-3-sulfonamid, N-(4-Chloro-3-methyl-5-isoxazolyl)-2-(3-cyano-2,4,6-trimethylphenylaminocarbonyl]thiophen-3-sulfonamid, N-(4-Chloro-3-methyl-5-isoxazolyl)-2-(3-carboxyl-2,4,6-trimethylphenylaminocarbonyl]thiophen-3-sulfonamid, N-(4-Chloro-3-methyl-5-isoxazolyl)-2-(3-hydroxymethyl-2,4,6-trimethylphenylaminocarbonyl]thiophen-3-sulfonamid, N-(4-Chloro-3-methyl-5-isoxazolyl)-2-(3-methansulfonyl-2,4,6-trimethylphenylaminocarbonyl]thiophen-3-sulfonamid, N-(4-Chloro-3-methyl-5-isoxazolyl)-2-(3-cyanomethyl-2,4,6-trimethylphenylaminocarbonyl]thiophen-3-sulfonamid, N-(4-Chloro-3-methyl-5-isoxazolyl)-2-[3-(2-hydroxyethyl-2,4,6-trimethylphenylaminocarbonyl]thiophen-3-sulfonamid, N-(4-Chloro-3-methyl-5-isoxazolyl)-2-[3-carboxylmethyl-2,4,6-trimethylphenylaminocarbonyl]thiophen-3-sulfonamid, N-(4-Chloro-3-methyl-5-isoxazolyl)-2-(4-cyano-2,6-dimethylphenylaminocarbonyl)-thiophen-3-sulfonamid, N-(4-Chloro-3-methyl-5-isoxazolyl)-2-(4-carboxyl-2,6-dimethylphenylaminocarbonyl)thiophen-3-sulfonamid, N-(4-Chloro-3-methyl-5-isoxazolyl)-2-[4-(hydroxymethyl)-2,6-dimethylphenylaminocarbonyl]thiophen-3-sulfonamid, N-(4-Chloro-3-methyl-5-isoxazolyl)-2-[4-(2-hydroxyethyl)-2,6-dimethylphenylaminocarbonyl]thiophen-3-sulfonamid,N-(4-Chloro-3-methyl-5-isoxazolyl)-2-[4-cyanomethyl)-2,6-dimethylphenylaminocarbonyl]thiophen-3-sulfonamid,N-(4-Chloro-3-methyl-5-isoxazolyl)-2-[4-(carboxylmethyl)-2,6-dimethylphenylaminocarbonyl]thiophen-3-sulfonamid, N-(4-Chloro-3-methyl-5-isoxazolyl)-2-(4-methansulfonyl-2,6-dimethylphenylaminocarbonyl)thiophen-3-sulfonamid, N-(4-Chloro-3-methyl-5-isoxazolyl)-2-(2,3,4-trimethoxy-6-methylphenylacetyl)thiophen-3-sulfonamid, N-(4-Chloro-3-methyl-5-isoxazolyl)-2-(2,3,4-trimethoxy-6-acetylphenylacetyl)thiophen-3-sulfonamid, N-(4-Chloro-3-methyl-5-isoxazolyl)-2-(2,3,4-trimethoxy-6-methoxycarbonylphenylacetyl)-thiophen-3-sulfonamid, N-(4-Chloro-3-methyl-5-isoxazolyl)-2-(2,3,4-trimethoxy-6-carboxylphenylacetyl)thiophen-3-sulfonamid, N-(4-Chloro-3-methyl-5-isoxazolyl)-2-(2,3,4-trimethoxy-6-methansulfonylphenylacetyl)thiophen-3-sulfonamid, N-(4-Chloro-3-methyl-5-isoxazolyl)-2-[2,3,4-trimethoxy-6-(cyanomethyl)phenylacetyl]-thiophen-3-sulfonamid, N-(4-Chloro-3-methyl-5-isoxazolyl)-2-[2,3,4-trimethoxy-6-(2-hydroxyethyl)phenylacetyl]thiophen-3-sulfonamid, N-(4-Chloro-3-methyl-5-isoxazolyl)-2-[3,4-(methylendioxy)-2-methoxy-6-methylphenylacetyl]thiophen-3-sulfonamid, N-(4-Chloro-3-methyl-5-isoxazolyl)-2-[3,4-(methylendioxy)-2-methoxy-6-acetylphenylacetyl)thiophen-3-sulfonamid, N-(4-Chloro-3-methyl-5-isoxazolyl)-2-[3,4-(methylendioxy)-2-methoxy-6-methoxycarbonylphenylacetyl)-thiophen-3-sulfonamid,N-(4-Chloro-3-methyl-5-isoxazolyl)-2-[3,4-(methylendioxy)-2-methoxy-6-carboxylphenylacetyl)thiophen-3-sulfonamid, N-(4-Chloro-3-methyl-5-isoxazolyl)-2-[3,4-(methylendioxy)-2-methoxy-6-methansulfonyl)phenylacetyl]thiophen-3-sulfonamid, N-(4-Chloro-3-methyl-5-isoxazolyl)-2-[3,4-(methylendioxy)-2-methoxy-6-(cyano)phenylacetyl]thiophen-3-sulfonamid, N-(4-Chloro-3-methyl-5-isoxazolyl)-2-[3,4-(methylendioxy)-2-methoxy-6-(cyanomethylphenylacetyl]thiophen-3-sulfonamid, N-(4-Chloro-3-methyl-5-isoxazolyl)-2-[3,4-(methylendioxy)-2-methoxy-6-(2-hydroxyethyl)-phenylacetyl]thiophen-3-sulfonamid, N-(4-Chloro-3-methyl-5-isoxazolyl)-2-[3,4-(methylendioxy)-2,6-dimethylphenylacetyl)thiophen-3-sulfonamid, N-(4-Chloro-3-methyl-5-isoxazolyl)-2-[3,4-(methylendioxy)-6-acetyl-2-methylphenylacetyl]-thiophen-3-sulfonamid, N-(4-Chloro-3-methyl-5-isoxazolyl)-2-[3,4-(methylendioxy)-6-methoxycarbonyl-2-methylphenylacetyl]thiophen-3-sulfonamid, N-(4-Chloro-3-methyl-5-isoxazolyl)-2-[3,4-(methylendioxy)-6-carboxyl-2-methylphenylacetyl]thiophen-3-sulfonamid, N-(4-Chloro-3-methyl-5-isoxazolyl)-2-[3,4-(methylendioxy)-6-methoxy-2-methylphenylacetyl]thiophen-3-sulfonamid, N-(4-Chloro-3-methyl-5-isoxazolyl)-2-[3,4-(methylendioxy)-6-methansulfonyl-2-methylphenylacetyl]thiophen-3-sulfonamid, N-(4-Chloro-3-methyl-5-isoxazolyl)-2-[3,4-(methylendioxy)-6-cyano-2-methylphenylacetyl]thiophen-3-sulfonamid, N-(4-Chloro-3-methyl-5-isoxazolyl)-2-[3,4-(methylendioxy)-6-(cyanomethyl)-2-methylphenylacetyl]thiophen-3-sulfonamid, N-(4-Chloro-3-methyl-5-isoxazolyl)-2-[3,4-(methylendioxy)-6-(2-hydroxyethyl)-2-methylphenylacetyl]thiophen-3-sulfonamid, N-(4-Chloro-3-methyl-5-isoxazolyl)-2-[3,4-(methylendioxy)-2-cyano-6-methylphenylacetyl]thiophen-3-sulfonamid, N-(4-Chloro-3-methyl-5-isoxazolyl)-2-[3,4-(methylendioxy)-6-methoxy-2-cyanophenylacetyl]thiophen-3-sulfonamid, N-(4-Chloro-3-methyl-5-isoxazolyl)-2-[3,4-(methylendioxy)-2-acetyl-6-methylphenylacetyl]thiophen-3-sulfonamid, N-(4-Chloro-3-methyl-5-isoxazolyl)-2-[3,4-(methylendioxy)-6-methoxy-2-acetylphenylacetyl]thiophen-3-sulfonamid, N-(4-Chloro-3-methyl-5-isoxazolyl)-2-(3-cyano-2,4,6-trimethylphenylacetyl)-thiophen-3-sulfonamid, N-(4-Chloro-3-methyl-5-isoxazolyl)-2-(3-carboxyl-2,4,6-trimethylphenylacetyl)thiophen-3-sulfonamid, N-(4-Chloro-3-methyl-5-isoxazolyl)-2-(3-hydroxymethyl-2,4,6-trimethylphenylacetyl)thiophen-3-sulfonamid, N-(4-Chloro-3-methyl-5-isoxazolyl)-2-(3-methansulfonyl-2,4,6-trimethylphenylacetyl)-thiophen-3-sulfonamid, N-(4-Chloro-3-methyl-5-isoxazolyl)-2-[3-(cyanomethyl)-2,4,6-trimethylphenylacetyl)thiophen-3-sulfonamid, N-(4-Chloro-3-methyl-5-isoxazolyl)-2-[3-(2-hydroxyethyl)-2,4,6-trimethylphenylacetyl)thiophen-3-sulfonamid, N-(4-Chloro-3-methyl-5-isoxazolyl)-2-[3-(carboxylmethyl)-2,4,6-trimethylphenylacetyl]thiophen-3-sulfonamid, N-(4-Chloro-3-methyl-5-isoxazolyl)-2-(4-cyano-2,6-dimethylphenylacetyl)thiophen-3-sulfonamid, N-(4-Chloro-3-methyl-5-isoxazolyl)-2-(4-carboxyl-2,6-dimethylphenylacetyl)thiophen-3-sulfonamid, N-(4-Chloro-3-methyl-5-isoxazolyl)-2-(4-hydroxymethyl-2,6-dimethylphenylacetyl)thiophen-3-sulfonamid, N-(4-Chloro-3-methyl-5-isoxazolyl)-2-[4-(2-hydroxyethyl)-2,6-(dimethyl)phenylacetyl] thiophen-3-sulfonamid, N-(4-Chloro-3-methyl-5-lsoxazolyl)-2-[4-cyanomethyl-2,6-(dimethyl)phenylacetyl]-thiophen-3-sulfonamid, N-(4-Chloro-3-methyl-5-isoxazolyl)-2-[4-(carboxylmethyl)-2,6-dimethylphenylacetyl)thiophen-3-sulfonamid und N-(4-Chloro-3-methyl-5-isoxazolyl)-2-(4-methansulfonyl-2,6-dimethylphenylacetyl)thiophen-3-sulfonamid oder jegliches entsprechende N-(4-Halo-3-methyl-5-isoxazolyl)-, N-(4-Halo-5-methyl-3-isoxazolyl)-, N-(3,4-Dimethyl-5-isoxazolyl)-, N-(4-Halo-5-methyl-3-isoxazolyl)-, N-(4-Halo-3-methyl-5-isoxazolyl)-, N-(4,5-Dimethyl-3-isoxazolyl)-Derivat einer beliebigen dieser Verbindungen.

**10.** Verbindung nach Anspruch 1 oder ein pharmazeutisch geeignetes Salz, Säure oder Ester davon, gewählt aus N-(4-Bromo-3-methyl-5-isoxazolyl)-2-[3,4-(methylendioxy)benzyl]benzo[b]thiophen-3-sulfonamid, N-(4-Chloro-3-methyl-5-isoxazolyl)-2-(3,4-methylendioxy)benzyl]benzo[b]thiophen-3-sulfonamid, N-(4-Chloro-3-methyl-5-isoxazolyl)-2-[β-hydroxy(3,4-methylendioxy)phenylethyl]-thiophen-3-sulfonamid und N-(4-Chloro-3-methyl-5-isoxazolyl)-2-(3,4-methylendioxybenzylcarbonyl)thiophen-3-sulfonamid oder jegliches entsprechende N-(4-Halo-3-methyl-5-isoxazolyl)-, N-(4-Halo-5-methyl-3-isoxazolyl)-, N-(3,4-Dimethyl-5-isoxazolyl)-, N-(4-Halo-3-methyl-5-isoxazolyl)-, N-(4,5-Dimethyl-3-isoxazolyl)-Derivat davon.

**11.** Verbindung nach Anspruch 1 oder ein pharmazeutisch geeignetes Salz, Säure oder Ester davon, die N-(4-Chloro-3-methyl-5-isoxazolyl)-2-[2-hydroxy-4-methylphenyl)aminocarbonyl]thiophen-3-sulfonamid ist.

**12.** Verbindung nach Anspruch 1 oder ein pharmazeutisch geeignetes Salz, Säure oder Ester davon, die N-(4-Chloro-3-methyl-5-isoxazolyl)-2-{[2-cyano-4,5-(methylendioxy)phenyl])aminocarbonyl}thiophen-3-sulfonamid ist.

**13.** Verbindung nach Anspruch 1 oder ein pharmazeutisch geeignetes Salz, Säure oder Ester davon, die N-(4-Chloro-3-methyl-5-isoxazolyl)-2-[2-methyl-4,5-(methylendioxy)phenylaminocarbonyl]thiophen-3-sulfonamid ist.

**14.** Verbindung nach Anspruch 1 oder ein pharmazeutisch geeignetes Salz, Säure oder Ester davon, die N-(4-Chloro-3-methyl-5-isoxazolyl)-2-(2-carboxamido-4,5-dimethoxyphenylaminocarbonyl)thiophen-3-sulfonamid ist.

**15.** Verbindung nach Anspruch 1 oder ein pharmazeutisch geeignetes Salz, Säure oder Ester davon, die N-(3,4-Dimethyl-5-isoxazolyl)-2-(2,4-dimethylphenylacetyl)-thiophen-3-sulfonamid ist.

**16.** Verbindung nach Anspruch 1 oder ein pharmazeutisch geeignetes Salz, Säure oder Ester davon, die N-(4-Chloro-3-methyl-5-isoxazolyl)-2-(2,4-dimethylphenylacetyl)thiophen-3-sulfonamid ist.

**17.** Verbindung nach Anspruch 1 oder ein pharmazeutisch geeignetes Salz, Säure oder Ester davon, die N-(4-Bromo-3-methyl-5-isoxazolyl)-2-(2,4-dimethylphenylacetyl)thiophen-3-sulfonamid ist.

**18.** Verbindung nach Anspruch 1 oder ein pharmazeutisch geeignetes Salz, Säure oder Ester davon, die N-(4-Chloro-3-methyl-5-isoxazolyl)-2-[3,4-(methylendioxy)]-phenylaminocarbonyl-3-thiophensulfonamid ist.

**19.** Verbindung nach Anspruch 1 oder ein pharmazeutisch geeignetes Salz, Säure oder Ester davon, die N-(4-Chloro-3-methyl-5-isoxazolyl)-2-[2-methyl-4,5-(methylendioxy)phenylacetyl]thiophen-3-sulfonamid ist.

**20.** Verbindung nach Anspruch 1 oder ein pharmazeutisch geeignetes Salz, Säure oder Ester davon, die N-(4-Chloro-3-methyl-5-isoxazolyl)-2-[3,4-methylendioxy)-6-(2-acetoxyethyl)phenylaminocarbonyl]thiophen-3-sulfonamid ist.

**21.** Verbindung nach Anspruch 1 oder ein pharmazeutisch geeignetes Salz, Säure oder Ester davon, die N-(4-Chloro-3-methyl-5-isoxazolyl)-2-[3,4-methylendioxy)-6-(2-hydroxyethyl)phenylaminocarbonyl]thiophen-3-sulfonamid ist.

**22.** Verbindung nach Anspruch 1 oder ein pharmazeutisch geeignetes Salz, Säure oder Ester davon, die N-(4-Chloro-3-methyl-5-isoxazolyl)-2-(2,5-dimethylphenylacetyl)thiophen-3-sulfonamid ist.

**23.** Verbindung nach Anspruch 1 oder ein pharmazeutisch geeignetes Salz, Säure oder Ester davon, die N-(4-Chloro-3-methyl-5-isoxazolyl)-2-[2-methansulfonylaminomethyl)-4,5-(methylendioxy)phenylaminocarbonyl]thiophen-3-sulfonamid ist.

**24.** Verbindung nach Anspruch 1 oder ein pharmazeutisch geeignetes Salz, Säure oder Ester davon, die N-(4-Chloro-3-methyl-5-isoxazolyl)-2-[2-cyanomethyl-4,5-(methylendioxy)-6-cyanomethyl]phenylaminocarbonyl-3-thiophensulfonamid ist.

**25.** Verbindung nach Anspruch 1 oder ein pharmazeutisch geeignetes Salz, Säure oder Ester davon, die N-(4-Bromo-3-methyl-5-isoxazolyl)-3-[2-methyl-4,5-(methylendioxy)cinnamyl]thiophen-2-sulfonamid ist.

**26.** Verbindung nach Anspruch 1 oder ein pharmazeutisch geeignetes Salz, Säure oder Ester davon, die N-(4-Chloro-3-methyl-5-isoxazolyl)-2-{2-[(dimethylamino)-carbonylmethyl]-4,5-(methylendioxy)phenylaminocarbonyl}thiophen-3-sulfonamid ist.

**27.** Verbindung nach Anspruch 1 oder ein pharmazeutisch geeignetes Salz, Säure oder Ester davon, die N-(4-Chloro-3-methyl-5-isoxazolyl)-2-[β-acetoxy-2-methyl)-4,5-(methylendioxy)styryl]thiophen-3-sulfonamid ist.

**28.** Verbindung nach Anspruch 1 oder ein pharmazeutisch geeignetes Salz, Säure oder Ester davon, die N-(4-Chloro-3-methyl-5-isoxazolyl)-2-[(2,3,4-trimethoxy-6-cyano)phenylaminocarbonyl]thiophen-3-sulfonamid ist.

**29.** Verbindung nach Anspruch 1 oder ein pharmazeutisch geeignetes Salz, Säure oder Ester davon, die N-(4-Chloro-3-methyl-5-isoxazolyl)-2-(2,4,6-trimethylphenylaminocarbonyl)thiophen-3-sulfonamid ist.

**30.** Verbindung nach Anspruch 1 oder ein pharmazeutisch geeignetes Salz, Säure oder Ester davon, die N-(4-Chloro-3-methyl-5-isoxazolyl)-2-(3-methoxycarbonyl-2,4,6-trimethylphenylaminocarbonyl)thiophen-3-sulfonamid ist.

**31.** Verbindung nach Anspruch 1 oder ein pharmazeutisch geeignetes Salz, Säure oder Ester davon, die N-(4-Chloro-3-methyl-5-isoxazolyl)-2-(2,4,6-trimethylphenylacetyl)thiophen-3-sulfonamid ist.

**32.** Verbindung nach Anspruch 1 oder ein pharmazeutisch geeignetes Salz, Säure oder Ester davon, die N-(4-Chloro-5-methyl-3-isoxazolyl)-2-[2-methyl-4,5-(methylendioxy)phenylacetyl]thiophen-3-sulfonamid ist.

**33.** Verbindung nach Anspruch 1 oder ein pharmazeutisch geeignetes Salz, Säure oder Ester davon, die N-(4-Chloro-3-methyl-5-isoxazolyl)-2-[(4-methoxycarbonyl-2,6-dimethyl)phenylaminocarbonyl]thiophen-3-sulfonamid ist.

**34.** Pharmazeutische Zusammensetzung, umfassend eine Verbindung nach einem der Ansprüche 1 bis 33 oder ein pharmazeutisch geeignetes Salz, Säure oder Ester davon, zusammen mit einem pharmazeutisch geeigneten Träger.

**35.** Pharmazeutische Zusammensetzung, umfassend die Verbindung nach Anspruch 19, oder ein pharmazeutisch geeignetes Salz, Säure oder Ester davon, zusammen mit einem pharmazeutisch geeigneten Träger.

**36.** Verbindung nach einem der Ansprüche 1 bis 33, oder ein pharmazeutisch geeignetes Salz, Säure oder Ester davon, zur Verwendung bei der Behandlung einer Endothelin-vermittelten Störung.

**37.** Verbindung nach Anspruch 19, oder ein pharmazeutisch geeignetes Salz, Säure oder Ester davon, zur Verwendung bei der Behandlung einer Endothelin-vermittelten Störung.

**38.** Verwendung einer oder mehrerer Verbindungen nach einem der Ansprüche 1 bis 33, oder eines pharmazeutisch

geeigneten Salzes, Säure oder Esters davon, zur Herstellung eines Medikaments zur Behandlung einer Endothelin-vermittelten Störung.

**39.** Verwendung der Verbindung nach Anspruch 19, oder eines pharmazeutisch geeigneten Salzes, Säure oder Esters davon, zur Herstellung eines Medikaments zur Behandlung einer Endothelin-vermittelten Störung.

**40.** Verwendung nach Anspruch 38 oder Anspruch 39, wobei die Störung gewählt ist aus Hypertonie, Herz-Kreislauf-Erkrankung, Asthma, Lungenhochdruck, entzündlichen Erkrankungen, Augenerkrankungen, Menstruationsstörungen, obstetrischen Zuständen, Wunden, Magen-Darm-Erkrankungen, Nierenversagen, Immunsuppressivum-vermittelter Nierengefäßverengung, Erythropoietin-vermittelter Gefäßverengung, Endotoxin-Schock, anaphylaktischem Schock und hämorrhagischem Schock.

**41.** Verwendung nach Anspruch 40, wobei die Störung gewählt ist aus Hypertonie, Herz-Kreislauf-Erkrankung, Lungenhochdruck, Erythropoietin-vermittelter Gefäßverengung, Endotoxin-Schock, anaphylaktischem Schock und hämorrhagischem Schock.

**42.** Verwendung nach Anspruch 40, wobei die Störung gewählt ist aus Asthma und entzündlichen Erkrankungen.

**43.** Verwendung einer oder mehrerer Verbindungen nach einem der Ansprüche 1 bis 33, oder eines pharmazeutisch geeigneten Salzes, Esters oder Säure davon, bei der Herstellung eines Medikaments zur Hemmung der Bindung eines Endothelinpeptids an einen $Endothelin_A$-($ET_A$)- oder $Endothelin_B$-($ET_B$)-Rezeptor.

**44.** Verwendung der Verbindung nach Anspruch 19, oder eines pharmazeutisch geeigneten Salzes, Esters oder Säure davon, bei der Herstellung eines Medikaments zur Hemmung der Bindung eines Endothelinpeptids an einen $Endothelin_A$-($ET_A$)- oder $Endothelin_B$-($ET_B$)-Rezeptor.

**45.** Verwendung einer oder mehrerer Verbindungen nach einem der Ansprüche 1 bis 33, oder eines pharmazeutisch geeigneten Salzes, Esters oder Säure davon, bei der Herstellung eines Medikaments zur Veränderung der Endothelinrezeptor-vermittelten Aktivität.

**46.** Verwendung der Verbindung nach Anspruch 19, oder eines pharmazeutisch geeigneten Salzes, Esters oder Säure davon, bei der Herstellung eines Medikaments zur Veränderung der Endothelinrezeptor-vermittelten Aktivität.

**47.** Pharmazeutische Zusammensetzung, formuliert zur Verabreichung von Einzeldosen, umfassend eine wirksame Menge einer oder mehrerer Verbindungen nach einem der Ansprüche 1 bis 33, oder eines pharmazeutisch geeigneten Salzes, Esters oder Säure davon, wobei die Menge zur Milderung der Symptome einer Endothelin-vermittelten Erkrankung wirksam ist.

**48.** Pharmazeutische Zusammensetzung, formuliert zur Verabreichung von Einzeldosen, umfassend eine wirksame Menge der Verbindung nach Anspruch 19, oder eines pharmazeutisch geeigneten Salzes, Esters oder Säure davon, wobei die Menge zur Milderung der Symptome einerEndothelin-vermittelten Erkrankung wirksam ist.

**49.** Erzeugnis, umfassend Verpackungsmaterial und eine oder mehrere Verbindungen nach einem der Ansprüche 1 bis 33 oder ein pharmazeutisch geeignetes Salz, Ester oder Säure davon, die in dem Verpackungsmaterial enthalten ist, wobei die Verbindung zum Antagonisieren der Wirkungen des Endothelins, Mildern der Symptome einer Endothelin-vermittelten Störung oder Hemmen der Bindung eines Endothelinpeptids an einen ET-Rezeptor mit einem $IC_{50}$ von weniger als etwa 10 µM wirksam ist, und das Verpackungsmaterial ein Etikett umfasst, das angibt, dass das Sulfonamid oder Salz davon zum Antagonisieren der Wirkungen von Endothelin, Hemmen der Bindung von Endothelin an einen Endothelinrezeptor oder Behandeln einer Endothelin-vermittelten Störung verwendet wird.

**50.** Erzeugnis, umfassend Verpackungsmaterial und eine Verbindung nach Anspruch 19 oder ein pharmazeutisch geeignetes Salz, Ester oder Säure davon, die in dem Verpackungsmaterial enthalten ist, wobei die Verbindung zum Antagonisieren der Wirkungen des Endothelins, Mildern der Symptome einer Endothelin-vermittelten Störung oder Hemmen der Bindung eines Endothelinpeptids an einen ET-Rezeptor mit einem $IC_{50}$ von weniger als etwa 10 µM wirksam ist, und das Verpackungsmaterial ein Etikett umfasst, das angibt, dass das Sulfonamid oder Salz davon zum Antagonisieren der Wirkungen von Endothelin, Hemmen der Bindung von Endothelin an einen Endothelinrezeptor oder Behandeln einer Endothelin-vermittelten Störung verwendet wird.

**51.** Verwendung nach Anspruch 38 oder Anspruch 39, wobei die Störung Glaukom ist.

## Revendications

**1.** Composé de formule I :

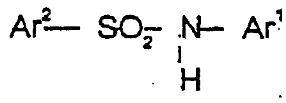

ou sel, acide ou ester pharmaceutiquement acceptable de celui-ci, dans laquelle :

$Ar^1$ est :

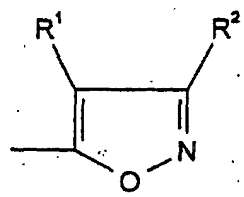 ou 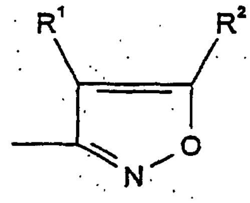

où $R^1$ est choisi parmi les halogènes, $CH_3$, $C_2H_5$, $CF_3$, $C_2F_5$, n-$C_3H_7$ et cyclo-$C_3H_7$, et $R^2$ est choisi parmi H, $CH_3$, $C_2H_5$, $CF_3$, $C_2F_5$, n-$C_3H_7$ et cyclo-$C_3H_7$ ; et
$Ar^2$ a la formule VI :

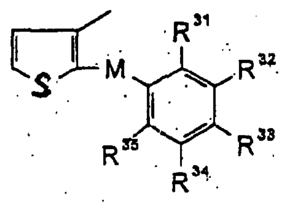

dans laquelle :

M est choisi parmi

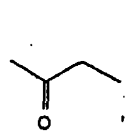, 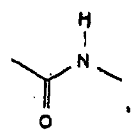, 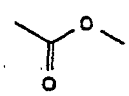, 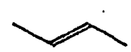,

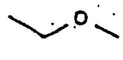, 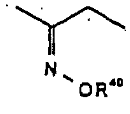, 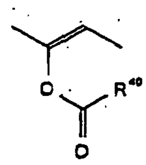 et 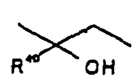

dans lesquelles $R^{40}$ est méthyle, éthyle ou hydrogène ;
$R^{31}$, $R^{32}$, $R^{33}$, $R^{34}$ et $R^{35}$ sont chacun indépendamment choisis entre (i) et (ii) comme suit :

(i) $R^{31}$, $R^{32}$ et $R^{34}$ sont chacun indépendamment choisis parmi un hydrogène, un groupe alkyle en $C_1$ à $C_6$, halogénoalkyle en $C_1$ à $C_6$, phényle, alcoxy en $C_1$ à $C_6$, alkyle en $C_1$ à $C_6$-sulfonylamino-alkyle en $C_1$ à $C_6$, cyanoalkyle en $C_1$ à $C_6$, acétyle, alcoxy en $C_1$ à $C_6$-carbonyle, cyano, OH, acétoxy-alkyle en $C_1$ à $C_6$, hydroxyalkyle en $C_1$ à $C_6$, acétoxy-alcoxy en $C_1$ à $C_6$ ou alcoxy en $C_1$ à $C_6$-carbonyle; $R^{33}$ et $R^{35}$sont différents d'un atome d'hydrogène et sont choisis entre un groupe alkyle en $C_1$ à $C_6$ et alcoxy en $C_1$ à $C_6$ ;ou

(ii) $R^{35}$ est un groupe alkyle en $C_1$ à $C_6$ ou alcoxy en $C_1$ à $C_6$, et $R^{32}$ et $R^{33}$ ou $R^{33}$ et $R^{34}$ forment un groupe méthylènedioxy ou éthylènedioxy, et les autres de $R^{31}$, $R^{32}$, $R^{33}$ et $R^{34}$ sont choisis comme en (i), avec la condition que lorsque M est $(CH_2)_mC(O)NH(CH_2)_r$, alors au moins deux de $R^{31}$, $R^{32}$, $R^{33}$, $R^{34}$ et $R^{35}$ ne sont pas un hydrogène.

**2.** Composé selon la revendication 1 ou sel, acide ou ester pharmaceutiquement acceptable de celui-ci, dans lequel $Ar^2$ a la formule VII:

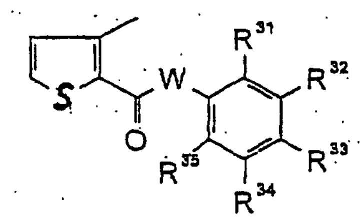

dans laquelle W est $CH_2$ ou NH.

**3.** Composé selon la revendication 1 ou sel, acide ou ester pharmaceutiquement acceptable de celui-ci, dans lequel M est choisi parmi

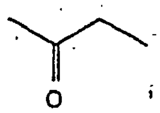

;

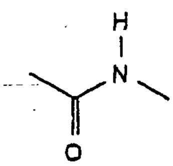

et

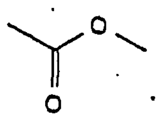

.

**4.** Composé ou sel, acide ou ester pharmaceutiquement acceptable de celui-ci, qui est choisi parmi le N-(4-bromo-3-méthyl-5-isoxazolyl)-2-[3,4-(méthylènedioxy)benzyl]benzo[b]thiophène-3-sulfonamide ; le N-(4-bromo-3-méthyl-5-isoxazolyl)-2-(3,4,5-triméthoxybenzyl)benzo[b]thiophène-3-sulfonamide ; le N-(4-chloro-3-méthyl-5-isoxazolyl)-2-[(3,4-méthylènedioxy)benzyl]benzo[b]-thiophène-3-sulfonamide ; le N-(4-bromo-3-méthyl-5-isoxazolyl)-2-(3,4-diméthoxybenzyl)-benzo[b]thiophène-3-sulfonamide ; le N-(4-chloro-3-méthyl-5-isoxazolyl)-6-méthoxy-2-[3,4-(méthylènedioxy)benzyl]benzo[b]thiophène-3-sulfonamide ; et le N-(4-chloro-5-méthyl-3-isoxazolyl)-2-[3,4-(méthylènedioxy)benzyl)benzo[b]thiophène-3-sulfonamide.

**5.** Composé selon l'une quelconque des revendications 1 à 3, ou sel, acide, ou ester pharmaceutiquement acceptable de celui-ci, dans lequel $R^1$ est un halogène ou $CH_3$, et $R^2$ est H, $CH_3$, $C_2H_5$, ou $CF_3$;

**6.** Composé selon l'une quelconque des revendications 1 à 3 et 5 ou sel, acide ou ester pharmaceutiquement acceptable de celui-ci qui est un (phénylacétyl)thiophènesulfonamide.

**7.** Composé selon l'une quelconque des revendications 1 à 3 et 5 ou sel, acide ou ester pharmaceutiquement acceptable de celui-ci qui est un (phényloxy)-thiophènesulfonamide.

**8.** Composé selon la revendication 1 ou sel, acide ou ester pharmaceutiquement acceptable de celui-ci qui est choisi parmi le N-(4-bromo-3-méthyl-5-isoxazolyl)-2-[(3,4-méthylènedioxy)phénoxycarbonyl]thiophène-3-sulfonamide, le N-(4-chloro-3-méthyl-5-isoxazolyl)-2-[(3,4-méthylènedioxy)-phénoxycarbonyl]-thiophène-3-sulfonamide, le N-(4-bromo-3-méthyl-5-isoxazolyl)-2-[(3,4-méthylènedioxy)phénylacétyl]thiophène-3-sulfonamide, le N-(4-chloro-3-méthyl-5-isoxazolyl)-{2-[3,4-(méthylènedioxy)-phényl]acétyl}thiophène-3-sulfonamide oxime, le N-(4-chloro-3-méthyl-5-isoxazolyl)-2-[(2-cyano-4,5-diméthoxyphényl)aminocarbonyl]thiophène-3-sulfonamide, le N-(4-chloro-3-méthyl-5-isoxazolyl)-2-[(2-hydroxy-4-méthylphényl)aminocarbonyl]thiophène-3-sulfonamide, le N-(4-chloro-3-méthyl-5-isoxazolyl)-2-{[2-cyano-4,5-(méthylènedioxy)phényl]aminocarbonyl}thiophène-3-sulfonamide, le N-(4-chloro-3-méthyl-5-isoxazolyl)-2-[2-methyl-4,5-(méthylènedioxy)phénylaminocarbonyl]-thiophène-3-sulfonamide, le N-(3,4-diméthyl-5-isoxazolyl)-2-[(2,4-diméthylphénylacétyl)thiophène-3-sulfonamide, le N-(4-chloro-3-méthyl-5-isoxazolyl)-2-[(2,4-diméthylphénylacétyl)thiophène-3-sulfonamide, le N-(4-bromo-3-méthyl-5-isoxazolyl)-2-(2, 4-diméthylphénylacétyl)thiophène-3-sulfonamide, le N-(4-chloro-3-méthyl-5-isoxazolyl)-2-[3,4-(méthylènedioxy)] phénylaminocarbonyl-3-thiophènesulfonamide, le N-(4-chloro-3-méthyl-5-isoxazolyl)-2-[2-méthyl-4,5-(méthylènedioxy)]phénylacétyl]thiophène-3-sulfonamide, le N-(4-chloro-3-méthyl-5-isoxazolyl)-2-[3,4-(méthylènedioxy)-6-(2-acétoxyéthyl)phénylaminocarbonyl]-thiophène-3-sulfonamide,leN-(4-chloro-3-méthyl-5-isoxazolyl)-2-[3,4-(méthylènedioxy)-6-(2-hydroxyéthyl)-phénylaminocarbonyl]thiophène-3-sulfonamide, le N-(4-chloro-3-méthyl-5-isoxazolyl)-2-(2,5-diméthylphénylacétyl)thiophène-3-sulfonamide, le N-(4-chloro-3-méthyl-5-isoxazolyl)-2-[(2-méthanesulfonylamino-méthyl)-4,5-(méthylènedioxy)]phénylaminocarbonyl]thiophène-3-sulfonamide, le N-(4-chloro-3-méthyl-5-isoxazolyl)-2-[2-cyanométhyl-4,5-(méthylènedioxy)-6-cyanométhyl]phénylaminocarbonyl-3-thiophènesulfonamide, le N-(4-chloro-3-méthyl-5-isoxazolyl)-2-[2-hydroxypropyl-4,5-(méthylènedioxy)phénylaminocarbonyl]thiophène-3-sulfonamide, le N-(4-chloro-3-méthyl-5-isoxazolyl)-2-{2-[(diméthylamino)carbonylméthyl]-4,5-(méthylènedioxy)]phénylaminocarbonyl}thiophène-3-sulfonamide, la N-(4-chloro-3-méthyl-5-isoxazolyl)-2-[β-acétoxy-2-méthyl-4,5-(méthylènedioxy)styryl]thiophène-3-sulfonamide, le N-(4-chloro-3-méthyl-5-isoxazolyl)-2-[(2,3,4-triméthoxy-6-cyano)phénylaminocarbonyl]thiophène-3-sulfonamide, le N-(3,4-diméthyl-5-isoxazolyl)-2-[2-méthyl-4,5-(méthylènedioxy)-phénylacétyl]thiophène-3-sulfonamide, le N-(4-chloro-3-méthyl-5-isoxazolyl)-2-(2,4,6-triméthylphénylaminocarbonyl)thiophène-3-sulfonamide, le N-(4-chloro-3-méthyl-5-isoxazolyl)-2-(2,4,6-triméthylphénylacétyl)thiophène-3-sulfonamide, le N-(4-chloro-5-méthyl-3-isoxazolyl)-2-[2-méthyl-4,5-(méthylènedioxy)phénylacétyl]-thiophène-3-sulfonamide, le N-(4-bromo-3-méthyl-5-isoxazolyl)-2-[2-méthyl-4,5-(méthylènedioxy)cinnamyl]thiophène-3-sulfonamide, le N-(4-bromo-3-méthyl-5-isoxazolyl)-2-(2,4-diméthylphényléthyl)-thiophène-3-sulfonamide et le N-(4-chloro-3-méthyl-5-isoxazolyl)-2-[(4-méthoxycarbonyl-2,6-diméthyl)phénylaminocarbonyl]-thiophène-3-sulfonamide ou un dérivé quelconque N-(4-halogéno-3-méthyl-5-isoxazolyle), N-(4-halogéno-5-méthyl-3-isoxazolyle), N-(3,4-diméthyl-5-isoxazolyle), N-(4-halogéno-5-méthyl-3-isoxazolyle), N-(4-halogéno-3-méthyl-5-isoxazolyle), N-(4,5-diméthyl-3-isoxazolyle) de l'un quelconque de ces composés.

**9.** Composé selon la revendication 1 ou sel, acide ou ester pharmaceutiquement acceptable de celui-ci qui est choisi parmi le N-(4-chloro-3-méthyl-5-isoxazolyl)-2-[(2,3,4-triméthoxy-6-méthylphénylaminocarbonyl)thiophène-3-sulfonamide, le N-(4-chloro-3-méthyl-5-isoxazolyl)-2-[(2,3,4-triméthoxy-6-acétylphénylaminocarbonyl)thiophène-3-sulfonamide, le N-(4-chloro-3-méthyl-5-isoxazolyl)-2-[(2,3,4-triméthoxy-6-méthoxycarbonylphénylaminocarbonyl)-thiophène-3-sulfonamide, le N-(4-chloro-3-méthyl-5-isoxazolyl)-2-[(2,3,4-triméthoxy-6-carboxylphénylaininocarbonyl)thiophène-3-sulfonamide, le N-(4-chloro-3-méthyl-5-isoxazolyl)-2-[(2,3,4-triméthoxy-6-méthanesulfonylphénylaminocarbonyl)thiophène-3-sulfonamide,leN-(4-chloro-3-méthyl-5-isoxazolyl)-2-[(2,3,4-triméthoxy-6-(cyanométhyl)phénylaminocarbonyl)thiophène-3-sulfonamide, le N-(4-chloro-3-méthyl-5-isoxazolyl)-2-[(2,3,4-triméthoxy-6-(2-hydroxyéthyl)phénylaminocarbonyl)thiophène-3-sulfonamide, le N-(4-chloro-3-méthyl-5-isoxazolyl)-2-[3,4-(méthylènedioxy)-2-méthoxy-6-méthylphénylaminocarbonyl]thiophène-3-sulfonamide, le N-(4-chloro-3-méthyl-5-isoxazolyl)-2-[3,4-(méthylènedioxy)-2-méthoxy-6-acétylphénylaminocarbonyl]thiophène-3-sulfonamide, le N-(4-chloro-3-méthyl-5-isoxazolyl)-2-[3,4-(méthylènedioxy)-2-méthoxy-6-méthoxycarbonylphénylaminocarbonyl] thiophène-3-sulfonamide, le N-(4-chloro-3-méthyl-5-isoxazolyl)-2-[3,4-(méthylènedioxy)-2-méthoxy-6-carboxylphénylaminocarbonyl]-thiophène-3-sulfonamide, le N-(4-chloro-3-méthyl-5-isoxazolyl)-2-[3,4-(méthylènedioxy)-2-méthoxy-6-méthanesulfonylphénylaminocarbonyl]thiophène-3-sulfonamide, le N-(4-chloro-3-méthyl-5-isoxazolyl)-2-[3,4-(méthylènedioxy)-2-méthoxy-6-cyanophénylaminocarbonyl]thiophène-3-sulfonamide, le N-(4-chloro-3-méthyl-5-isoxazolyl)-2-[3,4-(méthylènedioxy)-2-méthoxy-6-cyanométhylphénylamiocarbonyl]thiophène-3-sulfonamide, le N-(4-chloro-3-méthyl-5-isoxazolyl)-2-[3,4-(méthylènedioxy)-2-méthoxy-6-(2-hydroxyéthyl)phénylaminocarbonyl]-thiophène-3-sulfonamide, le N-(4-chloro-3-méthyl-5-isoxazolyl)-2-[3,4-(méthylènedioxy)-2,6-diméthylphénylaminocarbonyl]thiophène-3-sulfonamide, le N-(4-chloro-3-méthyl-5-isoxazolyl)-2-[3,4-(méthylènedioxy)-6-acétyl-2-méthylphénylaminocarbonyl]thiophène-3-sulfonamide, le N-(4-chloro-3-méthyl-5-isoxazolyl)-2-[3,4-(méthylènedioxy)-6-méthoxycarbonyl-2-méthylphénylaminocarbonyl]thiophène-3-sulfonamide, le N-(4-chloro-3-méthyl-5-isoxazolyl)-2-[3,4-(méthylènedioxy)-6-carboxyl-2-méthylphénylaminocarbonyl]thiophène-3-sulfonamide, le N-(4-chloro-3-méthyl-5-isoxazolyl)-2-[3,4-(méthylènedioxy)-6-méthoxy-2-méthylphénylaminocarbonyl]thiophène-3-sulfonamide, le N-(4-chloro-3-méthyl-5-isoxazolyl)-2-[3,4-(méthylènedioxy)-6-méthanesulfonyl-2-méthylphénylaminocarbonyl]thiophène-3-sulfonamide,leN-(4-chloro-3-méthyl-5-isoxazolyl)-2-[3,4-(méthylènedioxy)-6-cyano-2-méthylphénylaminocarbonyl]-thiophène-3-sulfonamide, le N-(4-chloro-3-méthyl-5-isoxazolyl)-2-[3,4-(méthylènedioxy)-6-(cyanométhyl)-2-méthylphénylaminocarbonyl]thiophène-3-sulfonamide, le N-(4-chloro-3-méthyl-5-isoxazolyl)-2-[3,4-(méthylènedioxy)-6-(2-hydroxyéthyl)-2-méthylphénylaminocarbonyl]thiophène-3-sulfonamide, le N-(4-chloro-3-méthyl-5-isoxazolyl)-2-[3,4-(méthylènedioxy)-2-cyano-6-méthylphénylaa-

minocarbonyl]thiophène-3-sulfonamide, le N-(4-chloro-3-méthyl-5-isoxazolyl)-2-[3,4-(méthylènedioxy)-6-méthoxy-2-cyanophénylaminocarbonyl]thiophène-3-sulfonamide, le N-(4-chloro-3-méthyl-5-isoxazolyl)-2-[3,4-(méthylènedioxy)-2-acétyl-6-méthylphénylaminocarbonyl]thiophène-3-sulfonamide, le N-(4-chloro-3-méthyl-5-isoxazolyl)-2-[3,4-(méthylènedioxy)-6-méthoxy-2-acétylphénylaminocarbonyl]thiophène-3-sulfonamide, le N-(4-chloro-3-méthyl-5-isoxazolyl)-2-[3-cyano-2,4,6-triméthylphénylaminocarbonyl]thiophène-3-sulfonamide, le N-(4-chloro-3-méthyl-5-isoxazolyl)-2-[3-carboxyl-2,4,6-triméthylphénylaminocarbonyl]-thiophène-3-sulfonamide, le N-(4-chloro-3-méthyl-5-isoxazolyl)-2-[3-hydroxyméthyl-2,4,6-triméthylphénylaminocarbonyl]thiophène-3-sulfonamide, le N-(4-chloro-3-méthyl-5-isoxazolyl)-2-[3-méthanesulfonyl-2,4,6-triméthylphénylaminocarbonyl]thiophène-3-surfonamide, le N-(4-chloro-3-méthyl-5-isoxazolyl)-2-[3-cyanométhyl-2,4,6-triméthylphénylaminocarbonyl]thiophène-3-sulfonamide, le N-(4-chloro-3-méthyl-5-isoxazolyl)-2-[3-(2-hydroxyéthyl)-2,4,6-triméthylphénylaminocarbonyl]thiophène-3-sulfonamide, le N-(4-chloro-3-méthyl-5-isoxazolyl)-2-[3-(carboxyméthyl)-2,4,6-triméthylphénylaminocarbonyl]thiophène-3-sulfonamide, le N-(4-chloro-3-méthyl-5-isoxazolyl)-2-(4-cyano-2,6-diméthylphénylaminocarbonyl)thiophène-3-sulfonamide, le N-(4-chloro-3-méthyl-5-isoxazolyl)-2-(4-carboxyl-2,6-diméthylphénylaminocarbonyl)thiophène-3-sulfonamide, le N-(4-chloro-3-méthyl-5-isoxazolyl)-2-[4-(hydroxyméthyl)-2,6-diméthylphénylaminocarbonyl]thiophène-3-sulfonamide, le N-(4-chloro-3-méthyl-5-isoxazolyl)-2-[4-(2-hydroxyéthyl)-2,6-diméthylphénylamino-carbonyl]thiophène-3-sulfonamide, le N-(4-chloro-3-méthyl-5-isoxazolyl)-2-[4-(cyanométhyl)-2,6-diméthylphénylaminocarbonyl]thiophène-3-sulfonamide, le N-(4-chloro-3-méthyl-5-isoxazolyl)-2-[4-(carboxylméthyl)-2,6-diméthylphénylaminocarbonyl]thiophène-3-sulfonamide, le N-(4-chloro-3-méthyl-5-isoxazolyl)-2-(4-méthanesulfonyl-2,6-diméthylphénylaminocarbonyl)thiophène-3-sulfonamide, le N-(4-chloro-3-méthyl-5-isoxazolyl)-2-(2,3,4-triméthoxy-6-méthylphénylacétyl)thiophène-3-sulfonamide, le N-(4-chloro-3-méthyl-5-isoxazolyl)-2-(2,3,4-triméthoxy-6-acétylphénylacétyl)thiophène-3-sulfonamide, le N-(4-chloro-3-méthyl-5-isoxazolyl)-2-(2,3,4-triméthoxy-6-méthoxycarbonylphénylacétyl)thiophène-3-sulfonamide, le N-(4-chloro-3-méthyl-5-isoxazolyl)-2-(2,3,4-triméthoxy-6-carboxylphénylacétyl)thiophène-3-sulfonamide, le N-(4-chloro-3-méthyl-5-isoxazolyl)-2-(2,3,4-triméthoxy-6-méthanesulfonylphénylacétyl)thiophène-3-sulfonamide, le N-(4-chloro-3-méthyl-5-isoxazolyl)-2-[2,3,4-triméthoxy-6-(cyanométhyl)phénylacétyl]thiophène-3-sulfonamide, le N-(4-chloro-3-méthyl-5-isoxazolyl)-2-[2,3,4-triméthoxy-6-(2-hydroxyéthyl)phénylacétyl]-thiophène-3-sulfonamide, le N-(4-chloro-3-méthyl-5-isoxazolyl)-2-[3,4-(méthylènedioxy)-2-méthoxy-6-méthylphénylacétyl)thiophène-3-sulfonamide, le N-(4-chloro-3-méthyl-5-isoxazolyl)-2-[3,4-(méthylènedioxy)-2-méthoxy-6-acétylphénylacétyl)thiophène-3-sulfonamide, le N-(4-chloro-3-méthyl-5-isoxazolyl)-2-[3,4-(méthylènedioxy)-2-méthoxy-6-méthoxycarbonylphénylacétyl]thiophène-3-sulfonamide, le N-(4-chloro-3-méthyl-5-isoxazolyl)-2-[3,4-(méthylènedioxy)-2-méthoxy-6-carboxylphénylacétyl] thiophène-3-sulfonamide, le N-(4-chloro-3-méthyl-5-isoxazolyl)-2-[3,4-(méthylènedioxy-2-méthoxy-6-méthanesulfonyl)phénylacétyl]-thiophène-3-sulfonamide, le N-(4-chloro-3-méthyl-5-isoxazolyl)-2-[3,4-(méthylènedioxy)-2-méthoxy-6-(cyano)phénylacétyl]-thiophène-3-sulfonamide, le N-(4-chloro-3-méthyl-5-isoxazolyl)-2-[3,4-(méthylènedioxy)-2-méthoxy-6-cyanométhylphénylacétyl]thiophène-3-sulfonamide, le N-(4-chloro-3-méthyl-5-isoxazolyl)-2-[3,4-(méthylènedioxy)-2-méthoxy-6-(2-hydroxyéthyl)phénylacétyl]-thiophène-3-sulfonamide, le N-(4-chloro-3-méthyl-5-isoxazolyl)-2-[3,4-(méthylènedioxy)-2,6-diméthylphénylacétyl]-thiophène-3-sulfonamide, le N-(4-chloro-3-méthyl-5-isoxazolyl)-2-[3,4-(méthylènedioxy)-6-acétyl-2-méthylphénylacétyl]thiophène-3-sulfonamide, le N-(4-chloro-3-méthyl-5-isoxazolyl)-2-[3,4-(méthylènedioxy)-6-méthoxycarbonyl-2-méthylphénylacétyl]-thiophène-3-sulfonamide, le N-(4-chloro-3-méthyl-5-isoxazolyl)-2-[3,4-(méthylènedioxy)-6-carboxyl-2-méthylphénylacétyl]-thiophène-3-sulfonamide, le N-(4-chloro-3-méthyl-5-isoxazolyl)-2-[3,4-(méthylènedioxy)-6-méthoxy-2-méthylphénylacétyl]-thiophène-3-sulfonamide, le N-(4-chloro-3-méthyl-5-isoxazolyl)-2-[3,4-(méthylènedioxy)-6-méthanesulfonyl-2-méthylphénylacétyl]thiophène-3-sulfonamide, le N-(4-chloro-3-méthyl-5-isoxazolyl)-2-[3,4-(méthylènedioxy)-6-cyano-2-méthylphénylacétyl]thiophène-3-sulfonamide, le N-(4-chloro-3-méthyl-5-isoxazolyl)-2-[3,4-(méthylènedioxy)-6-(cyanométhyl)-2-méthylphénylacétyl]-thiophène-3-sulfonamide, le N-(4-chloro-3-méthyl-5-isoxazolyl)-2-[3,4-(méthylènedioxy)-6-(2-hydroxyéthyl)-2-méthylphénylacétyl]thiophène-3-sulfonamide, le N-(4-chloro-3-méthyl-5-isoxazolyl)-2-[3,4-(méthylènedioxy)-2-cyano-6-méthylphénylacétyl]thiophène-3-sulfonamide, le N-(4-chloro-3-méthyl-5-isoxazolyl)-2-[3,4-(méthylènedioxy)-6-méthoxy-2-cyanophénylacétyl]thiophène-3-sulfonamide, le N-(4-chloro-3-méthyl-5-isoxazolyl)-2-[3,4-(méthylènedioxy)-2-acétyl-6-méthylphénylacétyl]thiophène-3-sulfonamide, le N-(4-chloro-3-méthyl-5-isoxazolyl)-2-[3,4-(méthylènedioxy)-6-méthoxy-2-acétylphénylacétyl]thiophène-3-sulfonamide, le N-(4-chloro-3-méthyl-5-isoxazolyl)-2-(3-cyano-2,4,6-triméthylphénylacétyl)thiophène-3-sulfonamide, le N-(4-chloro-3-méthyl-5-isoxazolyl)-2-(3-carboxyl-2,4,6-triméfhylphénylacétyl)thiophène-3-sulfonamide, le N-(4-chloro-3-méthyl-5-isoxazolyl)-2-(3-hydroxyméthyl-2,4,6-triméthylphénylacétyl)thiophène-3-sulfonamide, le N-(4-chloro-3-méthyl-5-isoxazolyl)-2-(3-méthanesulfonyl-2,4,6-triméthylphénylacétyl)thiophène-3-sulfonamide, le N-(4-chloro-3-méthyl-5-isoxazolyl)-2-[3-(cyanométhyl)-2,4,6-triméthylphénylacétyl]thiophène-3-sulfonamide, le N-(4-chloro-3-méthyl-5-isoxazolyl)-2-[3-(2-hydroxyéthyl)-2,4,6-triméthylphénylacétyl]thiophène-3-sulfonamide, le N-(4-chloro-3-méthyl-5-isoxazolyl)-2-[3-(carboxyméthyl)-2,4,6-triméthylphénylacétyl] thiophène-3-sulfonamide, le N-(4-chloro-3-méthyl-5-isoxazolyl)-2-(4-cyano-2,6-diméthylphénylacétyl)thiophène-3-sulfonamide, le N-(4-chloro-3-méthyl-5-isoxazolyl)-2-(4-carboxyl-2,6-diméthyl-phénylacétyl)thiophène-3-sulfonamide, le N-(4-chloro-3-méthyl-5-isoxazolyl)-2-(4-hydroxyméthyl-2,6-diméthyl-phénylacétyl)thiophène-3-sulfonamide, le N-(4-chloro-3-méthyl-5-isoxazolyl)-2-[4-(2-hydroxyéthyl)-2,6-(diméthyl)phénylacétyl]thiophène-3-sulfonamide, le N-(4-chloro-3-méthyl-5-isoxazolyl)-2-[4-cyanométhyl-2,6-(diméthyl)phénylacétyl]thiophène-3-sulfonamide, le N-(4-chloro-3-méthyl-5-isoxazolyl)-2-[4-(carboxyméthyl)-2,6-diméthylphénylacétyl]-thiophène-3-sulfonamide, et
le N-4-chloro-3-méthyl-5-isoxazolyl)-2-(4-méthanesulfonyl-2,6-diméthylphénylacétyl)thiophène-3-sulfonamide ou tout dérivé correspondant N-(4-halogéno-3-méthyl-5-isoxazolyle), N-(4-halogéno-5-méthyl-3-isoxazolyle), N-(3,4-diméthyl-5-isoxazolyle), N-(4-halogéno-5-méthyl-3-isoxazolyle), N-(4-halogéno-3-méthyl-5-isoxazolyle), N-(4,5-diméthyl-3-isoxazolyle) de l'un quelconque de ces composés.

**10.** Composé selon la revendication 1 ou sel, acide ou ester pharmaceutiquement acceptable de celui-ci choisi parmi le N-(4-bromo-3-méthyl-5-isoxazolyl)-2-[3,4-(méthylènedioxy)benzyl]benzo[b]thiophène-3-sulfonamide, le N-(4-chloro-3-méthyl-5-isoxazolyl)-2-[(3,4-méthylènedioxy)benzyl]benzo[b]-thiophène-3-sulfonamide, le N-(4-chloro-3-méthyl-5-isoxazolyl)-2-[β-hydroxy(3,4-méthylènedioxy)phényléthyl]thiophène-3-sulfonamide et le N-(4-chloro-3-méthyl-5-isoxazolyl)-2-(3,4-méthylènedioxybenzylcarbonyl)thiophène-3-sulfonamide ou un dérivé quelconque N-(4-halogéno-3-méthyl-5-isoxazolyle), N-(4-halogéno-5-méthyl-3-isoxazolyle), N-(3,4-diméthyl-5-isoxazolyle), N-(4-halogéno-3-méthyl-5-isoxazolyle), N-(4,5-diméthyl-3-isoxazolyle) de ceux-ci.

**11.** Composé selon la revendication 1 ou sel, acide ou ester pharmaceutiquement acceptable de celui-ci qui est le N-(4-chloro-3-méthyl-5-isoxazolyl)-2-[2-hydroxy-4-méthylphényl)aminocarbonyl]thiophène-3-sulfonamide.

**12.** Composé selon la revendication 1 ou sel, acide ou ester pharmaceutiquement acceptable de celui-ci qui est le N-(4-chloro-3-méthyl-5-isoxazolyl)-2-{[2-cyano-4,5-(méthylènedioxy)phényl]aminocarbonyl]thiophène-3-sulfonamide.

**13.** Composé selon la revendication 1 ou sel, acide ou ester pharmaceutiquement acceptable de celui-ci qui est le N-(4-chloro-3-méthyl-5-isoxazolyl)-2-[2-méthyl-4,5-(méthylènedioxy)phénylaminocarbonyl]thiophène-3-sulfonamide.

**14.** Composé selon la revendication 1 ou sel, acide ou ester pharmaceutiquement acceptable de celui-ci qui est le N-(4-chloro-3-méthyl-5-isoxazolyl)-2-(2-carboxamido-4,5-diméthoxyphénylaminocarbonyl)thiophène-3-sulfonamide.

**15.** Composé selon la revendication 1 ou sel, acide ou ester pharmaceutiquement acceptable de celui-ci qui est le N-(3,4-diméthyl-5-isoxazolyl)-2-(2,4-diméthylphénylacétyl)thiophène-3-sulfonamide.

**16.** Composé selon la revendication 1 ou sel, acide ou ester pharmaceutiquement acceptable de celui-ci qui est le N-(4-chloro-3-méthyl-5-isoxazolyl)-2-(2,4-diméthylphénylacétyl)thiophène-3-sulfonamide.

**17.** Composé selon la revendication 1 ou sel, acide ou ester pharmaceutiquement acceptable de celui-ci qui est le N-(4-bromo-3-méthyl-5-isoxazolyl)-2-(2,4-diméthylphénylacétyl)thiophène-3-sulfonamide.

**18.** Composé selon la revendication 1 ou sel, acide ou ester pharmaceutiquement acceptable de celui-ci qui est le N-(4-chloro-3-méthyl-5-isoxazolyl)-2-[3,4-(méthylènedioxy)]-phénylaminocarbonyl-3-thiophènesulfonamide.

**19.** Composé selon la revendication 1 ou sel, acide ou ester pharmaceutiquement acceptable de celui-ci qui est le N-(4-chloro-3-méthyl-5-isoxazolyl)-2-[2-méthyl-4,5-(méthylènedioxy)phénylacétyl]thiophène-3-sulfonamide.

**20.** Composé selon la revendication 1 ou sel, acide ou ester pharmaceutiquement acceptable de celui-ci qui est le N-(4-chlore-3-méthyl-5-isoxazolyl)-2-[3,4-(méthylènedioxy)-6-(2-acétoxyéthyl)phénylaminocarbonyl]-thiophène-3-sulfonamide.

**21.** Composé selon la revendication 1 ou sel, acide ou ester pharmaceutiquement acceptable de celui-ci qui est le N-(4-chloro-3-méthyl-5-isoxazolyl)-2-[3,4-(méthylènedioxy)-6-(2-hydroxyéthyl)phénylaminocarbonyl]-thiophène-3-sulfonamide.

**22.** Composé selon la revendication 1 ou sel, acide ou ester pharmaceutiquement acceptable de celui-ci qui est le N-(4-chloro-3-méthyl-5-isoxazolyl)-2-(2,5-diméthylphénylacétyl)thiophène-3-sulfonamide.

**23.** Composé selon la revendication 1 ou sel, acide ou ester pharmaceutiquement acceptable de celui-ci qui est le N-(4-chloro-3-méthyl-5-isoxazolyl)-2-[(2-méthanesulfonyleaminométhyl)-4,5-(méthyrènedioxy)phénylamino-carbonyl]thiophène-3-sulfonamide.

**24.** Composé selon la revendication 1 ou sel, acide ou ester pharmaceutiquement acceptable de celui-ci qui est le N-(4-chloro-3-méthyl-5-isoxazolyl)-2-[2-cyanométhyl-4,5-(méthylènedioxy)-6-cyanométhyl]phénylaminocarbonyl-3-thiophènesulfonamide.

**25.** Composé selon la revendication 1 ou sel, acide ou ester pharmaceutiquement acceptable de celui-ci qui est le N-(4-bromo-3-méthyl-5-isoxazolyl)-3-[2-méthyl-4,5-(méthylènedioxy)cinnamyl]thiophène-2-sulfonamide.

**26.** Composé selon la revendication 1 ou sel, acide ou ester pharmaceutiquement acceptable de celui-ci qui est le N-(4-chloro-3-méthyl-5-isoxazolyl)-2-{2-[(diméthylamino)-carboxyméthyl]-4,5-(méthylènedioxy)-phénylaminocarbonyl}thiophène-3-sulfonamide.

**27.** Composé selon la revendication 1 ou sel, acide ou ester pharmaceutiquement acceptable de celui-ci qui est le N-(4-chloro-3-méthyl-5-isoxazolyl)-2-[β-acétoxy-2-méthyl-4,5-(méthylènedioxy)styryl]thiophène-3-sulfonamide.

**28.** Composé selon la revendication 1 ou sel, acide ou ester pharmaceutiquement acceptable de celui-ci qui est le N-(4-chloro-3-méthyl-5-isoxazolyl)-2-[2,3,4-triméthoxy-6-cyano)phénylaminocarbonyl]thiophène-3-sulfonamide.

**29.** Composé selon la revendication 1 ou sel, acide ou ester pharmaceutiquement acceptable de celui-ci qui est le N-(4-chloro-3-méthyl-5-isoxazolyl)-2-(2,4,6-triméthylphénylaminocarbonyl)thiophène-3-sulfonamide.

**30.** Composé selon la revendication 1 ou sel, acide ou ester pharmaceutiquement acceptable de celui-ci qui est le N-(4-chloro-3-méthyl-5-isoxazolyl)-2-(3-méthoxycarbonyl-2,4,6-triméthylphénylaminocarbonyl)thiophène-3-sulfonamide.

**31.** Composé selon la revendication 1 ou sel, acide ou ester pharmaceutiquement acceptable de celui-ci qui est le N-(4-chlore-3-méthyl-5-isoxazolyl)-2-(2,4,6-triméthylphénylacétyl)thiophène-3-sulfonamide.

**32.** Composé selon la revendication 1 ou sel, acide ou ester pharmaceutiquement acceptable de celui-ci qui est le N-(4-chloro-5-méthyl-3-isoxazolyl)-2-[2-mémyl-4,5-(méthylènedioxy)phénylacétyl]thiophène-3-sulfonamide.

**33.** Composé selon la revendication 1 ou sel, acide ou ester pharmaceutiquement acceptable de celui-ci qui est le N-(4-chloro-3-méthyl-5-isoxazolyl)-2-[(4-méthoxycarbonyl-2,6-diméthyl)phénylaminocarbonyl]thiophène-3-sulfonamide.

**34.** Composition pharmaceutique, comprenant un composé selon l'une quelconque des revendications 1 à 33 ou un sel, acide, ou ester pharmaceutiquement acceptable de celui-ci ensemble avec un support pharmaceutiquement acceptable.

**35.** Composition pharmaceutique, comprenant le composé selon la revendication 19, ou un sel, acide, ou ester pharmaceutiquement acceptable de celui-ci, ensemble avec un support pharmaceutiquement acceptable.

**36.** Composé selon l'une quelconque des revendications 1 à 33, ou un sel, acide, ou ester pharmaceutiquement acceptable de celui-ci à utiliser dans le traitement d'un trouble sous la médiation de l'éndothéline.

**37.** Composé selon la revendication 19, ou un sel, acide, ou ester pharmaceutiquement acceptable de celui-ci à utiliser dans le traitement d'un trouble sous la médiation de l'endothéline.

**38.** Utilisation d'un ou plusieurs composés selon l'une quelconque des revendications 1 à 33, ou d'un sel, acide, ou ester pharmaceutiquement acceptable de ceux-ci pour la préparation d'un médicament pour le traitement d'un trouble sous la médiation de l'endothéline.

**39.** Utilisation du composé selon la revendication 19, ou d'un sel, acide, ou ester pharmaceutiquement acceptable de celui-ci pour la préparation d'un médicament pour le traitement d'un trouble sous la médiation de l'endothéline.

**40.** Utilisation selon la revendication 38 ou la revendication 39, dans laquelle le trouble est choisi parmi l'hypertension, les troubles cardiovasculaires, l'asthme, l'hypertension pulmonaire, les maladies inflammatoires, les troubles ophtalmologiques, les troubles menstruels, les états obstétriques, les blessures, les troubles gastro-entériques, l'insuffisance rénale, la vasoconstriction rénale sous la médiation d'un immunosuppresseur, la vasoconstriction sous la médiation de l'érythropoïétine, le choc endotoxinique, le choc anaphylactique et le choc hémorragique.

**41.** Utilisation selon la revendication 40, dans laquelle le trouble est choisi parmi l'hypertension, les troubles cardiovasculaires, l'hypertension pulmonaire, la vasoconstriction sous la médiation de l'érythropoïétine, le choc endotoxinique, le choc anaphylactique et le choc hémorragique.

**42.** Utilisation selon la revendication 40, dans laquelle le trouble est choisi parmi l'asthme et les maladies inflammatoires.

**43.** Utilisation d'un ou de plusieurs composés selon l'une quelconque des revendications 1 à 33 ou d'un sel, ester, ou acide pharmaceutiquement acceptable de ceux-ci dans la préparation d'un médicament pour inhiber la liaison d'un peptide d'endothéline à un récepteur d'endothéline$_A$ ($ET_A$) ou d'endothéline$_B$ ($ET_B$).

**44.** Utilisation du composé selon la revendication 19 ou d'un sel, ester ou acide pharmaceutiquement acceptable de celui-ci dans la préparation d'un médicament pour inhiber la liaison d'un peptide d'endothéline à un récepteur d'endothéline$_A$ ($ET_A$) ou d'endothéline$_B$ ($ET_B$).

**45.** Utilisation d'un ou de plusieurs composés selon l'une quelconque des revendications 1 à 33, ou d'un sel, ester ou acide pharmaceutiquement acceptable de celui-ci, dans la préparation d'un médicament pour altérer une activité sous la médiation d'un récepteur d'endothéline.

**46.** Utilisation du composé selon la revendication 19 ou d'un sel, ester ou acide pharmaceutiquement acceptable de celui-ci dans la préparation d'un médicament pour altérer une activité sous la médiation d'un récepteur d'endothéline.

**47.** Composition pharmaceutique formulée pour une administration à dose unique, comprenant une quantité efficace d'un ou de plusieurs composés selon l'une quelconque des revendications 1 à 33 ou d'un sel, ester ou acide pharmaceutiquement acceptable de celui-ci, dans laquelle la quantité est efficace pour améliorer les symptômes d'une maladie sous la médiation de l'endothéline.

**48.** Composition pharmaceutique formulée pour une administration à dose unique, comprenant une quantité efficace du composé selon la revendication 19 ou d'un sel, ester ou acide pharmaceutiquement acceptable de celui-ci, dans laquelle la quantité est efficace pour améliorer les symptômes d'une maladie sous la médiation de l'endothéline.

**49.** Article manufacturé, comprenant un matériau de conditionnement et un ou plusieurs composés selon l'une quelconque des revendications 1 à 33 ou d'un sel, ester ou acide pharmaceutiquement acceptable de celui-ci contenu à l'intérieur du matériau de conditionnement, dans lequel le composé est efficace comme antagoniste des effets de l'endothéline, pour améliorer les symptômes d'un trouble sous la médiation de l'endothéline, ou pour inhiber la liaison d'un peptide d'endothéline à un récepteur ET avec une $CI_{50}$ de moins d'environ 10 µM, et le matériau de conditionnement comprend une étiquette qui indique que le sulfonamide ou son sel est utilisé comme antagoniste des effets de l'endothéline, pour inhiber la liaison d'endothéline à un récepteur d'endothéline ou traiter une maladie sous la médiation de l'endothéline.

**50.** Article manufacturé, comprenant un matériau de conditionnement et un composé selon la revendication 19 ou un sel, ester ou acide pharmaceutiquement acceptable de celui-ci contenu à l'intérieur du matériau de conditionnement, dans lequel le composé est efficace comme antagoniste des effets de l'endothéline, pour améliorer les symptômes d'un trouble sous la médiation de l'endothéline, ou inhiber la liaison d'un peptide d'endothéline à un récepteur ET avec une $CI_{50}$ de moins d'environ 10 µM, et le matériau de conditionnement comprend une étiquette qui indique que le sulfonamide ou son sel est utilisé comme antagoniste des effets de l'endothéline, pour inhiber la liaison d'endothéline à un récepteur d'endothéline ou traiter une maladie sous la médiation de l'endothéline.

**51.** Utilisation selon la revendication 38 ou la revendication 39, dans laquelle la maladie est le glaucome.